(19) Europäisches Patentamt
European Patent Office
Office européen des brevets

(11) EP 4 365 290 A1

(12) EUROPEAN PATENT APPLICATION
published in accordance with Art. 153(4) EPC

(43) Date of publication:
**08.05.2024 Bulletin 2024/19**

(21) Application number: **22832207.9**

(22) Date of filing: **01.07.2022**

(51) International Patent Classification (IPC):
***C12N 15/113*** (2010.01) ***A61K 47/54*** (2017.01)
***A61K 31/713*** (2006.01) ***A61P 1/16*** (2006.01)

(52) Cooperative Patent Classification (CPC):
(C-Sets available)
**A61K 47/549; A61P 1/16; C12N 15/111;**
**C12N 15/113;** C12N 2310/14; C12N 2310/351;
C12N 2320/32 (Cont.)

(86) International application number:
**PCT/CN2022/103275**

(87) International publication number:
**WO 2023/274395 (05.01.2023 Gazette 2023/01)**

(84) Designated Contracting States:
**AL AT BE BG CH CY CZ DE DK EE ES FI FR GB GR HR HU IE IS IT LI LT LU LV MC MK MT NL NO PL PT RO RS SE SI SK SM TR**
Designated Extension States:
**BA ME**
Designated Validation States:
**KH MA MD TN**

(30) Priority: **02.07.2021 CN 202110753225**

(71) Applicant: **Tuojie Biotech (Shanghai) Co., Ltd.**
**Shanghai 201203 (CN)**

(72) Inventors:
- **HUANG, Jinyu**
  **Shanghai 201203 (CN)**
- **LUO, Min**
  **Shanghai 201203 (CN)**
- **YIN, Ke**
  **Shanghai 201203 (CN)**
- **HOU, Zhe**
  **Shanghai 201203 (CN)**
- **LI, Yunfei**
  **Shanghai 201203 (CN)**

(74) Representative: **Regimbeau**
**20, rue de Chazelles**
**75847 Paris Cedex 17 (FR)**

# (54) NUCLEIC ACID LIGAND AND CONJUGATE THEREOF, AND PREPARATION METHOD THEREFOR AND USE THEREOF

(57) A nucleic acid ligand and a conjugate thereof, and a preparation method therefor and the use thereof. Specifically, provided is a ligand having the structure as represented by formula (I). The provided ligand achieves a good expression inhibition effect by means of the conjugation with a nucleic acid.

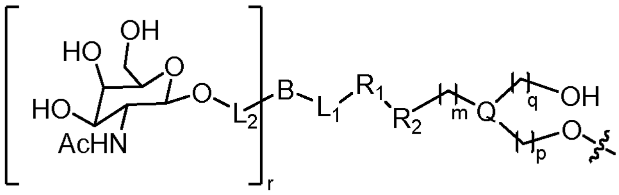

(I)

EP 4 365 290 A1

(52) Cooperative Patent Classification (CPC): (Cont.)

C-Sets
C12N 2310/321, C12N 2310/3521;
C12N 2310/322, C12N 2310/3533

## Description

### TECHNICAL FIELD

The present disclosure relates to a ligand and a preparation method therefor. The present disclosure also relates to a nucleic acid-ligand conjugate formed by linking a ligand to a nucleic acid by a covalent bond. Delivery of the nucleic acid-ligand conjugate can be targeted to hepatocytes so that it can produce an RNA interference effect.

### BACKGROUND

RNA interference is an effective way to silence gene expression. Statistically, about more than 80% of the disease-related proteins in humans are non-druggable proteins as they cannot be targeted by conventional small-molecule drugs and biomacromolecule formulations. By using the RNA interference technology, proper siRNAs can be designed according to the mRNAs coding for these proteins to specifically target and degrade the target mRNAs so the generation of the related proteins is inhibited. Therefore, siRNAs have very important prospects for drug development. However, to achieve the therapeutic RNA interference effect *in vivo,* it is necessary to deliver siRNA molecules to specific cells *in vivo.*

One effective way of delivering drugs is by conjugating siRNA with a targeting ligand so that it can enter cells through endocytosis using the binding of the targeting ligand to the receptor molecule on the surface of the cell membrane. For example, the asialoglycoprotein receptors (ASGPR) are receptors specifically expressed by hepatocytes and, on the surface of hepatocytes, are characterized by their high abundance and rapid intracellular and extracellular transformation. Monosaccharide and polysaccharide molecules such as galactose, galactosamine and N-acetylgalactosamine have high affinities for ASGPR. According to the literature (Yuanyu H, LIANG Zicai L, Asialoglycoprotein Receptor and Its Application in Liver-targeted Drug Delivery, Prog. Biochem. Biophys. 2015; 42 (6)), RNA can be effectively delivered to hepatocytes using galactosamine clusters (GalNAc); when GalNAc molecules are designed as trivalent or tetravalent clusters, the ability of monovalent or divalent GalNAc molecules to target hepatocytes can be significantly improved.

Different cluster structures and different modes of connection with RNA significantly affect the *in vivo* activity of siRNA. Higher activity means a better therapeutic effect or a lower dosage of medication, and further, a lower dosage of medication that achieves an equivalent therapeutic effect means lower toxicity. Thus, it is of great significance to design a reasonable covalent linkage between the targeting ligand and siRNA.

The present disclosure provides a novel molecular structure in which a GalNAc molecule is connected to RNA, which has a simpler synthetic structure and has better delivery activity and better RNA interference activity.

### SUMMARY

In a first aspect, the present disclosure provides a ligand having a structure represented by formula (I'),

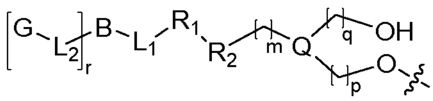

(I')

wherein $L_1$ is a $C_1$-$C_{30}$ alkyl chain, or a $C_1$-$C_{30}$ alkyl chain interrupted by one or more oxygen, sulfur or nitrogen atoms or C=O;

$R_1$ and $R_2$ are independently chemical bonds, $-NR_6-$, -C(=O)- or -OC(=O)-;

Q is

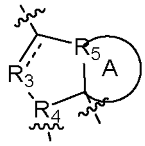 or 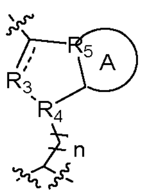 ;

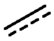 is a single or double bond; when 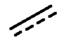 is a single bond, $R_3$ is independently $CR_7R_8$, $NR_6$, O or S; when 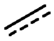 is a double bond, $R_3$ is independently $CR_9$ or N;

$R_4$ is independently $CR_9$ or N;

ring A is absent, or is cycloalkyl, heterocyclyl, aryl or heteroaryl; when ring A is present, $R_5$ is independently $CR_9$ or N; when ring A is absent, $R_5$ is independently $CR_7R_8$, $NR_6$ or O;

$R_6$ and $R_9$ are independently hydrogen, deuterium, alkyl, alkoxy, cycloalkyl, heterocyclyl, aryl, heteroaryl, SR', S(=O)R', $S(=O)_2R'$, $S(=O)_2NR'(R")$, NR'(R"), C(=O)R', C(=O)OR' or C(=O)NR'(R"), wherein the alkyl, alkoxy, cycloalkyl, heterocyclyl, aryl or heteroaryl is optionally substituted with one or more groups selected from the group consisting of halogen, hydroxy, oxo, nitro, cyano, $C_{1-6}$ alkyl, $C_{1-6}$ alkoxy, $C_{3-7}$ cycloalkyl, 3-12 membered heterocyclyl, 5-12 membered aryl, 5-12 membered heteroaryl, SR', S(=O)R', $S(=O)_2R'$, $S(=O)_2NR'(R")$, NR'(R"), C(=O)R', C(=O)OR' and C(=O)NR'(R");

$R_7$ and $R_8$ are independently hydrogen, deuterium, alkyl, alkoxy, cycloalkyl, heterocyclyl, aryl, heteroaryl, SR', S(=O)R', $S(=O)_2R'$, $S(=O)_2NR'(R")$, NR'(R"), C(=O)R', C(=O)OR' or C(=O)NR'(R"), wherein the alkyl, alkoxy, cycloalkyl, heterocyclyl, aryl or heteroaryl is optionally substituted with one or more groups selected from the group consisting of halogen, hydroxy, oxo, nitro, cyano, $C_{1-6}$ alkyl, $C_{1-6}$ alkoxy, $C_{3-7}$ cycloalkyl, 3-12 membered heterocyclyl, 5-12 membered aryl, 5-12 membered heteroaryl, SR', S(=O)R', $S(=O)_2R'$, $S(=O)_2NR'(R")$, NR'(R"), C(=O)R', C(=O)OR' and C(=O)NR'(R");

R' and R" are independently hydrogen, deuterium, hydroxy, alkyl, alkoxy, cycloalkyl, heterocyclyl, aryl or heteroaryl, wherein the alkyl, alkoxy, cycloalkyl, heterocyclyl, aryl or heteroaryl is optionally substituted with one or more substituents selected from the group consisting of halogen, hydroxy, oxo, nitro and cyano;

m, n, p and q are independently 0, 1, 2, 3 or 4;

B is

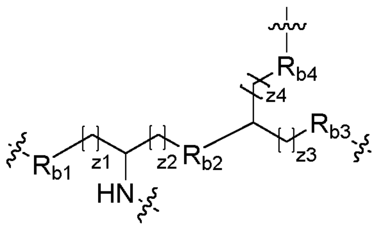

or

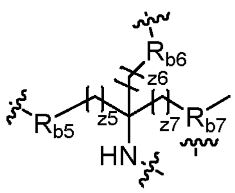

;

$R_{b1}$, $R_{b2}$, $R_{b3}$, $R_{b4}$, $R_{b5}$, $R_{b6}$ and $R_{b7}$ are independently -C(=O)-, -NHC(=O)-, -C(=O)O-, $-C(=O)-(CH_2)_{z8}-O-$ or $-NHC(=O)-(CH_2)_{z9}-O-$;

z1, z2, z3, z4, z5, z6, z7, z8 and z9 are independently integers of 0-10;

$L_2$ is a $C_1$-$C_{30}$ alkyl chain, or a $C_1$-$C_{30}$ alkyl chain interrupted by one or more oxygen, sulfur or nitrogen atoms or C=O;

G is a targeting moiety that binds to a cellular receptor;

r is an integer of 1-10.

In some embodiments, certain groups are defined as follows, and undefined groups are as described in any of the preceding embodiments (hereinafter referred to as "in some embodiments"); $L_1$ may be $L_3$ or $L_3$-$R_{10}$-$R_{11}$-$L_3$, wherein $L_3$ is independently a $C_1$-$C_{12}$ alkyl chain, $-(CH_2)_{j1}-C(=O)-(CH_2)_{j2}-$ or $-(CH_2)_{j3}-(CH_2CH_2O)_{1-4}-(CH_2)_{j4}-$; $R_{10}$ and $R_{11}$ are independently chemical bonds, $-NR_{12}-$, -C(=O)- or -OC(=O)-; $R_{12}$ is hydrogen or $C_1$-$C_{12}$ alkyl; j 1, j2, j3 and j4 are independently integers of 0-10, preferably 0-2 or 4-10, and more preferably 0, 1, 2, 6, 7, 8, 9 or 10.

In some embodiments, $L_1$ may be $-(CH_2)_{j1}-C(=O)-(CH_2)_{j2}-$, wherein j 1 and j2 are as defined in any of the preceding embodiments.

In some embodiments, $L_1$ may be

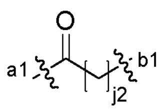

,

and j 1 and j2 are as defined in any of the preceding embodiments, wherein the end a1 is attached to B, and the end b1 is attached to $R_1$.

In some embodiments, $L_1$ may be

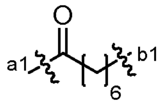,
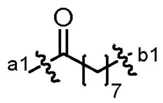,
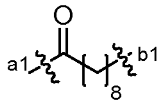,

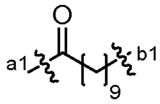 or 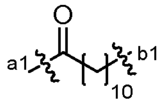

, wherein the end a1 is attached to B, and the end b1 is attached to $R_1$.

In some embodiments, $R_1$ may be a chemical bond and $R_2$ may be C=O.

In some embodiments, $R_1$ may be a chemical bond and $R_2$ may be $NR_6$, wherein $R_6$ is as defined in any of the preceding embodiments.

In some embodiments, $R_1$ may be a chemical bond and $R_2$ may be -OC(=O)-.

In some embodiments, $R_1$ may be $NR_6$ and $R_2$ may be C=O, wherein $R_6$ is as defined in any of the preceding embodiments.

In some embodiments, $R_1$ may be $NR_6$ and $R_2$ may be -OC(=O)-, wherein $R_6$ is as defined in any of the preceding embodiments.

In some embodiments, $R_2$ may be $NR_6$ and $R_1$ may be C=O, wherein $R_6$ is as defined in any of the preceding embodiments.

In some embodiments, $R_2$ may be $NR_6$ and $R_1$ may be -OC(=O)-, wherein $R_6$ is as defined in any of the preceding embodiments.

In some embodiments, $R_6$ may be hydrogen or $C_{1-6}$ alkyl.

In some embodiments, $R_6$ may be hydrogen, methyl, ethyl, propyl or isopropyl.

In some embodiments, $R_6$ may be hydrogen.

In some embodiments, $R_7$ and $R_8$ may be hydrogen.

In some embodiments, $R_9$ may be hydrogen.

In some embodiments, when ring A is present, ring A may be $C_{6-10}$ aryl, preferably phenyl.

In some embodiments, m may be 0 or 1.

In some embodiments, m may be 3.

In some embodiments, n may be 0 or 1.

In some embodiments, p and q are independently 0 or 1.

In some embodiments, p = 1 and q = 1.

In some embodiments, p = 1 and q = 0.

In some embodiments, p = 0 and q = 1.

In some embodiments, p = 0 and q = 0.

In some embodiments, z1, z2, z3, z4, z5, z6, z7, z8 and z9 may independently be integers of 0-4, preferably 0, 1 or 2.

In some embodiments, B may be

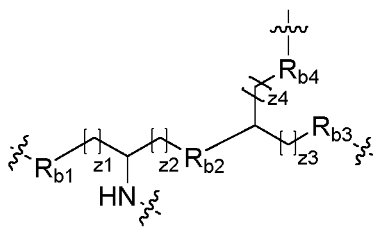,

wherein $R_{b1}$, $R_{b2}$, $R_{b3}$ and $R_{b4}$ are independently -C(=O)- or -NHC(=O)-; the N atom is attached to $L_1$; z1, z2, z3 and z4 are as defined in any of the preceding embodiments.

In some embodiments, B may be

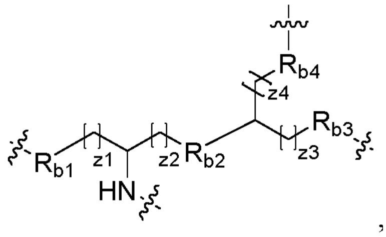

, wherein $R_{b1}$, $R_{b2}$, $R_{b3}$ and $R_{b4}$ are independently -C(=O)- or -NHC(=O)-; the N atom is attached to $L_1$; $R_{b1}$, $R_{b3}$ and $R_{b4}$ are identical; z1, z2, z3 and z4 are as defined in any of the preceding embodiments.

In some embodiments, B may be

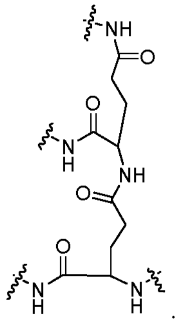

In some embodiments, B may be

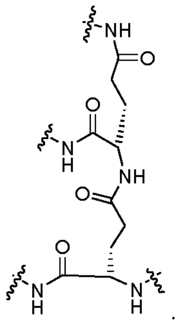

In some embodiments, B may be

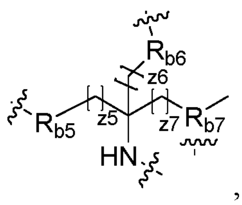

wherein $R_{b5}$, $R_{b6}$ and $R_{b7}$ are independently -C(=O)-$(CH_2)_{z8}$-O- or -NHC(=O)-$(CH_2)_{z9}$-O-; the N atom is attached to $L_1$; z5, z6, z7, z8 and z9 are as defined in any of the preceding embodiments.

In some embodiments, B may be

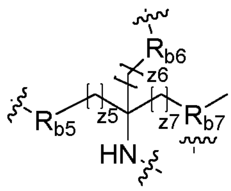

,

wherein $R_{b5}$, $R_{b6}$ and $R_{b7}$ are independently $-C(=O)-(CH_2)_{z8}-O-$ or $-NHC(=O)-(CH_2)_{z9}-O-$; the N atom is attached to $L_1$; $R_{b5}$, $R_{b6}$ and $R_{b7}$ are identical; z5, z6, z7, z8 and z9 are as defined in any of the preceding embodiments.

In some embodiments, B may be

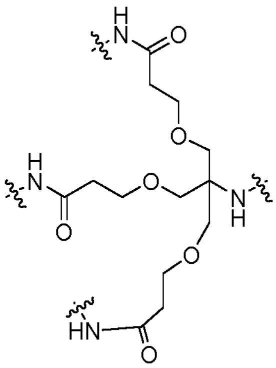

.

In some embodiments, $L_2$ may be $L_4$ or $L_4-R_{13}-R_{14}-L_4$, wherein $L_4$ is independently a $C_1$-$C_{12}$ alkyl chain or $-(CH_2)_{j5}-(OCH_2CH_2)_{1-4}-(CH_2)_{j6}-$; $R_{13}$ and $R_{14}$ are independently chemical bonds, $-NR_{15}-$, $-C(=O)-$ or $-OC(=O)-$; $R_{15}$ is independently hydrogen or $C_1$-$C_{12}$ alkyl; j5 and j6 are independently integers of 0-10, preferably 0-6, and more preferably 0, 1, 2, 3 or 4.

In some embodiments, $L_2$ may be $-(CH_2)_{j5}-(OCH_2CH_2)_{1-4}-(CH_2)_{j6}-$, wherein j5 and j6 are as defined in any of the preceding embodiments.

In some embodiments, $L_2$ may be

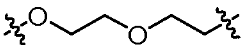
, 
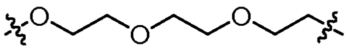
,

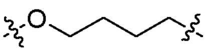
, 
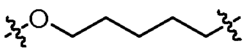
 or 
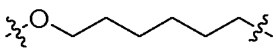
,

wherein the O atom is attached to G, and the C atom is attached to B; preferably $L_2$ is

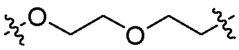

or

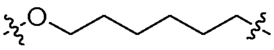

.

In some embodiments, $L_2$ may be

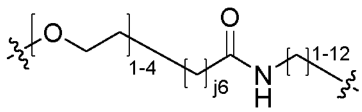

,

wherein j6 is as defined in any of the preceding embodiments; the O atom is attached to G, and the C atom is attached to B.

In some embodiments, $L_2$ may be

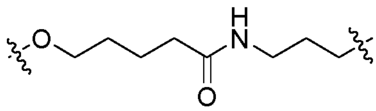

,

wherein the O atom is attached to G, and the C atom is attached to B.

In some embodiments, G may be an asialoglycoprotein receptor targeting moiety.

In some embodiments, G may be galactose, galactosamine, N-formylgalactosamine, N-acetylgalactosamine, N-propionylgalactosamine, N-n-butyrylgalactosamine or N-isobutyrylgalactosamine.

In some embodiments, G may be

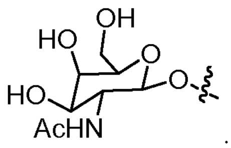

.

In some embodiments, r may be 3, 4, 5 or 6, e.g., 3.

In some embodiments, Q may be

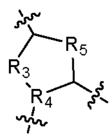

or

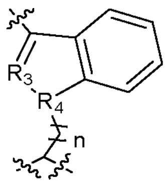

,

preferably

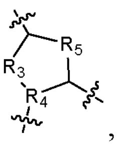

,

wherein $R_3$, $R_4$, $R_5$ and n are as defined in any of the preceding embodiments.

In some embodiments,

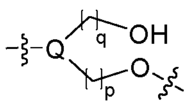

may be

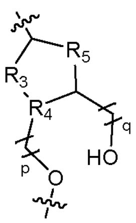

, wherein $R_3$, $R_4$, $R_5$, p and q are as defined in any of the preceding embodiments.

In some embodiments,

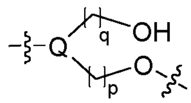

may be

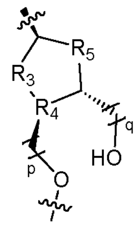, 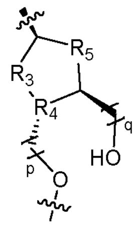, 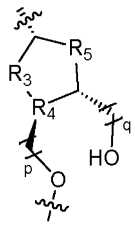

or

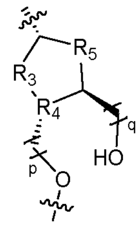,

wherein $R_3$, $R_4$, $R_5$, p and q are as defined in any of the preceding embodiments.

In some embodiments,

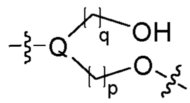

may be

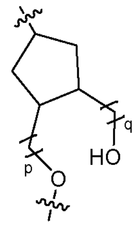, 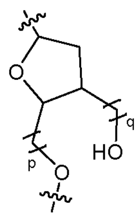 or 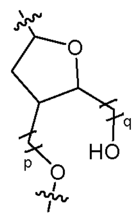,

preferably

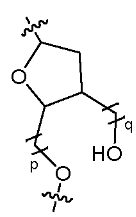 or 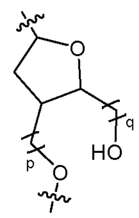,

and more preferably

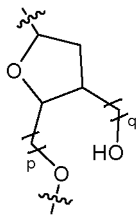
,

wherein p and q are as defined in any of the preceding embodiments.

In some embodiments,

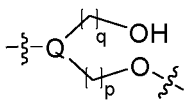

may be

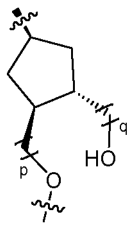
, 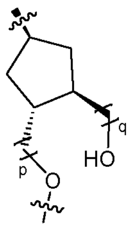
, 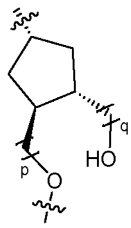
,

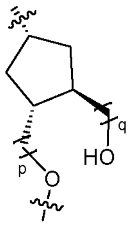
, 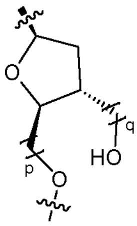
, 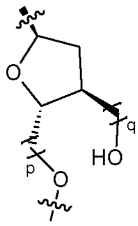
, 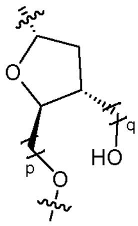
, 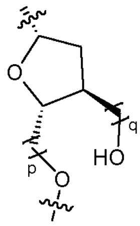
, 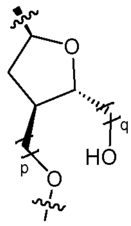
, 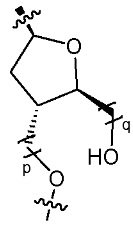
,

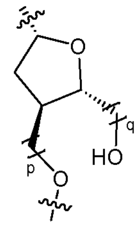
or 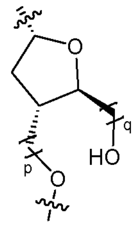
,

preferably

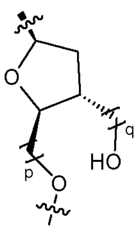
, 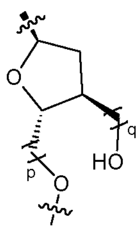
, 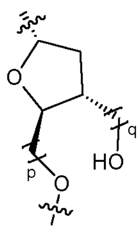
,

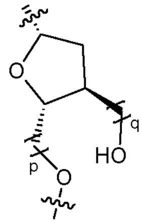, 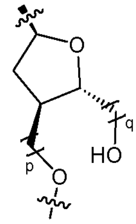, 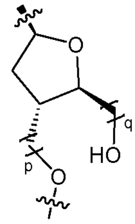, 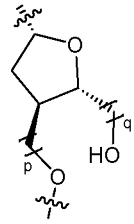 or 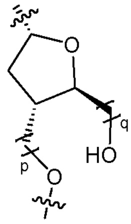,

and more preferably

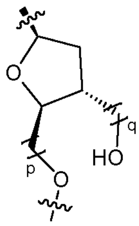, 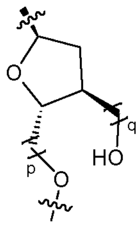, 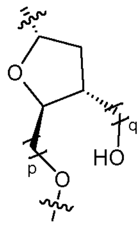 or 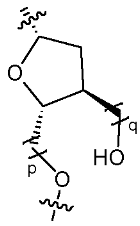,

wherein p and q are as defined in any of the preceding embodiments.

In some embodiments,

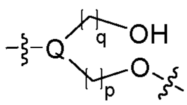

may be

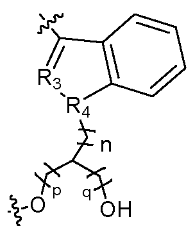,

wherein $R_3$, $R_4$, n, p and q are as defined in any of the preceding embodiments.

In some embodiments,

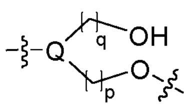

may be

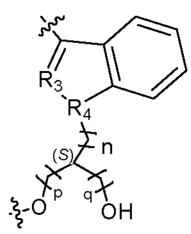 or 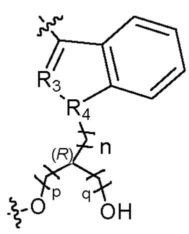,

wherein $R_3$, $R_4$, $R_5$, n, p, and q are as defined in any of the preceding embodiments.

In some embodiments,

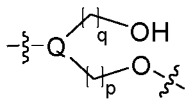

may be

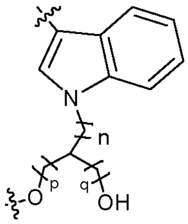

,

wherein n, p and q are as defined in any of the preceding embodiments.

In some embodiments,

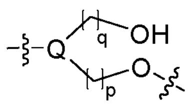

may be

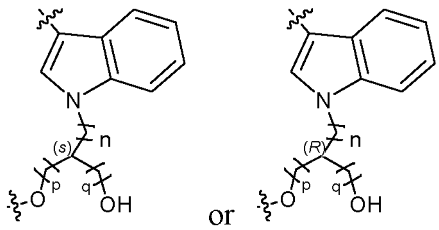

,

wherein n, p and q are as defined in any of the preceding embodiments.

The present disclosure provides a ligand having a structure represented by formula (I),

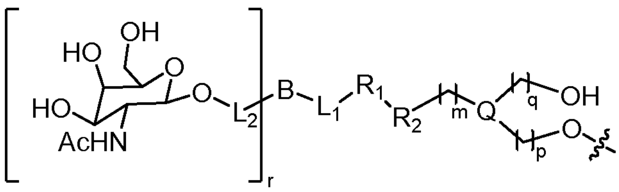

(I)

wherein $L_1$ is a $C_1$-$C_{30}$ alkyl chain, or a $C_1$-$C_{30}$ alkyl chain interrupted by one or more oxygen, sulfur or nitrogen atoms or C=O;

$R_1$ and $R_2$ are independently chemical bonds, -$NR_6$-, -C(=O)- or -OC(=O)-;

Q is

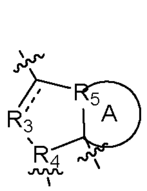 or 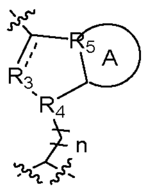 ;

is a single or double bond; when is a single bond, $R_3$ is independently $CR_7R_8$, $NR_6$, O or S; when is a double bond, $R_3$ is independently $CR_9$ or N;

$R_4$ is independently $CR_9$ or N;

ring A is absent, or is cycloalkyl, heterocyclyl, aryl or heteroaryl; when ring A is present, $R_5$ is independently $CR_9$ or N; when ring A is absent, $R_5$ is independently $CR_7R_8$, $NR_6$ or O;

$R_6$ and $R_9$ are independently hydrogen, deuterium, alkyl, alkoxy, cycloalkyl, heterocyclyl, aryl, heteroaryl, SR', S(=O)R', $S(=O)_2R'$, $S(=O)_2NR'(R'')$, NR'(R''), C(=O)R', C(=O)OR' or C(=O)NR'(R''), wherein the alkyl, alkoxy, cycloalkyl, heterocyclyl, aryl or heteroaryl is optionally substituted with one or more groups selected from the group consisting of halogen, hydroxy, oxo, nitro, cyano, $C_{1-6}$ alkyl, $C_{1-6}$ alkoxy, $C_{3-7}$ cycloalkyl, 3-12 membered heterocyclyl, 5-12 membered aryl, 5-12 membered heteroaryl, SR', S(=O)R', $S(=O)_2R'$, $S(=O)_2NR'(R'')$, NR'(R''), C(=O)R', C(=O)OR' and C(=O)NR'(R'');

$R_7$ and $R_8$ are independently hydrogen, deuterium, alkyl, alkoxy, cycloalkyl, heterocyclyl, aryl, heteroaryl, SR', S(=O)R', $S(=O)_2R'$, $S(=O)_2NR'(R'')$, NR'(R''), C(=O)R', C(=O)OR' or C(=O)NR'(R''), wherein the alkyl, alkoxy, cycloalkyl, heterocyclyl, aryl or heteroaryl is optionally substituted with one or more groups selected from the group consisting of halogen, hydroxy, oxo, nitro, cyano, $C_{1-6}$ alkyl, $C_{1-6}$ alkoxy, $C_{3-7}$ cycloalkyl, 3-12 membered heterocyclyl, 5-12 membered aryl, 5-12 membered heteroaryl, SR', S(=O)R', $S(=O)_2R'$, $S(=O)_2NR'(R'')$, NR'(R''), C(=O)R', C(=O)OR' and C(=O)NR'(R'');

R' and R'' are independently hydrogen, deuterium, hydroxy, alkyl, alkoxy, cycloalkyl, heterocyclyl, aryl or heteroaryl, wherein the alkyl, alkoxy, cycloalkyl, heterocyclyl, aryl or heteroaryl is optionally substituted with one or more substituents selected from the group consisting of halogen, hydroxy, oxo, nitro and cyano;

m, n, p and q are independently 0, 1, 2, 3 or 4;

B is

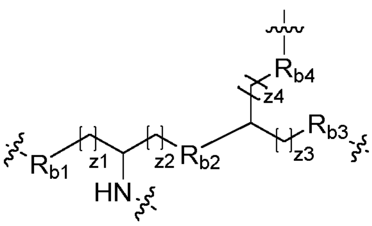 or 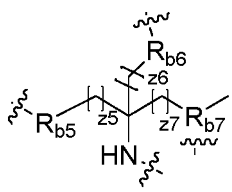 ;

$R_{b1}$, $R_{b2}$, $R_{b3}$, $R_{b4}$, $R_{b5}$, $R_{b6}$ and $R_{b7}$ are independently -C(=O)-, -NHC(=O)-, -C(=O)O-, $-C(=O)-(CH_2)_{z8}-O-$ or $-NHC(=O)-(CH_2)_{z9}-O-$;

z1, z2, z3, z4, z5, z6, z7, z8 and z9 are independently integers of 0-10;

$L_2$ is a $C_1$-$C_{30}$ alkyl chain, or a $C_1$-$C_{30}$ alkyl chain interrupted by one or more oxygen, sulfur or nitrogen atoms or C=O;

r is an integer of 1-10.

In some embodiments, $L_1$ may be $L_3$ or $L_3$-$R_{10}$-$R_{11}$-$L_3$, wherein $L_3$ is independently a $C_1$-$C_{12}$ alkyl chain, $-(CH_2)_{j1}-C(=O)-(CH_2)_{j2}-$ or $-(CH_2)_{j3}-(CH_2CH_2O)_{1-4}-(CH_2)_{j4}-$; $R_{10}$ and $R_{11}$ are independently chemical bonds, $-NR_{12}-$, -C(=O)- or -OC(=O)-; $R_{12}$ is hydrogen or $C_1$-$C_{12}$ alkyl; j 1, j2, j3 and j4 are independently integers of 0-10, preferably 0-2 or 4-10, and more preferably 0, 1, 2, 6, 7, 8, 9 or 10.

In some embodiments, $L_1$ may be $-(CH_2)_{j1}-C(=O)-(CH_2)_{j2}-$, wherein j 1 and j2 are as defined in any of the preceding embodiments.

In some embodiments, $L_1$ may be

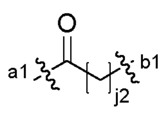

, and j 1 and j2 are as defined in any of the preceding embodiments, wherein the end a1 is attached to B, and the end b1 is attached to $R_1$.

In some embodiments, $L_1$ may be

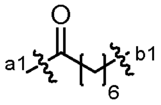 , 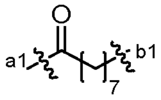 , 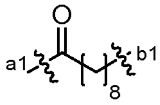 ,

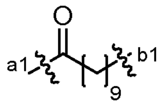 or 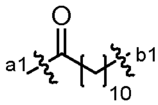 ,

wherein the end a1 is attached to B, and the end b1 is attached to $R_1$.

In some embodiments, $R_1$ may be a chemical bond and $R_2$ may be C=O.

In some embodiments, $R_1$ may be a chemical bond and $R_2$ may be $NR_6$, wherein $R_6$ is as defined in any of the preceding embodiments.

In some embodiments, $R_1$ may be a chemical bond and $R_2$ may be -OC(=O)-.

In some embodiments, $R_1$ may be $NR_6$ and $R_2$ may be C=O, wherein $R_6$ is as defined in any of the preceding embodiments.

In some embodiments, $R_1$ may be $NR_6$ and $R_2$ may be -OC(=O)-, wherein $R_6$ is as defined in any of the preceding embodiments.

In some embodiments, $R_2$ may be $NR_6$ and $R_1$ may be C=O, wherein $R_6$ is as defined in any of the preceding embodiments.

In some embodiments, $R_2$ may be $NR_6$ and $R_1$ may be -OC(=O)-, wherein $R_6$ is as defined in any of the preceding embodiments.

In some embodiments, $R_6$ may be hydrogen or $C_{1-6}$ alkyl.

In some embodiments, $R_6$ may be hydrogen, methyl, ethyl, propyl or isopropyl.

In some embodiments, $R_6$ may be hydrogen.

In some embodiments, $R_7$ and $R_8$ may be hydrogen.

In some embodiments, $R_9$ may be hydrogen.

In some embodiments, when ring A is present, ring A may be $C_{6-10}$ aryl, preferably phenyl.

In some embodiments, m may be 0 or 1.

In some embodiments, m may be 3.

In some embodiments, n may be 0 or 1.

In some embodiments, p and q are independently 0 or 1.

In some embodiments, p = 1 and q = 1.

In some embodiments, p = 1 and q = 0.

In some embodiments, p = 0 and q = 1.

In some embodiments, p = 0 and q = 0.

In some embodiments, z1, z2, z3, z4, z5, z6, z7, z8 and z9 may independently be integers of 0-4, preferably 0, 1 or 2.

In some embodiments, B may be

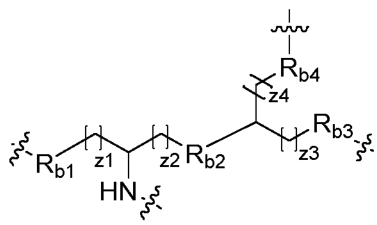 ,

wherein $R_{b1}$, $R_{b2}$, $R_{b3}$ and $R_{b4}$ are independently -C(=O)- or -NHC(=O)-; the N atom is attached to $L_1$; z1, z2, z3 and z4 are as defined in any of the preceding embodiments (e.g.,

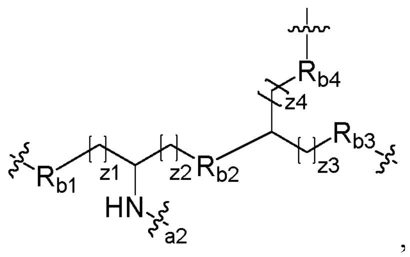

, wherein the N atom of the end a2 is attached to $L_1$).

In some embodiments, B may be

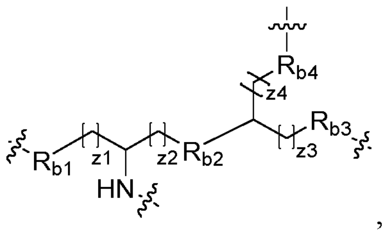

wherein $R_{b1}$, $R_{b2}$, $R_{b3}$ and $R_{b4}$ are independently -C(=O)- or -NHC(=O)-; the N atom is attached to $L_1$; $R_{b1}$, $R_{b3}$ and $R_{b4}$ are identical; z1, z2, z3 and z4 are as defined in any of the preceding embodiments (e.g.,

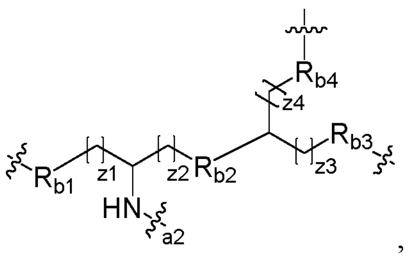

wherein the N atom of the end a2 is attached to $L_1$).

In some embodiments, B may be

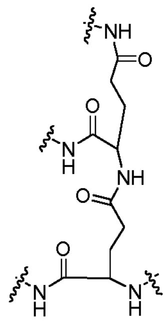

(e.g.,

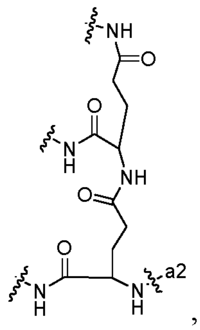

wherein the N atom of the end a2 is attached to $L_1$).

In some embodiments, B may be

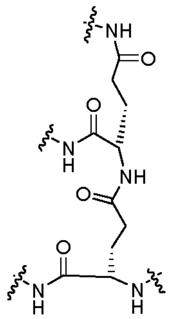

(e.g.,

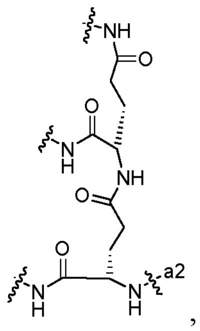

wherein the N atom of the end a2 is attached to $L_1$).

In some embodiments, B may be

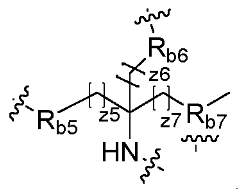

wherein $R_{b5}$, $R_{b6}$ and $R_{b7}$ are independently -C(=O)-$(CH_2)_{z8}$-O- or -NHC(=O)-$(CH_2)_{z9}$-O-; the N atom is attached to $L_1$; z5, z6, z7, z8 and z9 are as defined in any of the preceding embodiments.

In some embodiments, B may be

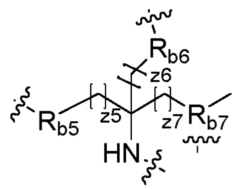

wherein $R_{b5}$, $R_{b6}$ and $R_{b7}$ are independently -C(=O)-$(CH_2)_{z8}$-O- or -NHC(=O)-$(CH_2)_{z9}$-O-; the N atom is attached to $L_1$; $R_{b5}$, $R_{b6}$ and $R_{b7}$ are identical; z5, z6, z7, z8 and z9 are as defined in any of the preceding embodiments.

In some embodiments, B may be

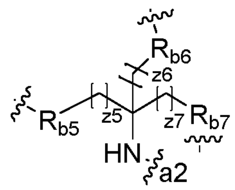

wherein $R_{b5}$, $R_{b6}$ and $R_{b7}$ are independently -C(=O)-$(CH_2)_{z8}$-O- or -NHC(=O)-$(CH_2)_{z9}$-O-; the N atom of the end a2 is attached to $L_1$; z5, z6, z7, z8 and z9 are as defined in any of the preceding embodiments.

In some embodiments, B may be

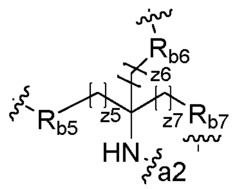

wherein $R_{b5}$, $R_{b6}$ and $R_{b7}$ are independently -C(=O)-$(CH_2)_{z8}$-O- or -NHC(=O)-$(CH_2)_{z9}$-O-; the N atom of the end a2 is attached to $L_1$; $R_{b5}$, $R_{b6}$ and $R_{b7}$ are identical; z5, z6, z7, z8 and z9 are as defined in any of the preceding embodiments.

In some embodiments, B may be

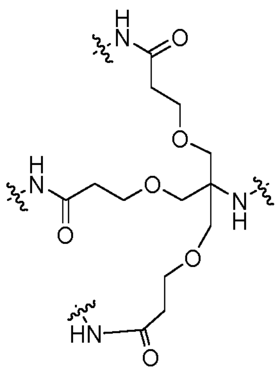

(e.g.,

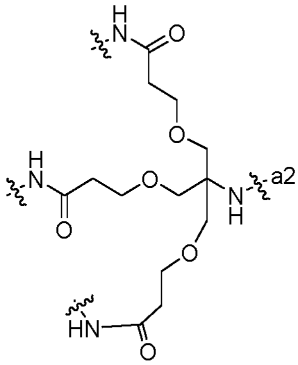

, wherein the N atom of the end a2 is attached to $L_1$).

In some embodiments, $L_2$ may be $L_4$ or $L_4$-$R_{13}$-$R_{14}$-$L_4$, wherein $L_4$ is independently a $C_1$-$C_{12}$ alkyl chain or $-(CH_2)_{j5}-(OCH_2CH_2)_{1-4}-(CH_2)_{j6}-$; $R_{13}$ and $R_{14}$ are independently chemical bonds, $-NR_{15}-$, -C(=O)- or -OC(=O)-; $R_{15}$ is independently hydrogen or $C_1$-$C_{12}$ alkyl; j5 and j6 are independently integers of 0-10, preferably 0-6, and more preferably 0, 1, 2, 3 or 4.

In some embodiments, $L_2$ may be $-(CH_2)_{j5}-(OCH_2CH_2)_{1-4}-(CH_2)_{j6}-$, wherein j5 and j6 are as defined in any of the preceding embodiments.

In some embodiments, $L_2$ may be

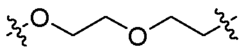 , 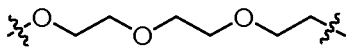 ,

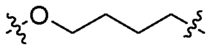 , 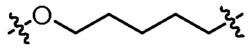 or 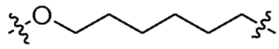 ,

preferably

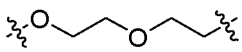 or 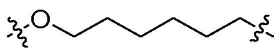 ,

wherein the O atom is attached to G, and the C atom is attached to B.

In some embodiments, $L_2$ may be a $C_1$-$C_{12}$ alkyl chain.

In some embodiments, $L_2$ may be

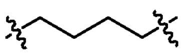 , 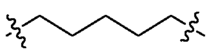 ,

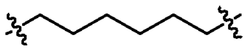 , 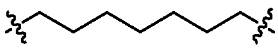 or 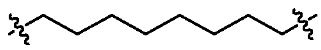 .

In some embodiments, $L_2$ may be

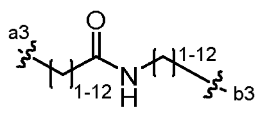

, preferably

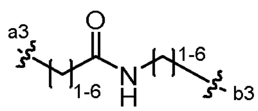 ,

and more preferably

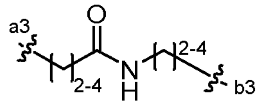

wherein the end a3 is attached to O, and the end b3 is attached to B.

In some embodiments, L2 may be

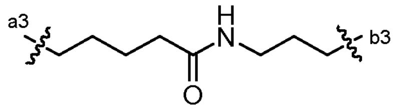

wherein the end a3 is attached to O, and the end b3 is attached to B.

In some embodiments, $L_2$ may be

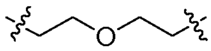

In some embodiments, r may be 3, 4, 5 or 6, preferably 3.

In some embodiments, Q may be

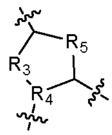

wherein $R_3$, $R_4$ and $R_5$ are as defined in any of the preceding embodiments.

In some embodiments,

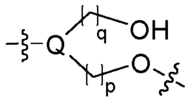

may be

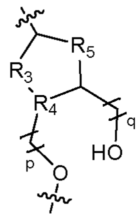

wherein $R_3$, $R_4$, $R_5$, p and q are as defined in any of the preceding embodiments.

In some embodiments,

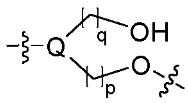

may be

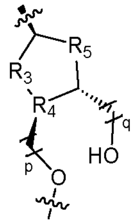,
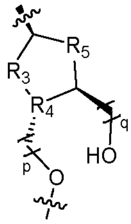,
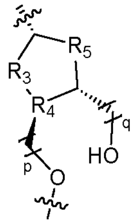

or

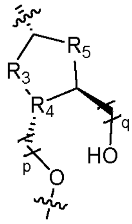,

wherein $R_3$, $R_4$, $R_5$, p and q are as defined in any of the preceding embodiments.

In some embodiments,

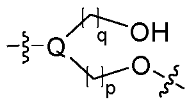,

may be

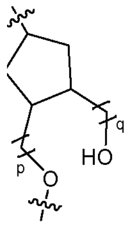,
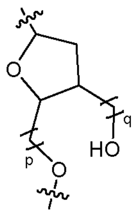
or
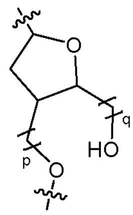,

preferably

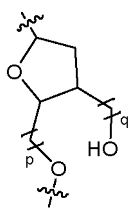
or
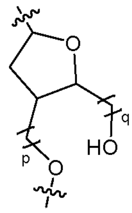,

and more preferably

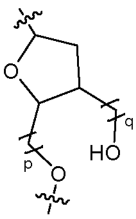,

wherein p and q are as defined in any of the preceding embodiments.

In some embodiments,

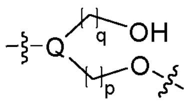

may be

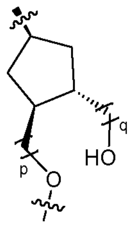, 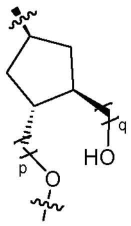, 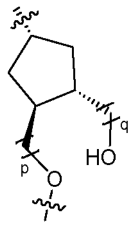,

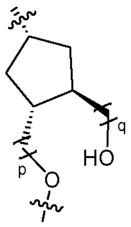, 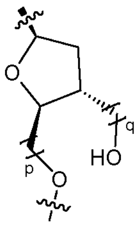, 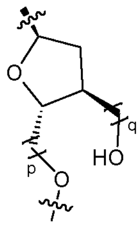, 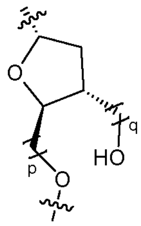, 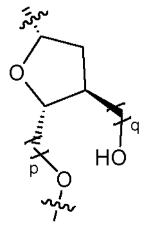, 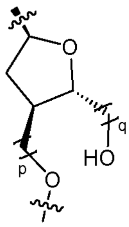, 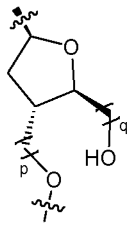,

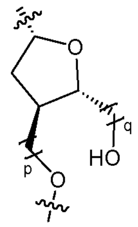 or 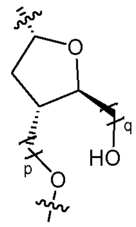,

preferably

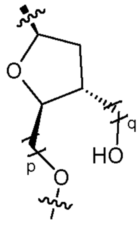, 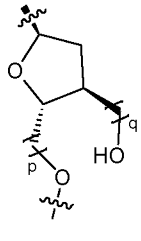, 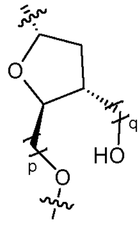,

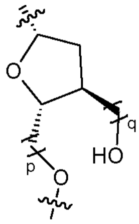 , 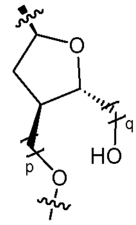 , 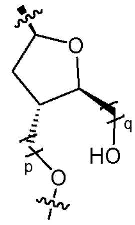 , 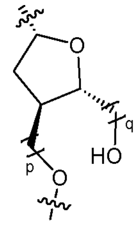 or 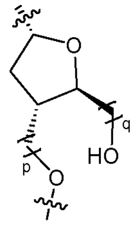 ,

and more preferably

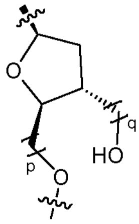 , 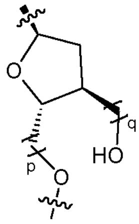 , 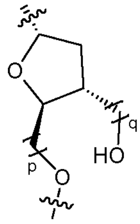 or 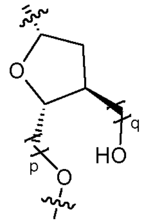 ,

wherein p and q are as defined in any of the preceding embodiments.

In some embodiments, $-R_1-R_2-$ may be $-C(=O)-NR_6-$, wherein the C atom is preferably attached to $L_1$, and $R_6$ is as defined in any of the preceding embodiments. In some embodiments, m may be 1.

In some embodiments, m may be 1;

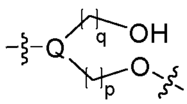

may be

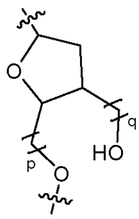 ,

preferably

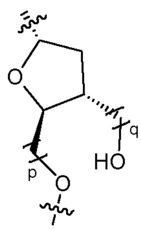 ;

p and q are as defined in any of the preceding embodiments.

In some embodiments,

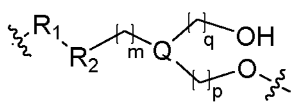

may be

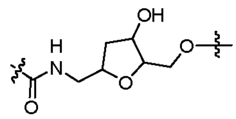

,

preferably

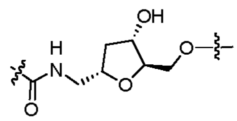

.

In some embodiments, the ligand may be any of the following structures:

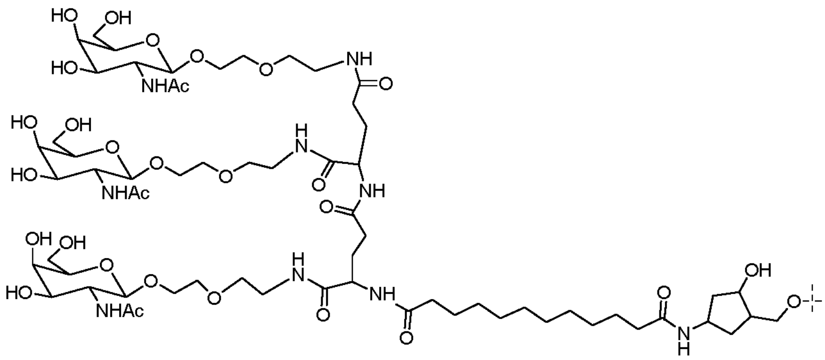

,

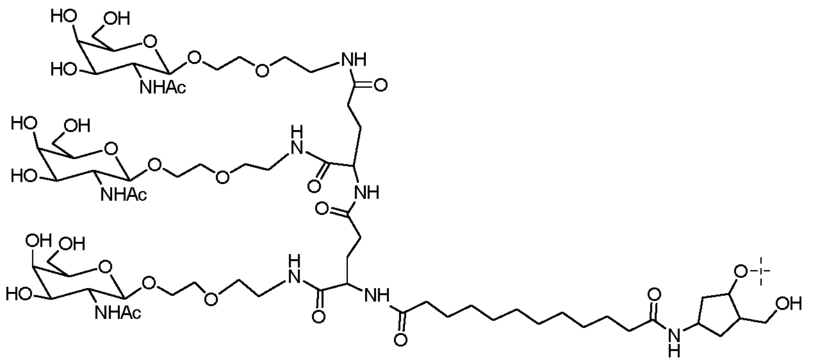

,

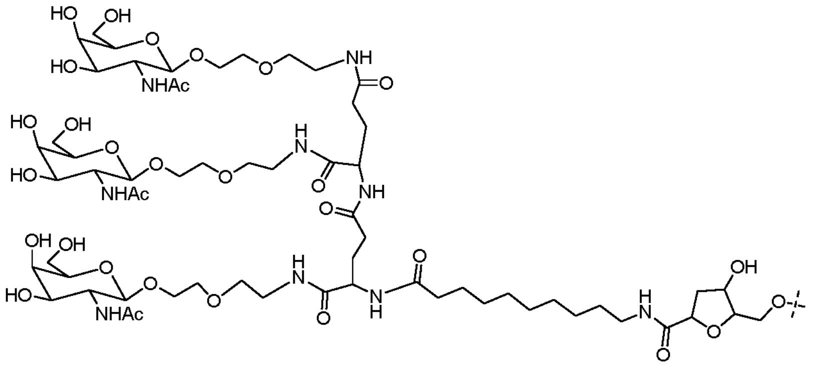

,

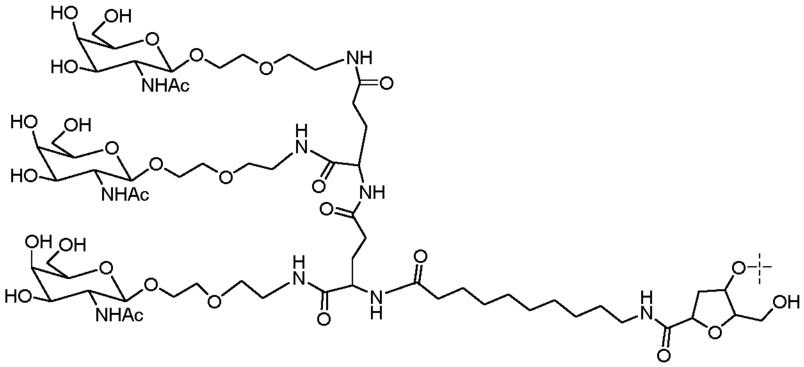

,

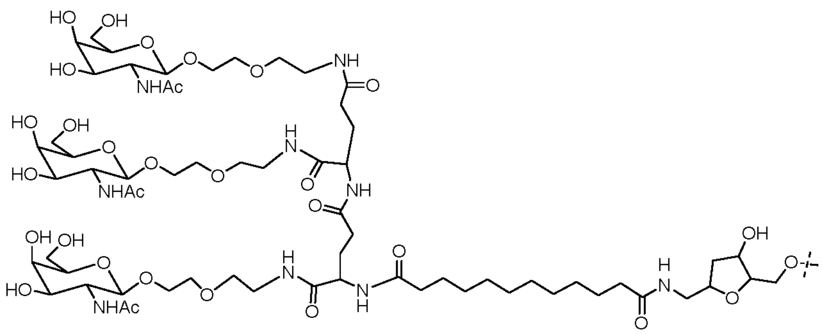

,

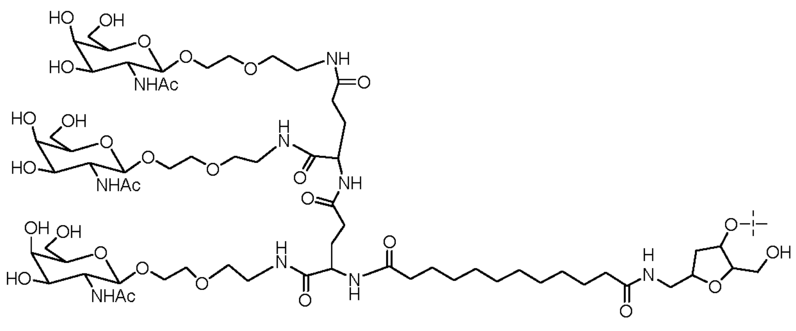

,

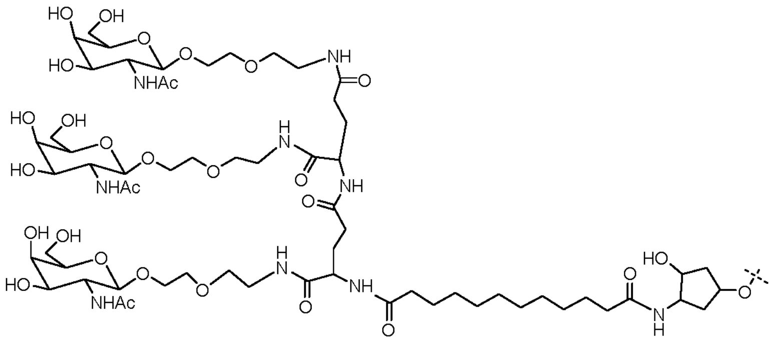

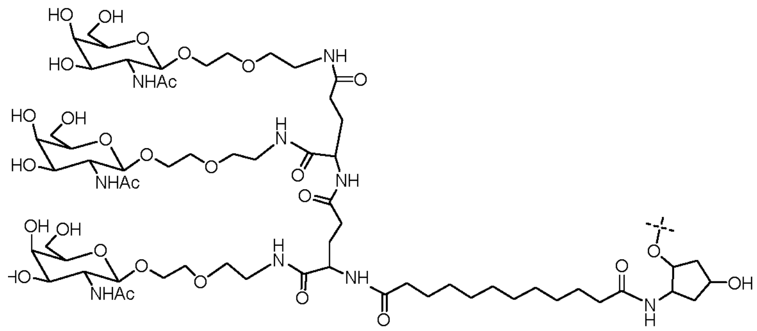

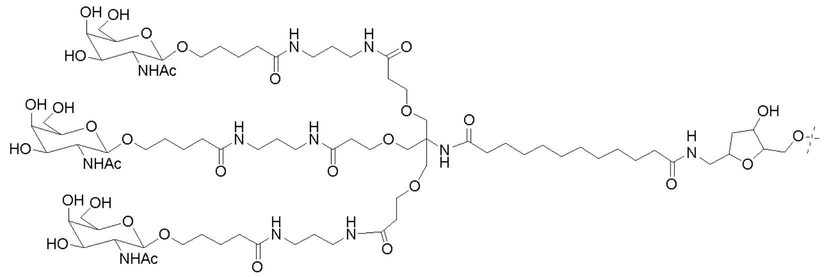

,

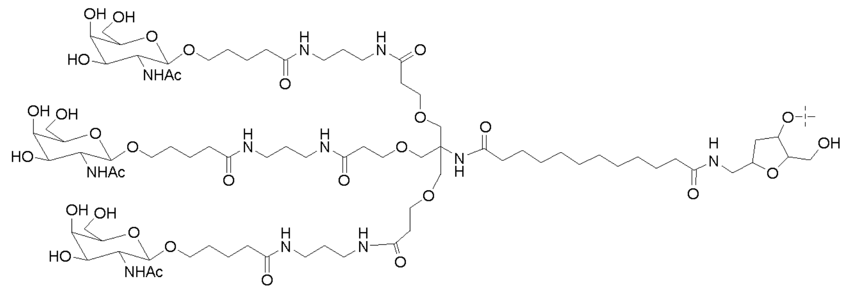

,

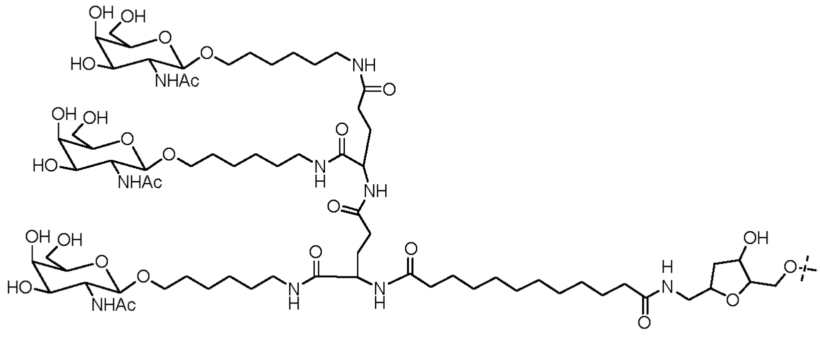

,

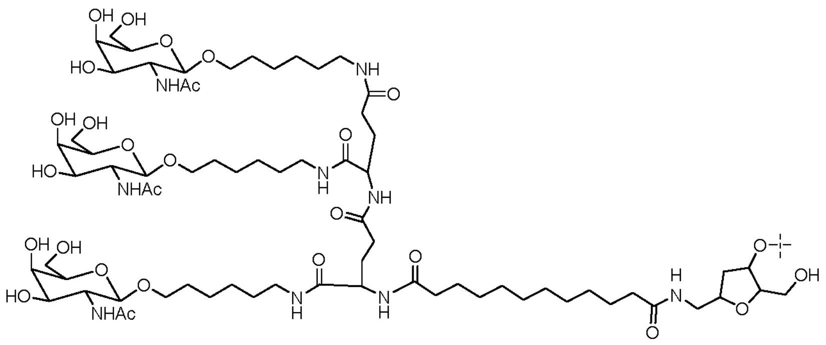

,

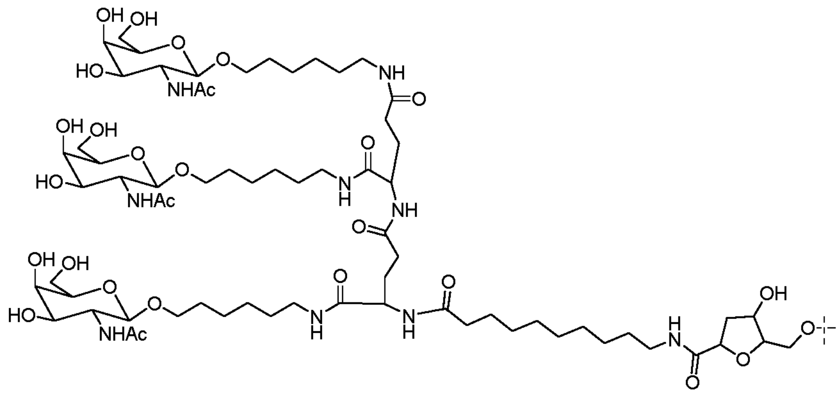

,

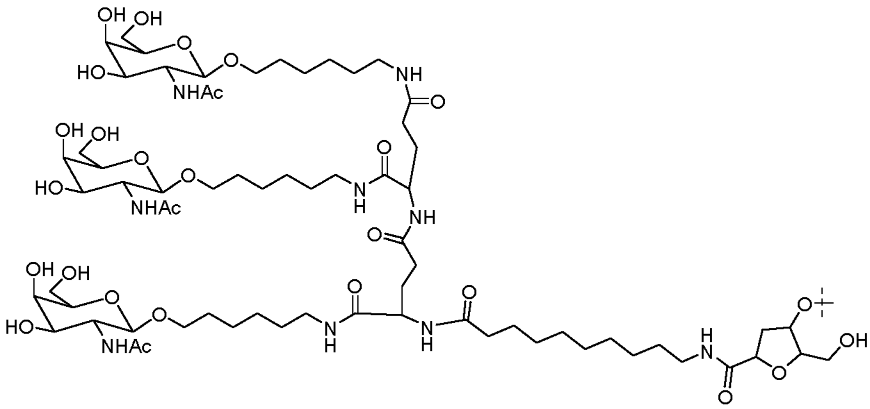

,

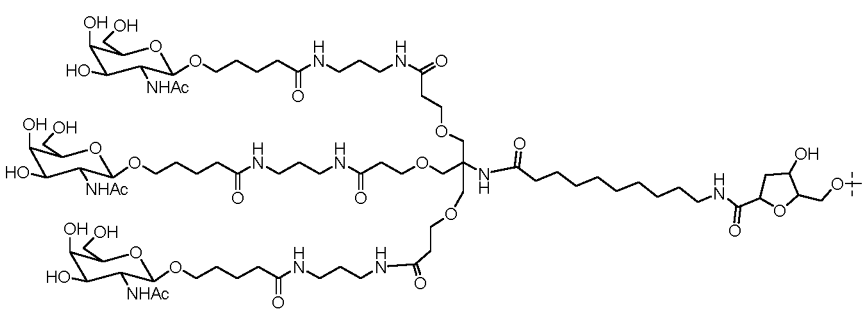

or

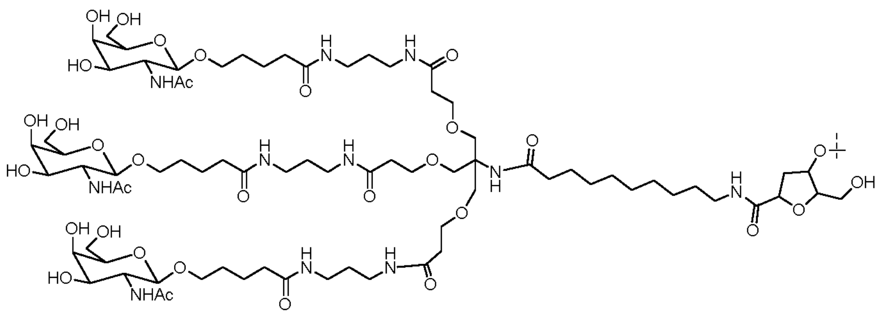

.

In some embodiments, the ligand may be any of the following structures:

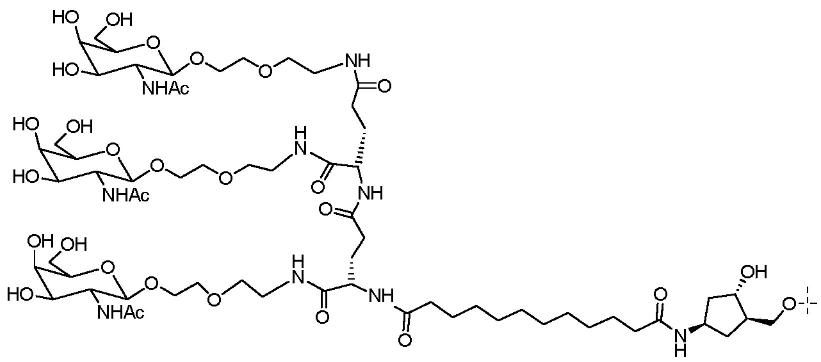

,

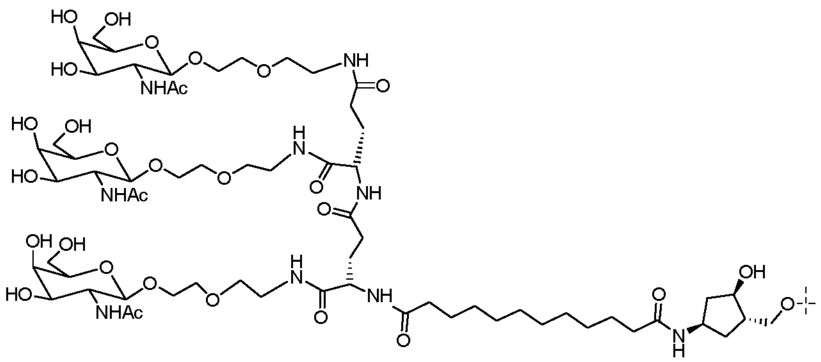

,

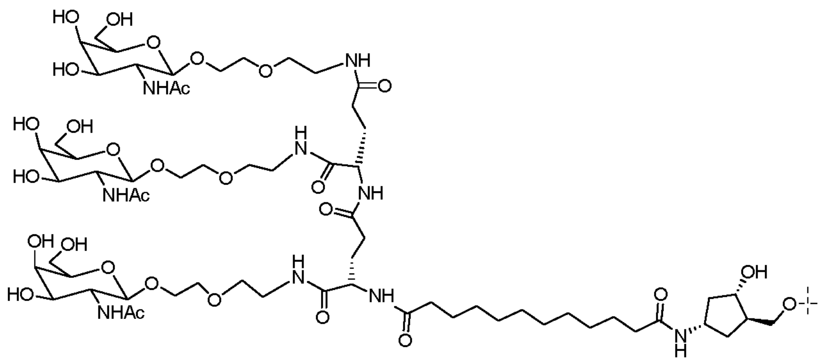

,

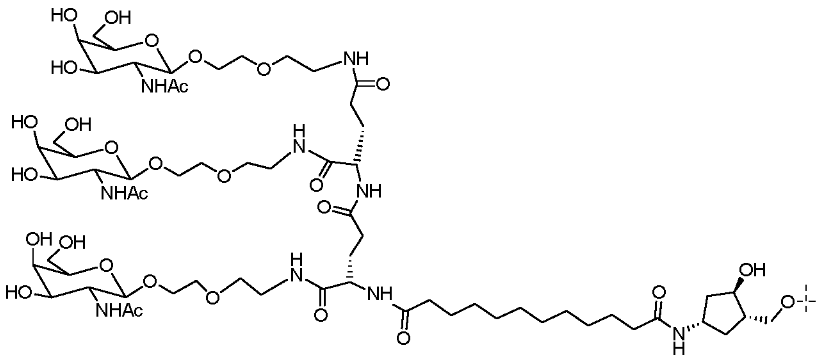

,

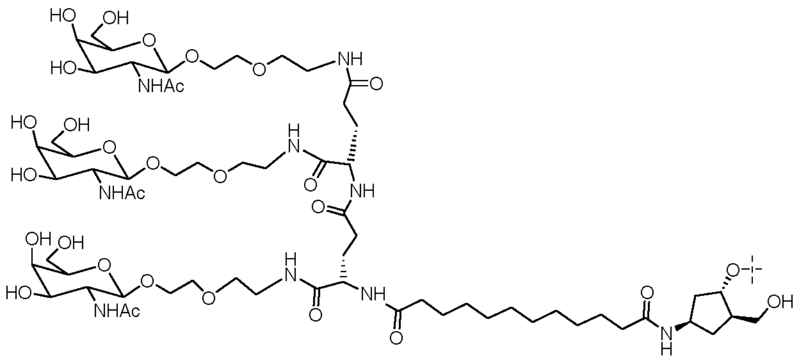

,

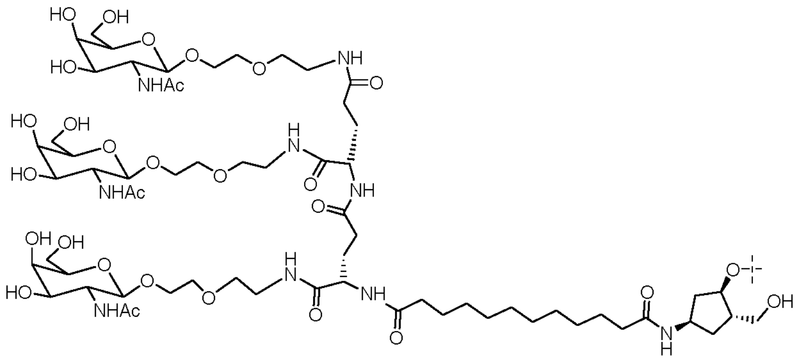

,

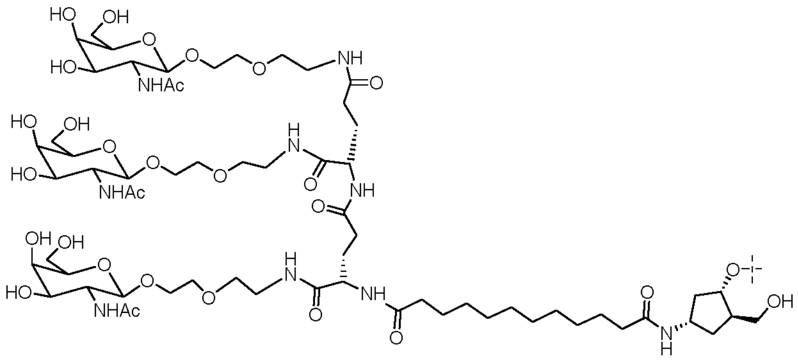

,

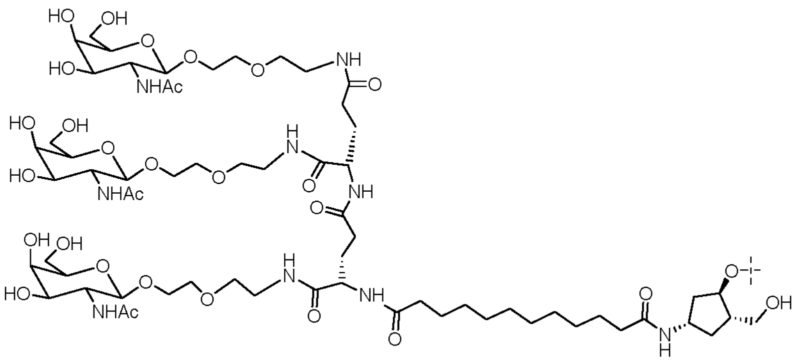

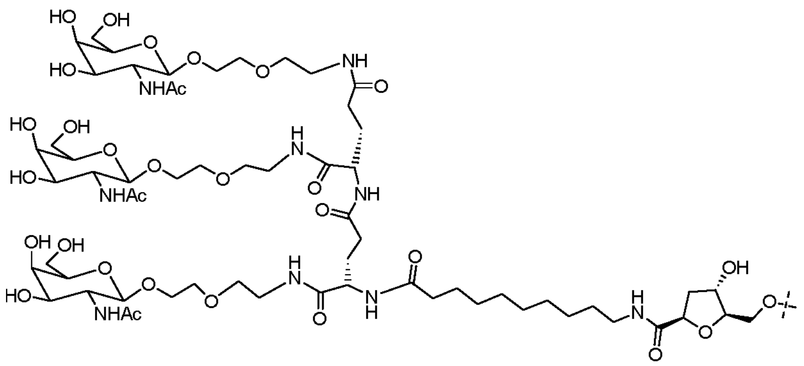

,

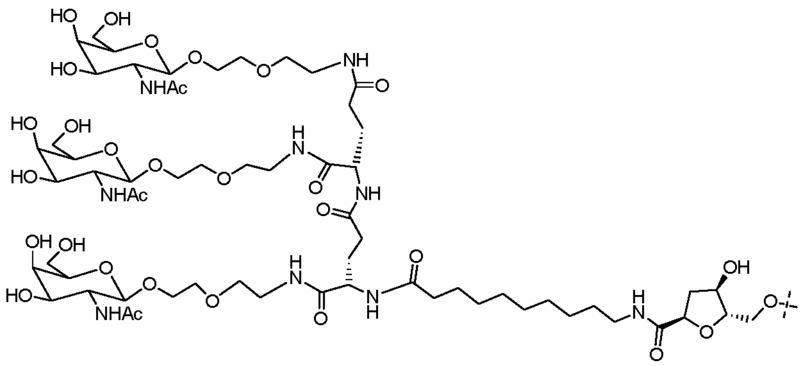

,

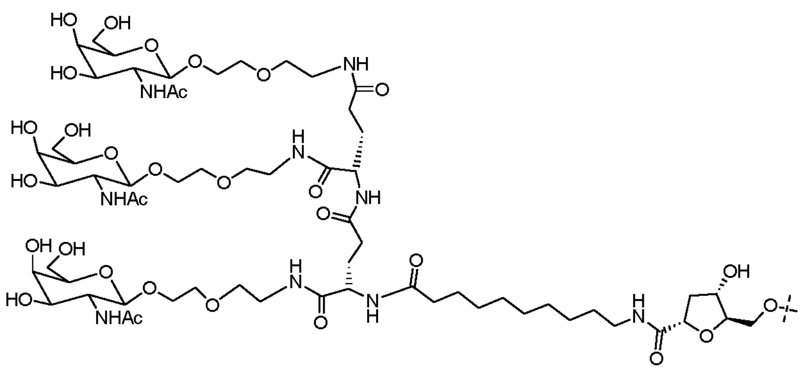

,

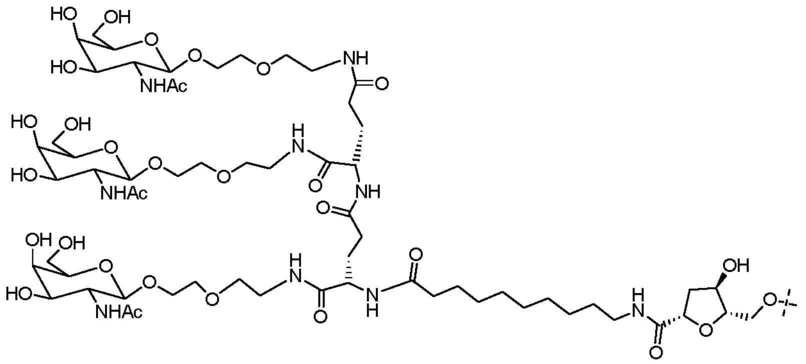

,

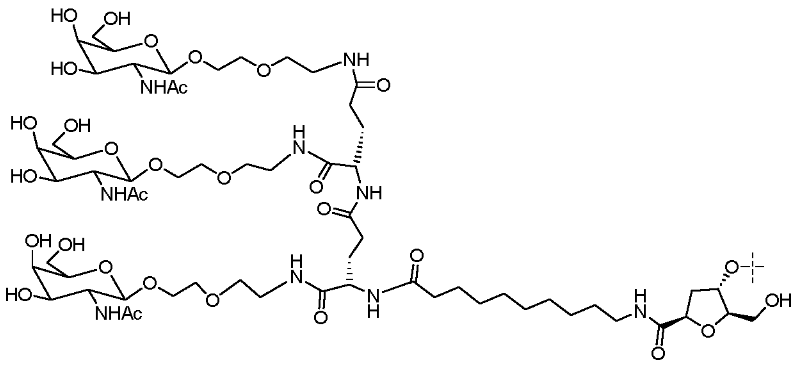

,

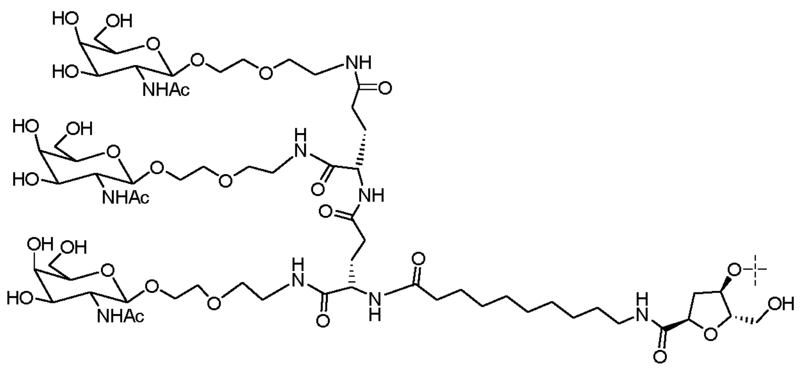

,

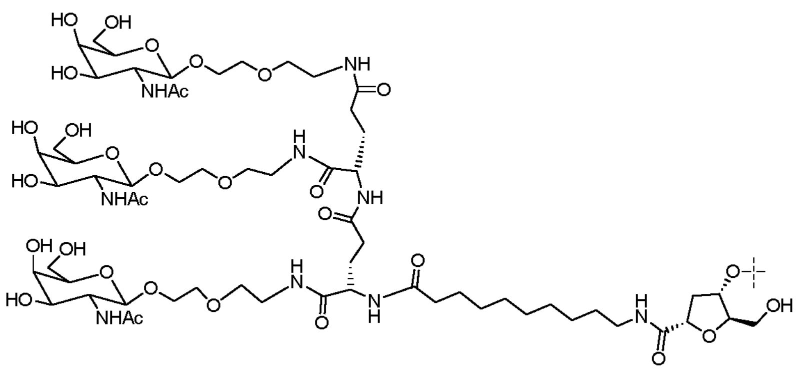

,

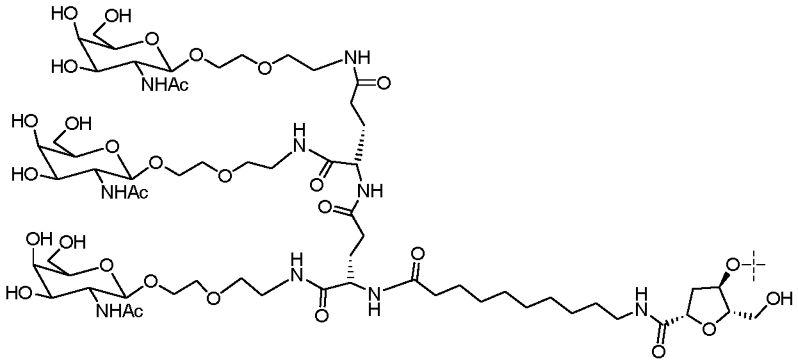

,

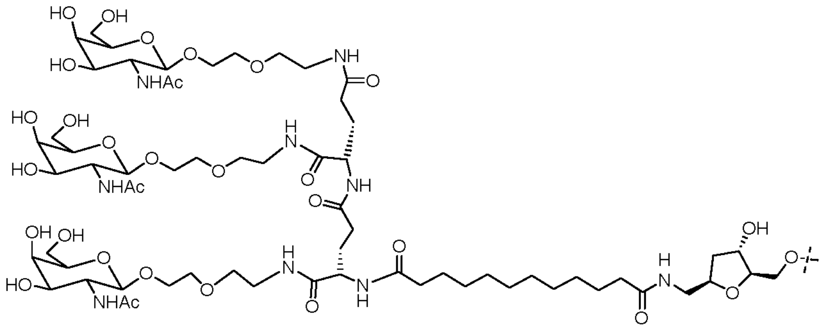

,

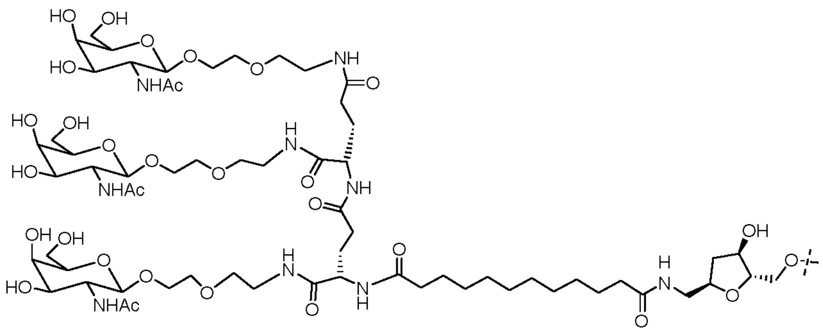

,

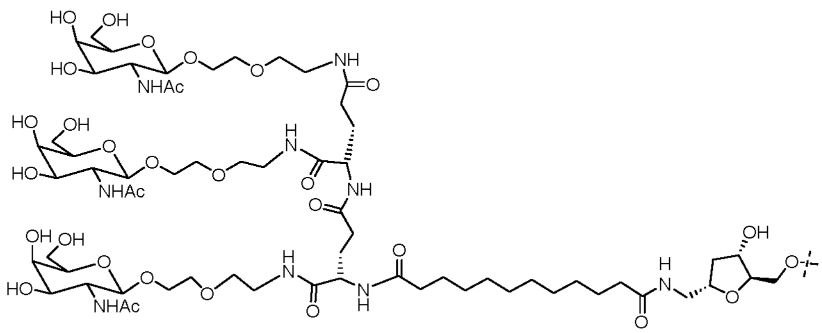

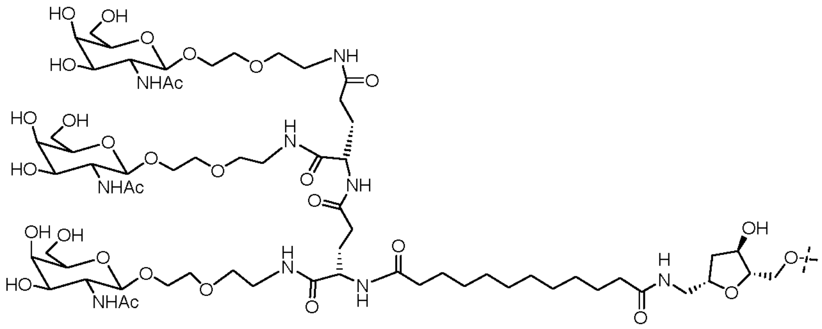

,

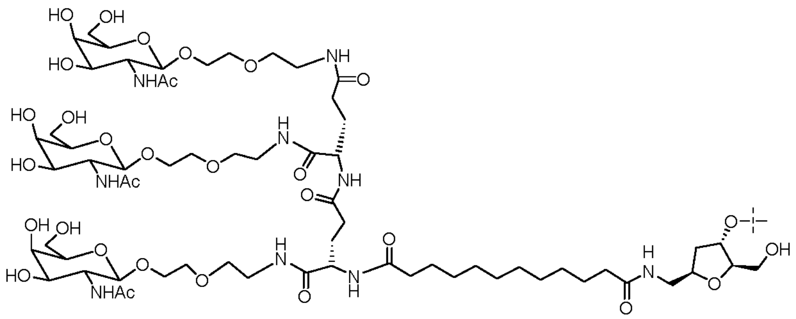

,

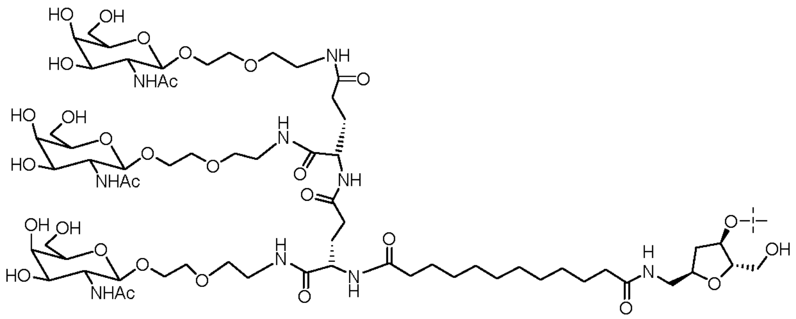

,

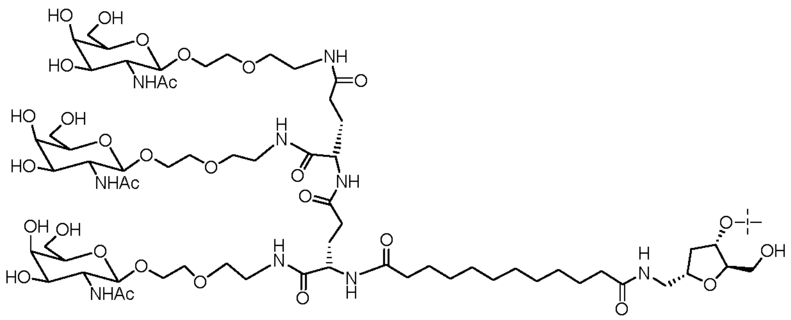

,

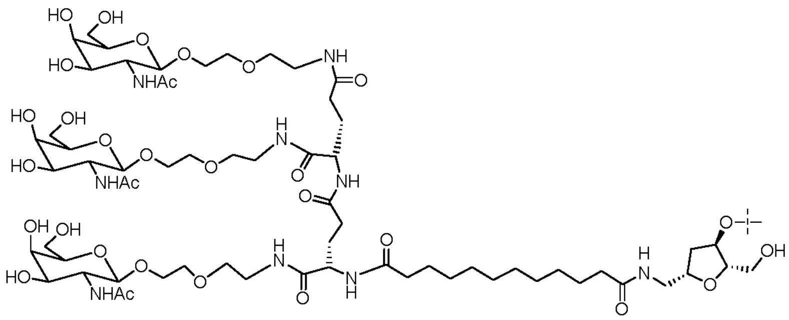

,

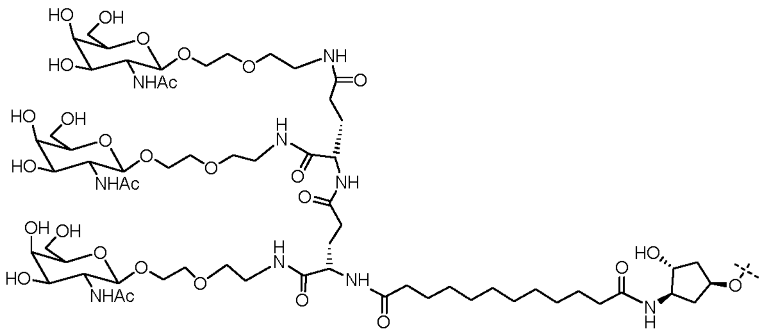

,

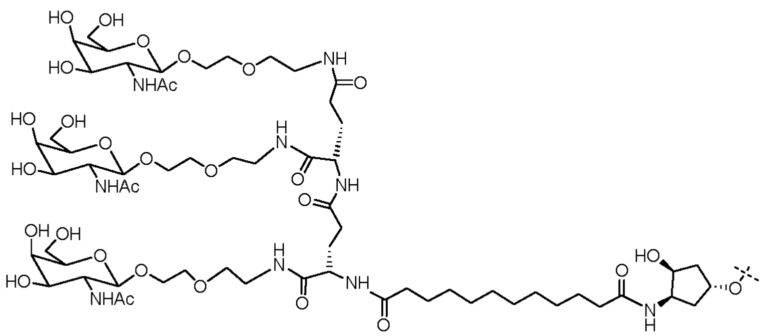

,

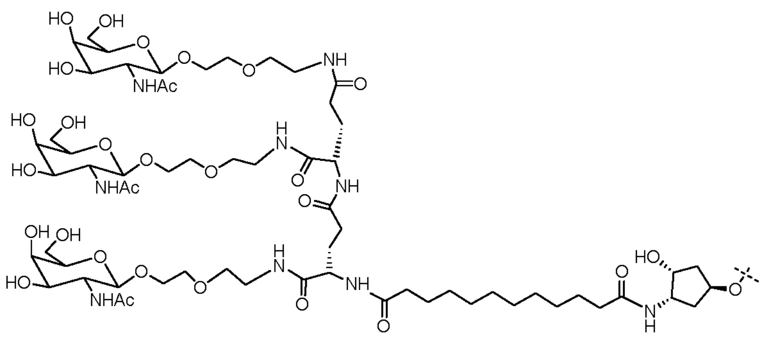

,

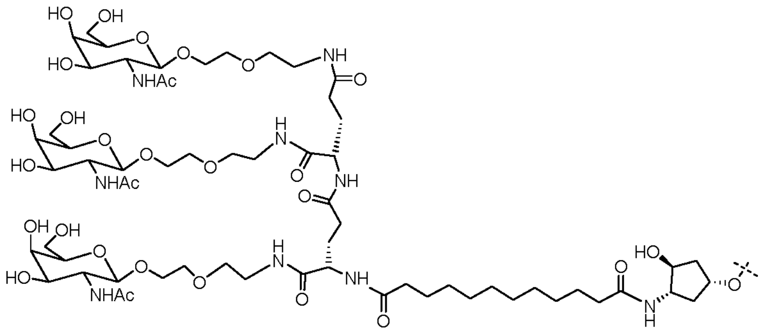

,

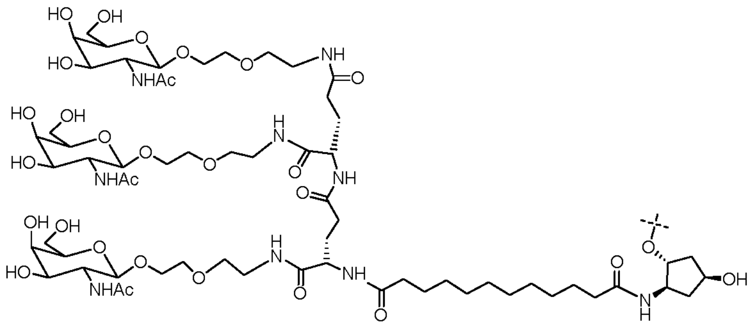

,

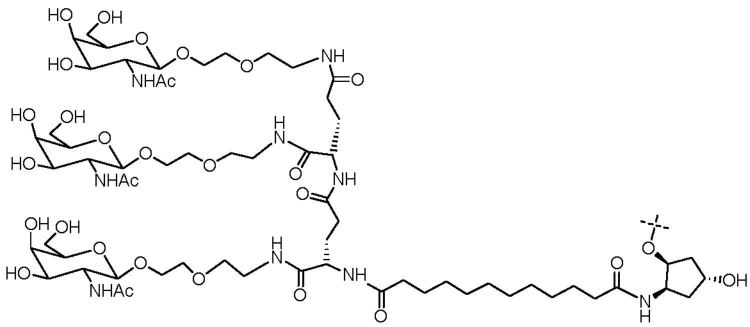

,

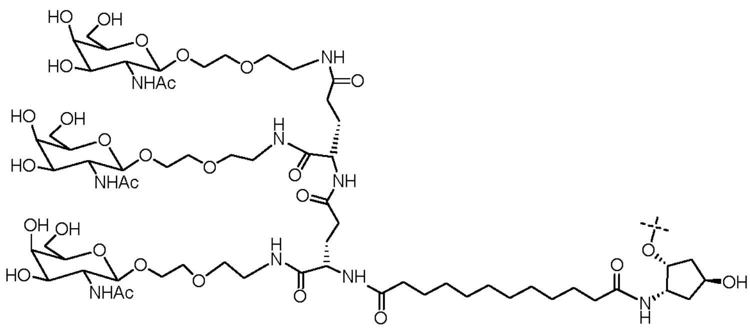

,

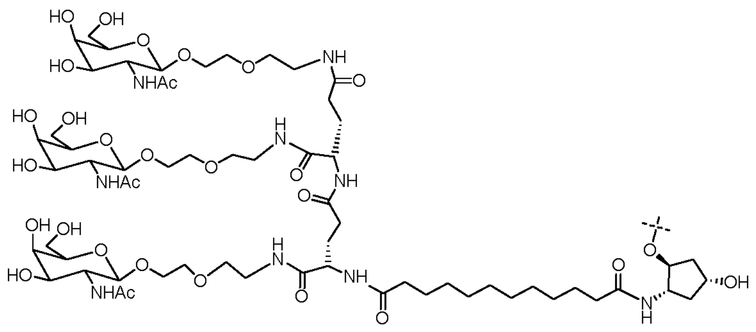

,

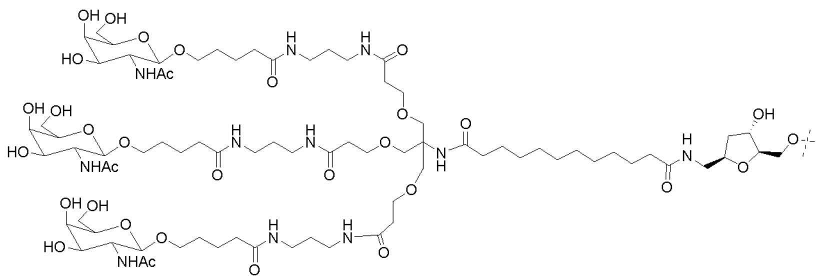

,

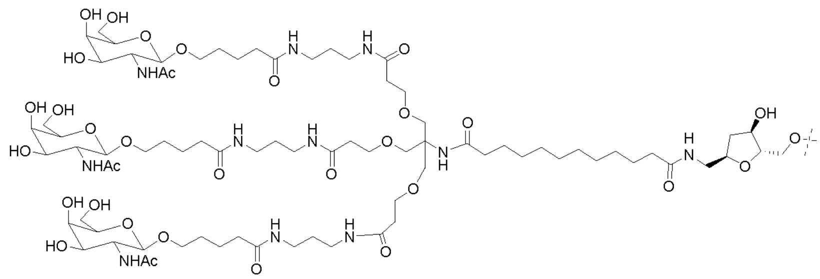

,

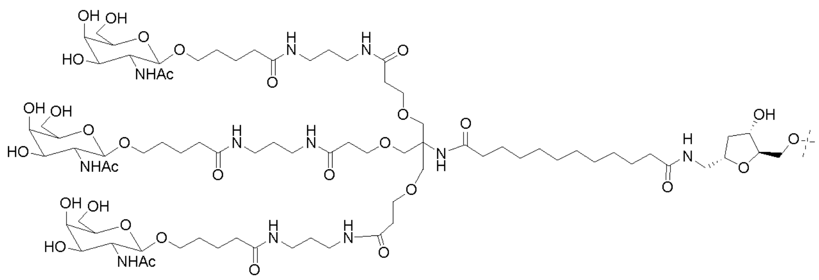

,

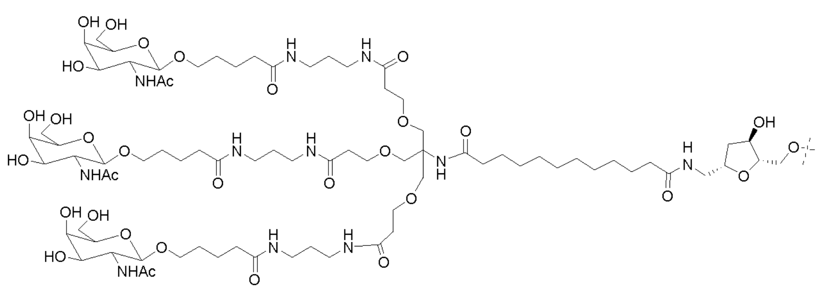

,

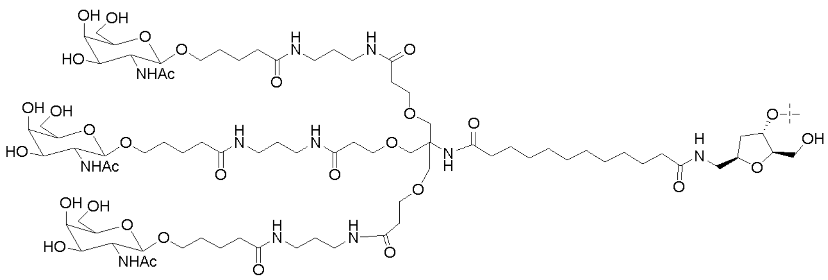

,

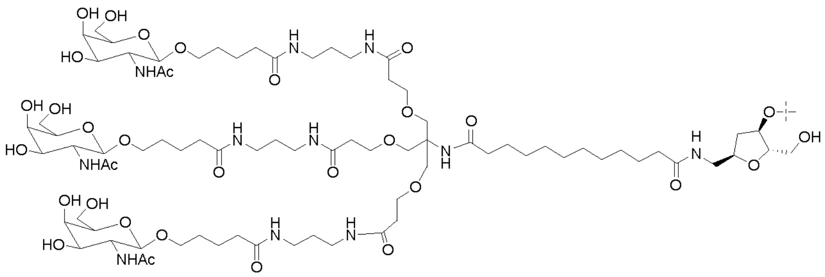

,

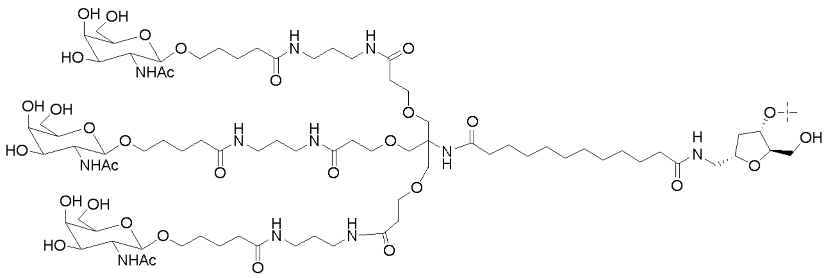

,

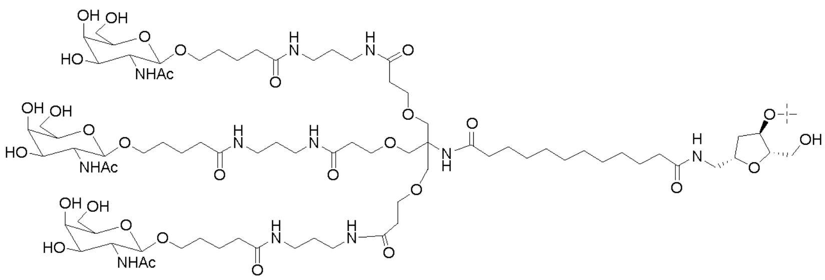

,

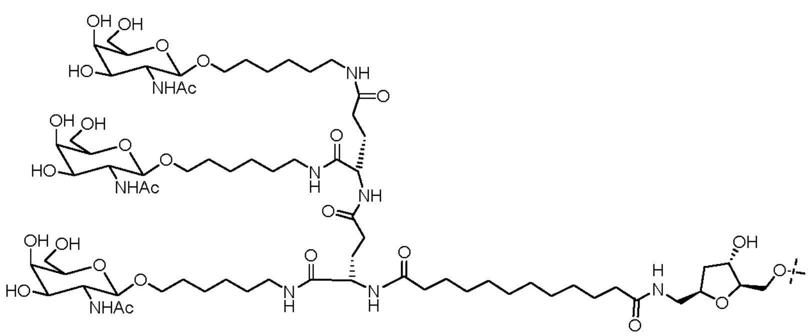

,

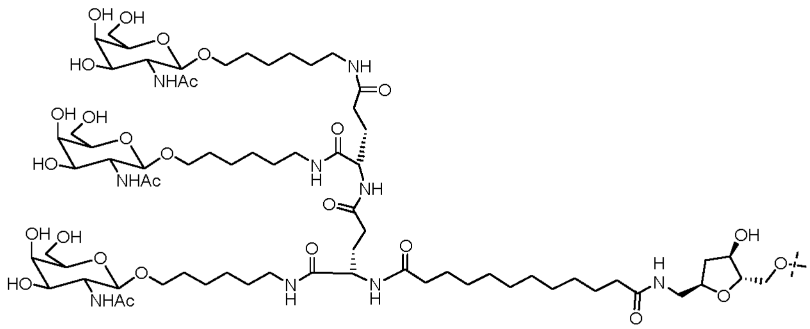

,

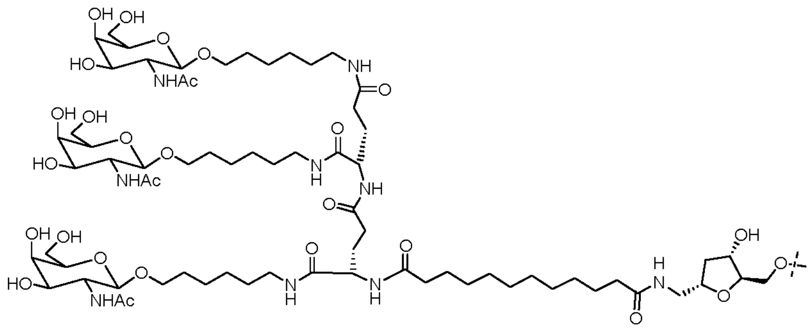

,

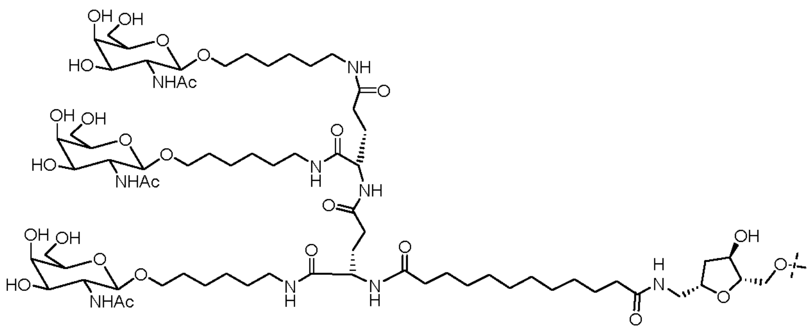

,

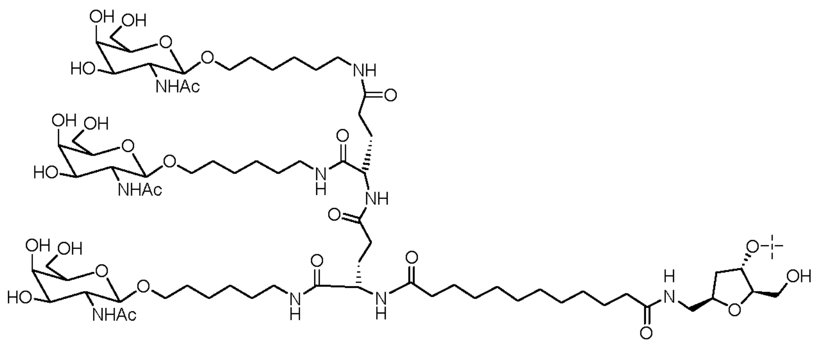

,

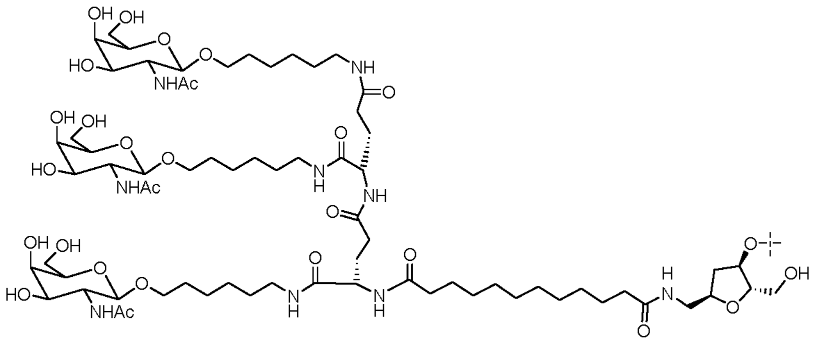

,

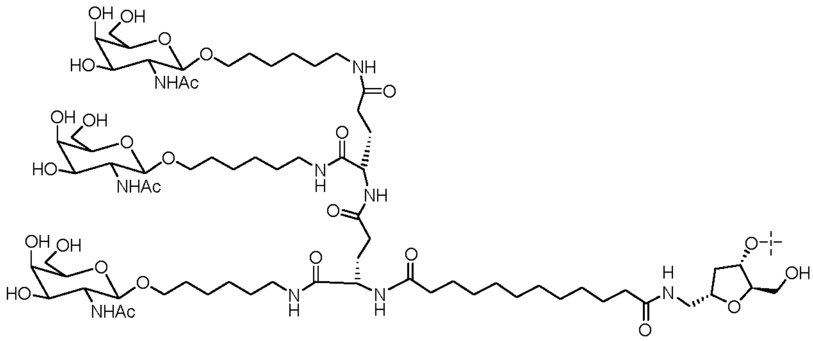

,

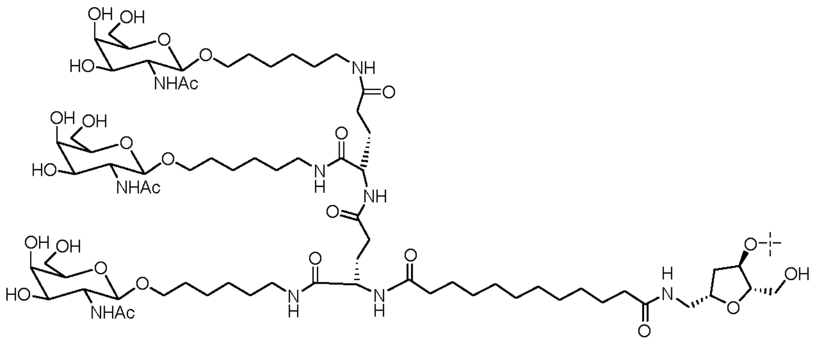

,

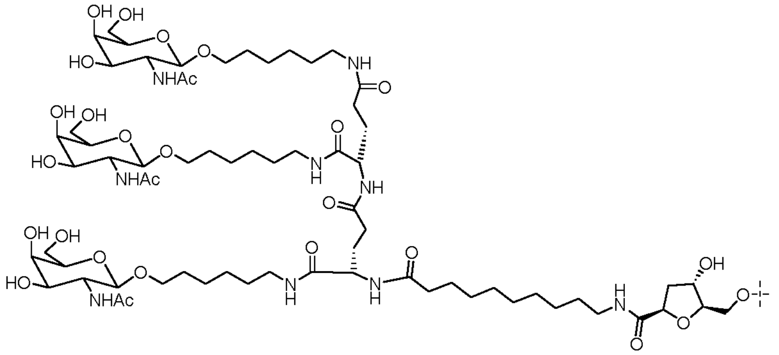

,

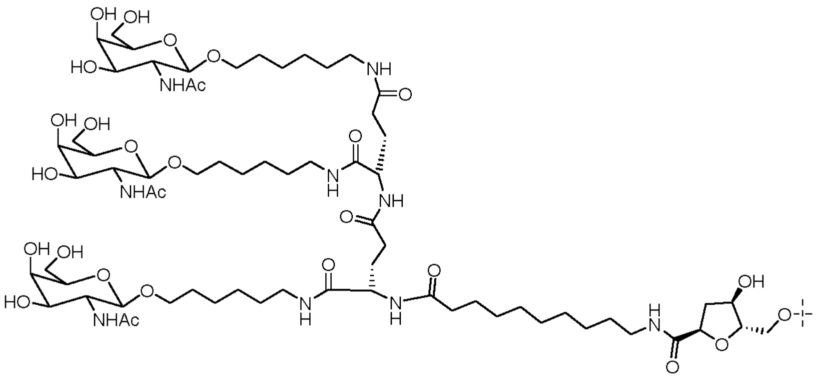

,

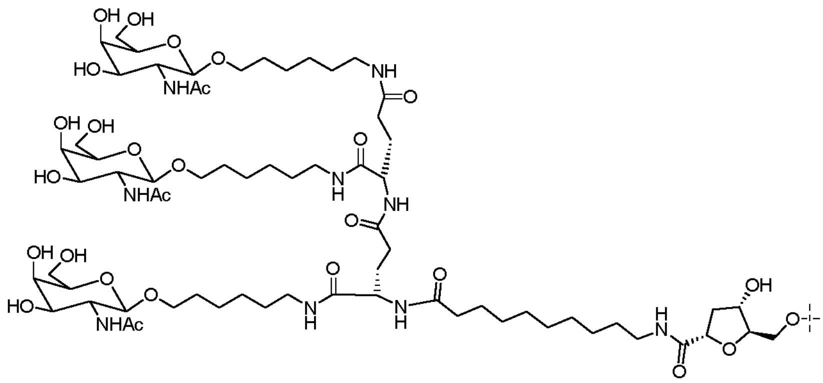

,

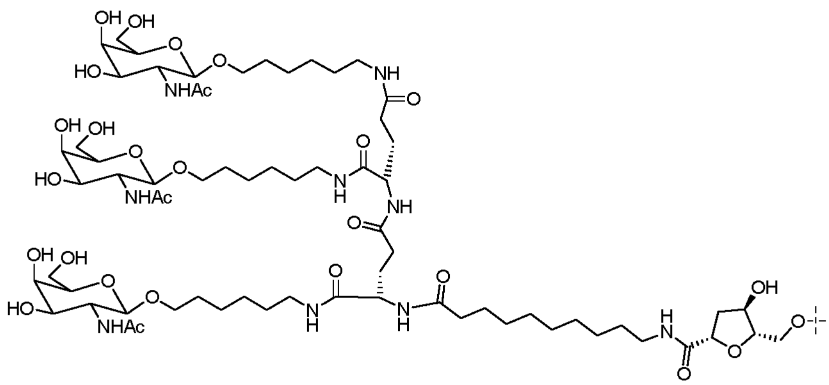

,

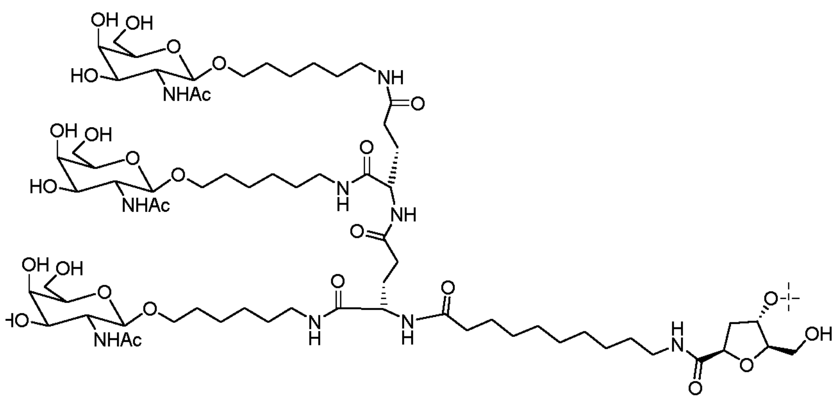

,

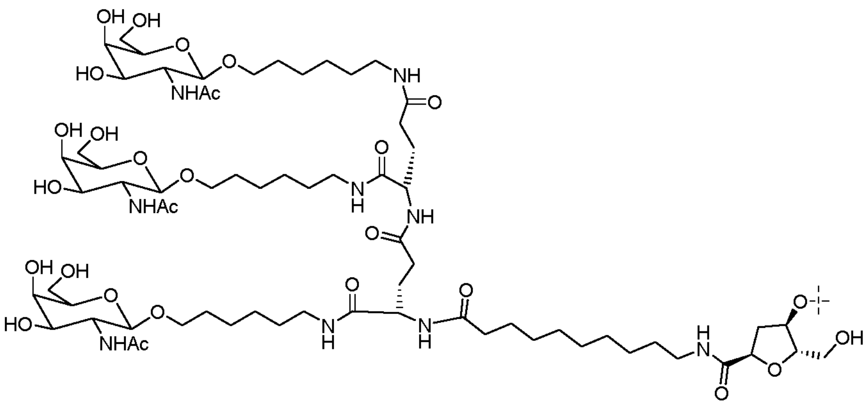

,

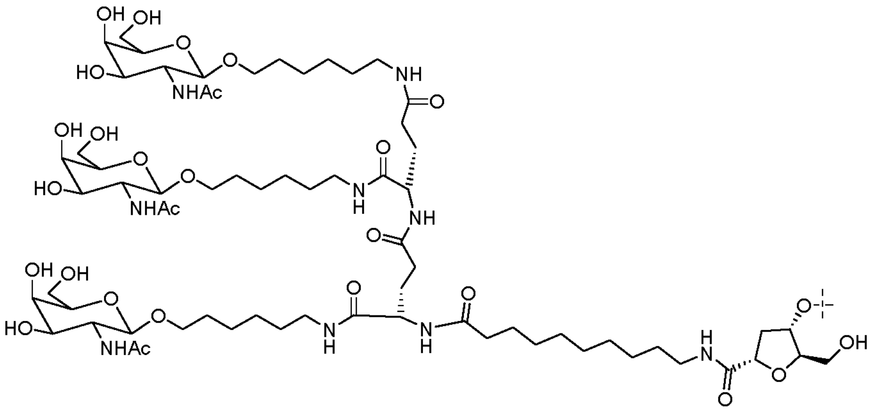

,

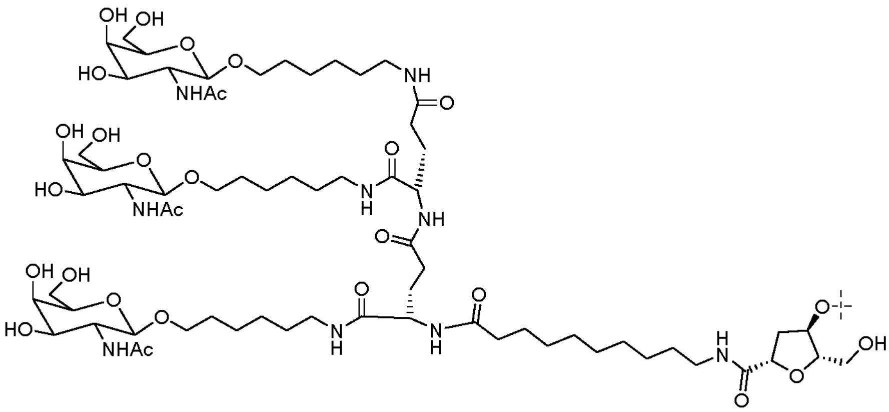

,

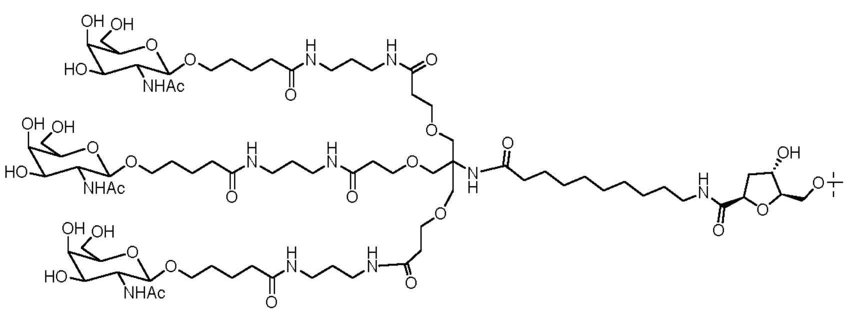

,

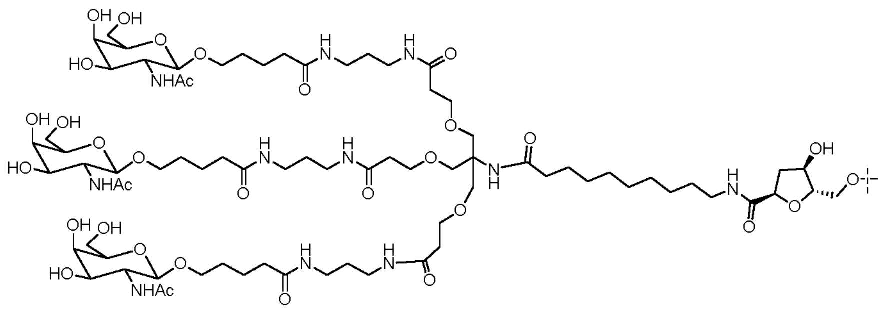

,

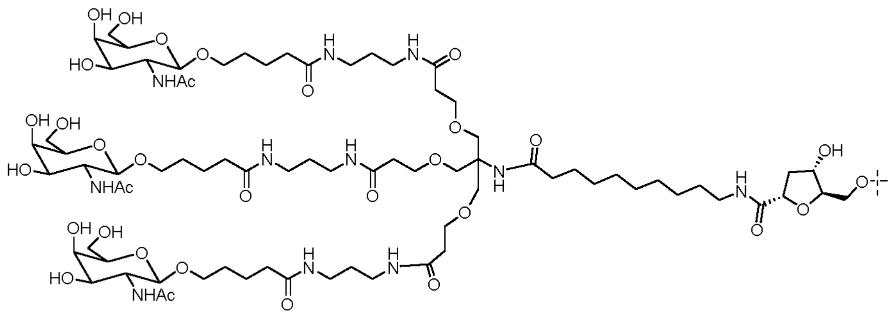

,

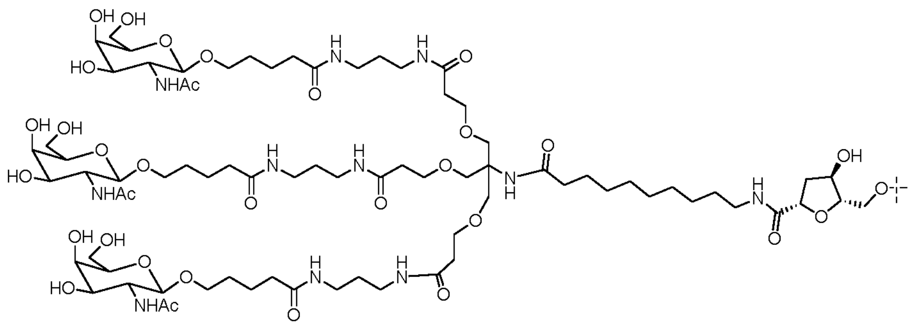

,

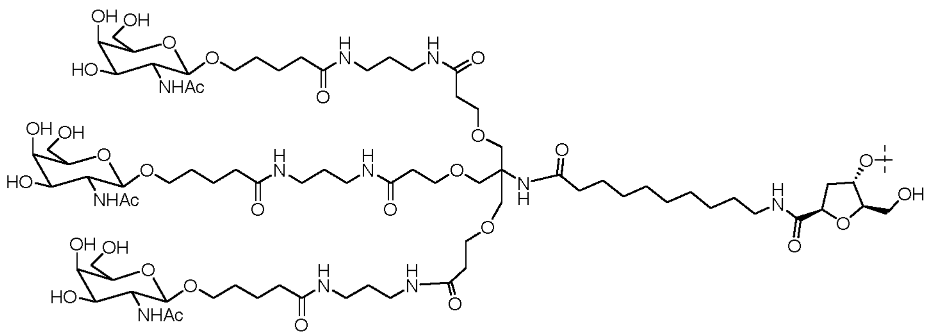

,

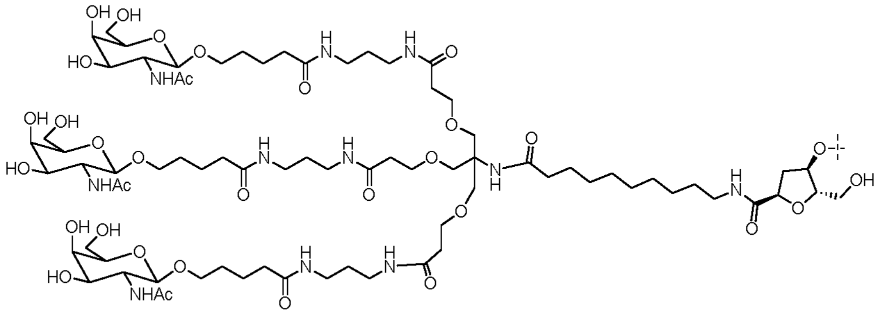

,

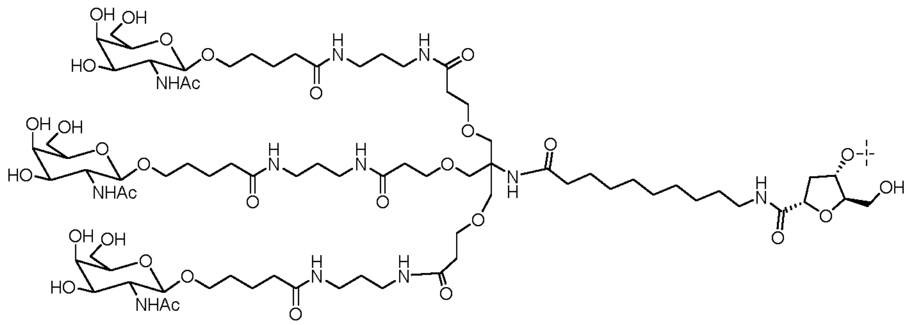

or

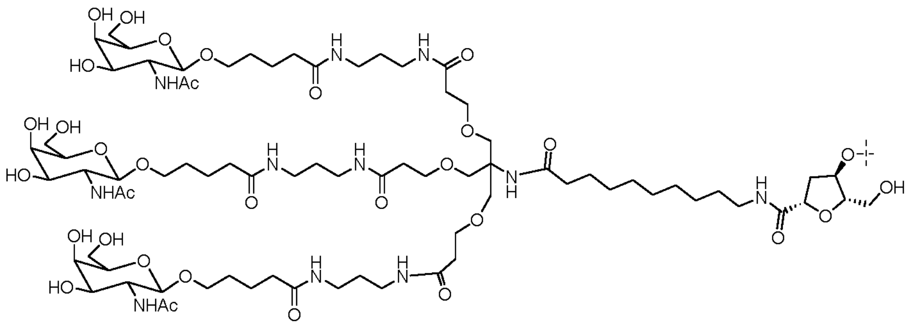

.

In some embodiments, the N-acetyl-galactosamine moiety in the above ligand may be replaced with N-trifluoroacetylgalactosamine, N-propionylgalactosamine, N-n-butyrylgalactosamine or N-isobutyrylgalactosamine.

In a second aspect, the present disclosure provides a compound represented by formula (II'),

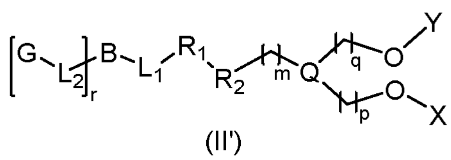

(II')

wherein X is a hydroxy protecting group; Y is hydrogen, deuterium,

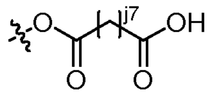

or

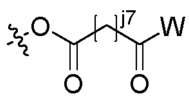;

j7 is 1, 2, 3 or 4;
W is a macromolecular compound;
G is a targeting moiety that binds to a cellular receptor;
$L_1$, $R_1$, $R_2$, Q, B, $L_2$, m, p, q and r are as defined in any of the embodiments of the ligand having a structure represented by formula (I').

In some embodiments, the hydroxy protecting group may be an ester group protecting group, an alkoxymethyl protecting group, an alkyl protecting group, a silyl protecting group or an aryl protecting group, preferably an aryl protecting group, more preferably MMTr and DMTr, and further preferably DMTr.
In some embodiments, j7 may be 2.
In some embodiments, G may be an asialoglycoprotein receptor targeting moiety.
In some embodiments, G may be N-acetyl-galactosamine triacetate, N-trifluoroacetylgalactosamine triacetate, N-propionylgalactosamine triacetate, N-n-butyrylgalactosamine triacetate or N-isobutyrylgalactosamine triacetate, preferably N-acetyl-galactosamine triacetate.
In some embodiments, the macromolecular compound may be resin, preferably macroporous resin, and more preferably macroporous aminomethyl resin.
In some embodiments,

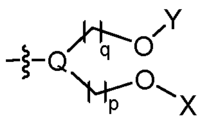

may be

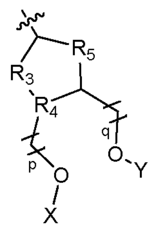,

wherein $R_3$, $R_4$, $R_5$, X, Y, p and q are as defined in any of the preceding embodiments.
In some embodiments,

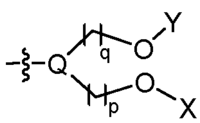

may be

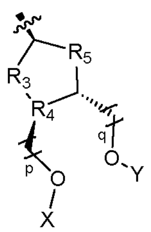, 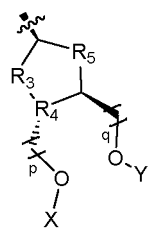,

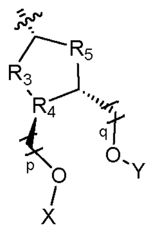

or

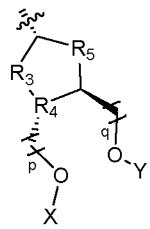

,

wherein $R_3$, $R_4$, $R_5$, X, Y, p and q are as defined in any of the preceding embodiments.

In some embodiments,

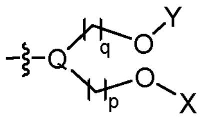

may be

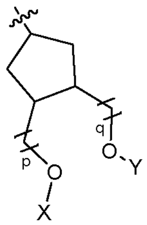

,

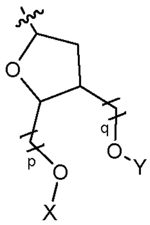

or

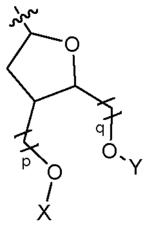

,

preferably

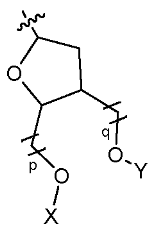

or

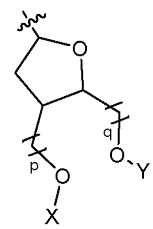

,

and more preferably

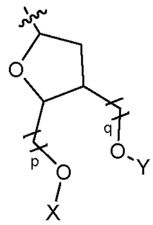

,

wherein X, Y, p and q are as defined in any of the preceding embodiments.

In some embodiments,

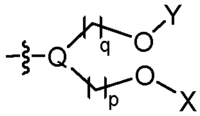

may be

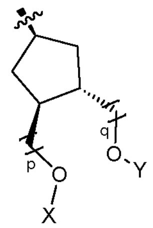 , 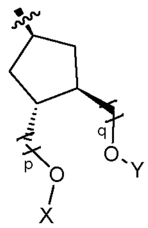 ,

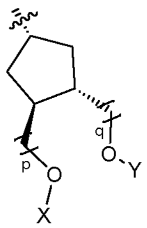 , 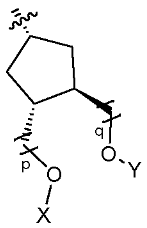 , 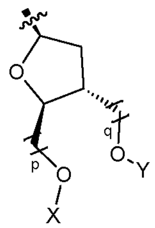 , 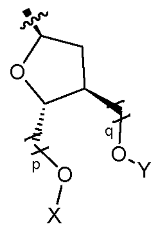 , 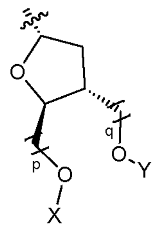 , 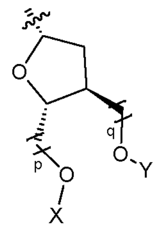 ,

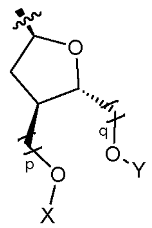 , 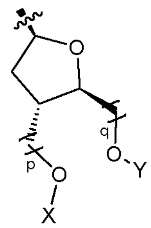 , 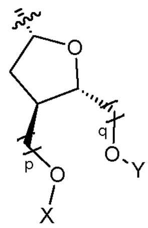 or 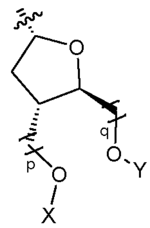 ,

preferably

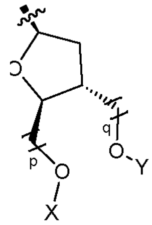

,

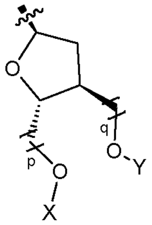 , 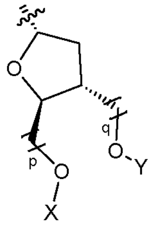 , 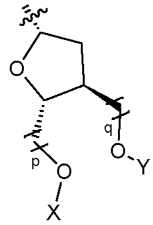 , 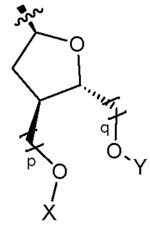 , 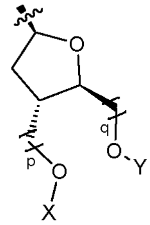 , 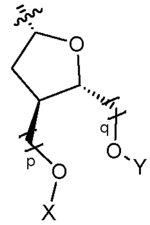

or

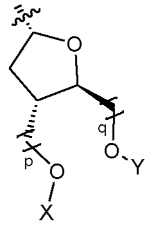 ,

and more preferably

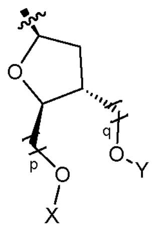 , 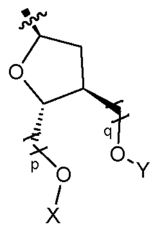 , 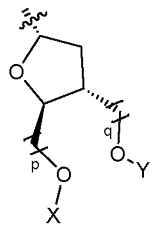

or

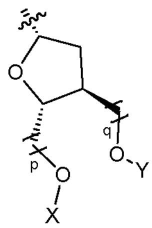 ,

wherein X, Y, p and c are as defined in any of the preceding embodiments.

In some embodiments,

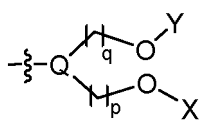

may be

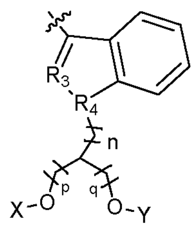

,

wherein $R_3$, $R_4$, X, Y, n, p and q are as defined in any of the preceding embodiments.

In some embodiments,

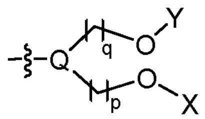

may be

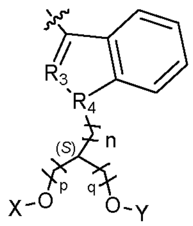

or

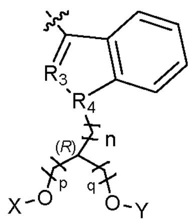

,

wherein $R_3$, $R_4$, X, Y, n, p and q are as defined in any of the preceding embodiments.

In some embodiments,

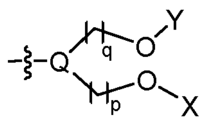

may be

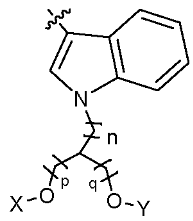

,

wherein X, Y, n, p and q are as defined in any of the preceding embodiments.

In some embodiments,

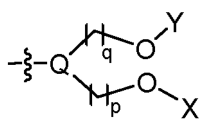

may be

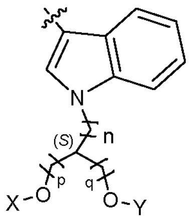 or 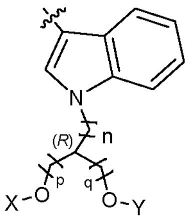 ,

wherein X, Y, n, p and q are as defined in any of the preceding embodiments.

In some embodiments, the compound may be a compound represented by formula (II'-1), (11'-2) or (II'-3),

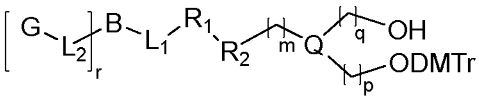

(II'-1)

,

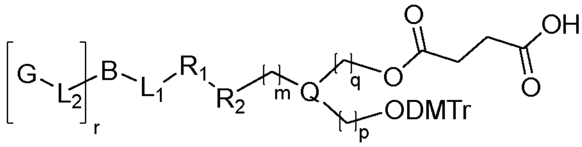

(II'-2)

or

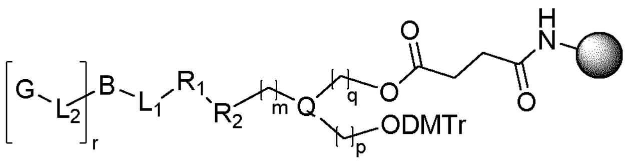

(II'-3)

;

wherein

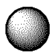

is resin, preferably macroporous resin, and more preferably macroporous aminomethyl resin; $L_1$, $R_1$, $R_2$, Q, B, $L_2$, G, m, p, q and r are as defined in any of the embodiments of the compound represented by formula (II').

The present disclosure provides a compound represented by formula (II),

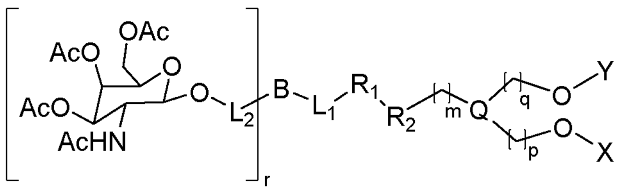

(II)

wherein X is a hydroxy protecting group; Y is hydrogen, deuterium,

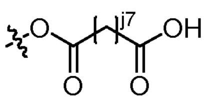

or

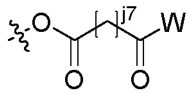
;

j7 is 1, 2, 3 or 4;

W is a macromolecular compound;

$L_1$, $R_1$, $R_2$, Q, B, $L_2$, m, p, q and r are as defined in any of the embodiments of the ligand having a structure represented by formula (I).

In some embodiments, the hydroxy protecting group may be an ester group protecting group, an alkoxymethyl protecting group, an alkyl protecting group, a silyl protecting group or an aryl protecting group, preferably an aryl protecting group, more preferably MMTr and DMTr, and further preferably DMTr.

In some embodiments, j7 may be 2.

In some embodiments, the macromolecular compound may be resin, preferably macroporous resin, and more preferably macroporous aminomethyl resin.

In some embodiments,

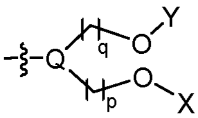

may be

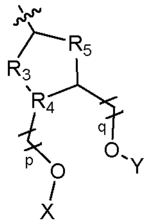
,

wherein $R_3$, $R_4$, $R_5$, X, Y, p and q are as defined in any of the preceding embodiments.

In some embodiments,

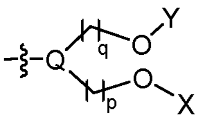

may be

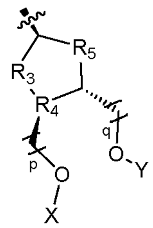
,
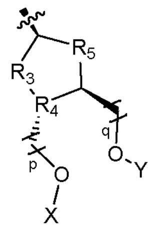
,

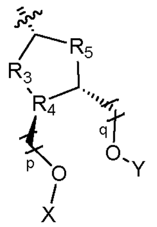

or

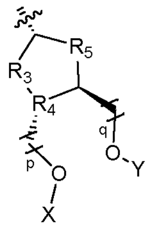

,

wherein $R_3$, $R_4$, $R_5$, X, Y, p and q are as defined in any of the preceding embodiments.

In some embodiments,

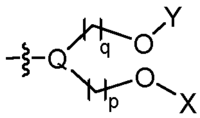

may be

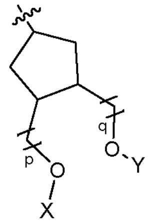

,

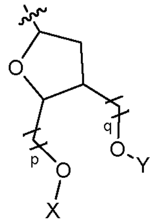

or

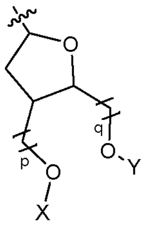

,

preferably

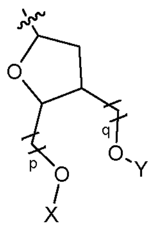

or

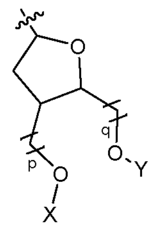

,

and more preferably

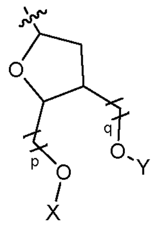,

wherein X, Y, p and q are as defined in any of the preceding embodiments.

In some embodiments,

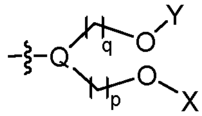

may be

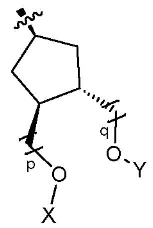, 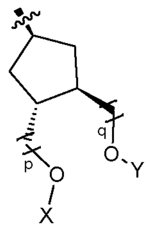,

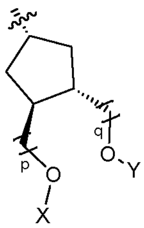, 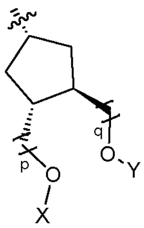, 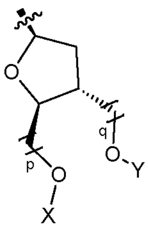, 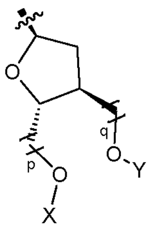, 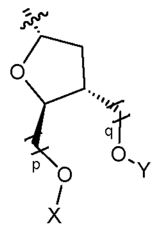, 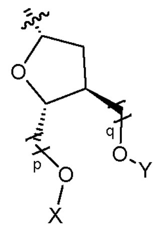,

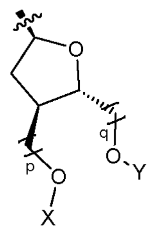, 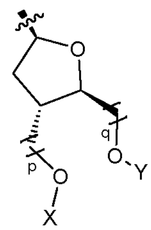, 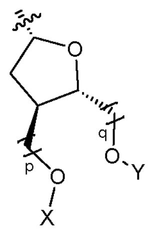 or 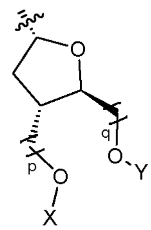,

preferably

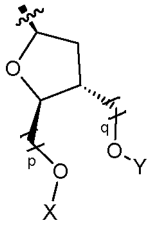,

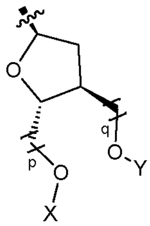, 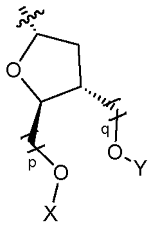, 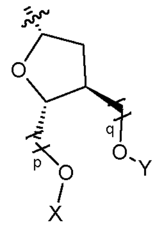, 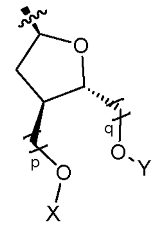, 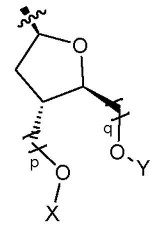, 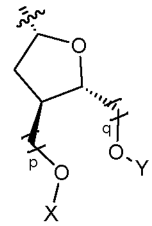

or

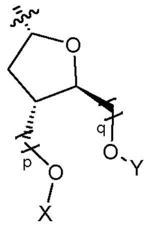,

and more preferably

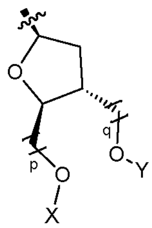, 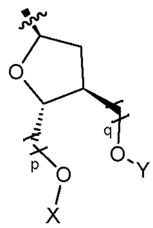, 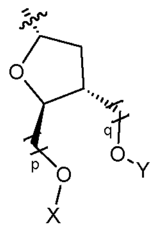

or

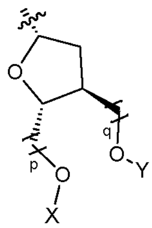,

wherein X, Y, p and q are as defined in any of the preceding embodiments.

In some embodiments,

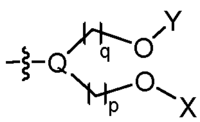

may be

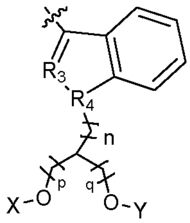

,

wherein $R_3$, $R_4$, X, Y, n, p and q are as defined in any of the preceding embodiments.

In some embodiments,

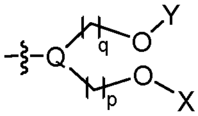

may be

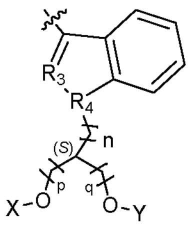

or

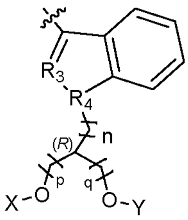

,

wherein $R_3$, $R_4$, X, Y, n, p and q are as defined in any of the preceding embodiments.

In some embodiments,

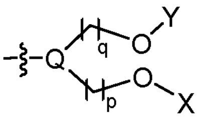

may be

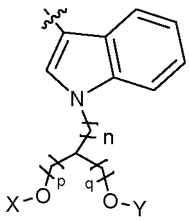

,

wherein X, Y, n, p and q are as defined in any of the preceding embodiments.

In some embodiments,

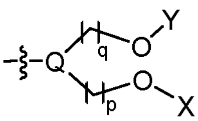

may be

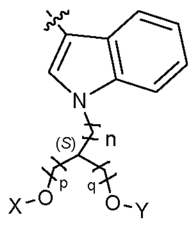 or 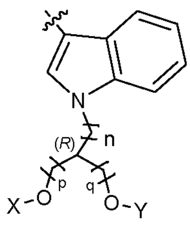 ,

wherein X, Y, n, p and q are as defined in any of the preceding embodiments.

In some embodiments, the compound may be a compound represented by formula (II-1), (II-2) or (II-3),

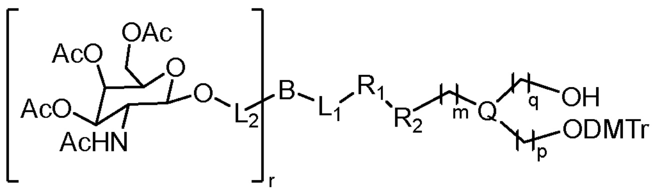

(II-1)

,

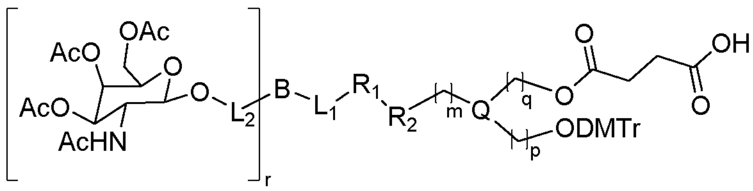

(II-2)

or

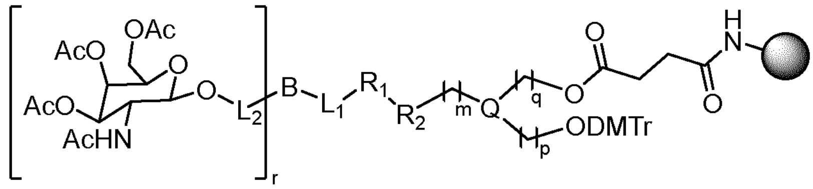

(II-3)

;

wherein

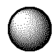

is resin, preferably macroporous resin, and more preferably macroporous aminomethyl resin; $L_1$, $R_1$, $R_2$, Q, B, $L_2$, m, p, q and r are as defined in any of the embodiments of the compound represented by formula (II).

In some embodiments, the compound may be any of the following structures:

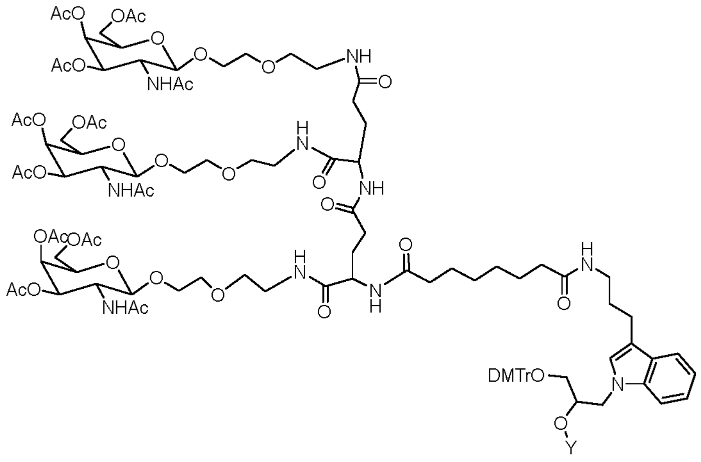

,

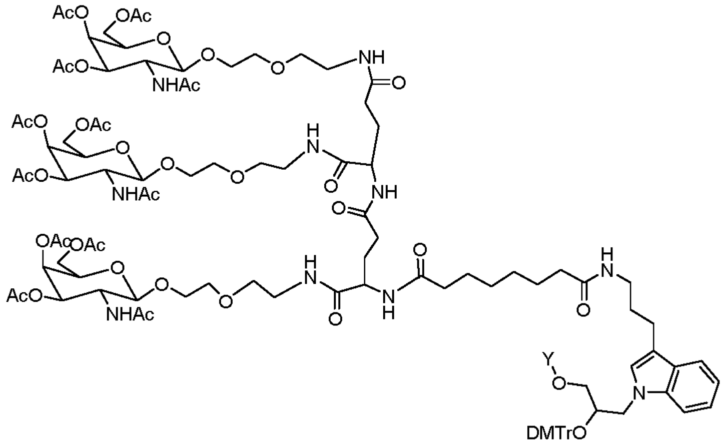

,

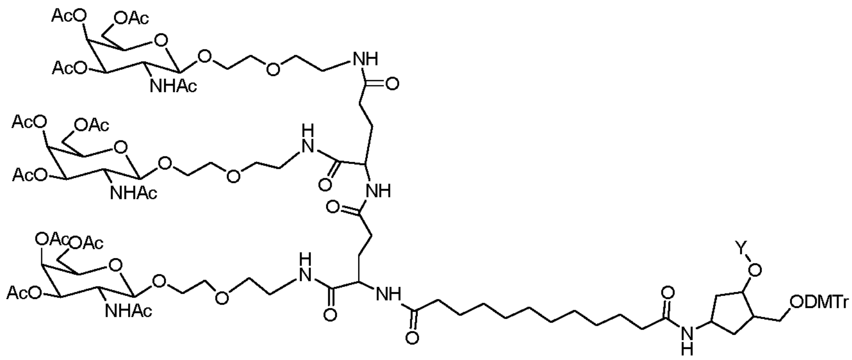

,

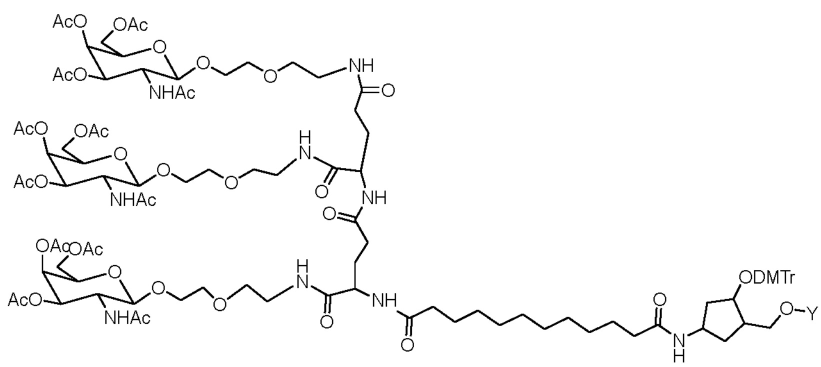

,

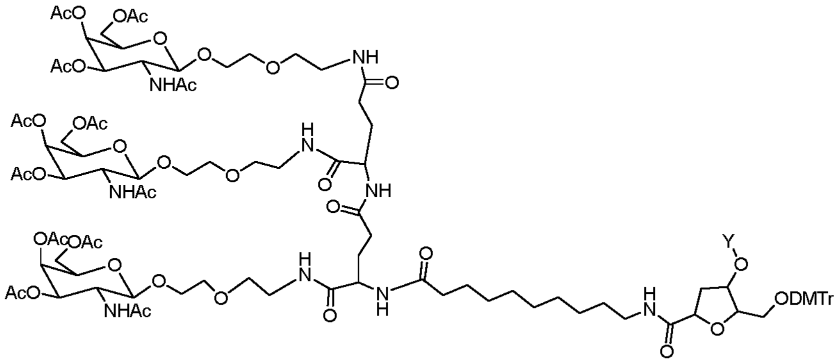

,

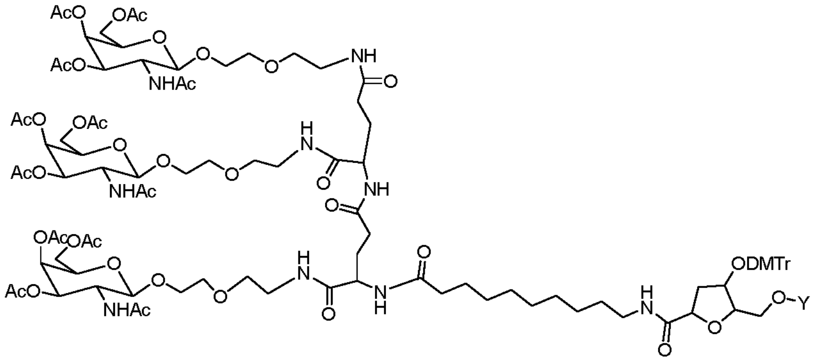

,

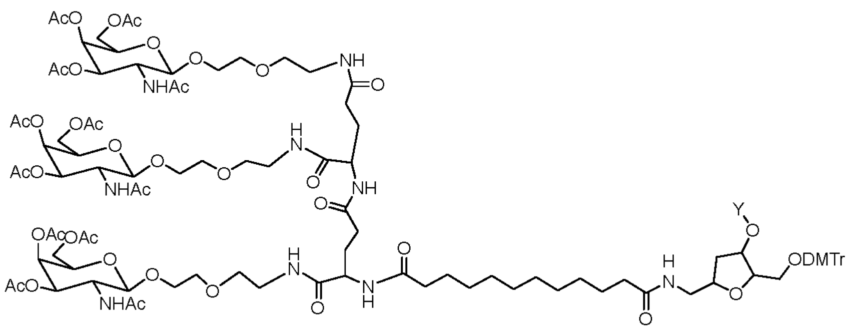

,

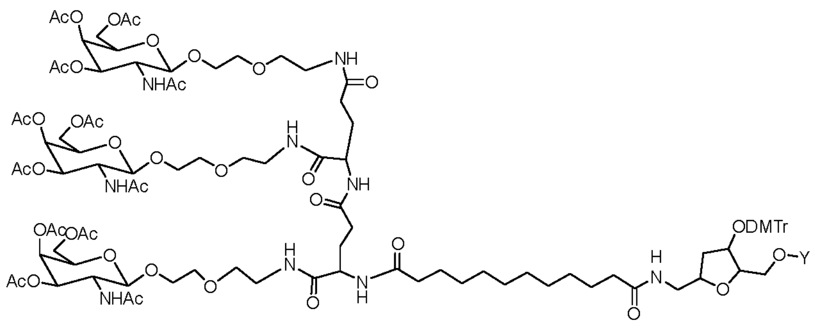

,

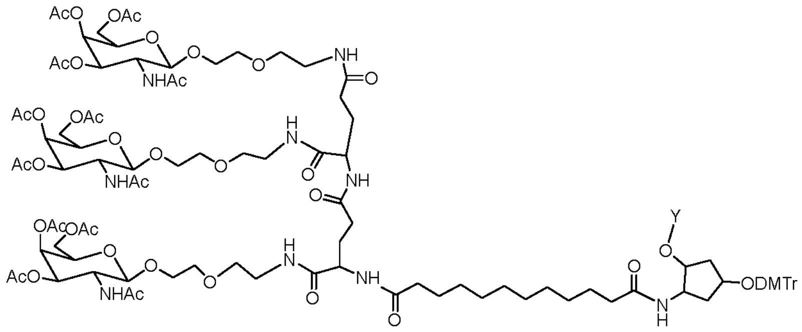

,

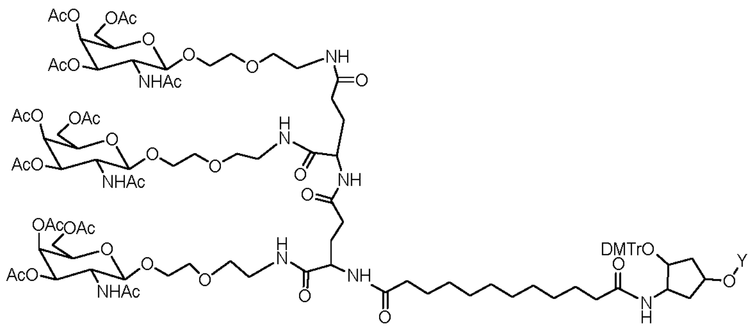

,

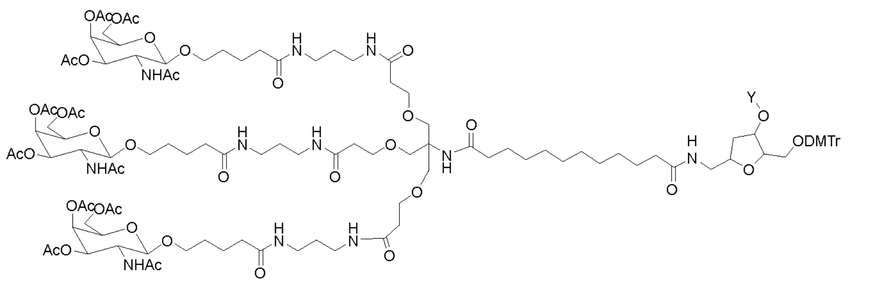

,

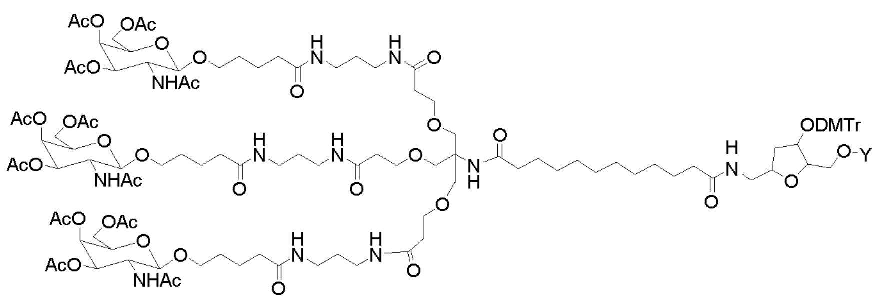

,

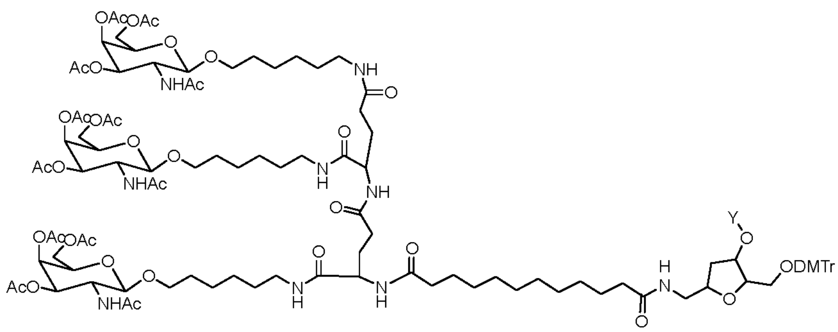

,

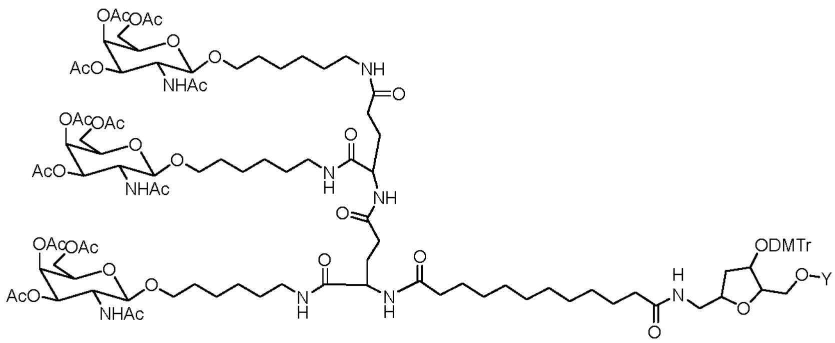

,

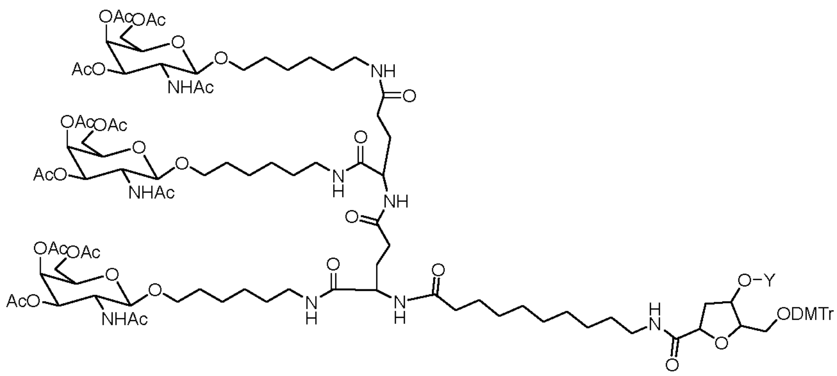

,

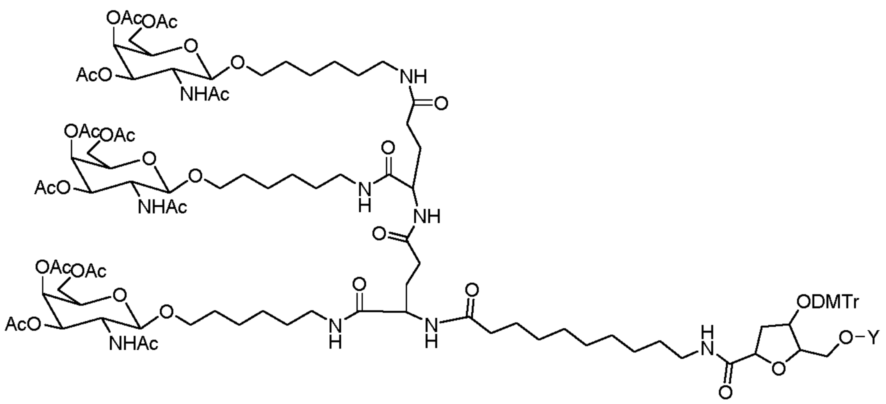

,

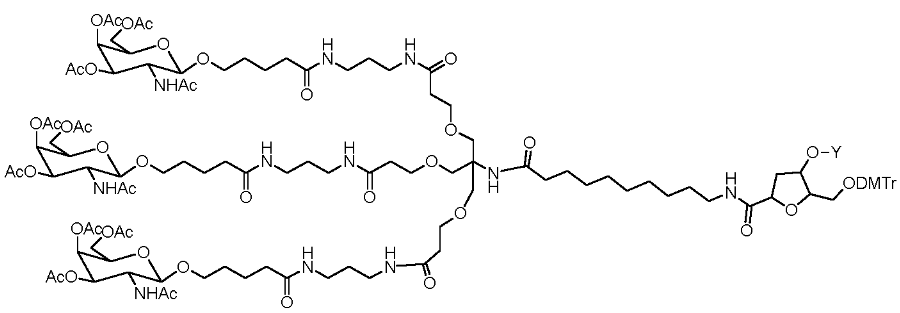

or

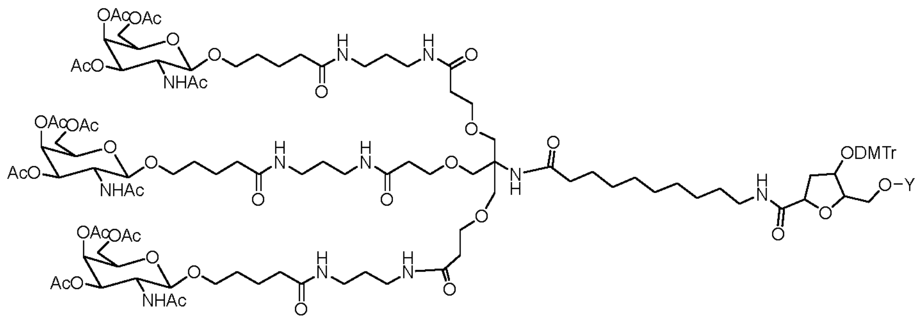

;

wherein Y is hydrogen or

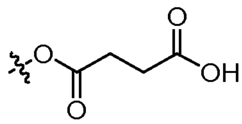

;

or, Y is

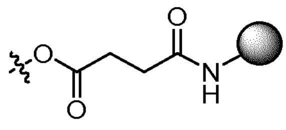

,

wherein

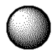

is resin, preferably macroporous resin, and more preferably macroporous aminomethyl resin.

In some embodiments, the compound may be any of the following structures:

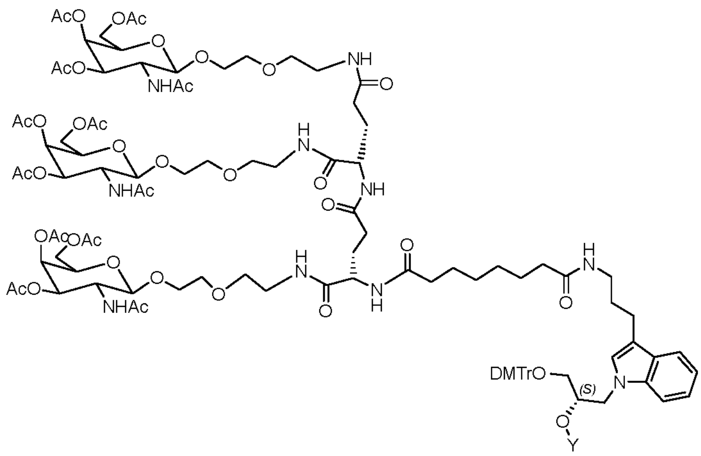

,

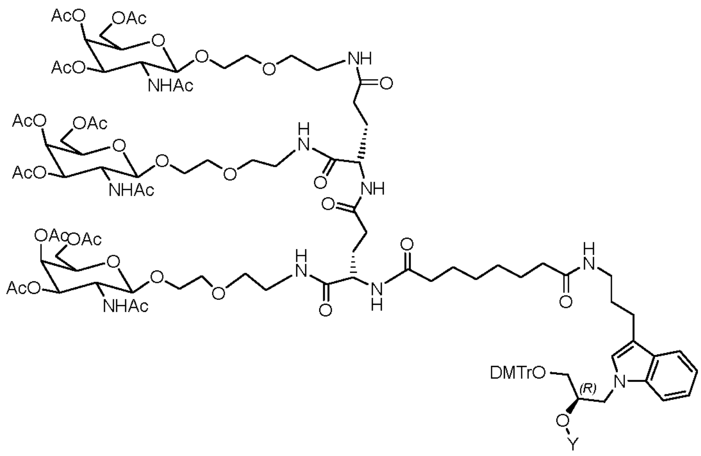

,

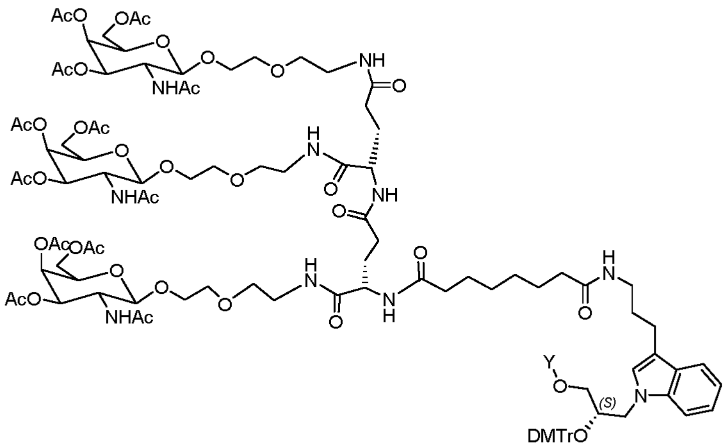

,

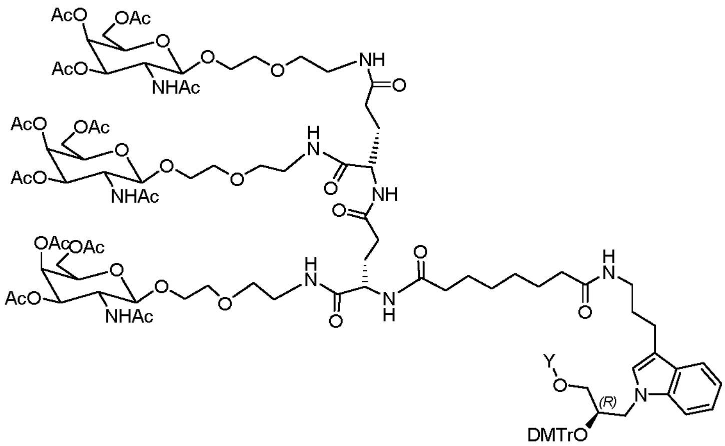

,

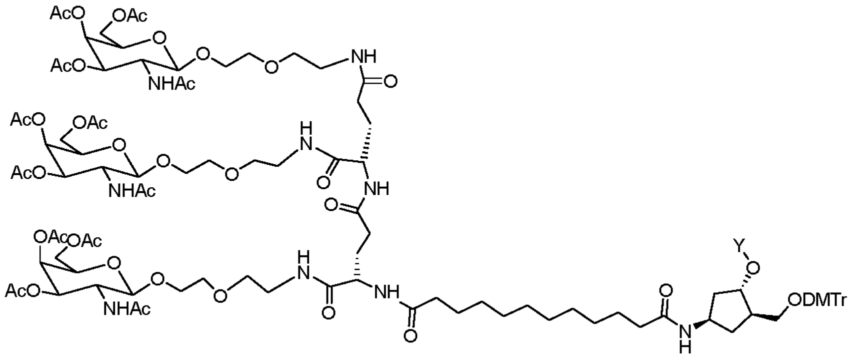

,

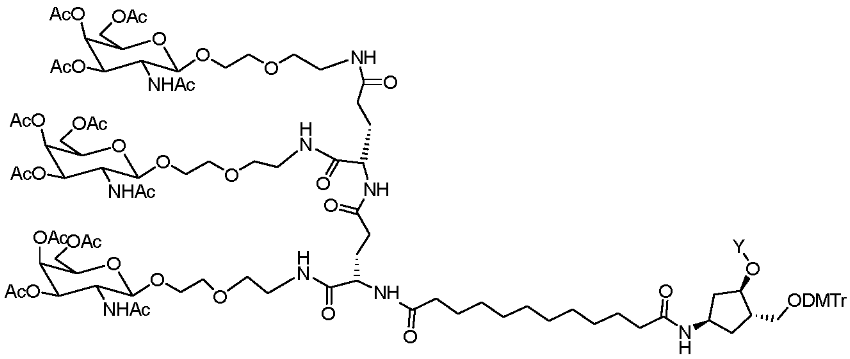

,

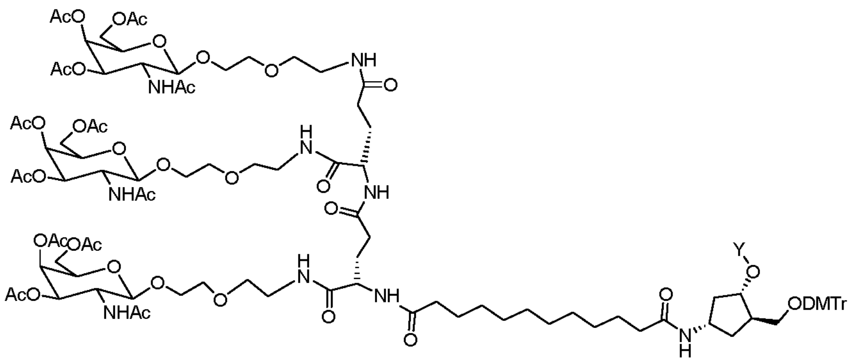

,

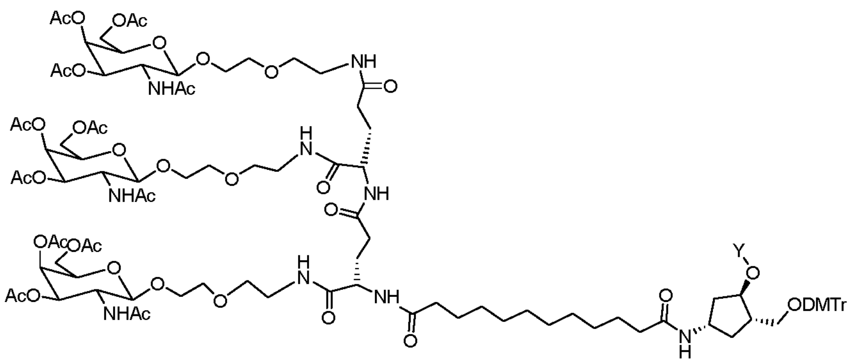

,

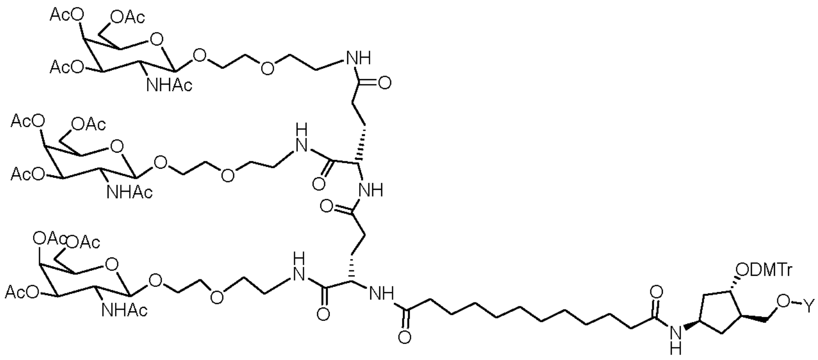

,

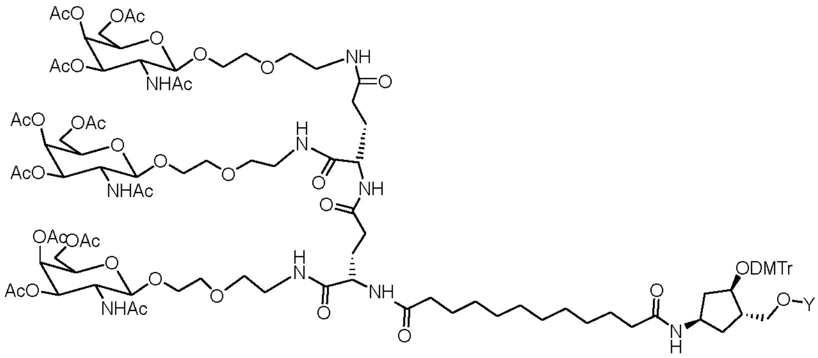

,

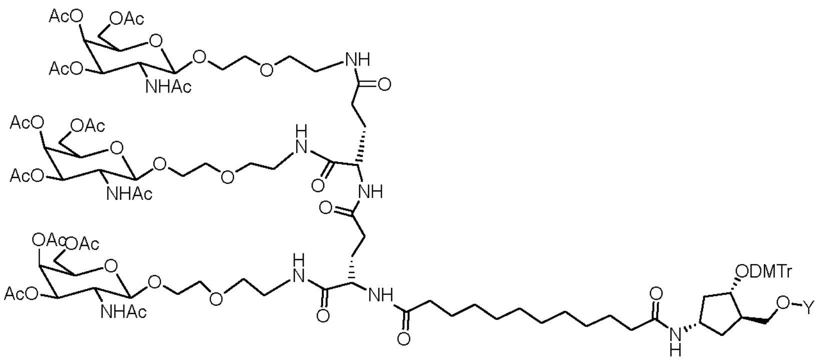

,

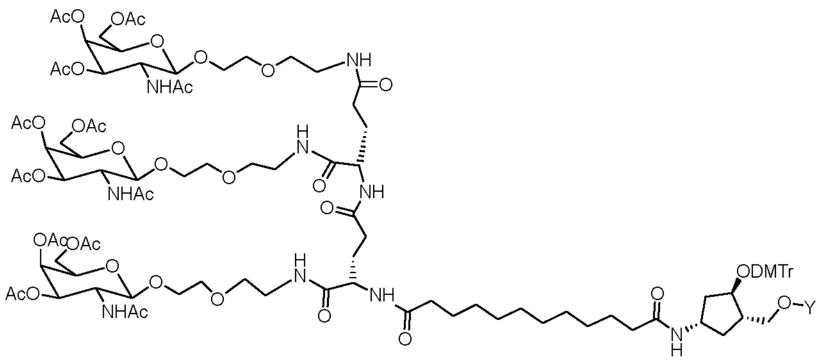

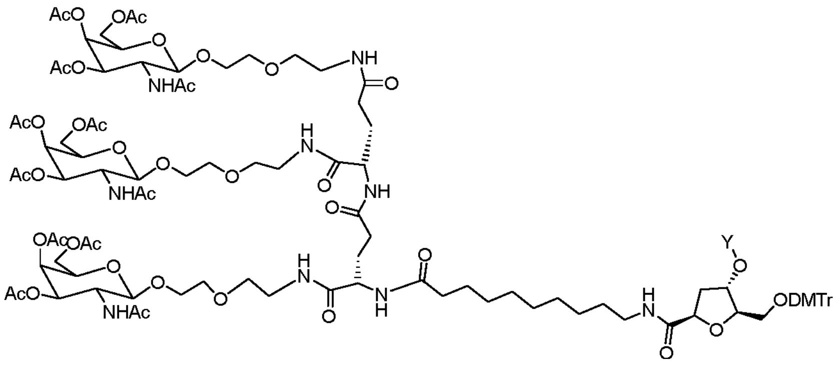

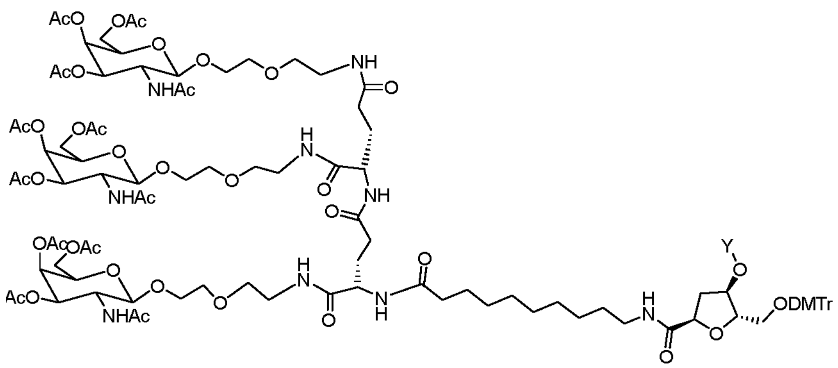

,

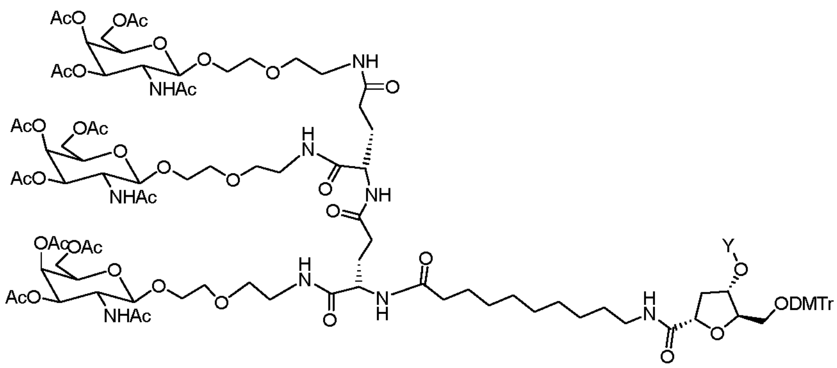

,

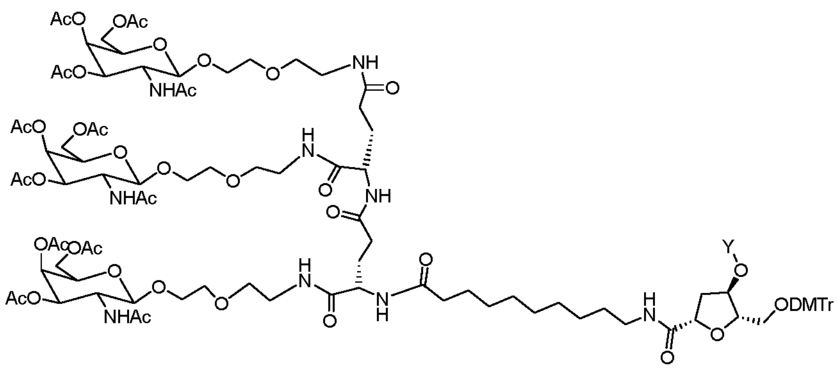

,

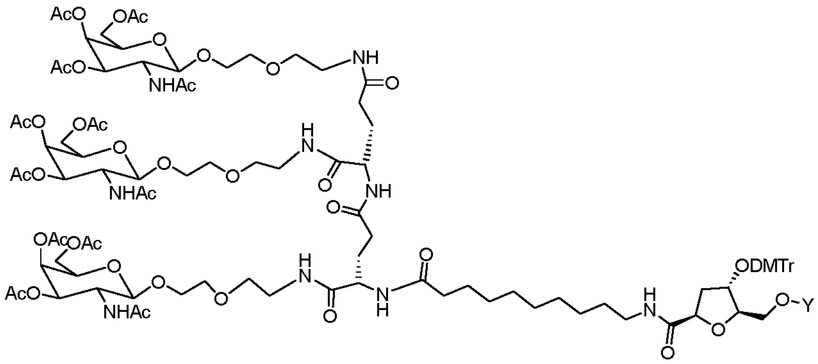

,

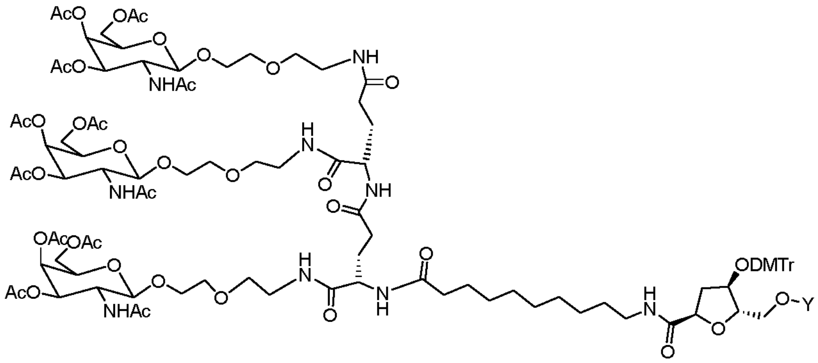

,

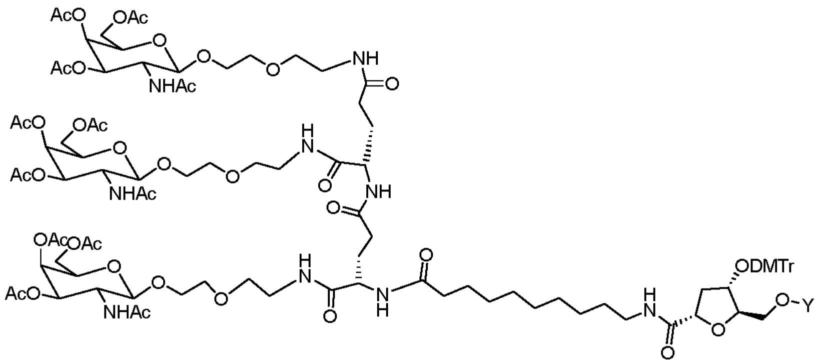

,

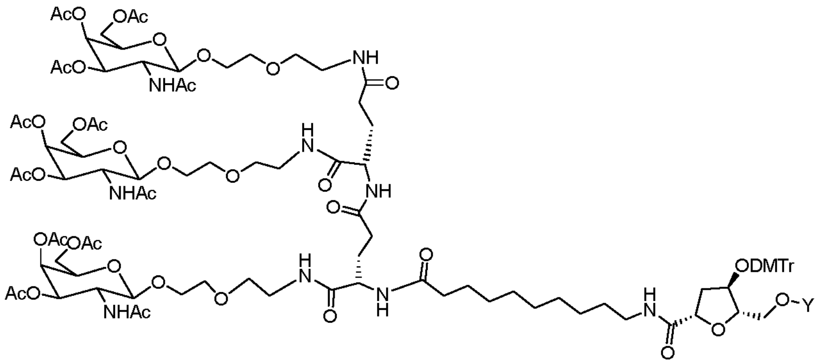

,

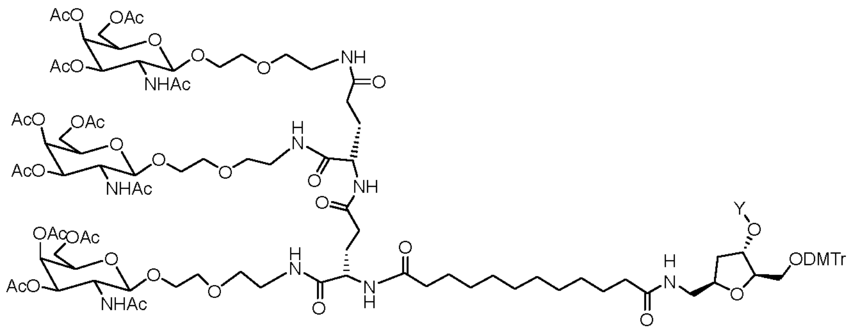

,

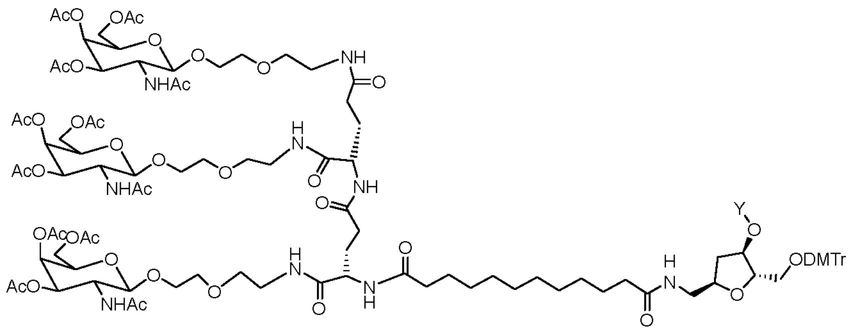

,

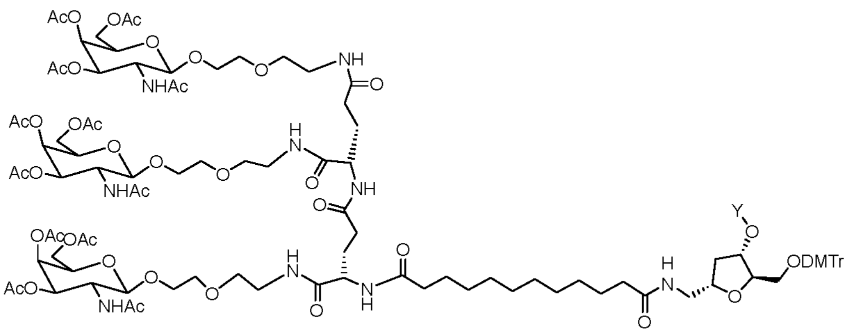

,

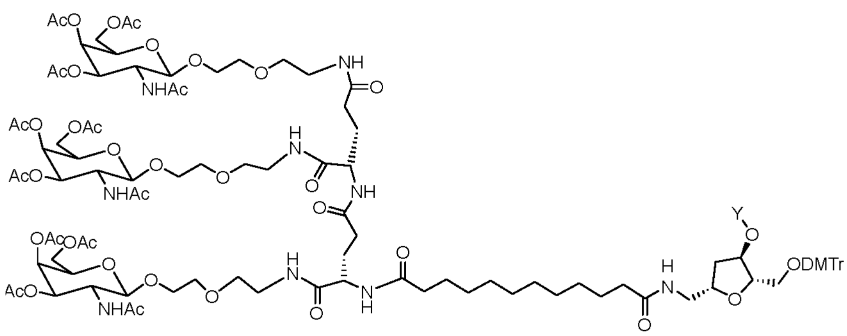

,

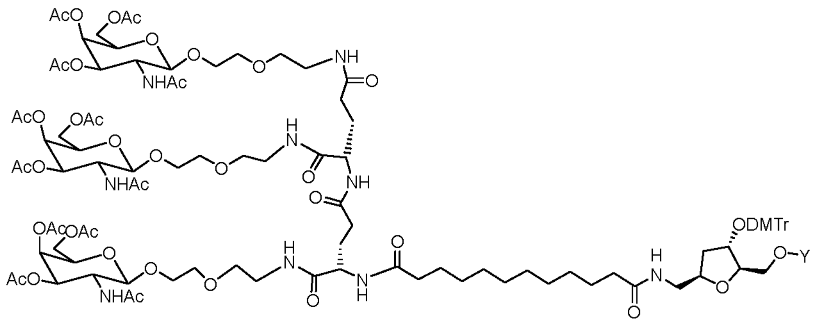

,

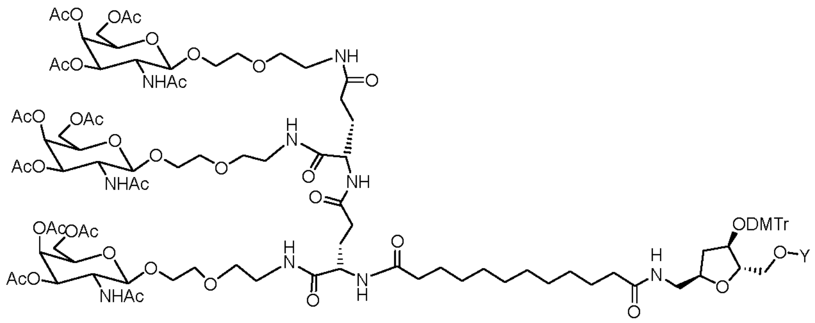

,

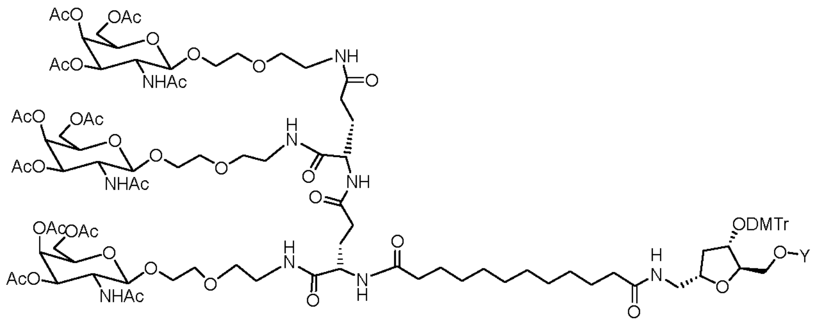

,

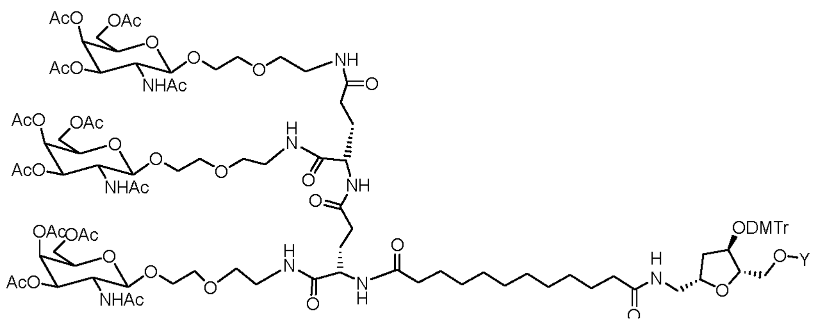

,

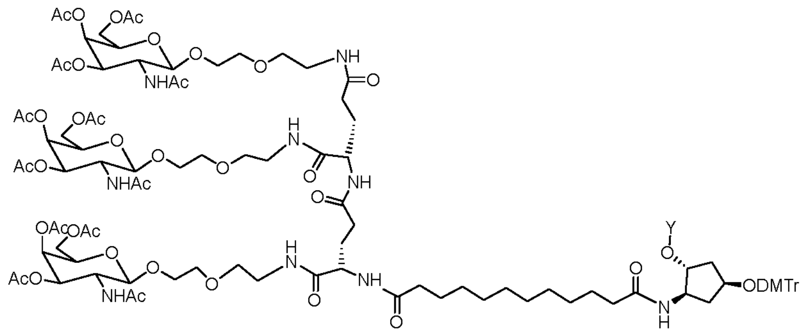

,

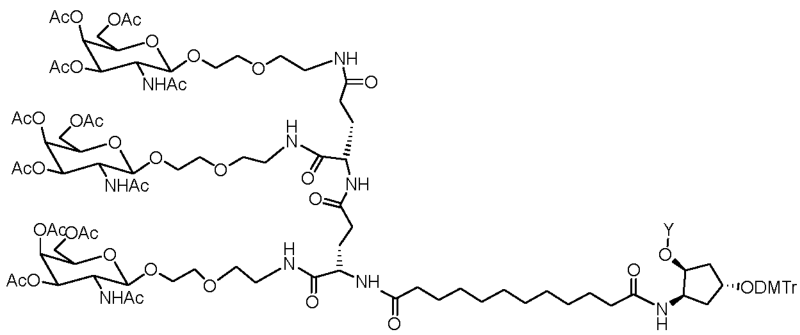

,

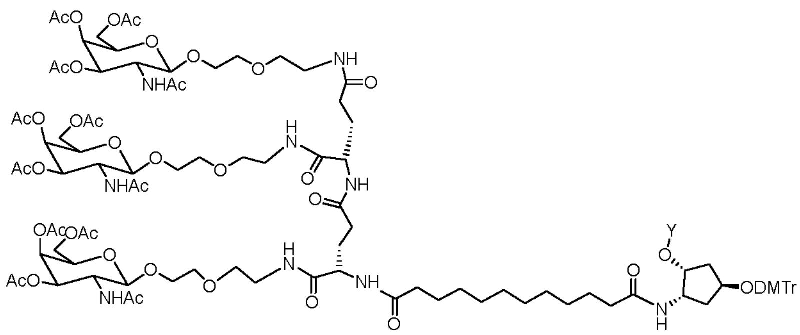

,

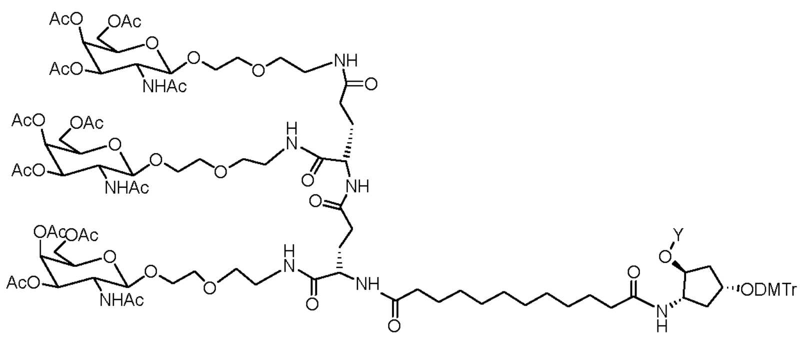

,

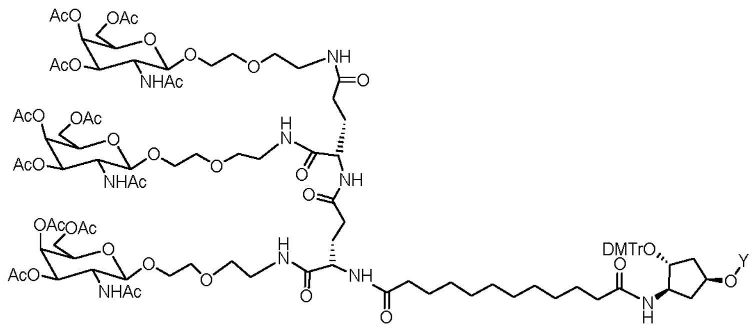

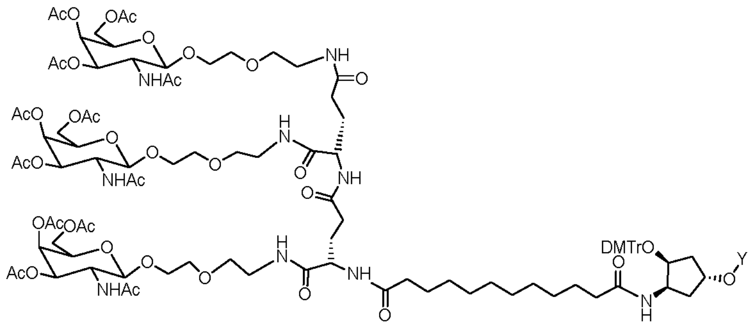

,

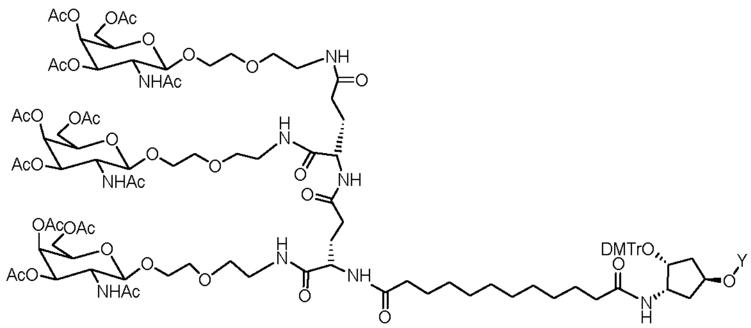

,

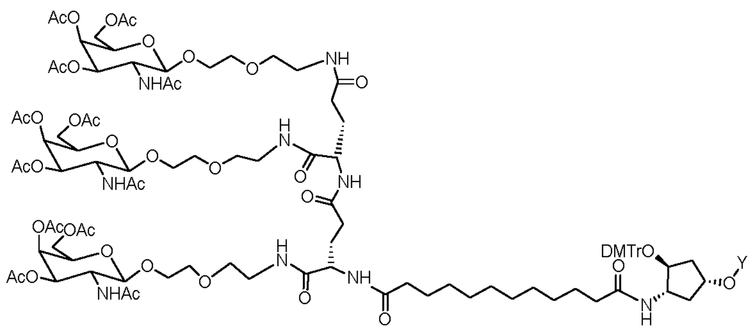

,

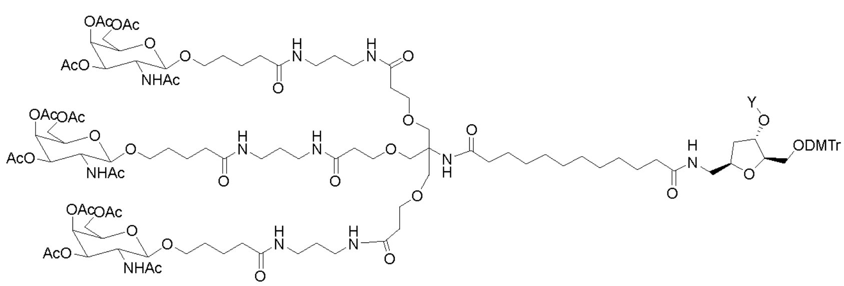

,

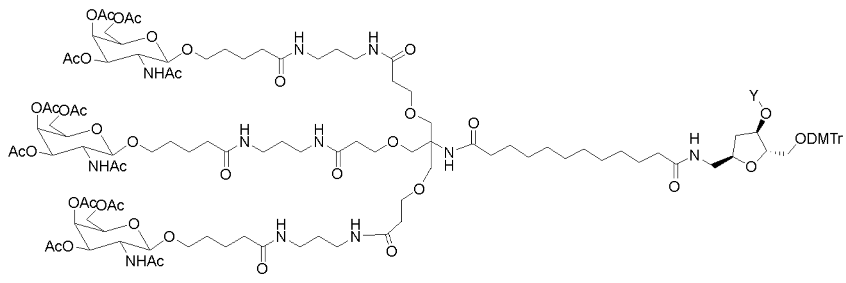

,

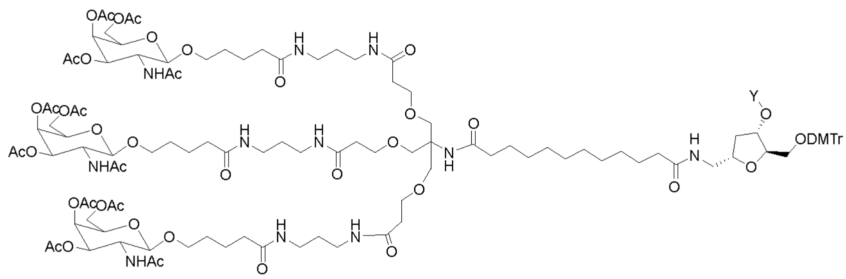

,

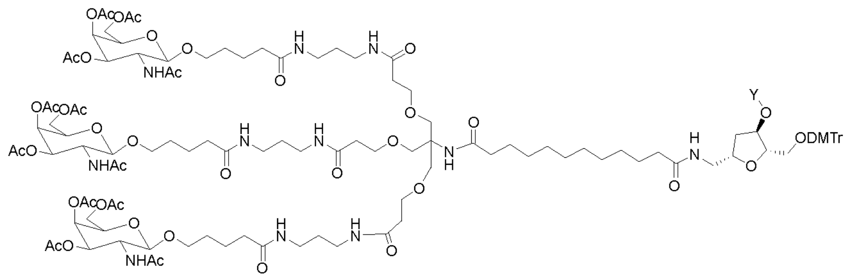

,

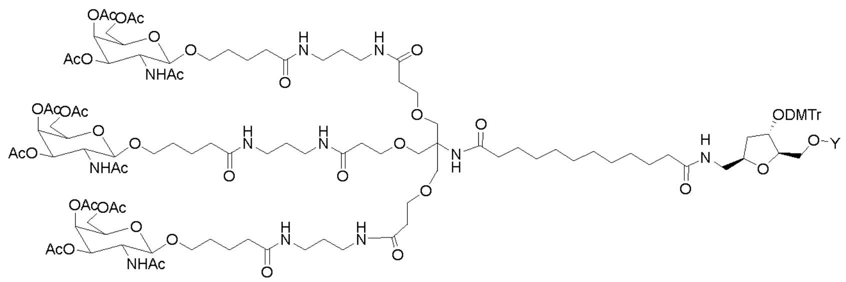

,

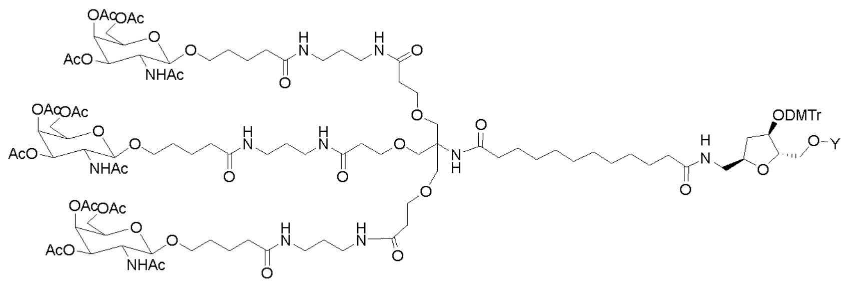

,

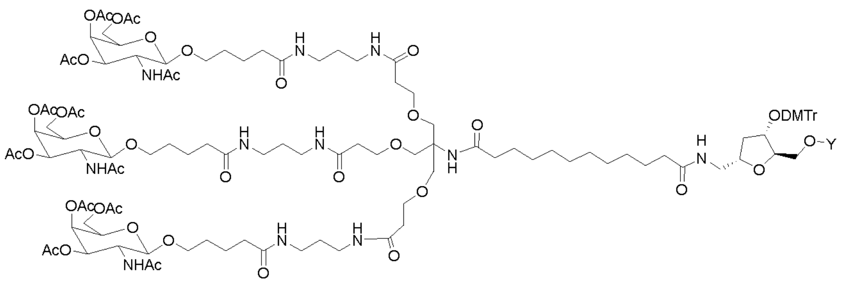

,

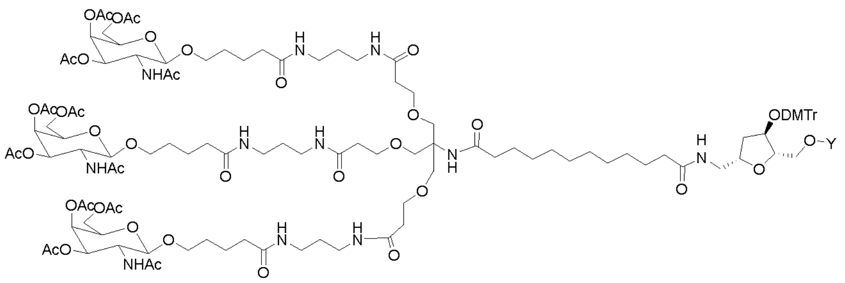

,

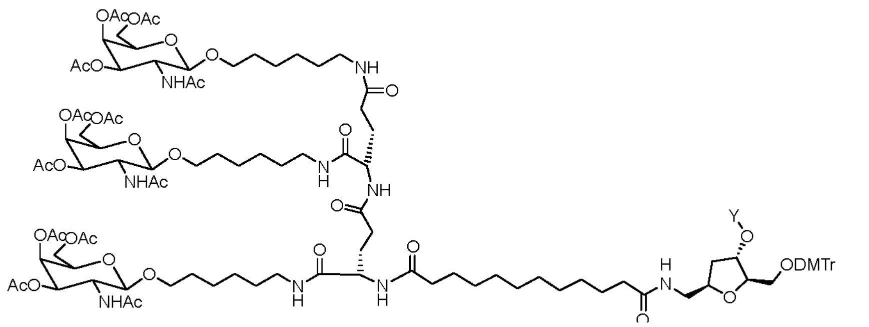

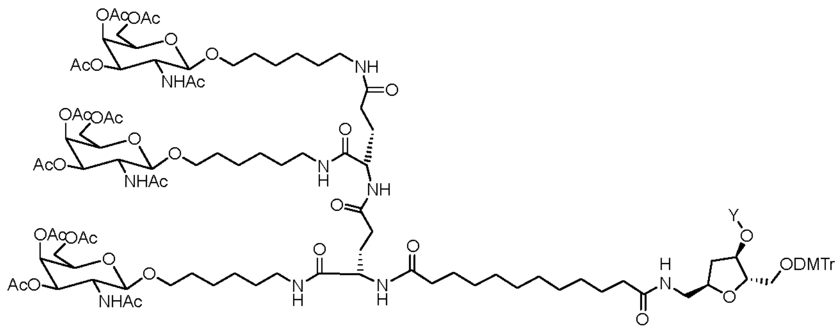

,

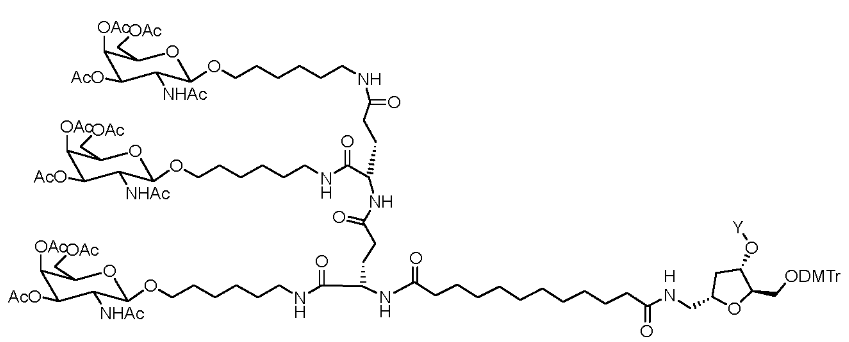

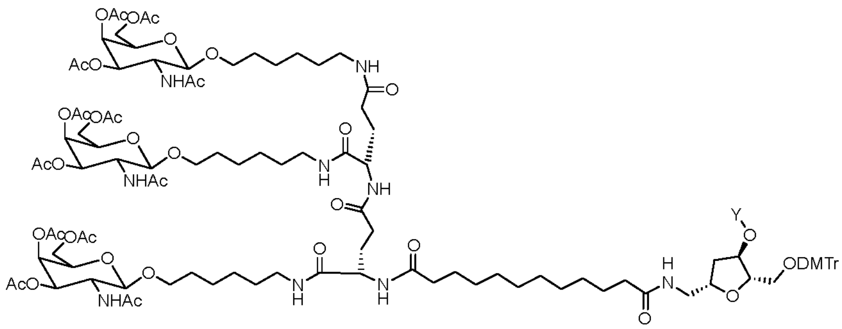

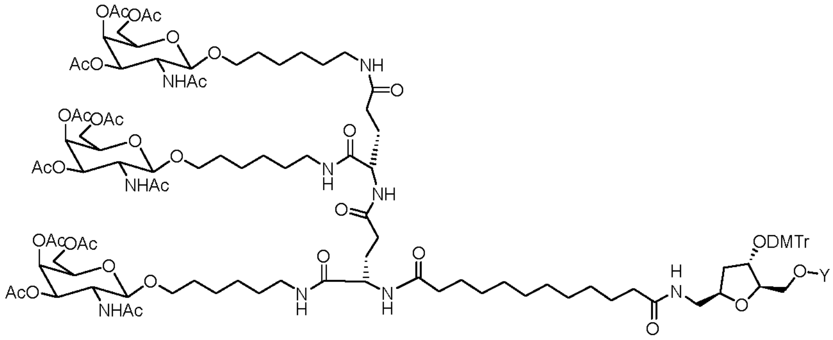

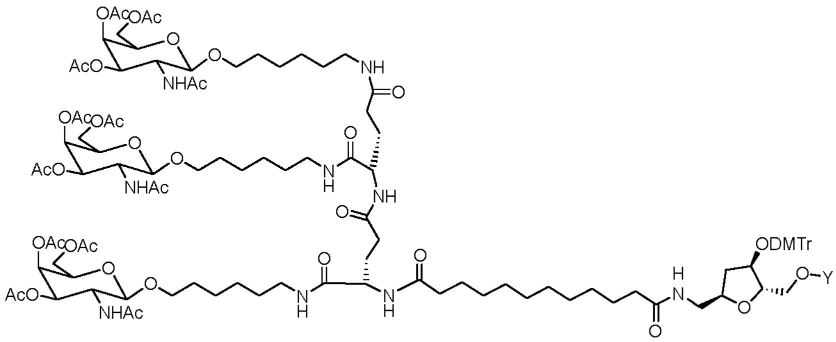

,

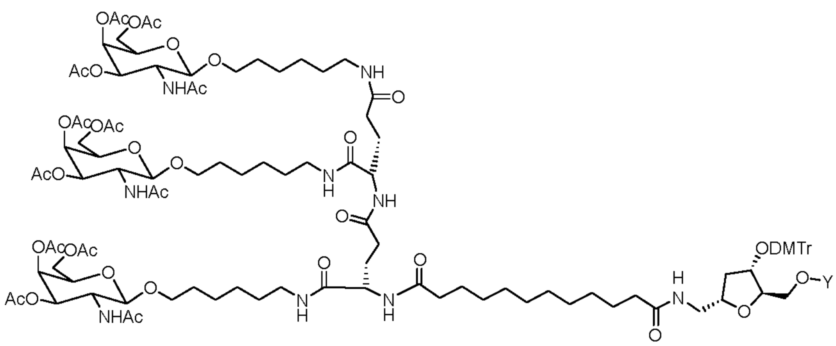

,

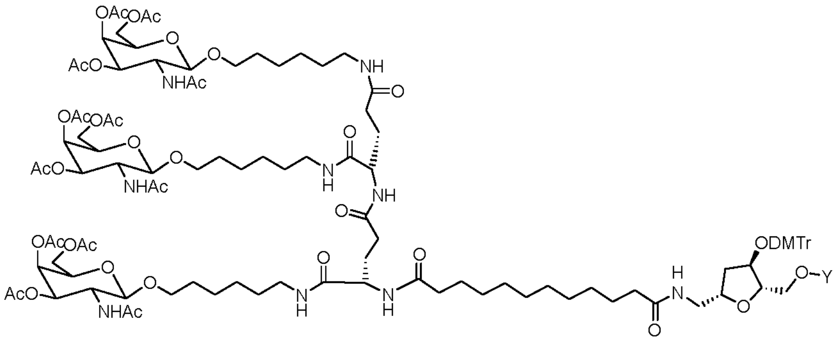

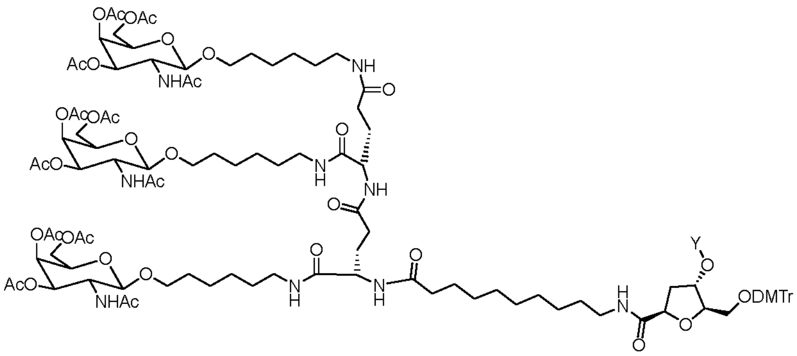

,

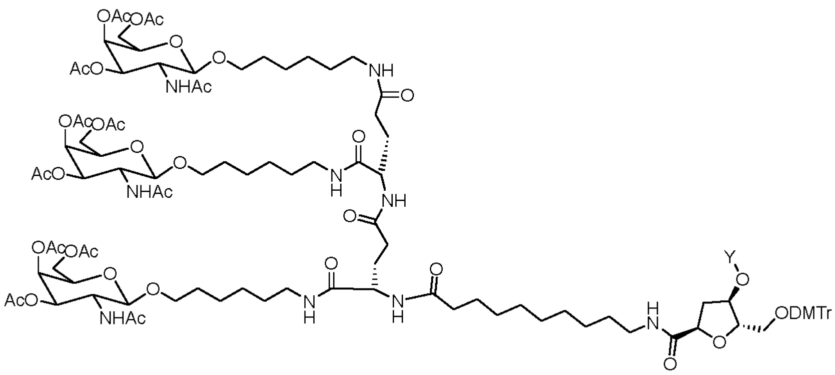

,

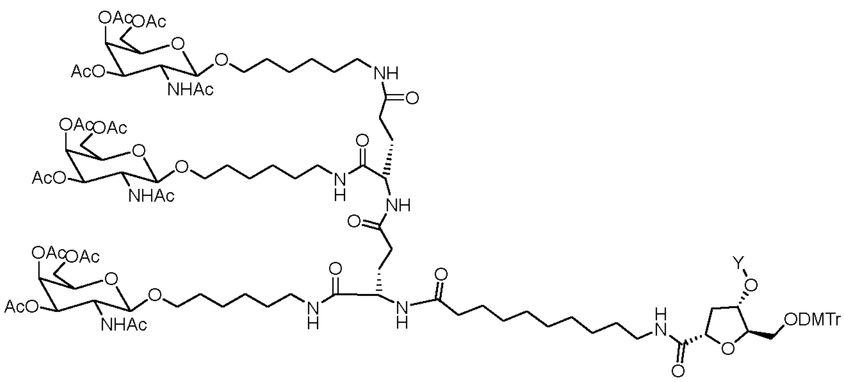

,

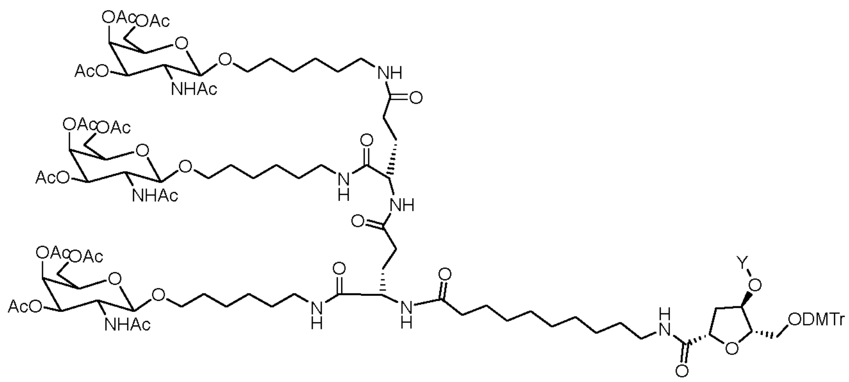

,

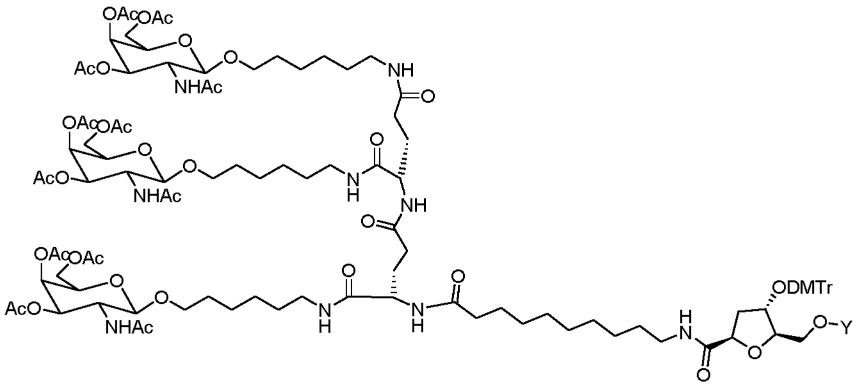

,

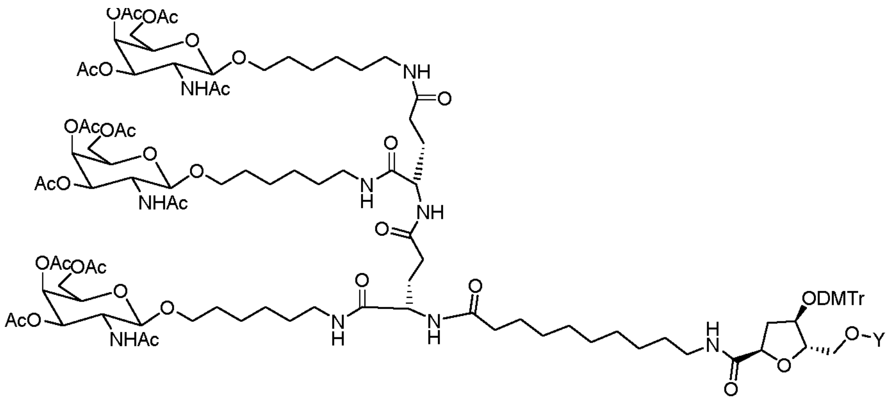

,

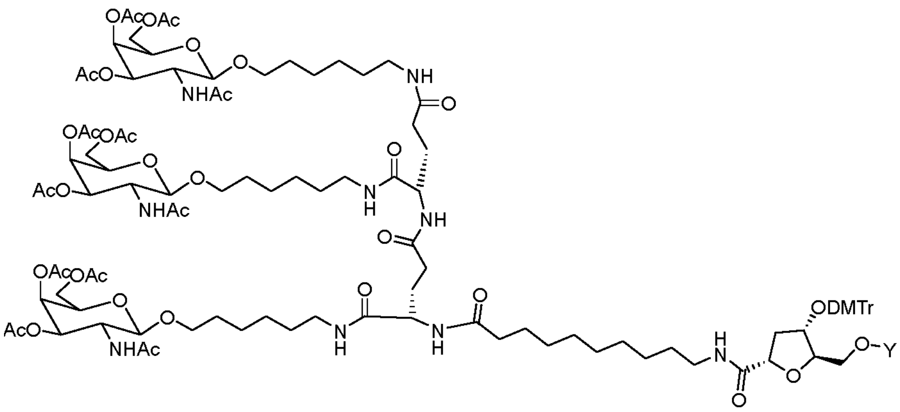

,

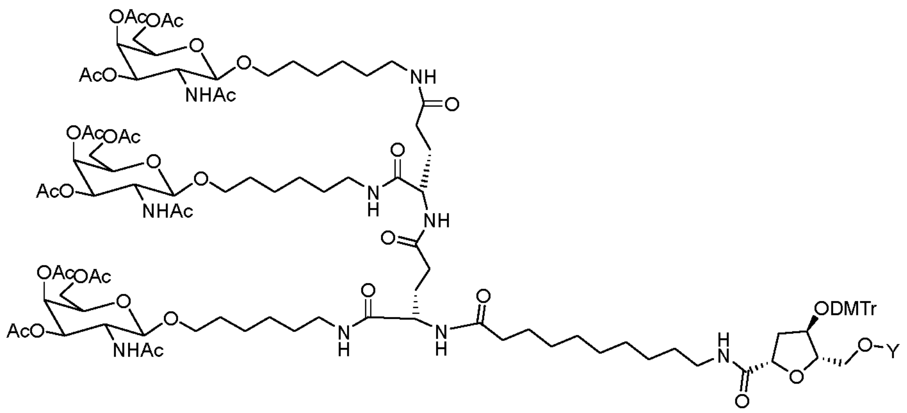

,

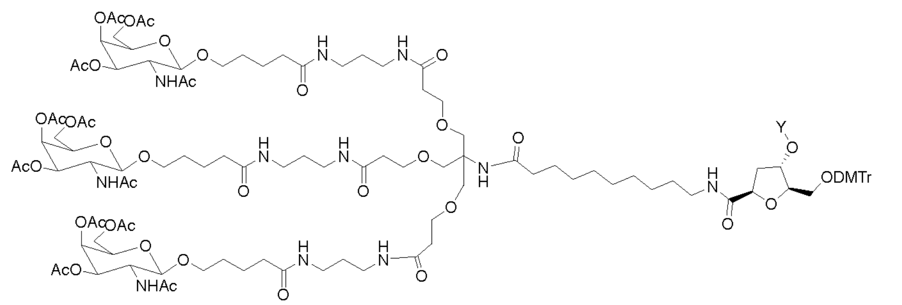

,

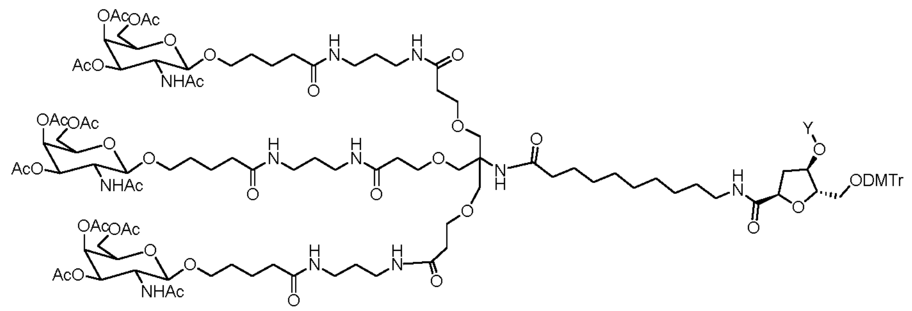

,

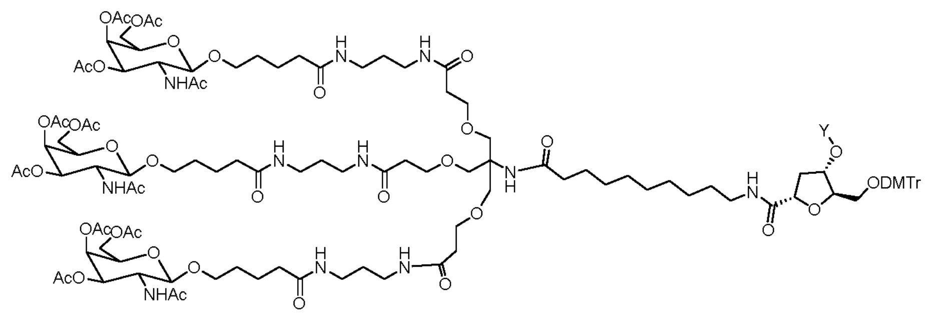

,

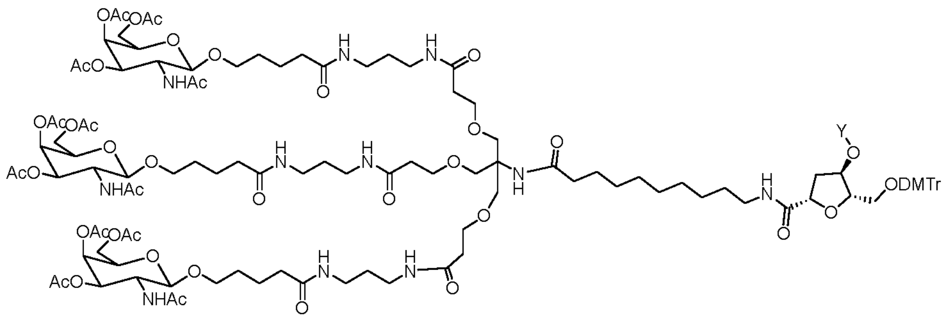

,

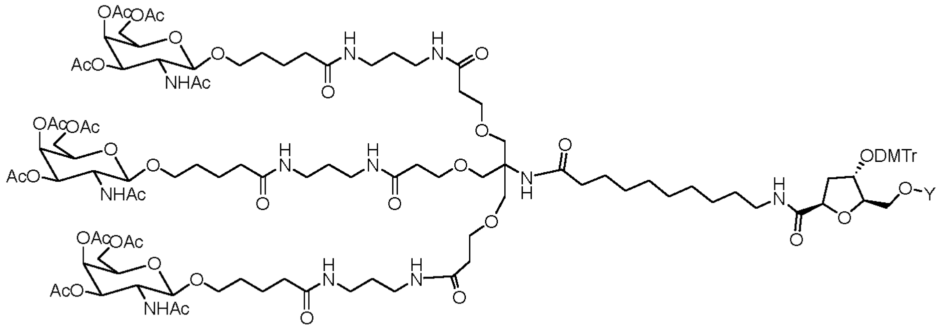

,

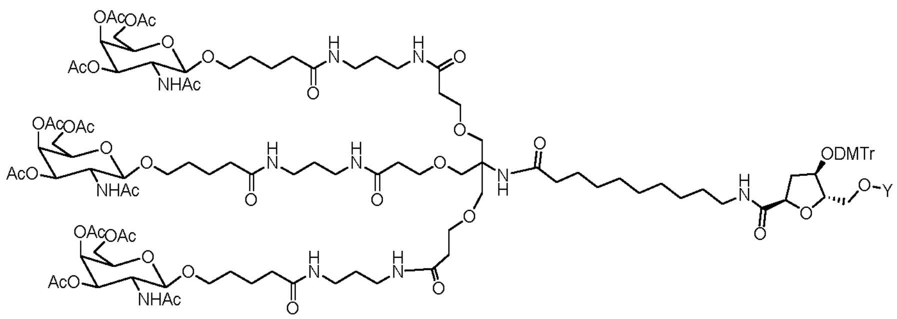

,

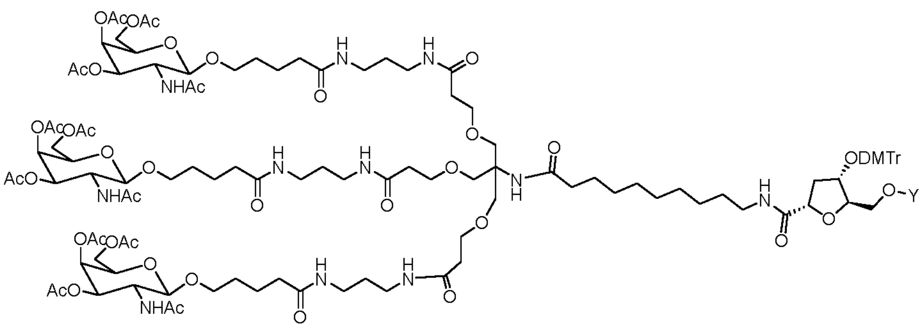

or

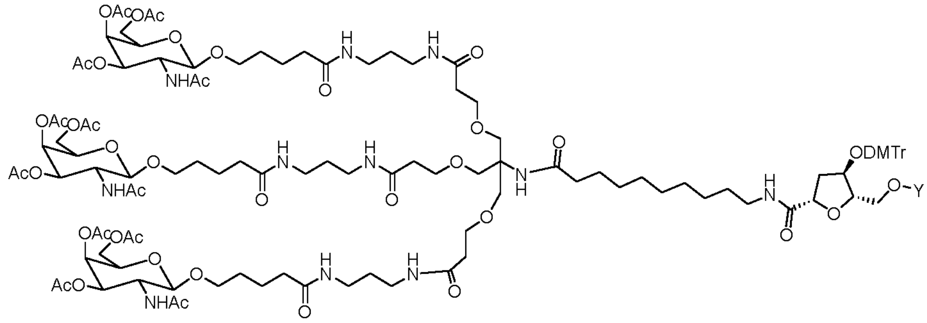

;

wherein Y is hydrogen or

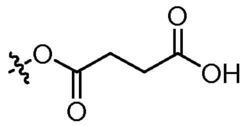

;

or, Y is

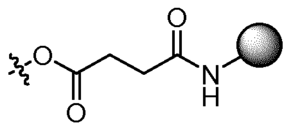

, wherein

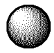

is resin, preferably macroporous resin, and more preferably macroporous aminomethyl resin.

In some embodiments, the N-acetyl-galactosamine triacetate moiety in the compound described above may be replaced with N-trifluoroacetylgalactosamine triacetate, N-propionylgalactosamine triacetate, N-n-butyrylgalactosamine triacetate or N-isobutyrylgalactosamine triacetate.

The present disclosure provides a nucleic acid-ligand conjugate comprising a nucleic acid and one or more ligands described above, wherein the ligands are conjugated to an end of the nucleic acid, and the ligands are identical or different.

In some embodiments, the nucleic acid and the ligands may be linked by phosphoester groups, phosphorothioate groups or phosphonic acid groups.

In some embodiments, the nucleic acid may include, but is not limited to: oligonucleotides, single-stranded oligonucleotides, single-stranded antisense oligonucleotides, short interfering RNAs (siRNAs), double-stranded RNAs (dsRNAs), microRNAs (miRNAs), short hairpin RNAs (shRNAs), ribozymes, interfering RNA molecules, and Dicer enzyme substrates.

In some embodiments, the 3' end of the nucleic acid may be conjugated to a ligand.

In some embodiments, the nucleic acid may be a single-stranded nucleic acid.

In some embodiments, the single-stranded nucleic acid may be the sense strand of an siRNA.

In some embodiments, the single-stranded nucleic acid may be the antisense strand of an siRNA.

In some embodiments, the nucleic acid may be a double-stranded nucleic acid. The double-stranded nucleic acid may comprise at least one duplex region within which a first strand nucleic acid is at least partially complementary to a second strand nucleic acid, wherein:

(1) neither end of the first strand nucleic acid is conjugated to a ligand; and the 5' end of the second strand nucleic acid is conjugated to a ligand, and the 3' end is not conjugated to a ligand; or the 3' end of the second strand nucleic acid is conjugated to a ligand, and the 5' end is not conjugated to a ligand; or both the 3' end and the 5' end of the second strand nucleic acid are conjugated to ligands;

(2) neither end of the second strand nucleic acid is conjugated to a ligand; and the 5' end of the first strand nucleic

acid is conjugated to a ligand, and the 3' end is not conjugated to a ligand; or the 3' end of the first strand nucleic acid is conjugated to a ligand, and the 5' end is not conjugated to a ligand; or both the 3' end and the 5' end of the first strand nucleic acid are conjugated to ligands;

(3) both the 5' ends of the first strand nucleic acid and the second strand nucleic acid are conjugated to ligands, and neither 3' end is conjugated to a ligand; or both the 3' ends of the first strand nucleic acid and the second strand nucleic acid are conjugated to ligands, and neither 5' end is conjugated to a ligand; or both the 3' ends and the 5' ends of the first strand nucleic acid and the second strand nucleic acid are conjugated to ligands; or both the 3' end of the first strand nucleic acid and the 5' end of the second strand nucleic acid are conjugated to ligands, and neither the 5' end of the first strand nucleic acid nor the 3' end of the second strand nucleic acid is conjugated to a ligand; or both the 5' end of the first strand nucleic acid and the 3' end of the second strand nucleic acid are conjugated to ligands, and neither the 3' end of the first strand nucleic acid nor the 5' end of the second strand nucleic acid is conjugated to a ligand;

(4) both the 3' end and the 5' end of the first strand nucleic acid are conjugated to ligands, and either the 5' end or the 3' end of the second strand nucleic acid is conjugated to a ligand; or both the 3' end and the 5' end of the second strand nucleic acid are conjugated to ligands, and either the 5' end or the 3' end of the first strand nucleic acid is conjugated to a ligand.

In some embodiments, the double-stranded nucleic acid may be an siRNA.

In some embodiments, the nucleic acid may comprise one or more modified nucleotides.

In some embodiments, the nucleic acid-ligand conjugate may be the following structure or a pharmaceutically acceptable salt thereof:

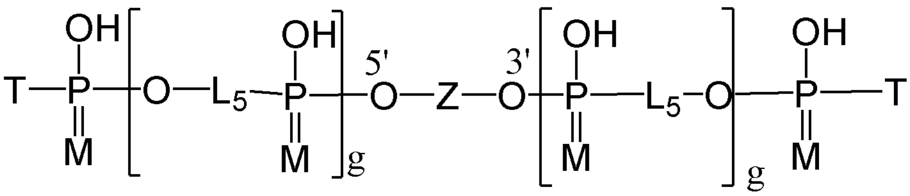

;

wherein T is the ligand described above, and each T is identical or different;

$L_5$ is independently a $C_1$-$C_{30}$ alkyl chain, or a $C_1$-$C_{30}$ alkyl chain interrupted by one or more oxygen, sulfur or nitrogen atoms or C=O;

M is independently O or S;

g is independently an integer of 0-4;

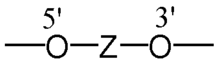

represents the nucleic acid.

In some embodiments, $L_5$ may be

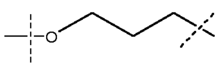

,

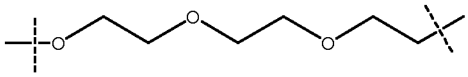

or

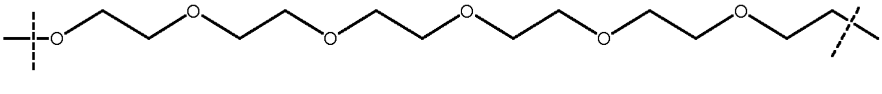

.

In some embodiments, g may be 0 or 1.

In some embodiments, g may be 0.

In some embodiments, M may be S.

In some embodiments, the nucleic acid-ligand conjugate may be any of the following structures or a pharmaceutically acceptable salt thereof:

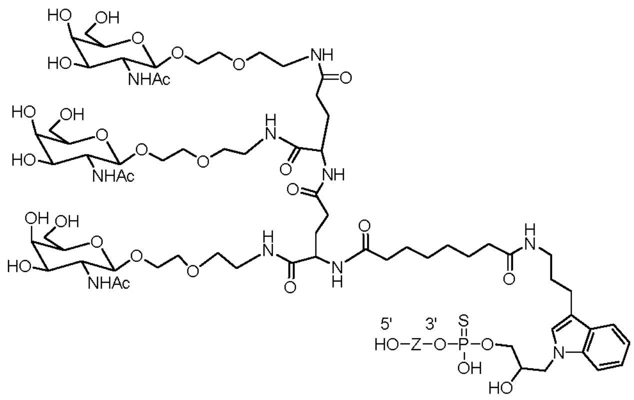

,

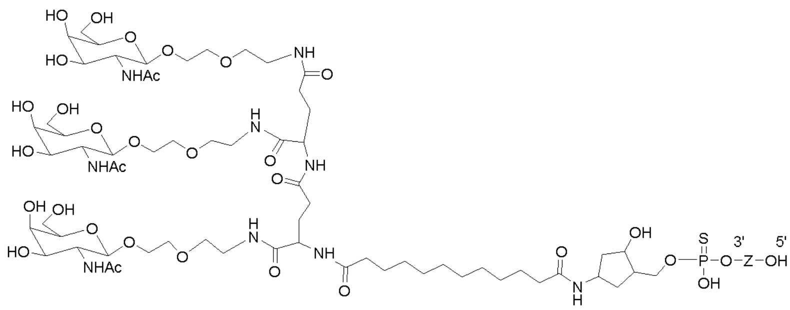

,

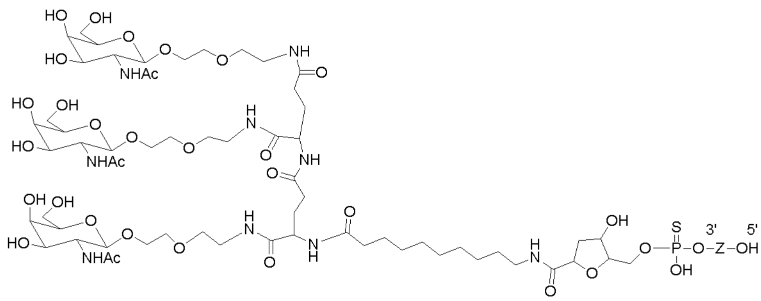

,

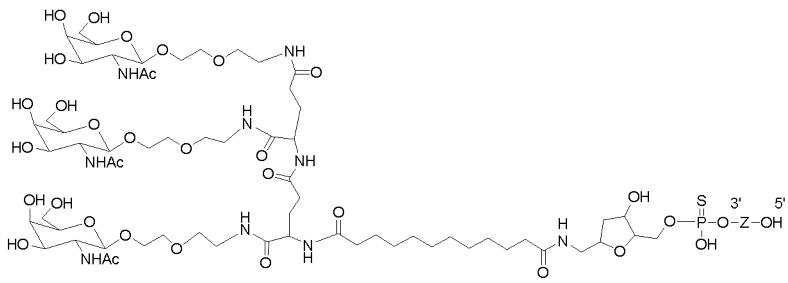

,

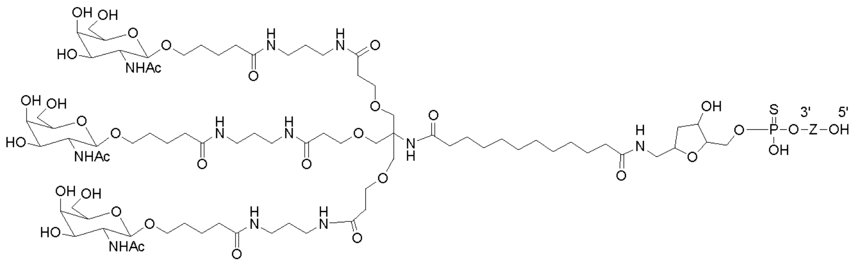

,

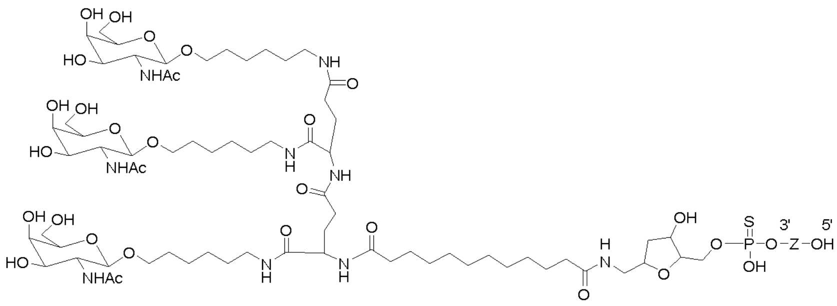

,

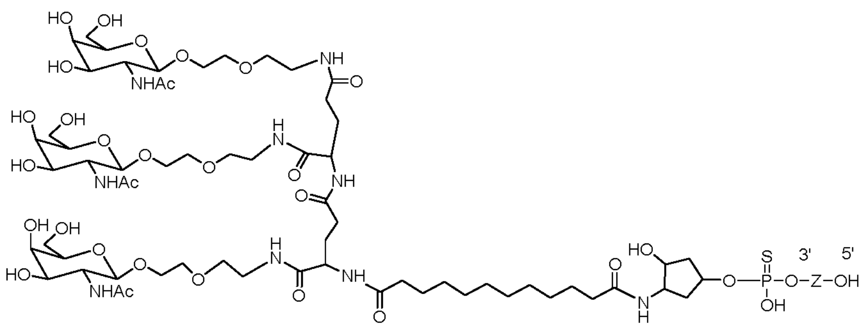

,

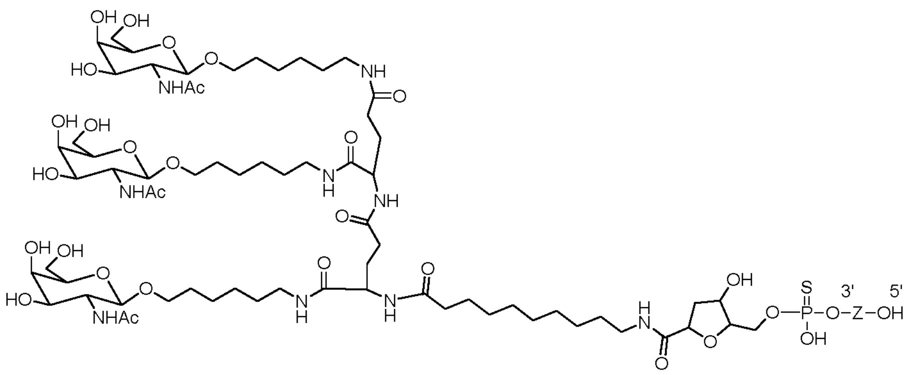

,

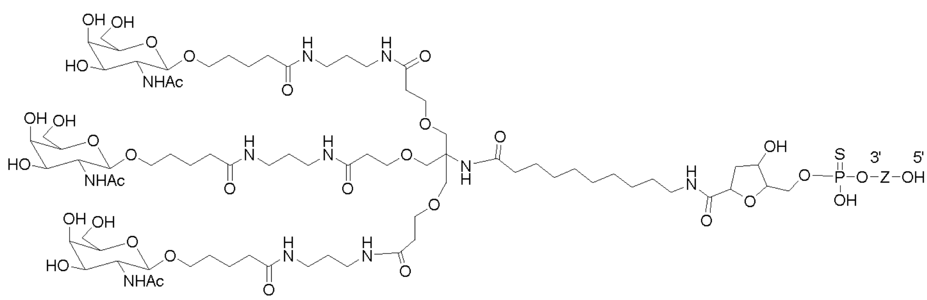

,

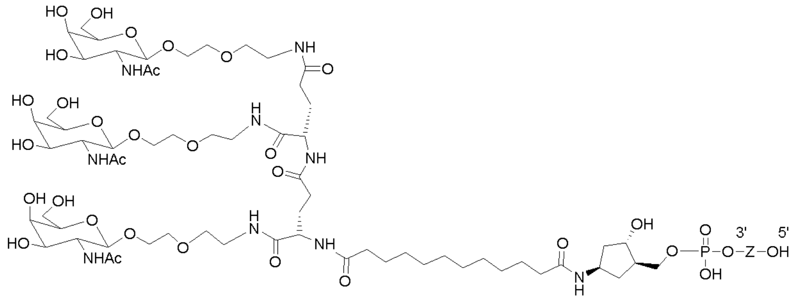

,

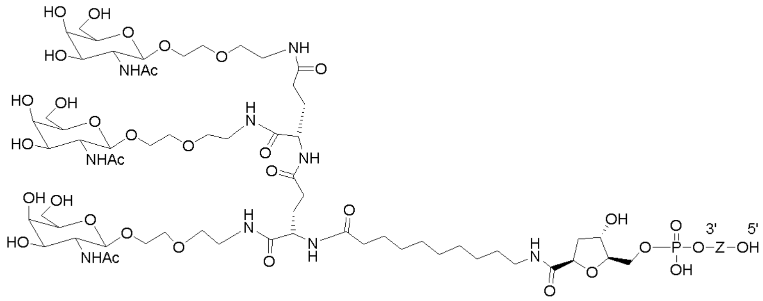

,

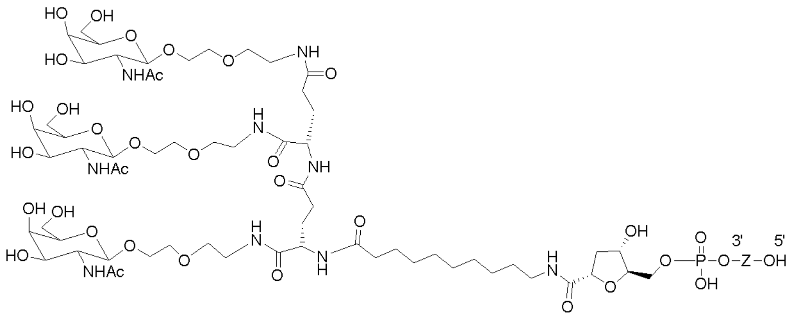

,

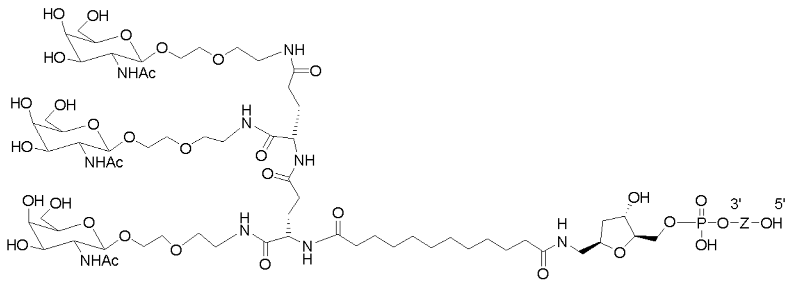

,

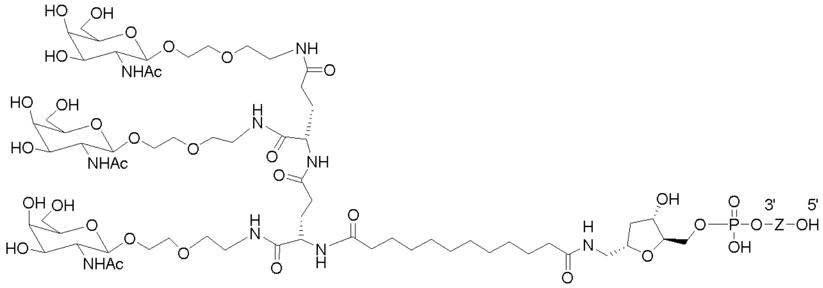

,

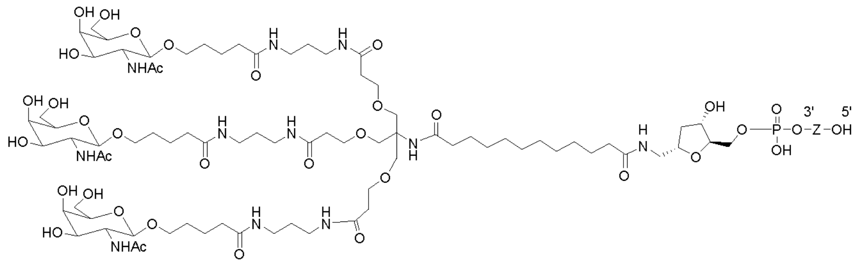

,

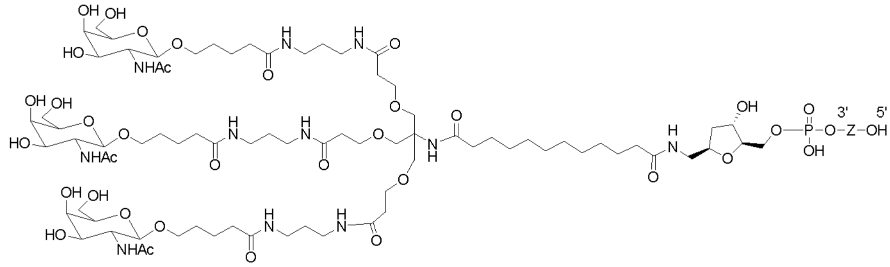

,

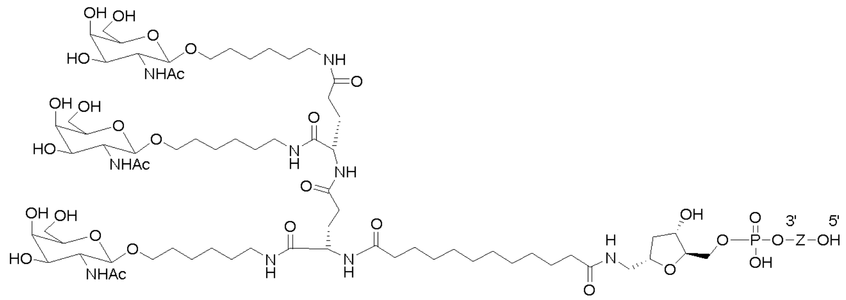

,

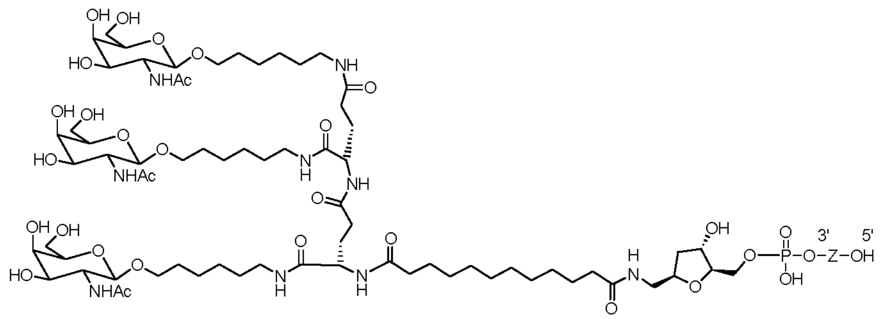

,

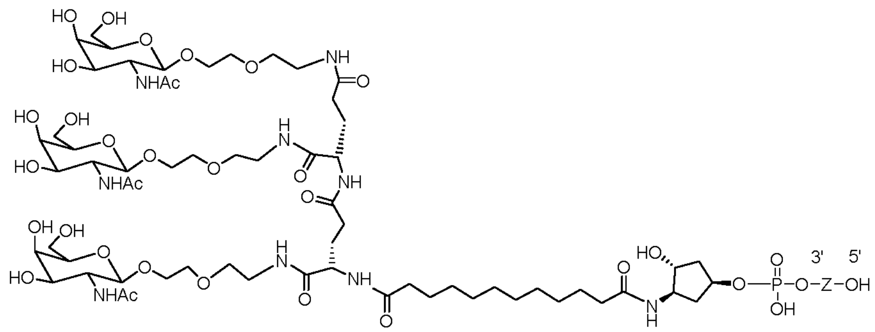

,

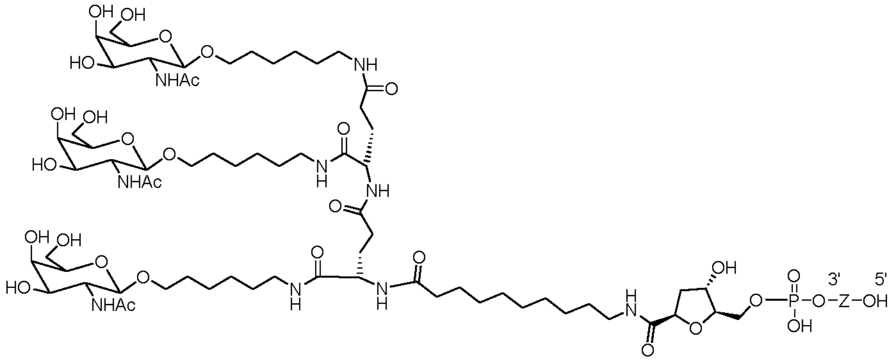

,

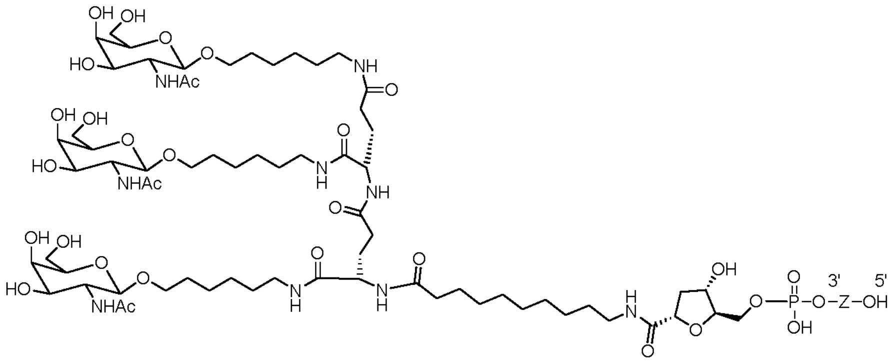

,

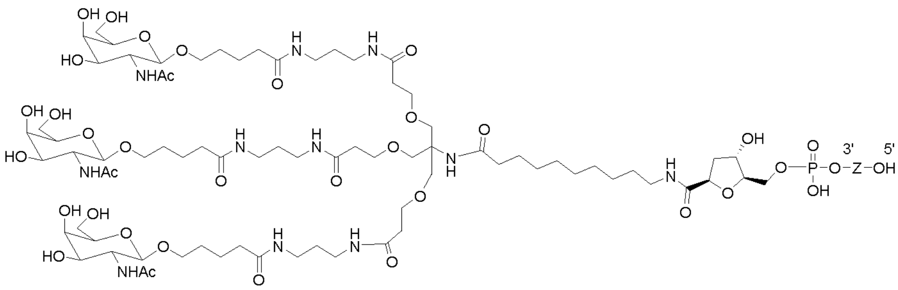

or

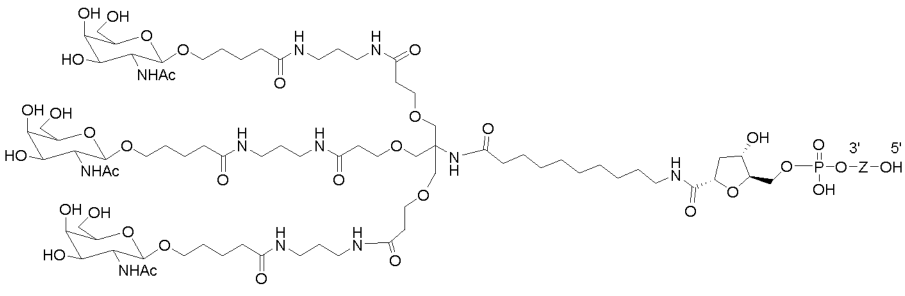

.

In some embodiments, the nucleic acid-ligand conjugate may be any of the following structures or a pharmaceutically acceptable salt thereof:

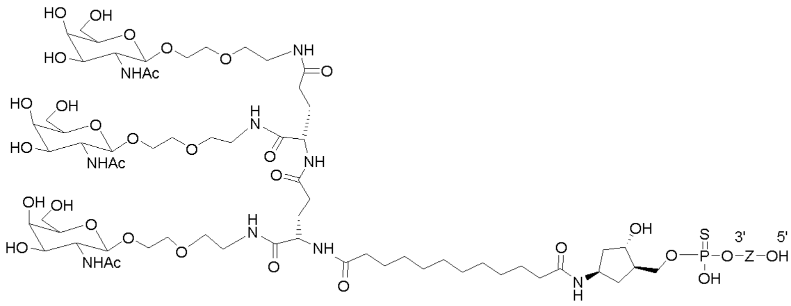

,

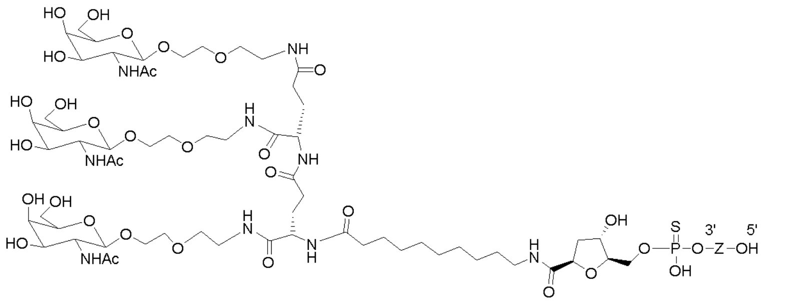

,

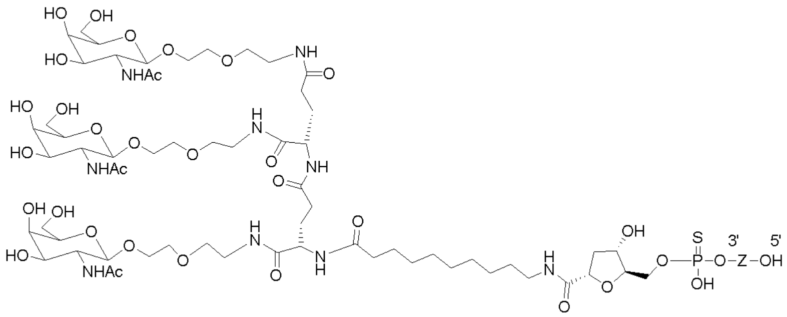

,

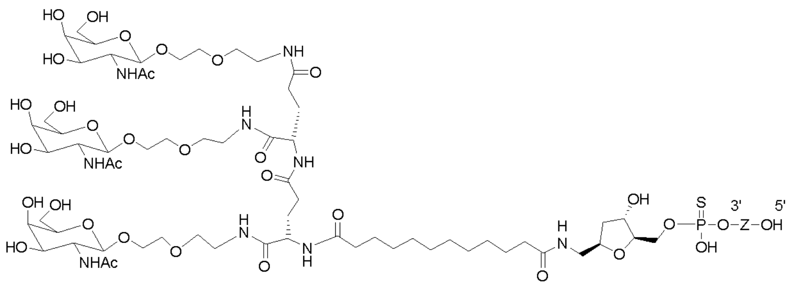

,

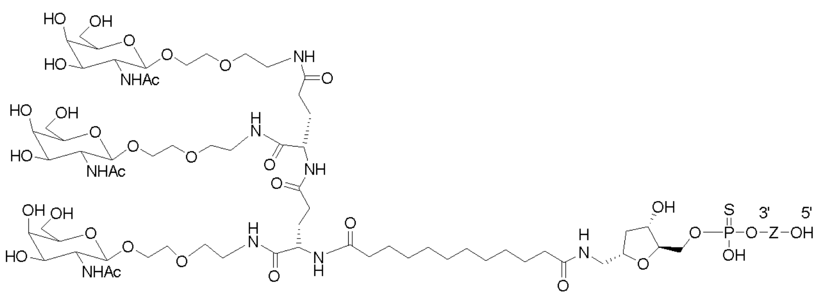

,

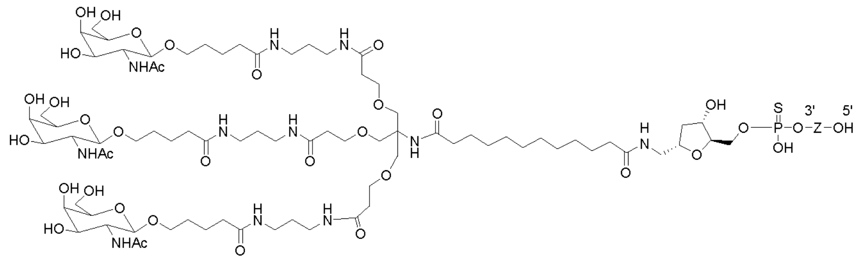

,

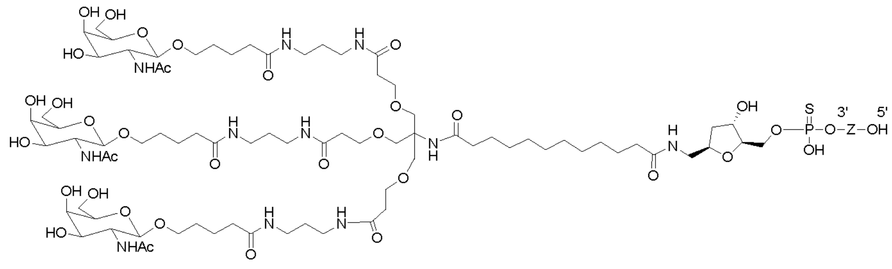

,

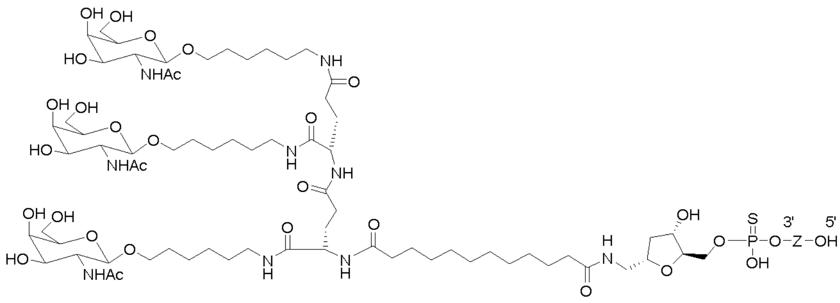

,

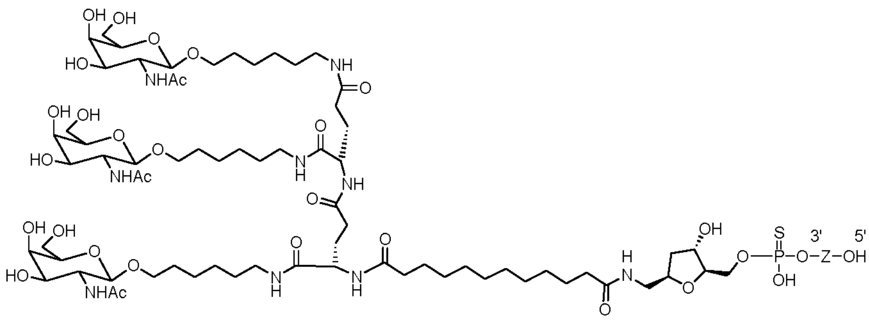

,

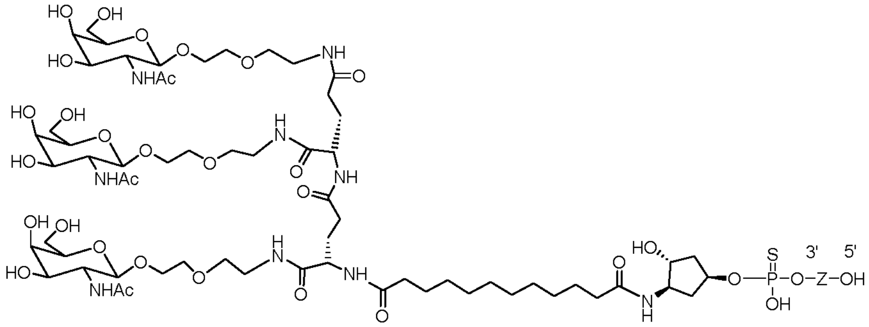

,

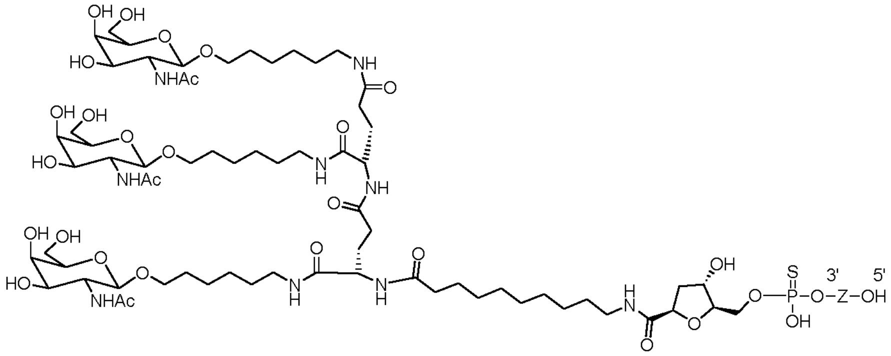

,

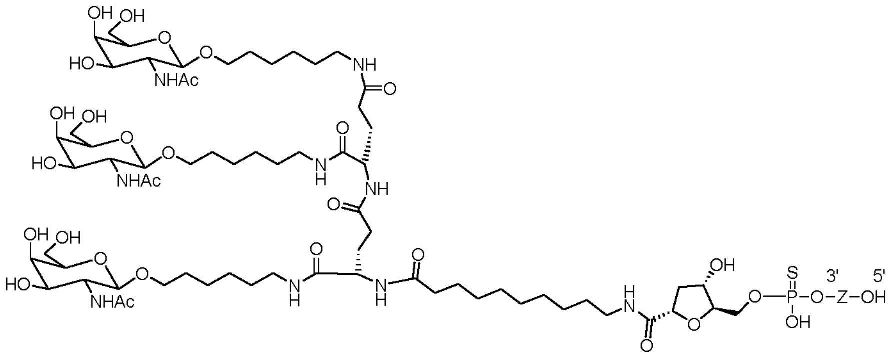

,

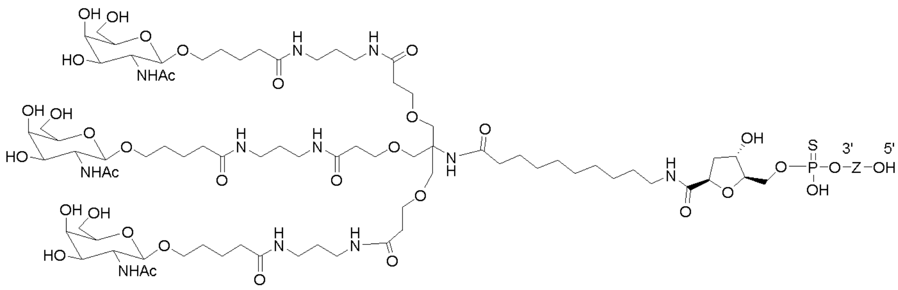

,

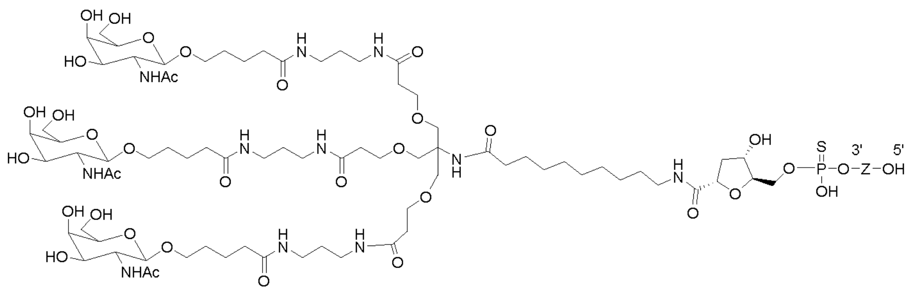

,

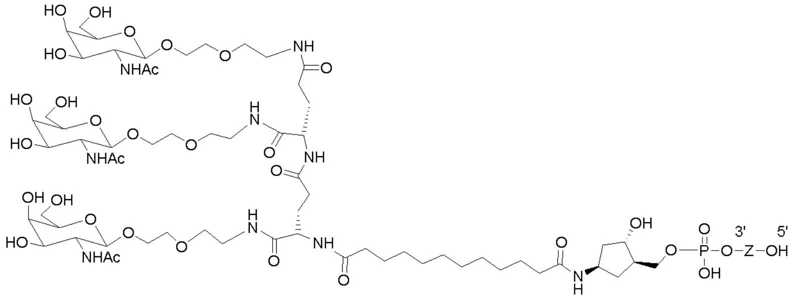

,

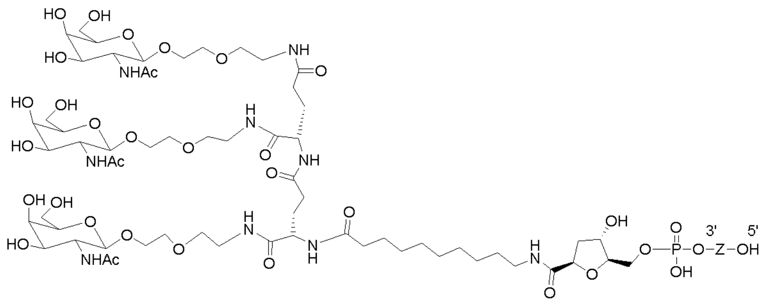

,

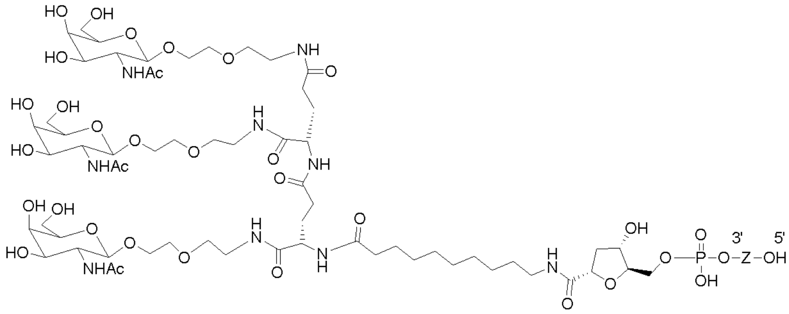

,

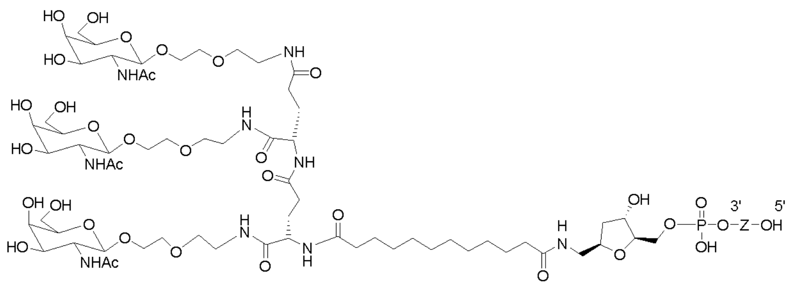

,

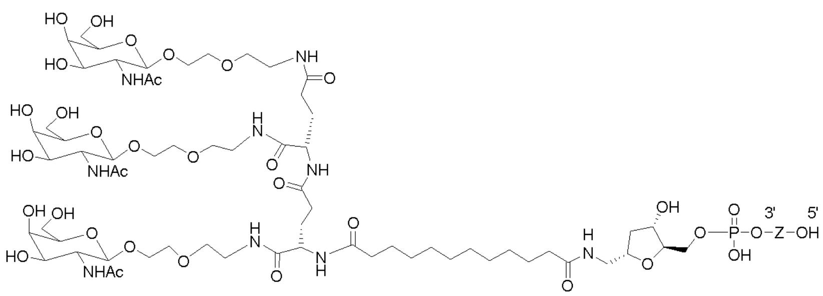

,

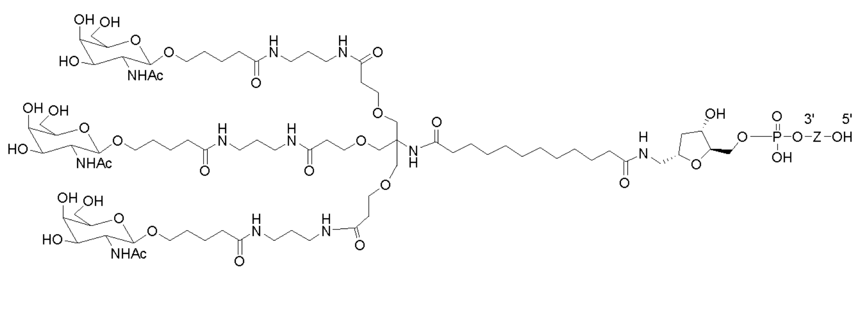

,

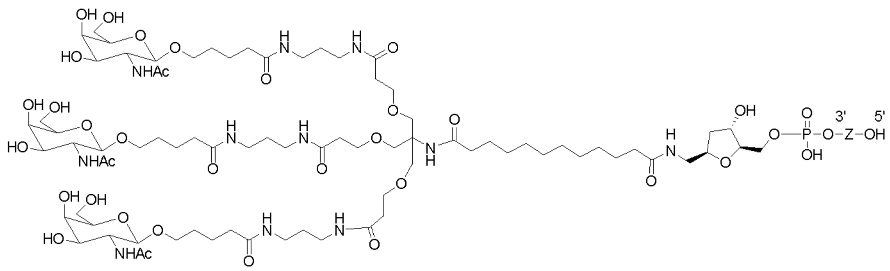

,

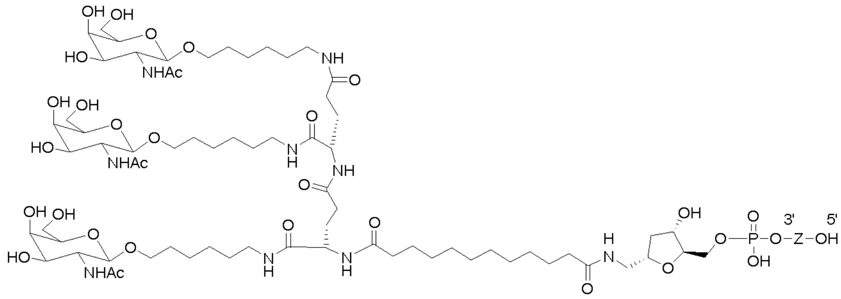

,

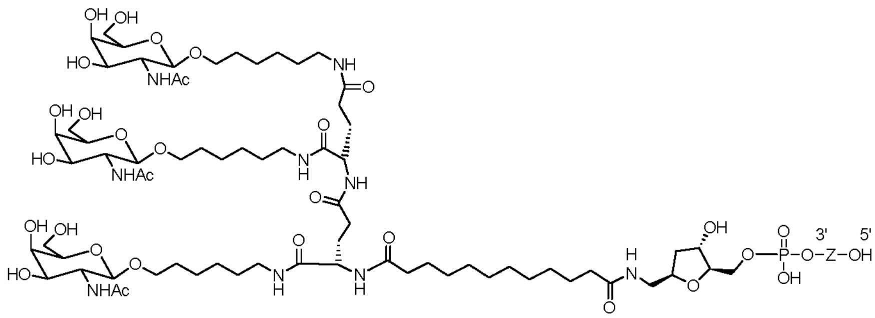

,

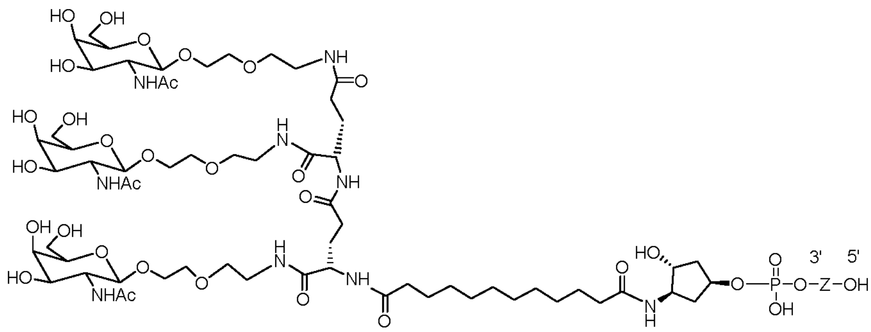

,

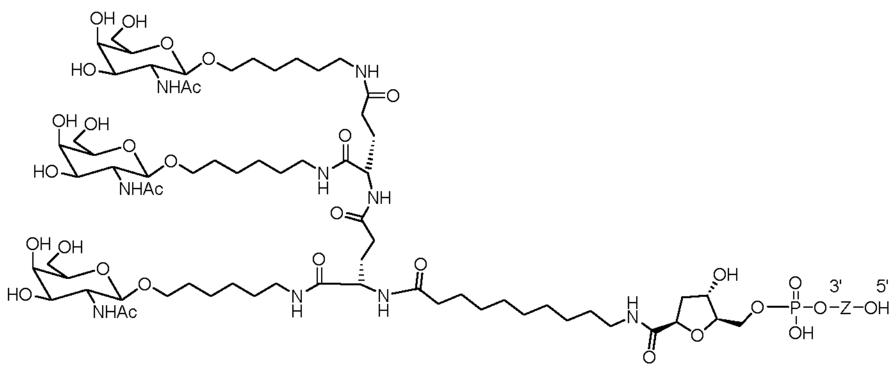

,

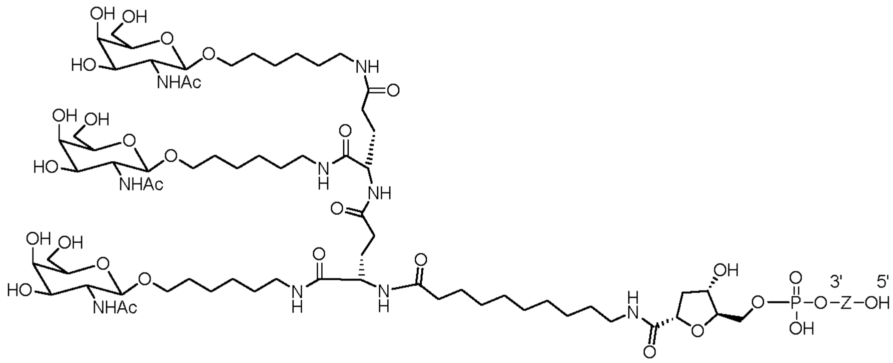

,

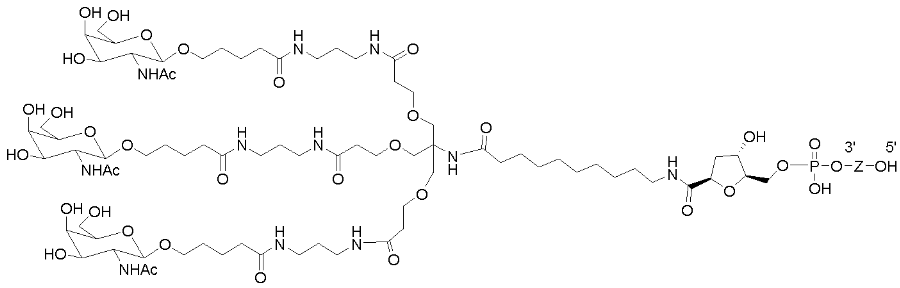

or

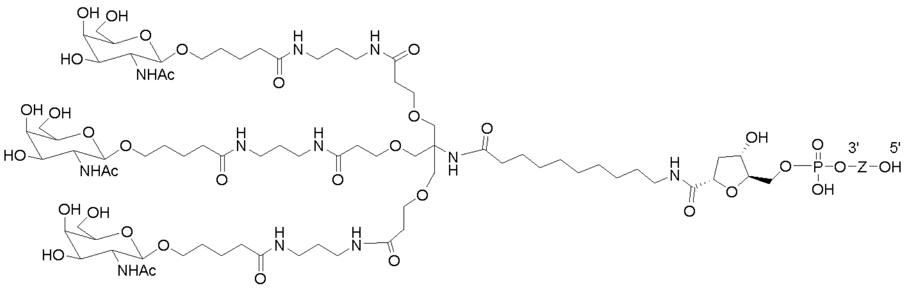

.

In some embodiments, the pharmaceutically acceptable salt may be a conventional salt in the art, including, but not limited to: sodium salts, potassium salts, ammonium salts, amine salts, etc.

In another aspect, the present disclosure provides an RNAi agent comprising the nucleic acid-ligand conjugate described above.

In some embodiments, the RNAi agent may include, but is not limited to: single-stranded oligonucleotides, single-stranded antisense oligonucleotides, short interfering RNAs (siRNAs), double-stranded RNAs (dsRNAs), micro-RNAs (miRNAs), short hairpin RNAs (shRNAs), and Dicer substrates.

In some embodiments, the RNAi agent may be an siRNA.

In another aspect, the present disclosure provides a composition comprising the nucleic acid-ligand conjugate described above or the RNAi agent described above, and one or more pharmaceutically acceptable excipients.

In some embodiments, the pharmaceutically acceptable excipients may be, for example, carriers, vehicles, diluents, and/or delivery polymers.

In some embodiments, the nucleic acid-ligand conjugate or the RNAi agent may be present in a therapeutically effective amount.

In some embodiments, a unit dose of the composition may be 0.001 mg-1000 mg.

In some embodiments, the nucleic acid-ligand conjugate or the RNAi agent may be present in an amount of 0.01-99.99%, or 0.1-99.9%, or 0.5%-99.5%, further 1%-99%, and still further 2%-98%, based on the total weight of the composition.

In some embodiments, the pharmaceutically acceptable excipients may be present in an amount of 0.01-99.99%, or 0.1-99.9%, or 0.5%-99.5%, further 1%-99%, and still further 2%-98%, based on the total weight of the composition.

In some embodiments, when the nucleic acid-ligand conjugate or RNAi agent or composition described in the

present disclosure is in contact with a target gene-expressing cell, the nucleic acid-ligand conjugate or RNAi agent described above inhibits target gene expression by at least 5%, at least 10%, at least 15%, at least 20%, at least 25%, at least 30%, at least 35%, at least 40%, at least 45%, at least 50%, at least 55%, at least 60%, at least 65%, at least 70%, at least 75%, at least 80%, at least 85%, at least 90%, at least 91%, at least 92%, at least 93%, at least 94%, at least 95%, at least 96%, at least 97%, at least 98%, or at least 99%, as measured by, for example, psiCHECK activity screening and luciferase reporter gene assay, other methods such as PCR or branched DNA (bDNA)-based methods, or protein-based methods such as immunofluorescence assay, e.g., western blot or flow cytometry.

In some embodiments, when the conjugate or RNAi agent or composition described above is in contact with a target gene-expressing cell, the siRNA described above results in a remaining percentage of target gene mRNA expression of no more than 99%, no more than 95%, no more than 90%, no more than 85%, no more than 80%, no more than 75%, no more than 70%, no more than 65%, no more than 60%, no more than 55%, no more than 50%, no more than 45%, no more than 40%, no more than 35%, no more than 30%, no more than 25%, no more than 20%, no more than 15%, or no more than 10%, as measured by, for example, psiCHECK activity screening and luciferase reporter gene assay, other methods such as PCR or branched DNA (bDNA)-based methods, or protein-based methods such as immunofluorescence assay, e.g., western blot or flow cytometry.

In another aspect, the present disclosure provides use of the nucleic acid-ligand conjugate described above or the RNAi agent described above or the composition described above in the preparation of a medicament for treating a disease in a patient. The disease is preferably a hepatogenic disease.

The present disclosure provides a nucleic acid-ligand conjugate or RNAi agent or composition for treating a disease in a patient, wherein the nucleic acid-ligand conjugate or RNAi agent or composition is as described above. The disease is preferably a hepatogenic disease.

The present disclosure provides a nucleic acid-ligand conjugate or RNAi agent or composition for inhibiting mRNA expression in a patient, wherein the nucleic acid-ligand conjugate or RNAi agent or composition is as described above.

The present disclosure provides a ligand, nucleic acid-ligand conjugate or RNAi agent or composition for delivering *in vivo* an expression-inhibiting oligomeric compound to the liver, wherein the ligand, nucleic acid-ligand conjugate or RNAi agent or composition is as described above.

In another aspect, the present disclosure provides a method for treating a disease in a patient, comprising administering to the patient the nucleic acid-ligand conjugate or RNAi agent or composition described above. The disease is preferably a hepatogenic disease. The nucleic acid-ligand conjugate or RNAi agent or composition may be present in a therapeutically effective amount.

In another aspect, the present disclosure provides a method for inhibiting mRNA expression in a patient, comprising administering to the patient the nucleic acid-ligand conjugate or RNAi agent or composition described above. The nucleic acid-ligand conjugate or RNAi agent or composition may be present in a therapeutically effective amount.

In another aspect, the present disclosure provides a method for delivering *in vivo* an expression-inhibiting oligomeric compound to the liver, comprising administering to a patient the nucleic acid-ligand conjugate or RNAi agent or composition described above. The nucleic acid-ligand conjugate or RNAi agent or composition may be present in a therapeutically effective amount.

The nucleic acid-ligand conjugate or RNAi agent or composition and the methods disclosed herein can reduce the level of a target mRNA in a cell, a group of cells, a tissue or a subject, comprising: administering to the subject a therapeutically effective amount of the expression-inhibiting oligomer described herein. The expression-inhibiting oligomer is linked to a ligand, thereby inhibiting target mRNA expression in the subject. The ligand is as described above.

In some embodiments, the subject has been previously identified as having pathogenic upregulation of the target gene in the targeted cell or tissue.

The patient described in the present disclosure refers to a subject having a disease or condition that would benefit from reduction or inhibition of target mRNA expression. Delivery may be by local administration (e.g., direct injection or implantation) or systemic administration, or by oral, rectal or parenteral routes. The parenteral routes include, but are not limited to, subcutaneous injection, intravenous injection, intramuscular injection, intraperitoneal injection, transdermal administration, inhalation administration (e.g., aerosol), mucosal administration (e.g., sublingual or intranasal administration), intracranial administration, etc.

In some embodiments, the nucleic acid-ligand conjugate or RNAi agent or composition provided by the present disclosure may be administered by injection, for example, intravenous, intramuscular, intradermal, subcutaneous, intraduodenal or intraperitoneal injection.

In some embodiments, the nucleic acid-ligand conjugate or RNAi agent or composition provided by the present disclosure may be packaged in a kit.

In some embodiments, the present disclosure further provides a cell comprising the nucleic acid-ligand conjugate or RNAi agent described above.

The present disclosure provides a method for preparing a nucleic acid-ligand conjugate, comprising the following

steps: taking the compound represented by formula (II-3) or formula (II'-3) described above as a start and attaching nucleoside monomers one by one in the 3'-5' direction in the order in which nucleotides are arranged.

In some embodiments, each time a monomer is attached, four reactions-deprotection, coupling, capping, and oxidation or sulfurization-are involved; after the last monomer is attached, the nucleic acid sequence attached to the solid-phase support is cleaved, deprotected, purified, desalted, and then lyophilized to give the nucleic acid-ligand conjugate.

The present disclosure provides a method for preparing the compound represented by formula (II-3) described above, comprising the following step: conjugating the compound represented by formula (II-2) described above with a macromolecular compound to give the compound represented by formula (II-3),

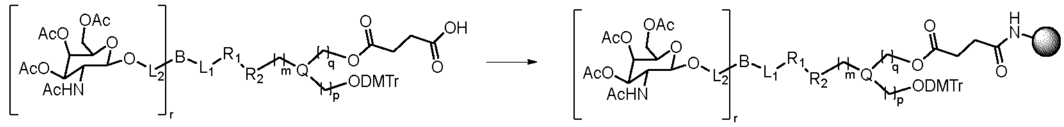

(II-2) (II-3)

wherein $L_1$, $R_1$, $R_2$, Q, B, $L_2$, m, p, q, r and

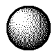

are as defined above.

In some embodiments, the conditions and procedures for the conjugation may be those conventional in the art.

In some embodiments, the compound represented by formula (II-2) may be prepared by the following step: subjecting the compound represented by formula (II-1) described above to the following reaction with succinic anhydride in a solvent under the action of alkali to give the compound represented by formula (II-2);

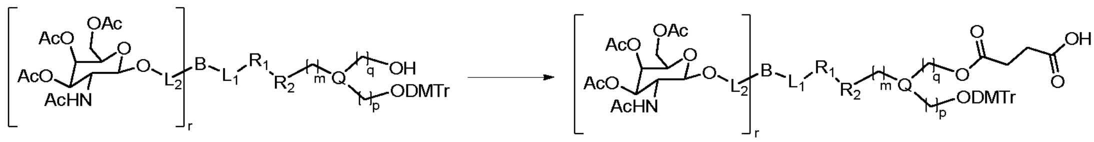

(II-1) (II-2)

$L_1$, $R_1$, $R_2$, Q, B, $L_2$, m, p, q and r are as defined above.

In some embodiments, the conditions and procedures for the reaction may be those conventional in the art for such reactions.

In some embodiments, the compound represented by formula (II-1) may be prepared by the following step: subjecting the compound represented by formula (III) to the following reaction with the compound represented by formula (IV) to give the compound represented by formula (II-1);

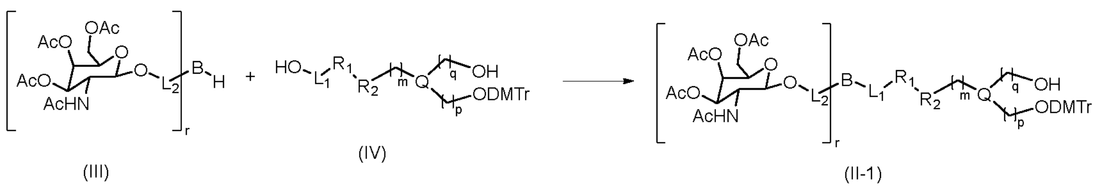

(III) (IV) (II-1)

$L_1$, $R_1$, $R_2$, Q, B, $L_2$, m, p, q and r are as defined above.

In some embodiments, the conditions and procedures for the reaction may be those conventional in the art for such reactions.

The present disclosure provides a method for preparing the compound represented by formula (II-1), comprising the following step: subjecting the compound represented by formula (III) to the following reaction with the compound represented by formula (IV) to give the compound represented by formula (II-1);

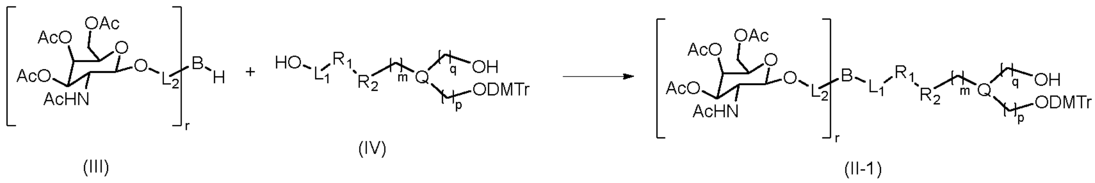

(III) (IV) (II-1)

$L_1$, $R_1$, $R_2$, Q, B, $L_2$, m, p, q and r are as defined above.

In some embodiments, the conditions and procedures for the reaction may be those conventional in the art for such reactions.

**Definition of Terms**

In another aspect, where the present disclosure does not define a particular configuration, the compounds of the present disclosure may exist in particular geometric or stereoisomeric forms. The present disclosure contemplates all such compounds, including cis and trans isomers, (-)- and (+)-enantiomers, (R)- and (S)-enantiomers, diastereomers, (D)-isomer, (L)-isomer, and racemic mixtures and other mixtures thereof, such as enantiomerically or diastereomerically enriched mixtures, all of which are within the scope of the present disclosure. Additional asymmetric carbon atoms may be present in substituents such as an alkyl group. All such isomers and mixtures thereof are included within the scope of the present disclosure.

The compounds and intermediates of the present disclosure may also exist in different tautomeric forms, and all such forms are included within the scope of the present disclosure. The term "tautomer" or "tautomeric form" refers to structural isomers of different energies that can interconvert via a low energy barrier. For example, proton tautomers (also known as proton transfer tautomers) include interconversion via proton migration, such as keto-enol and imine-enamine, lactam-lactim isomerization. An example of a lactam-lactim equilibrium is present between A and B as shown below.

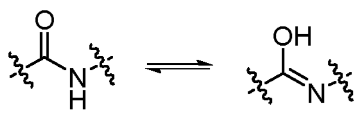

**A** **B**

All compounds in the present disclosure can be drawn as form A or form B. All tautomeric forms are within the scope of the present disclosure. The nomenclature of the compounds does not exclude any tautomers.

The compounds of the present disclosure may be asymmetric; for example, the compounds have one or more stereoisomers. Unless otherwise specified, all stereoisomers include, for example, enantiomers and diastereomers. The compounds of the present disclosure containing asymmetric carbon atoms can be isolated in optically active pure form or in racemic form. The optically active pure form can be isolated from a racemic mixture or synthesized using chiral starting materials or chiral reagents.

Optically active (R)- and (S)-enantiomers, and D- and L-isomers may be prepared by chiral synthesis, chiral reagents or other conventional techniques. If one enantiomer of a certain compound of the present disclosure is desired, it may be prepared by asymmetric synthesis or derivatization with a chiral auxiliary, wherein the resulting mixture of diastereomers is separated and the auxiliary group is cleaved to provide the pure desired enantiomer. Alternatively, when the molecule contains a basic functional group (e.g., amino) or an acidic functional group (e.g., carboxyl), salts of diastereomers are formed with an appropriate optically active acid or base, followed by resolution of diastereomers by conventional methods known in the art, and the pure enantiomers are obtained by recovery. In addition, separation of enantiomers and diastereomers is generally accomplished by chromatography using a chiral stationary phase, optionally in combination with chemical derivatization (e.g., carbamate formation from amines).

The present disclosure further includes isotopically labeled compounds that are identical to those recited herein but have one or more atoms replaced by an atom having an atomic mass or mass number different from the atomic mass or mass number usually found in nature. Examples of isotopes that can be incorporated into the compounds of the present disclosure include isotopes of hydrogen, carbon, nitrogen, oxygen, phosphorus, sulfur, fluorine, iodine, and chlorine, such as $^{2}H$, $^{3}H$, $^{11}C$,$^{13}C$, $^{14}C$, $^{13}N$, $^{15}N$, $^{15}O$, $^{17}O$, $^{18}O$, $^{31}P$, $^{32}P$, $^{35}S$, $^{18}F$, $^{123}I$, $^{125}I$ and $^{36}Cl$.

Unless otherwise specified, when a position is specifically assigned deuterium (D), the position should be construed as deuterium with an abundance that is at least 1000 times greater than the natural abundance of deuterium (which is 0.015%) (i.e., at least 10% deuterium incorporation). The compounds of examples comprise deuterium having

an abundance that is greater than at least 1000 times the natural abundance, at least 2000 times the natural abundance, at least 3000 times the natural abundance, at least 4000 times the natural abundance, at least 5000 times the natural abundance, at least 6000 times the natural abundance, or higher times the natural abundance. The present disclosure further includes various deuterated forms of the compound of formula (I). Each available hydrogen atom connected to a carbon atom may be independently replaced by a deuterium atom. Those skilled in the art can synthesize the deuterated forms of the compound of formula (I) according to the relevant literature. Commercially available deuterated starting materials can be used in preparing the deuterated forms of the compound of formula (I), or they can be synthesized using conventional techniques with deuterated reagents including, but not limited to, deuterated borane, tri-deuterated borane in tetrahydrofuran, deuterated lithium aluminum hydride, deuterated iodoethane, deuterated iodomethane, and the like.

"Optionallay" or "optional" means that the event or circumstance subsequently described may, but does not necessarily, occur, and that the description includes instances where the event or circumstance occurs or does not occur. For example, "$C_{1-6}$ alkyl that is optionally substituted with a halogen or cyano" means that the halogen or cyano may, but does not necessarily, exist, and the description includes the instance where alkyl is substituted with a halogen or cyano and the instance where alkyl is not substituted with a halogen or cyano.

In the chemical structure of the compound of the present disclosure, a bond "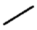" represents an unspecified configuration; that is, if chiral isomers exist in the chemical structure, the bond "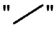" may be "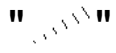" or "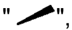", or contains both the configurations of "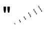" and "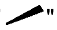" Although all of the above structural formulae are drawn as certain isomeric forms for the sake of simplicity, the present disclosure may include all isomers, such as tautomers, rotamers, geometric isomers, diastereomers, racemates and enantiomers. In the chemical structure of the compound of the present disclosure, a bond "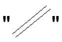" does not specify a configuration-that is, the configuration for the bond "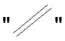" can be an E configuration or a Z configuration, or includes both the E configuration and the Z configuration.

The term "composition" refers to a mixture of a drug containing one or more of the compounds described herein or physiologically pharmaceutically acceptable salts or precursors thereof, and other chemical components, as well as other components such as physiologically pharmaceutically acceptable carriers and excipients. The composition is intended to promote the administration to an organism, so as to facilitate the absorption of the active ingredient, thereby exerting biological activity. The term "pharmaceutically acceptable excipient" includes, but is not limited to, any auxiliary, carrier, excipient, glidant, sweetener, diluent, preservative, dye/colorant, flavoring agent, surfactant, wetting agent, dispersant, suspending agent, stabilizer, isotonic agent, solvent or emulsifier that has been approved by the U.S. Food and Drug Administration as acceptable for use in humans or livestock animals.

Unless otherwise specified, "compound", "ligand", "nucleic acid-ligand conjugate" and "nucleic acid" of the present disclosure can each independently exist in the form of a salt, mixed salts, or a non-salt (e.g., a free acid or free base). When existing in the form of a salt or mixed salts, it can be a pharmaceutically acceptable salt.

The term "pharmaceutically acceptable salt" includes pharmaceutically acceptable acid addition salts and pharmaceutically acceptable base addition salts.

"Pharmaceutically acceptable acid addition salt" refers to salts that are capable of retaining the biological effectiveness of free bases without having any undesirable effects and that are formed with inorganic or organic acids. Inorganic acid salts include, but are not limited to, hydrochlorides, hydrobromides, sulfates, nitrates, phosphates, etc.; organic acid salts include, but are not limited to, formates, acetates, 2,2-dichloroacetates, trifluoroacetates, propionates, caproates, caprylates, caprates, undecenates, glycolates, gluconates, lactates, sebacates, adipates, glutarates, malonates, oxalates, maleates, succinates, fumarates, tartrates, citrates, palmitates, stearates, oleates, cinnamates, laurates, malates, glutamates, pyroglutamates, aspartates, benzoates, mesylates, benzenesulfonates, p-toluenesulfonates, alginates, ascorbates, salicylates, 4-aminosalicylates, napadisylates, etc. These salts can be prepared using methods known in the art.

"Pharmaceutically acceptable base addition salt" refers to salts that are capable of retaining the biological effectiveness of free acids without having any undesirable effects and that are formed with inorganic bases or organic bases. Salts derived from inorganic bases include, but are not limited to, sodium salts, potassium salts, lithium salts, ammonium salts, calcium salts, magnesium salts, iron salts, zinc salts, copper salts, manganese salts, aluminum salts, etc. Preferred inorganic salts are ammonium salts, sodium salts, potassium salts, calcium salts and magnesium salts; sodium salts are preferred. Salts derived from organic bases include, but are not limited to, salts of the following: primary, secondary and tertiary amines, substituted amines including naturally occurring substituted amines, cyclic amines and basic ion exchange resins, such as ammonia, isopropylamine, trimethylamine, diethylamine, triethylamine, tripropylamine, ethanolamine, diethanolamine, triethanolamine, dimethylethanolamine, 2-dimethylaminoethanol, 2-diethylaminoethanol, dicyclohexylamine, lysine, arginine, histidine, caffeine, procaine, choline, betaine, ethylenediamine, glucosamine, methylglucamine, theobromine, purine, piperazine, piperidine, N-ethylpiperidine, polyamine resins, etc. Preferred organic bases include isopropylamine, diethylamine, ethanolamine, trimethylamine, dicyclohexylamine, choline,

and caffeine. These salts can be prepared using methods known in the art.

The term "alkyl" refers to a saturated aliphatic hydrocarbon group, which is a linear or branched group containing 1 to 30 carbon atoms, preferably an alkyl group containing 1 to 12 carbon atoms, more preferably an alkyl group of 1 to 10 carbon atoms, more preferably an alkyl group of 1 to 6 carbon atoms, and further preferably an alkyl group of 1 to 4 carbon atoms. Non-limiting examples include methyl, ethyl, n-propyl, isopropyl, n-butyl, isobutyl, tert-butyl, sec-butyl, n-pentyl, 1,1-dimethylpropyl, 1,2-dimethylpropyl, 2,2-dimethylpropyl, 1-ethylpropyl, 2-methylbutyl, 3-methylbutyl, n-hexyl, 1-ethyl-2-methylpropyl, 1,1,2-trimethylpropyl, 1,1-dimethylbutyl, 1,2-dimethylbutyl, 2,2-dimethylbutyl, 1,3-dimethylbutyl, 2-ethylbutyl, 2-methylpentyl, 3-methylpentyl, 4-methylpentyl, 2,3-dimethylbutyl, n-heptyl, 2-methylhexyl, 3-methylhexyl, 4-methylhexyl, 5-methylhexyl, 2,3-dimethylpentyl, 2,4-dimethylpentyl, 2,2-dimethylpentyl, 3,3-dimethylpentyl, 2-ethylpentyl, 3-ethylpentyl, n-octyl, 2,3-dimethylhexyl, 2,4-dimethylhexyl, 2,5-dimethylhexyl, 2,2-dimethylhexyl, 3,3-dimethylhexyl, 4,4-dimethylhexyl, 2-ethylhexyl, 3-ethylhexyl, 4-ethylhexyl, 2-methyl-2-ethylpentyl, 2-methyl-3-ethylpentyl, n-nonyl, 2-methyl-2-ethylhexyl, 2-methyl-3-ethylhexyl, 2,2-diethylpentyl, n-decyl, 3,3-diethylhexyl, 2,2-diethylhexyl, and various branched isomers thereof, and the like. More preferred is an alkyl group containing 1 to 6 carbon atoms; non-limiting examples include methyl, ethyl, n-propyl, isopropyl, n-butyl, isobutyl, tert-butyl, sec-butyl, n-pentyl, 1,1-dimethylpropyl, 1,2-dimethylpropyl, 2,2-dimethylpropyl, 1-ethylpropyl, 2-methylbutyl, 3-methylbutyl, n-hexyl, 1-ethyl-2-methylpropyl, 1,1,2-trimethylpropyl, 1,1-dimethylbutyl, 1,2-dimethylbutyl, 2,2-dimethylbutyl, 1,3-dimethylbutyl, 2-ethylbutyl, 2-methylpentyl, 3-methylpentyl, 4-methylpentyl, 2,3-dimethylbutyl, and the like. Alkyl may be substituted or unsubstituted, and when it is substituted, the substituent may be substituted at any accessible point of attachment, and the substituent is preferably one or more of the following groups independently selected from the group consisting of alkyl, alkenyl, alkynyl, alkoxy, alkylthio, alkylamino, halogen, sulfhydryl, hydroxy, nitro, cyano, cycloalkyl, heterocycloalkyl, aryl, heteroaryl, cycloalkoxy, heterocycloalkoxy, cycloalkylthio, heterocycloalkylthio, oxo, carboxyl and carboxylate group.

The term "alkylene" refers to the remainder of an alkane molecule after 2 hydrogen atoms are removed, including linear and branched -ylene groups of 1 to 20 carbon atoms. Non-limiting examples of alkylene containing 1 to 6 carbon atoms include methylene ($-CH_2-$), ethylene (e.g., $-CH_2CH_2-$ or $-CH(CH_3)-$), propylene (e.g, $-CH_2CH_2CH_2-$ or $-CH(CH_2CH_3)-$), and butylene (e.g., $-CH_2CH_2CH_2CH_2-$). Unless otherwise specified, alkylene may be substituted or unsubstituted, and when it is substituted, the substituent may be substituted at any accessible point of attachment, preferably one or more of the following groups, independently selected from the group consisting of deuterium, aryl, heteroaryl and halogen.

The term "cycloalkyl" or "carbocycle" refers to a saturated or partially unsaturated, monocyclic or polycyclic hydrocarbon substituent. The cycloalkyl ring contains 3 to 20 carbon atoms, preferably 3 to 7 carbon atoms. Non-limiting examples of monocyclic cycloalkyl include cyclopropyl, cyclobutyl, cyclopentyl, cyclopentenyl, cyclohexyl, cyclohexenyl, cyclohexadienyl, and the like. Polycyclic cycloalkyl includes spirocycloalkyl, fused cycloalkyl and bridged cycloalkyl. Cycloalkyl may be substituted or unsubstituted, and when it is substituted, the substituent may be substituted at any accessible point of attachment, preferably one or more of the following groups, independently selected from the group consisting of halogen, deuterium, hydroxy, oxo, nitro, cyano, $C_{1-6}$ alkyl, $C_{1-6}$ alkoxy, $C_{2-6}$ alkenyloxy, $C_{2-6}$ alkynyloxy, $C_{3-6}$ cycloalkoxy, 3- to 6-membered heterocycloalkoxy, $C_{3-8}$ cycloalkenyloxy, and 5- to 6-membered aryl or heteroaryl, wherein the $C_{1-6}$ alkyl, $C_{1-6}$ alkoxy, $C_{2-6}$ alkenyloxy, $C_{2-6}$ alkynyloxy, $C_{3-6}$ cycloalkoxy, 3- to 6-membered heterocycloalkoxy, $C_{3-8}$ cycloalkenyloxy and 5- to 6-membered aryl or heteroaryl are optionally substituted with one or more groups selected from the group consisting of halogen, deuterium, hydroxy, oxo, nitro and cyano.

The cycloalkyl ring may be fused to an aryl or heteroaryl ring, wherein the ring attached to the parent structure is cycloalkyl; non-limiting examples include indanyl, tetrahydronaphthyl, benzocycloheptyl, etc. Cycloalkyl may be optionally substituted or unsubstituted, and when it is substituted, the substituent is preferably one or more of the following groups independently selected from the group consisting of halogen, deuterium, hydroxy, oxo, nitro, cyano, $C_{1-6}$ alkyl, $C_{1-6}$ alkoxy, $C_{2-6}$ alkenyloxy, $C_{2-6}$ alkynyloxy, $C_{3-6}$ cycloalkoxy, 3- to 6-membered heterocycloalkoxy, $C_{3-8}$ cycloalkenyloxy, and 5- to 6-membered aryl or heteroaryl, wherein the $C_{1-6}$ alkyl, $C_{1-6}$ alkoxy, $C_{2-6}$ alkenyloxy, $C_{2-6}$ alkynyloxy, $C_{3-6}$ cycloalkoxy, 3- to 6-membered heterocycloalkoxy, $C_{3-8}$ cycloalkenyloxy and 5- to 6-membered aryl or heteroaryl are optionally substituted with one or more groups selected from the group consisting of halogen, deuterium, hydroxy, oxo, nitro and cyano.

The term "heterocycloalkyl" or "heterocycle" refers to a saturated or partially unsaturated, monocyclic or polycyclic hydrocarbon substituent, which contains 3 to 20 ring atoms, one or more of which are heteroatoms selected from the group consisting of nitrogen, oxygen and $S(O)_m$ (where m is an integer of 0 to 2), but does not contain a ring moiety of -O-O-, -O-S- or -S-S-, and the other ring atoms are carbons. It preferably contains 3 to 12 ring atoms, 1 to 4 of which are heteroatoms; more preferably, it contains 3 to 7 ring atoms. Non-limiting examples of monocyclic heterocycloalkyl include pyrrolidinyl, imidazolidinyl, tetrahydrofuranyl, tetrahydrothienyl, dihydroimidazolyl, dihydrofuranyl, dihydropyrazolyl, dihydropyrrolyl, piperidinyl, piperazinyl, morpholinyl, thiomorpholinyl, homopiperazinyl, and the like. Polycyclic heterocycloalkyl includes spiroheterocyclyl, fused heterocyclyl and bridged heterocycloalkyl. Non-limiting examples of "heterocycloalkyl" include:

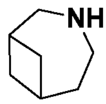, 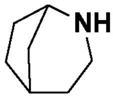, 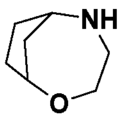, 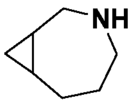, 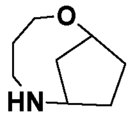, 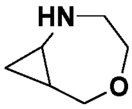,

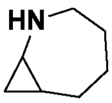, 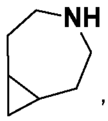, 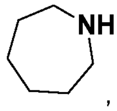, 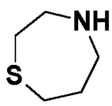, 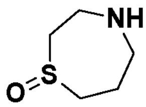, 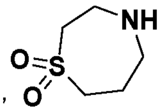,

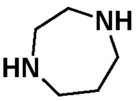, 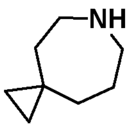, 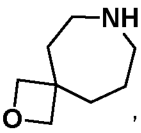, 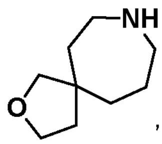, 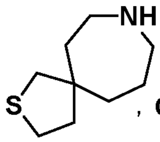, 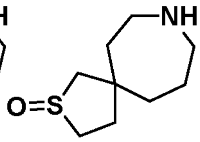,

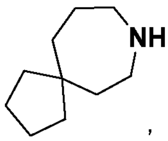, 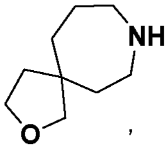, 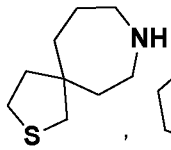, 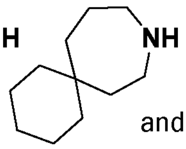 and 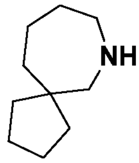,

and the like. The heterocycloalkyl ring may be fused to an aryl or heteroaryl ring, wherein the ring attached to the parent structure is heterocycloalkyl; its non-limiting examples include:

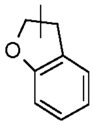 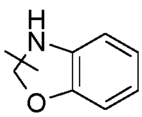 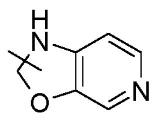, 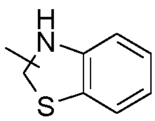,

etc. Heterocycloalkyl may be optionally substituted or unsubstituted, and when it is substituted, the substituent is preferably one or more of the following groups independently selected from the group consisting of halogen, deuterium, hydroxy, oxo, nitro, cyano, $C_{1-6}$ alkyl, $C_{1-6}$ alkoxy, $C_{2-6}$ alkenyloxy, $C_{2-6}$ alkynyloxy, $C_{3-6}$ cycloalkoxy, 3- to 6-membered heterocycloalkoxy, $C_{3-8}$ cycloalkenyloxy, and 5- to 6-membered aryl or heteroaryl, wherein the $C_{1-6}$ alkyl, $C_{1-6}$ alkoxy, $C_{2-6}$ alkenyloxy, $C_{2-6}$ alkynyloxy, $C_{3-6}$ cycloalkoxy, 3- to 6-membered heterocycloalkoxy, $C_{3-8}$ cycloalkenyloxy and 5- to 6-membered aryl or heteroaryl are optionally substituted with one or more groups selected from the group consisting of halogen, deuterium, hydroxy, oxo, nitro and cyano.

The term "aryl" refers to a 6- to 14-membered, preferably 6- to 12-membered, all-carbon monocyclic or fused polycyclic (i.e., rings sharing a pair of adjacent carbon atoms) group having a conjugated $\pi$-electron system, such as phenyl and naphthyl. The aryl ring may be fused to a heteroaryl, heterocycloalkyl or cycloalkyl ring, wherein the ring attached to the parent structure is an aryl ring; its non-limiting examples include:

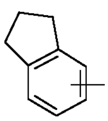, 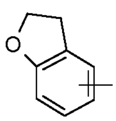, 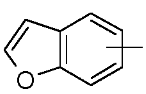, 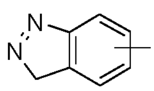, 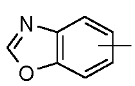, 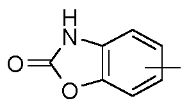,

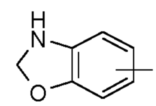, 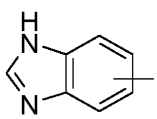, 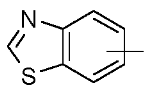, 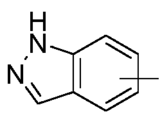, 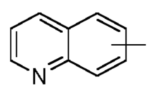,

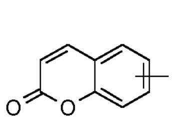 and 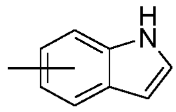 .

Aryl may be substituted or unsubstituted, and when it is substituted, the substituent is preferably one or more of the following groups independently selected from the group consisting of halogen, deuterium, hydroxy, oxo, nitro, cyano, $C_{1-6}$ alkyl, $C_{1-6}$ alkoxy, $C_{2-6}$ alkenyloxy, $C_{2-6}$ alkynyloxy, $C_{3-6}$ cycloalkoxy, 3- to 6-membered heterocycloalkoxy, $C_{3-8}$ cycloalkenyloxy, and 5- to 6-membered aryl or heteroaryl, wherein the $C_{1-6}$ alkyl, $C_{1-6}$ alkoxy, $C_{2-6}$ alkenyloxy, $C_{2-6}$ alkynyloxy, $C_{3-6}$ cycloalkoxy, 3- to 6-membered heterocycloalkoxy, $C_{3-8}$ cycloalkenyloxy and 5- to 6-membered aryl or heteroaryl are optionally substituted with one or more groups selected from the group consisting of halogen, deuterium, hydroxy, oxo, nitro and cyano.

The term "heteroaryl" refers to a heteroaromatic system containing 1 to 4 heteroatoms and 5 to 14 ring atoms, wherein the heteroatoms are selected from the group consisting of oxygen, sulfur and nitrogen. Heteroaryl is preferably 5- to 12-membered, and is more preferably 5- or 6-membered. For example, its non-limiting examples include: imidazolyl, furanyl, thienyl, thiazolyl, pyrazolyl, oxazolyl, isoxazolyl, pyrrolyl, tetrazolyl, pyridinyl, pyrimidinyl, thiadiazole, pyrazinyl, triazolyl, indazolyl, benzimidazolyl,

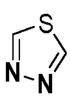 , 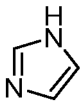 , 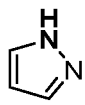 ,

and the like.

The heteroaryl ring may be fused to an aryl, heterocycloalkyl or cycloalkyl ring, wherein the ring attached to the parent structure is heteroaryl; its non-limiting examples include:

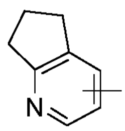 , 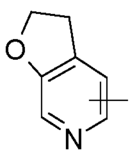 , 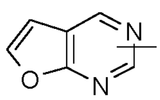 , 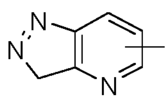 , 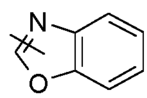 , 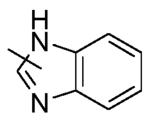 ,

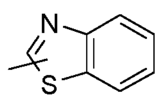 , 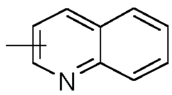 , and 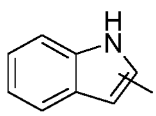 .

Heteroaryl may be optionally substituted or unsubstituted, and when it is substituted, the substituent is preferably one or more of the following groups independently selected from the group consisting of halogen, deuterium, hydroxy, oxo, nitro, cyano, $C_{1-6}$ alkyl, $C_{1-6}$ alkoxy, $C_{2-6}$ alkenyloxy, $C_{2-6}$ alkynyloxy, $C_{3-6}$ cycloalkoxy, 3- to 6-membered heterocycloalkoxy, $C_{3-8}$ cycloalkenyloxy, and 5- to 6-membered aryl or heteroaryl, wherein the $C_{1-6}$ alkyl, $C_{1-6}$ alkoxy, $C_{2-6}$ alkenyloxy, $C_{2-6}$ alkynyloxy, $C_{3-6}$ cycloalkoxy, 3- to 6-membered heterocycloalkoxy, $C_{3-8}$ cycloalkenyloxy and 5- to 6-membered aryl or heteroaryl are optionally substituted with one or more groups selected from the group consisting of halogen, deuterium, hydroxy, oxo, nitro and cyano.

The term "spiro" refers to compounds in which two rings share one atom.

The term "spirocycloalkyl" refers to a 5- to 20-membered polycyclic group in which monocyclic rings share one carbon atom (referred to as a spiro atom). It may contain one or more double bonds, but none of the rings has a fully conjugated $\pi$-electron system. It is preferably 6- to 14-membered, and is more preferably 7- to 10-membered. According to the number of spiro atoms shared among the rings, spirocycloalkyl may be monospirocycloalkyl, bispirocycloalkyl or polyspirocycloalkyl. Monospirocycloalkyl and bispirocycloalkyl are preferred. 4-membered/4-membered, 4-membered/5-membered, 4-membered/6-membered, 5-membered/5-membered or 5-membered/6-membered monospirocycloalkyl is more preferred. "Spirocarbocycle" refers to the ring system in spirocycloalkyl. Non-limiting examples of spirocycloalkyl include:

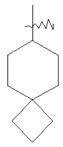 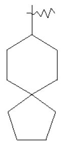 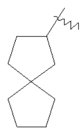 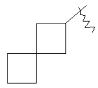 and 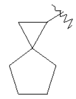 .

The term "spiroheterocyclyl" refers to a 5- to 20-membered polycyclic heterocyclyl group in which monocyclic rings share one atom (referred to as a spiro atom), wherein one or more ring atoms are heteroatoms selected from the group consisting of nitrogen, oxygen and $S(O)_m$ (where m is an integer of 0 to 2), and the other ring atoms are carbons. It may contain one or more double bonds, but none of the rings has a fully conjugated $\pi$-electron system. It is preferably 6- to 14-membered, and is more preferably 7- to 10-membered. According to the number of spiro atoms shared among the rings, spiroheterocyclyl may be monospiroheterocyclyl, bispiroheterocyclyl or polyspiroheterocyclyl. Monospiroheterocyclyl and bispiroheterocyclyl are preferred. 4-membered/4-membered, 4-membered/5-membered, 4-membered/6-membered, 5-membered/5-membered or 5-membered/6-membered monospiroheterocyclyl is more preferred. "Spiroheterocycle" refers to the ring system in spiroheterocyclyl. Non-limiting examples of spiroheterocyclyl include:

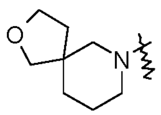 and 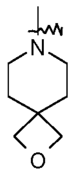 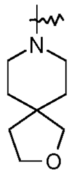 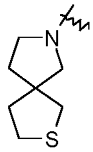 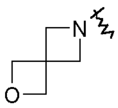 and 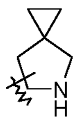 .

The term "fused" refers to compounds formed by fusing two or more rings by sharing two adjacent atoms.

The term "fused cycloalkyl" refers to a 5- to 20-membered all-carbon polycyclic group in which each ring in the system shares a pair of adjacent carbon atoms with other rings in the system, wherein one or more rings may contain one or more double bonds, but none of them has a fully conjugated $\pi$-electron system. It is preferably 6- to 14-membered, and is more preferably 7- to 10-membered. According to the number of constituent rings, fused cycloalkyl may be bicyclic, tricyclic, tetracyclic or polycyclic fused cycloalkyl, preferably bicyclic or tricyclic fused cycloalkyl, and more preferably 5-membered/5-membered or 5-membered/6-membered bicycloalkyl. Non-limiting examples of fused cycloalkyl include:

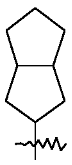 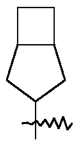 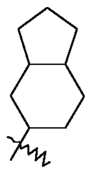 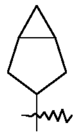 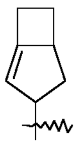 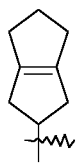 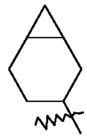 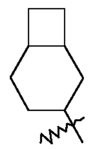 and 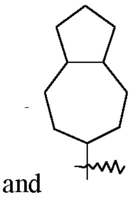 .

The term "fused heterocyclyl" refers to a 5- to 20-membered polycyclic heterocyclic group in which each ring in the system shares a pair of adjacent atoms with the other rings in the system, wherein one or more rings may contain one or more double bonds, but none of them has a fully conjugated $\pi$-electron system; one or more ring atoms are heteroatoms selected from the group consisting of nitrogen, oxygen and $S(O)_m$ (where m is an integer of 0 to 2), and the other ring atoms are carbons. It is preferably 6- to 14-membered, and is more preferably 7- to 10-membered. According to the number of constituent rings, fused heterocyclyl may be bicyclic, tricyclic, tetracyclic or polycyclic fused heterocyclyl, preferably bicyclic or tricyclic fused heterocyclyl, and more preferably 5-membered/5-membered or 5-membered/6-membered bicyclic fused heterocyclyl. "Fused heterocycle" refers to the ring system in fused heterocyclyl. Non-limiting examples of fused heterocyclyl include:

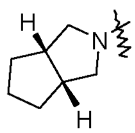 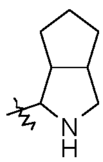 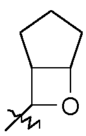 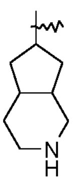 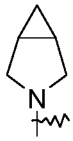 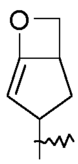 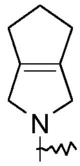 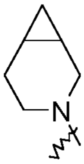

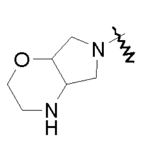, 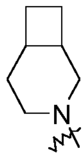, 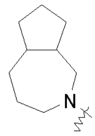, 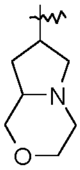, 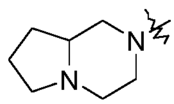 and 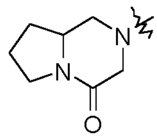.

The term "fused heteroaryl" may be an unsaturated aromatic fused ring structure containing 5-14 ring atoms (including at least one heteroatom) formed by connecting two or more cyclic structures that share two adjacent atoms, including the case where a carbon atom, a nitrogen atom and a sulfur atom may be substituted with oxo, preferably "5-12 membered fused heteroaryl", "7-12 membered fused heteroaryl", "9-12 membered fused heteroaryl", and the like, for example, benzofuranyl, benzoisothiafuranyl, benzothienyl, indolyl, isoindolyl, benzoxazolyl, benzimidazolyl, indazolyl, benzotriazolyl, quinolyl, 2-quinolinonyl, 4-quinolinonyl, 1-isoquinolinonyl, isoquinolinyl, acridinyl, phenanthridinyl, benzopyridazinyl, phthalazinyl, quinazolinyl, quinoxalinyl, phenazinyl, pteridinyl, purinyl, naphthyridinyl, phenazinyl, phenothiazinyl, and the like. "Fused heteroaromatic ring" refers to the ring system in fused heteroaryl.

Fused heteroaryl may be optionally substituted or unsubstituted, and when it is substituted, the substituent is preferably one or more of the following groups independently selected from the group consisting of alkyl, alkenyl, alkynyl, alkoxy, alkylthio, alkylamino, halogen, sulfhydryl, hydroxy, nitro, cyano, cycloalkyl, heterocycloalkyl, aryl, heteroaryl, cycloalkoxy, heterocycloalkoxy, cycloalkylthio, heterocycloalkylthio, carboxyl and carboxylate group.

The term "bridged" refers to a structure formed by two or more cyclic structures sharing two non-adjacent ring atoms.

The term "bridged cycloalkyl" refers to a 5- to 20-membered all-carbon polycyclic group in which any two rings share two carbon atoms that are not directly connected. It may contain one or more double bonds, but none of the rings has a fully conjugated π-electron system. It is preferably 6- to 14-membered, and is more preferably 7- to 10-membered. According to the number of constituent rings, bridged cycloalkyl may be bicyclic, tricyclic, tetracyclic or polycyclic bridged cycloalkyl, preferably bicyclic, tricyclic or tetracyclic bridged cycloalkyl, and more preferably bicyclic or tricyclic bridged cycloalkyl. Non-limiting examples of bridged cycloalkyl include:

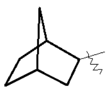 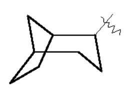 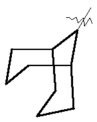 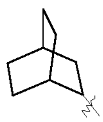 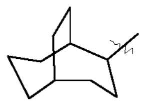

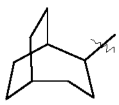 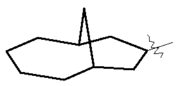 and 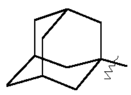.

The term "bridged heterocyclyl" refers to a 5- to 14-membered polycyclic heterocyclic group in which any two rings share two atoms that are not directly connected. It may contain one or more double bonds, but none of the rings has a fully conjugated π-electron system, wherein one or more ring atoms are heteroatoms selected from the group consisting of nitrogen, oxygen and $S(O)_m$ (where m is an integer of 0 to 2), and the other ring atoms are carbons. It is preferably 6- to 14-membered, and is more preferably 7- to 10-membered. According to the number of constituent rings, bridged heterocyclyl may be bicyclic, tricyclic, tetracyclic or polycyclic bridged heterocyclyl, preferably bicyclic, tricyclic or tetracyclic bridged heterocyclyl, and more preferably bicyclic or tricyclic bridged heterocyclyl. Non-limiting examples of bridged heterocyclyl include:

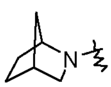 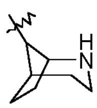 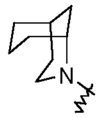 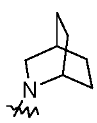

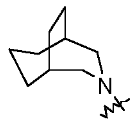 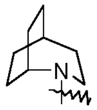 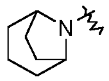 and 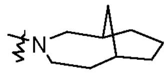.

The term "alkoxy" refers to -O-(alkyl) and -O-(unsubstituted cycloalkyl), wherein the alkyl is as defined above. Non-limiting examples of alkoxy include: methoxy, ethoxy, propoxy, butoxy, cyclopropyloxy, cyclobutoxy, cyclopentyloxy and cyclohexyloxy. Alkoxy may be optionally substituted or unsubstituted, and when it is substituted, the substituent is preferably one or more of the following groups independently selected from the group consisting of halogen, deuterium, hydroxy, oxo, nitro, cyano, $C_{1-6}$ alkyl, $C_{1-6}$ alkoxy, $C_{2-6}$ alkenyloxy, $C_{2-6}$ alkynyloxy, $C_{3-6}$ cycloalkoxy, 3- to 6-membered heterocycloalkoxy, $C_{3-8}$ cycloalkenyloxy, and 5- to 6-membered aryl or heteroaryl, wherein the $C_{1-6}$ alkyl, $C_{1-6}$ alkoxy, $C_{2-6}$ alkenyloxy, $C_{2-6}$ alkynyloxy, $C_{3-6}$ cycloalkoxy, 3- to 6-membered heterocycloalkoxy, $C_{3-8}$ cycloalkenyloxy and 5- to 6-membered aryl or heteroaryl are optionally substituted with one or more groups selected from the group consisting of halogen, deuterium, hydroxy, oxo, nitro and cyano. Likewise, the definitions of "alkynyloxy", "alkenyloxy", "cycloalkoxy", "heterocycloalkoxy" and "cycloalkenyloxy" are similar to the above definition of "alkoxy".

The term "substituted" means that one or more, preferably up to 5, more preferably 1 to 3 hydrogen atoms in the group are independently substituted with a corresponding number of substituents. It goes without saying that a substituent is only in its possible chemical position, and those skilled in the art will be able to determine (experimentally or theoretically) possible or impossible substitution without undue effort.

"Substituted with one or more" means that it may be substituted with a single substituent or multiple substituents. In the case of substitution with multiple substituents, there may be a plurality of identical substituents, or one combination of or a plurality of combinations of different substituents.

The term "link", "connect" or "attach", when referring to a relationship between two molecules, means that the two molecules are linked by a covalent bond or that the two molecules are associated via a non-covalent bond (e.g., a hydrogen bond or an ionic bond), and includes direct linkage and indirect linkage.

The term "directly connected" means that a first compound or group is connected to a second compound or group without any atom or group of atoms interposed between. The term "indirectly connected" means that a first compound or group is connected to a second compound or group by an intermediate group, a compound, or a molecule (e.g., a linking group).

The term "hydroxy" refers to -OH.

The term "halogen" refers to fluorine, chlorine, bromine or iodine.

The term "haloalkyl" refers to an alkyl group substituted with halogen, wherein the alkyl group is as defined above.

The term "cyano" refers to -CN.

The term "nitro" refers to $-NO_2$.

The term "oxo" refers to the =O group. For example, a carbon atom is connected to an oxygen atom via a double bond to form a ketone or aldehyde group.

The term "amino" refers to $-NH_2$.

The term "cyano" refers to -CN.

The term "carboxyl" refers to -C(O)OH.

The term "aldehyde" refers to -CHO.

In the present disclosure, the terms "comprise" and "include" can be replaced with "consist of".

In the present disclosure, "phosphoester group" and "phosphate linkage" are used interchangeably and include phosphomonoesters, phosphodiesters or phosphotriesters. The "phosphoester group" in the "phosphorothioate group" also has the same meaning. Unless otherwise specified, the natural internucleotide phosphoester group is a phosphodiester group.

In the present disclosure, the phosphorothioate group refers to a phosphodiester group modified by replacing one non-bridging oxygen atom with a sulfur atom, and is used interchangeably with

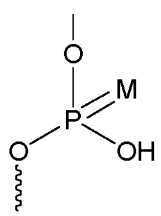 and 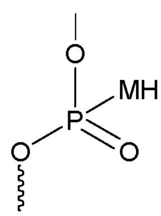

(M is an S atom).

As used herein, in the context of RNA-mediated gene silencing, the sense strand (also referred to as SS or SS strand) of an siRNA refers to a strand that comprises a sequence that is identical or substantially identical to a target mRNA sequence; the antisense strand (also referred to as AS or AS strand) of an siRNA refers to a strand having a sequence complementary to a target mRNA sequence.

In the present disclosure, the "5' region" of the sense or antisense strand, i.e., the "5' end" or "5' terminus", is used interchangeably. For example, the nucleotides at positions 2 to 8 of the 5' region of the antisense strand may be replaced with the nucleotides at positions 2 to 8 of the 5' end of the antisense strand. Likewise, the "3' region", "3' terminus" and "3' end" of the sense or antisense strand are also used interchangeably.

As used herein, the terms "complementary" and "reverse complementary" are used interchangeably and have the meaning well known to those skilled in the art-that is, in a double-stranded nucleic acid molecule, the bases of one strand are paired with the bases of the other strand in a complementary manner. In DNA, the purine base adenine (A) is always paired with the pyrimidine base thymine (T) (or uracil (U) in RNA), and the purine base guanine (C) is always paired with the pyrimidine base cytosine (G). Each base pair comprises a purine and a pyrimidine. When adenines of one strand are always paired with thymines (or uracils) of another strand and guanines are always paired with cytosines, the two strands are considered complementary to each other, and the sequences of the strands can be deduced from the sequences of their complementary strands. Accordingly, "mismatch" in the art means that in a double-stranded nucleic acid, the bases in the corresponding positions are not paired in a complementary manner.

As used herein, "chemical modification" or "modification" means a structure that has chemical differences when compared with a naturally occurring counterpart, including all changes made by chemical means, such as addition or removal of chemical moieties, or substitution of one chemical moiety for another.

In the context of the present disclosure, Bz represents benzoyl; MMTr represents methoxyphenyl benzhydryl; DMTr represents dimethoxytrityl.

As used herein, the term "base" encompasses any known DNA and RNA bases and base analogs such as purines or pyrimidines, which also include the natural compounds adenine, thymine, guanine, cytosine, uracil, inosine and natural analogs. Unless otherwise stated, in the context of the present disclosure, the uppercase letters C, G, U, A and T represent base components of a nucleotide; the lowercase letter d indicates that the right nucleotide adjacent to the letter d is a deoxyribonucleotide; the lowercase letter m indicates that the left nucleotide adjacent to the letter m is a methoxy-modified nucleotide; the lowercase letter f indicates that the left nucleotide adjacent to the letter f is a fluoro-modified nucleotide; the lowercase letter s indicates that the two nucleotides adjacent to the letter s are linked by a phosphorothioate group.

As used herein, the term "fluoro-modified nucleotide" refers to a nucleotide in which the hydroxy group at the 2' position of the ribosyl group of the nucleotide is substituted with fluorine; the methoxy-modified nucleotide refers to a nucleotide in which the 2'-hydroxy group of the ribosyl group is substituted with a methoxy group. As used herein, the term "inhibit" is used interchangeably with "decrease", "silence", "down-regulate", "repress" and other similar terms, and includes any level of inhibition. Inhibition can be assessed in terms of a decrease in the absolute or relative level of one or more of these variables relative to a control level. The control level can be any type of control level used in the art, such as a pre-dose baseline level or a level determined from a similar untreated or control (e.g., buffer-only control or inert agent control) treated subject, cell, or sample. For example, the remaining expression level of mRNA can be used to characterize the degree of inhibition of target gene expression by the siRNA; for example, the remaining expression level of mRNA is not greater than 99%, not greater than 95%, not greater than 90%, not greater than 85%, not greater than 80%, not greater than 75%, not greater than 70%, not greater than 65%, not greater than 60%, not greater than 55%, not greater than 50%, not greater than 45%, not greater than 40%, not greater than 35%, not greater than 30%, not greater than 25%, not greater than 20%, not greater than 15%, or not greater than 10%. The inhibition of target gene expression can be measured using Dual-Glo® Luciferase Assay System: the Firefly chemiluminescence value and the Renilla chemiluminescence value are each read, and the relative value Ratio = Ren/Fir is calculated; in the present disclosure, the ratio of the remaining mRNA expression level (or residual activity%) = Ratio (siRNA-treated group)/Ratio (siRNA-free control group), and inhibition rate (%) = 100% - remaining mRNA expression level (%).

"Effective amount", "effective dose", "effective therapeutic amount" or "therapeutically effective amount" refers to the amount of a drug, a compound or a pharmaceutical composition necessary to obtain any one or more beneficial or desired therapeutic results. For preventive use, the beneficial or desired results include elimination or reduction of risk, reduction of severity or delay of the onset of a disorder, including the biochemistry, histology and/or behavioral symptoms of the disorder, complications thereof and intermediate pathological phenotypes that appear during the progression of the disorder. For therapeutic applications, the beneficial or desired results include clinical results, such as reducing the incidence of various conditions related to the target gene, target mRNA or target protein of the present disclosure or alleviating one or more symptoms of the condition, reducing the dosage of other agents required to treat the condition, enhancing the therapeutic effect of another agent, and/or delaying the progression of conditions related to the target gene, target mRNA or target protein of the present disclosure in the patient. An effective amount also refers to an amount sufficient to allow or facilitate diagnosis. The effective amount for a particular patient or veterinary subject

may vary depending on factors such as the disorder to be treated, the general health of the patient, the method and route and dosage of administration, and the severity of side effects. An effective amount may be the maximum dose or administration regimen to avoid significant side effects or toxic effects.

As used herein, "object", "patient", "subject" and "individual" are used interchangeably and include human or non-human animals, e.g., mammals, e.g., humans or monkeys.

In some embodiments, upon delivery of an oligomeric compound to a gene-expressing cell, the oligomeric compound can inhibit the expression of underlying genes, and is referred to herein as an "expression-inhibiting oligomeric compound". It can inhibit gene expression *in vitro* or *in vivo.* "Oligomeric compound" includes, but is not limited to: oligonucleotides, single-stranded oligonucleotides, single-stranded antisense oligonucleotides, short interfering RNAs (siRNAs), double-stranded RNAs (dsRNAs), microRNAs (miRNAs), short hairpin RNAs (shRNAs), ribozymes, interfering RNA molecules, and Dicer enzyme substrates.

Unless otherwise indicated, the symbol 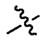 as used herein means that it may be linked to any group or groups according to the scope of the disclosure described herein.

As used herein, the first strand may be referred to as the antisense strand, and the second strand may be referred to as the sense strand. The terms first strand and antisense strand or second strand and sense strand should be considered interchangeable.

The "RNAi agent" used in the present disclosure refers to an agent that contains an RNA or RNA-like (e.g., chemically modified RNA) oligonucleotide molecule that is capable of degrading or inhibiting transcription and translation of a target messenger RNA (mRNA) in a sequence-specific manner. In the present disclosure, RNAi agents may operate through the RNA interference mechanism (i.e., inducing RNA interference through interaction with the RNA interference pathway machinery (RNA-induced silencing complex or RISC) of mammalian cells), or act by any other mechanisms or pathways. RNAi agents include, but are not limited to: single-stranded oligonucleotides, single-stranded antisense oligonucleotides, short interfering RNAs (siRNAs), double-stranded RNAs (dsRNAs), microRNAs (miRNAs), short hairpin RNAs (shRNAs), and Dicer substrates.

The RNAi agents described herein comprise an oligonucleotide having a strand that is at least partially complementary to the targeted mRNA.

## BRIEF DESCRIPTION OF THE DRAWINGS

FIG. 1 shows the expression levels of mRNA in TTR 7 days after administration of the conjugates TRD002218, TRD007203, TRD007204 and TRD007205.

FIG. 2 shows the expression levels of mRNA in TTR 28 days after administration of the conjugates TRD002218, TRD007203, TRD007204 and TRD007205.

FIG. 3 shows the inhibitory activity of GalNAc-conjugated siRNA against mTTR gene expression in primary murine hepatocytes in Test Example 6.

FIG. 4 shows the *in vivo* inhibitory activity of GalNAc-conjugated siRNA against the mouse mTTR gene in Test Example 7.

## DETAILED DESCRIPTION

The present disclosure is further illustrated by the following examples, which, however, are not intended to limit the present disclosure. Experimental procedures without conditions specified in the examples of the present disclosure are generally conducted according to conventional conditions, or according to conditions recommended by the manufacturers of the starting materials or commercial products. If the source of a reagent is not shown, the reagent is obtained from any molecular biology reagent supplier in quality/purity for molecular biology applications.

The compounds **NAG0024** and **NAG0026** were purchased from WuXi AppTec (Tianjin) Co., Ltd. Unless otherwise specified, all reagents used in the following examples are commercially available.

### Preparation Example 1: Synthesis of Compound NAG0039

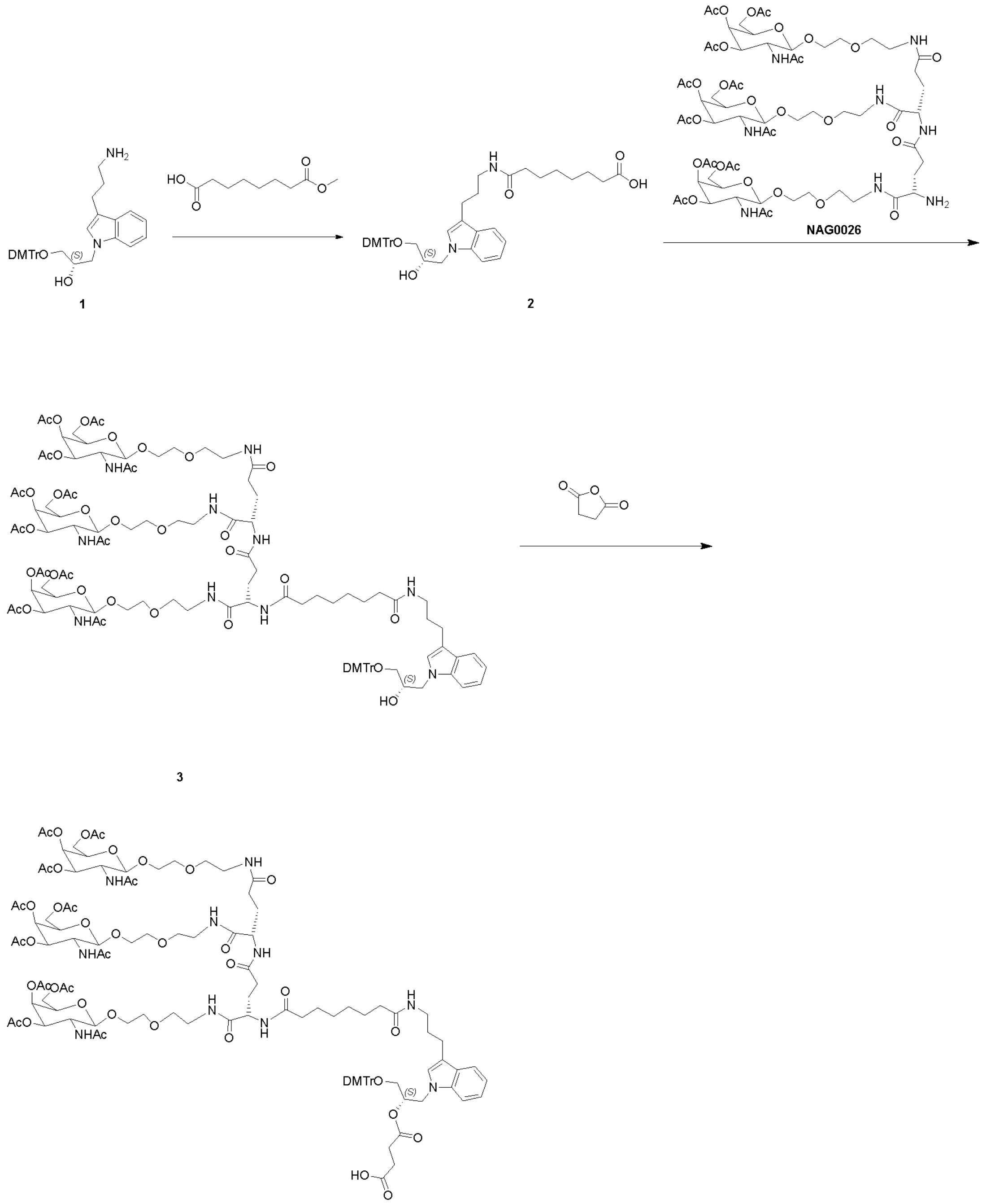

1

2

NAG0026

3

NAG0039

## Compound 2

To a solution of compound **1** (1.00 g, 1.82 mmol) in dry THF (20 mL) at room temperature in a nitrogen atmosphere were added sequentially DIEA (469 mg, 3.63 mmol), 3A molecular sieve, monomethyl suberate (342 mg, 1.82 mmol), DCC (487 mg, 2.36 mmol) and HOBt (319 mg, 2.36 mmol). The mixture was left to react overnight at 40 °C. The reaction solution was filtered, concentrated, purified by reversed-phase column chromatography (Boston C18 column, 0-100% MeCN/$H_2O$), and lyophilized to give an intermediate (1.14 g, 1.58 mmol, 87% yield).

The intermediate (1.14 g, 1.58 mmol) was dissolved in THF (3 mL) and MeOH (3 mL), and a solution of NaOH (126 mg, 3.16 mmol) in water (3 mL) was added dropwise. The reaction solution was stirred at room temperature for 4 h. Most of the organic solvent was removed under reduced pressure, and the residue was then purified by reversed-phase column chromatography (Boston C18 column, 0-100% MeCN/$H_2O$) to give compound **2** (851 mg, 1.17 mmol, sodium salt, 74% yield).

**Compound 3**

To a solution of the compound **NAG0026** (863 mg, 0.566 mmol) in dry THF (15 mL) at room temperature in a nitrogen atmosphere were added sequentially DIEA (146 mg, 1.13 mmol), 3A molecular sieve, compound 2 (400 mg, 0.566 mmol), DCC (140 mg, 0.679 mmol) and HOBt (92 mg, 0.679 mmol). The mixture was left to react overnight at 45 °C. The reaction solution was filtered, concentrated, purified by reversed-phase column chromatography (Boston C18 column, 0-100% MeCN/$H_2O$), and lyophilized to give compound **3** (918 mg, 0.415 mmol, 73% yield).

**Compound NAG0039**

To a solution of compound **3** (400 mg, 0.181 mmol) in pyridine at room temperature were added 3A molecular sieve, succinic anhydride (36 mg, 0.361 mmol) and DMAP (22 mg, 0.181 mmol). The mixture was left to react overnight at 45 °C in a nitrogen atmosphere. The reaction solution was filtered, concentrated under reduced pressure, and separated and purified by reversed-phase column chromatography (Boston C18 column, 0-100% MeCN/$H_2O$) to give a crude product (248 mg). Two batches of the crude product were combined and separated by preparative HPLC (column: Xbridge 150 × 50 mm, 5 μm; mobile phase: A: 0.1% $NH_3H_2O$ + 0.005% FA in water; B: MeCN; gradient: 20% B-95% B in 9 min) to give the compound **NAG0039** (240 mg, 0.104 mmol, 33% yield).

MS (ESI) m/z = 2312.3 $[M-1]^-$, calculated: 2313.0.
$^1H$ NMR (400 MHz, Acetonitrile-$d_3$) δ 7.63-6.59 (m, 27H), 5.40-5.22 (m, 4H), 5.16-5.02 (m, 3H), 4.74-4.57 (m, 3H), 4.45-3.05 (m, 47H), 2.70-1.96 (m, 57H), 1.63-1.27 (m, 11H).

**Preparation Example 2: Synthesis of Compound NAG0046**

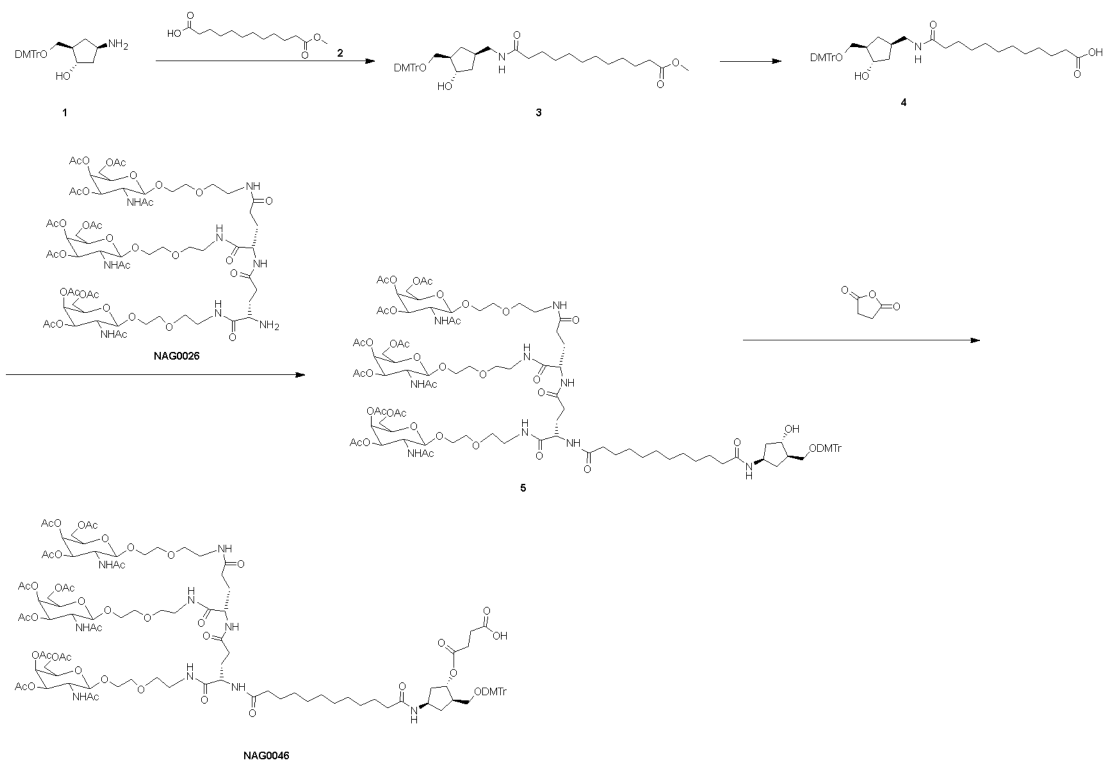
1
2
3
4
NAG0026
5
NAG0046

**Compound 3**

To the solvent DCM (5 mL) in a nitrogen atmosphere were added compound **2** (203 mg, 0.830 mmol), DIEA (0.206 mL, 1.246 mmol) and HATU (237 mg, 0.623 mmol). The mixture was stirred at 20 °C, and compound **1** (200 mg, 0.415 mmol) was then added to the system described above. After the addition, the mixture was left to react at 20 °C for 1 h in a nitrogen atmosphere. $H_2O$ (10 mL) was added to the reaction solution, and the mixture was extracted with

DCM (3 × 20 mL). The organic phase was dried over anhydrous $Na_2SO_4$, filtered, and concentrated. The residue was purified by column chromatography (DCM:MeOH (100:0-90:10)) to give compound **3** (250 mg, 82% yield).

LCMS: chromatographic conditions 10-80AB_2min, retention time 1.534 min; MS (ESI) m/z = 682.6 $[M+Na]^+$.
H NMR (400 MHz, CDCl3) δ 7.42 (d, *J* = 7.0 Hz, 1H), 7.34-7.29 (m, 6H), 7.21-7.17 (m, 2H), 6.86 (d, *J* = 9.0 Hz, 4H), 4.44 (br d, *J* = 7.5 Hz, 1H), 4.31-4.08 (m, 1H), 4.01-3.84 (m, 1H), 3.82 (d, *J* = 2.0 Hz, 6H), 3.69 (d, *J* = 2.3 Hz, 3H), 3.17-3.06 (m, 1H), 2.36-2.25 (m, 3H), 2.19-1.97 (m, 4H), 1.57-1.54 (m, 3H), 1.47 (d, *J* = 6.8 Hz, 4H), 1.28 (br d, *J* = 6.8 Hz, 10H).

**Compound 4**

Compound **3** (250 mg, 0.348 mmol) was dissolved in MeOH (1 mL) and $H_2O$ (0.5 mL), and LiOH (146 mg, 3.48 mmol) was then added. The mixture was left to react at room temperature for 12 h. The reaction solution was purified by reversed-phase column chromatography to give compound **4** (166 mg, 71% yield).

LCMS: chromatographic conditions 10-80CD_3min, retention time 1.353 min; MS (ESI) m/z = 668.6 $[M+Na]^+$.
HPLC: chromatographic conditions 10-80CD_6min, retention time 1.897 min.
H NMR (400 MHz, $CD_3OD$) δ ppm 7.45 (d, *J* = 7.2 Hz, 2H), 7.34-7.27 (m, 6H), 7.24-7.19 (m, 1H), 6.92-6.83 (m, 4H), 4.32 (q, *J* = 8.0 Hz, 1H), 4.13-4.02 (m, 1H), 3.80 (s, 6H), 3.21-3.14 (m, 1H), 3.13-3.06 (m, 1H), 2.37-2.28 (m, 1H), 2.14 (td, *J* = 7.5, 15.6 Hz, 5H), 1.99-1.89 (m, 1H), 1.72-1.70 (m, 1H), 1.59-1.50 (m, 4H), 1.36-1.23 (m, 13H).

**Compound 5**

The compound **NAG0026** (120 mg, 0.079 mmol) was dissolved in anhydrous THF (2 mL) and anhydrous DMF (2 mL), and 3A molecular sieve (50 mg) was added. Then compound **4** (54 mg, 0.084 mmol), HOBt (28 mg, 0.205 mmol), DCC (39 mg, 0.189 mmol) and DIEA (0.08 mL, 0.472 mmol) were added sequentially. The reaction solution was left to react at 40 °C for 20 h. After the reaction was complete as shown by LC-MS, the reaction solution was quenched with water and filtered. The filtrate was concentrated under reduced pressure and purified by reversed-phase column chromatography (Boston C18 column, 0-100% $MeCN/H_2O$) to give compound **5** (90 mg, 53% yield).

**Compound NAG0046**

Compound **5** (90 mg, 0.042 mmol) was dissolved in anhydrous pyridine (3 mL), and 3A molecular sieve (50 mg), DMAP (26 mg, 0.209 mmol) and succinic anhydride (42 mg, 0.364 mmol) were added. The reaction solution was stirred at 50 °C for 48 h. LC-MS showed that about 50% of the starting material had been consumed. The reaction solution was filtered, concentrated, and purified by reversed-phase column chromatography (Boston C18 column, $H_2O$/MeCN, elution from 5% to 70%) to give **NAG0046** (30 mg).

MS (ESI) m/z = 2251.4 $[M-1]^-$, calculated: 2252.0.
$^1H$ NMR (400 MHz, Acetonitrile-$d_3$) δ 7.50-7.14 (m, 14H), 6.89 (d, *J* = 8.6 Hz, 7H), 6.56 (d, *J* = 7.8 Hz, 1H), 5.32 (d, *J* = 3.4 Hz, 3H), 5.09 (t, *J* = 12.5 Hz, 4H), 4.65 (dd, *J* = 8.6, 4.8 Hz, 3H), 4.32-3.11 (m, 52H), 2.54 (t, *J* = 6.8 Hz, 3H), 2.32 (d, *J* = 7.6 Hz, 16H), 2.14-2.04 (m, 17H), 1.89 (d, *J* = 2.9 Hz, 10H), 1.75 (dt, *J* = 14.6, 7.9 Hz, 4H), 1.62-1.49 (m, 5H), 1.29 (s, 13H).

**Preparation Example 3: Synthesis of Compound NAG0047**

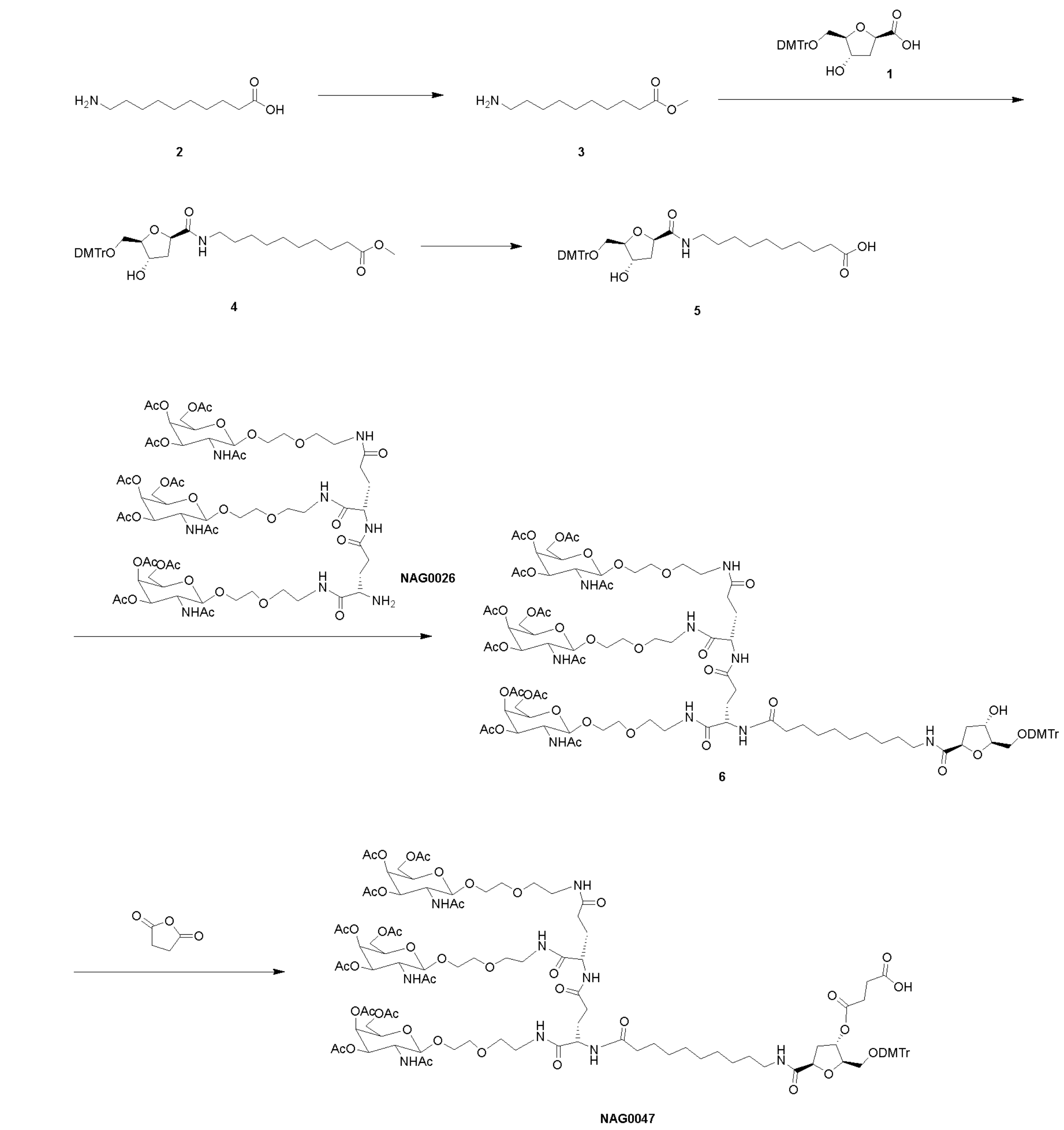

1 2 3 4 5 NAG0026 6 NAG0047

## Compound 3

To a solution of compound **2** (300 mg, 1.60 mmol) in MeOH (10 mL) at 0 °C in a nitrogen atmosphere was added dropwise $SOCl_2$ (0.581 mL, 8.009 mmol). The mixture was stirred at 60 °C for 3 h. The reaction solution was concentrated to give compound **3** (0.32 g, 1.510 mmol, 95% yield).

$^1H$ NMR: (400 MHz, $CD_3OD$) $\delta$ ppm 3.75-3.59 (m, 3H), 2.93 (t, $J$ = 7.2 Hz, 2H), 2.33 (t, $J$ = 7.4 Hz, 2H), 1.64 (td, $J$ = 7.0, 14.4 Hz, 4H), 1.48-1.27 (m, 10H).

## Compound 4

To a solution of compound **1** (250 mg, 0.538 mmol) in THF (25 mL) at 0 °C in a nitrogen atmosphere were added sequentially DIEA (0.267 mL, 1.61 mmol), 4A molecular sieve (500 mg), compound **3** (271 mg, 1.08 mmol), DCC (333 mg, 1.61 mmol) and HOBt (218 mg, 1.61 mmol). The reaction solution was stirred at 50 °C for 5 h. After the reaction was complete, the reaction solution was quenched with $H_2O$ (50 mL) and extracted with EtOAc (100 mL × 3). The combined organic phases were washed with saturated brine (50 mL), dried over $Na_2SO_4$, filtered, concentrated, and then purified by silica gel column chromatographed (0-80% EtOAc/PE) to give compound **4** (420 mg, 0.486 mmol, 90% yield).

LCMS: chromatographic conditions 10-80CD_7min, retention time 5.586 min; MS (ESI) m/z = 670.3 $[M+Na]^+$.
$^1H$ NMR: (400 MHz, $CD_3OD$) $\delta$ ppm 7.44 (d, $J$ = 7.6 Hz, 2H), 7.36-7.29 (m, 6H), 7.28-7.22 (m, 1H), 6.89 (d, $J$ = 8.8 Hz, 4H), 4.53 (t, $J$ = 7.8 Hz, 1H), 4.35-4.27 (m, 1H), 4.02 (q, $J$ = 4.0 Hz, 1H), 3.86-3.74 (m, 6H), 3.66 (s, 3H), 3.52-3.42 (m, 1H), 3.33-3.30 (m, 1H), 3.29-3.23 (m, 1H), 3.18 (td, $J$ = 7.0, 13.5 Hz, 1H), 3.00 (td, $J$ = 7.0, 13.5 Hz, 1H), 2.33-2.22 (m, 3H), 2.14 (ddd, $J$ = 5.8, 7.8, 13.3 Hz, 1H), 1.92-1.82 (m, 2H), 1.73 (td, $J$ = 3.6, 13.2 Hz, 2H), 1.66-1.53 (m, 3H), 1.43-1.26 (m, 7H).

**Compound 5**

To a solution of compound **4** (420 mg, 0.486 mmol) in THF (5 mL) and $H_2O$ (2 mL) at 20 °C was added LiOH (82 mg, 1.95 mmol). The reaction solution was stirred at 20 °C for 15 h. After the reaction was complete, the reaction solution was concentrated and purified by reversed-phase column chromatography (Boston C18 column, 0-100% $MeCN/H_2O$) to give compound **5** (236 mg, 0.155 mmol, 32% yield, lithium salt).

LCMS: chromatographic conditions 0-60CD_7min, retention time 3.950 min; MS (ESI) m/z = 656.4 $[M+Na]^+$.

**Compound 6**

The compound **NAG0026** (236 mg, 0.155 mmol) was dissolved in anhydrous THF (3 mL), and 3A molecular sieve (100 mg) was added. Then compound **5** (98 mg, 0.155 mmol), HOBt (25 mg, 0.186 mmol), DCC (41 mg, 0.201 mmol) and DIEA (0.08 mL, 0.464 mmol) were added sequentially. The reaction solution was left to react at 40 °C for 16 h. After the reaction was complete as shown by LC-MS, the reaction solution was quenched with water and filtered. The filtrate was concentrated and then purified by reversed-phase column chromatography (Boston C18 column, 0-100% $MeCN/H_2O$) to give compound **6** (160 mg, 0.075 mmol, 48% yield).

**Compound NAG0047**

Compound **6** (160 mg, 0.075 mmol) was dissolved in anhydrous pyridine (3 mL), and 3A molecular sieve (100 mg), DMAP (46 mg, 0.374 mmol) and succinic anhydride (75 mg, 0.747 mmol) were added sequentially. The reaction solution was stirred at 50 °C for 48 h. The reaction solution was filtered, concentrated, and purified by reversed-phase column chromatography (Boston C18 column, 0-100% $MeCN/H_2O$) to give **NAG0047** (80 mg).

MS (ESI) m/z = 2239.1 $[M-1]^-$, calculated: 2240.0.
$^1H$ NMR (400 MHz, Acetonitrile-$d_3$) $\delta$ 7.53-7.09 (m, 14H), 6.89 (dd, $J$ = 7.2, 5.1 Hz, 8H), 5.31 (dt, $J$ = 3.2, 1.5 Hz, 3H), 5.22-5.05 (m, 4H), 4.70-4.61 (m, 3H), 4.44 (dd, $J$ = 9.7, 6.7 Hz, 1H), 4.29 (dq, $J$ = 14.6, 7.6 Hz, 2H), 4.18-3.84 (m, 16H), 3.80 (s, 6H), 3.69 (dd, $J$ = 11.2, 4.3 Hz, 3H), 3.64-3.03 (m, 24H), 2.57 (h, $J$ = 2.3 Hz, 5H), 2.36-2.27 (m, 7H), 2.24-2.16 (m, 4H), 2.11 (s, 3H), 2.01-1.96 (m, 22H), 1.88 (q, $J$ = 2.9, 2.2 Hz, 8H), 1.56 (s, 2H), 1.24 (d, $J$ = 17.7 Hz, 13H).

**Preparation Example 4: Synthesis of Compound NAG0048**

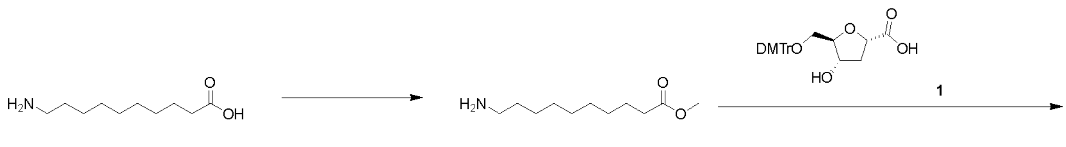

1

2 3

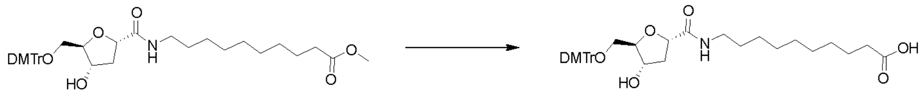

4 5

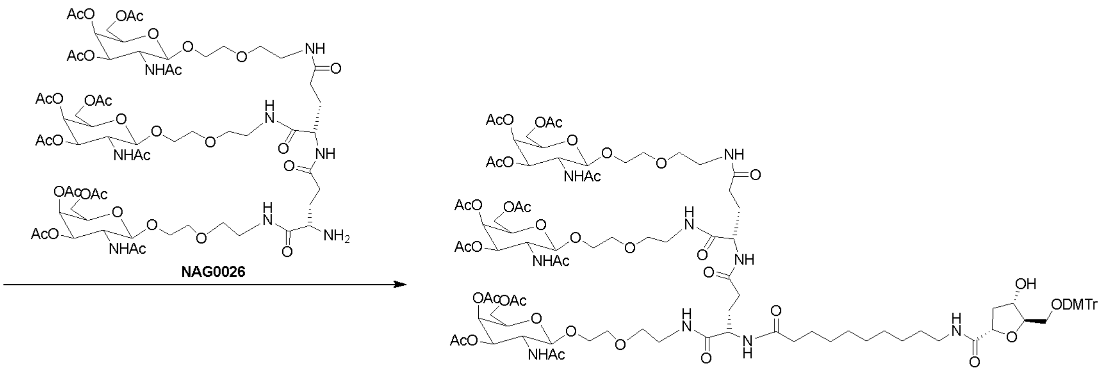

NAG0026

6

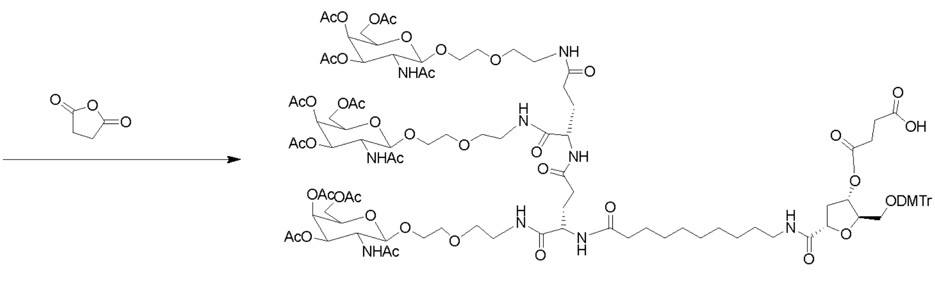

NAG0048

## Compound 3

To a solution of compound **2** (500 mg, 2.67 mmol) in MeOH (10 mL) at 0 °C in a nitrogen atmosphere was added dropwise $SOCl_2$ (0.968 mL, 13.3 mmol). The mixture was stirred at 60 °C for 12 h. The reaction solution was concentrated to give compound **3** (550 mg, 2.46 mmol, 92%).

$^1$H NMR: (400 MHz, $CD_3OD$) $\delta$ ppm 3.69-3.62 (m, 3H), 2.94 (s, 2H), 2.33 (t, $J$ = 7.2 Hz, 2H), 1.73-1.57 (m, 4H), 1.46-1.31 (m, 10H).

## Compound 4

To a solution of compound **1** (250 mg, 0.538 mmol) in THF (25 mL) at 0 °C in a nitrogen atmosphere were added sequentially DIEA (0.267 mL, 1.614 mmol), 4A molecular sieve (500 mg), compound **3** (271 mg, 1.08 mmol), DCC (333 mg, 1.61 mmol) and HOBt (218 mg, 1.61 mmol). The reaction solution was stirred at 50 °C for 5 h. After the reaction was complete, the reaction solution was quenched with $H_2O$ (50 mL) and extracted with EtOAc (100 mL × 3). The combined organic phases were washed with saturated brine (50 mL), dried over anhydrous $Na_2SO_4$, filtered, concentrated, and then purified by silica gel column chromatographed (0-80% EtOAc/PE) to give compound **4** (350 mg, 0.486 mmol, 90% yield).

LCMS: chromatographic conditions 10-80CD_7min, retention time 5.670 min; MS (ESI) m/z = 670.3 $[M+Na]^+$.
$^1H$ NMR: (400 MHz, $CD_3OD$) $\delta$ ppm 7.45 (d, *J* = 7.4 Hz, 2H), 7.36-7.27 (m, 6H), 7.25-7.19 (m, 1H), 6.90-6.85 (m, 4H), 4.50 (dd, *J* = 4.6, 9.0 Hz, 1H), 4.27 (td, *J* = 3.0, 5.6 Hz, 1H), 4.20-4.14 (m, 1H), 3.80 (s, 6H), 3.68-3.62 (m, 3H), 3.53-3.43 (m, 1H), 3.27-3.18 (m, 3H), 3.16-3.09 (m, 1H), 2.54 (ddd, *J* = 5.8, 8.8, 13.2 Hz, 1H), 2.31 (t, *J* = 7.4 Hz, 2H), 2.08 (td, *J* = 4.0, 13.2 Hz, 1H), 1.91-1.82 (m, 2H), 1.73 (td, *J* = 3.6, 13.2 Hz, 2H), 1.67-1.50 (m, 5H), 1.39-1.32 (m, 2H), 1.27-1.12 (m, 3H).

**Compound 5**

To a solution of compound **4** (350 mg, 0.486 mmol) in THF (5 mL) and $H_2O$ (2 mL) at 20 °C was added LiOH (82 mg, 1.95 mmol). The reaction solution was stirred at 20 °C for 15 h. After the reaction was complete, the reaction solution was directly concentrated to give compound **5** (300 mg, 0.469 mmol, 96% yield).
LCMS: chromatographic conditions 0-60CD_7min, retention time 4.081min; MS (ESI) m/z = 656.4 $[M+Na]^+$.

**Compound 6**

The compound **NAG0026** (300 mg, 0.197 mmol) was dissolved in anhydrous THF (5 mL), and 3A molecular sieve (100 mg) was added. Then compound **5** (125 mg, 0.197 mmol), HOBt (32 mg, 0.236 mmol), DCC (53 mg, 0.256 mmol) and DIEA (0.10 mL, 0.590 mmol) were added sequentially. The reaction solution was left to react at 40 °C for 16 h. After the reaction was complete as shown by LC-MS, the reaction solution was quenched with water and filtered. The filtrate was concentrated under reduced pressure and then purified by reversed-phase column chromatography (Boston C18 column, 0-100% $MeCN/H_2O$) to give compound **6** (260 mg, 62% yield).

**Compound NAG0048**

Compound **6** (260 mg, 0.121 mmol) was dissolved in anhydrous pyridine (4 mL), and 3A molecular sieve (100 mg), DMAP (30 mg, 0.242 mmol) and succinic anhydride (73 mg, 0.726 mmol) were added sequentially. The reaction solution was stirred at 50 °C for 48 h. LC-MS showed that about 70% of the starting material had been consumed. The reaction solution was filtered, concentrated, and purified by reversed-phase column chromatography (Boston C18 column, 0-100% $MeCN/H_2O$) to give **NAG0048** (170 mg, 62% yield).

MS (ESI) m/z = 2239.1 $[M-1]^-$, calculated: 2240.0.
$^1H$ NMR (400 MHz, Acetonitrile-$d_3$) $\delta$ 7.68 (s, 3H), 7.51-7.17 (m, 12H), 7.11-6.85 (m, 7H), 5.31 (dt, *J* = 2.5, 1.3 Hz, 3H), 5.18-5.04 (m, 4H), 4.74-4.53 (m, 4H), 4.30 (d, *J* = 4.9 Hz, 3H), 4.21-3.93 (m, 12H), 3.79 (s, 9H), 3.69 (dt, *J* = 10.4, 4.7 Hz, 3H), 3.62-3.14 (m, 22H), 3.09 (dd, *J* = 10.1, 4.4 Hz, 2H), 2.91 (d, *J* = 7.2 Hz, 1H), 2.46 (s, 6H), 2.28 (ddt, *J* = 28.8, 20.0, 7.2 Hz, 8H), 2.11 (s, 3H), 2.01-1.96 (m, 18H), 1.90-1.87 (m, 8H), 1.54 (d, *J* = 25.2 Hz, 4H), 1.37-1.18 (m, 16H).

**Preparation Example 5: Synthesis of Compound NAG0049**

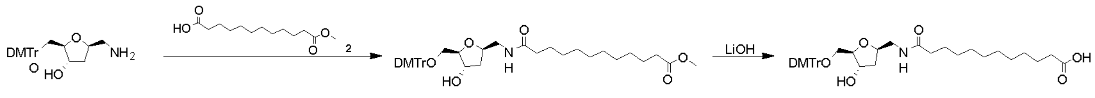


1 3 4

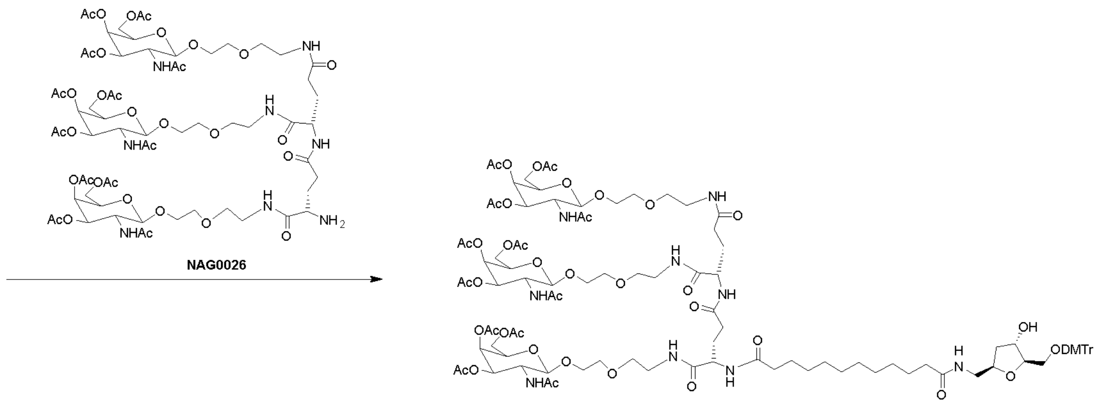


5

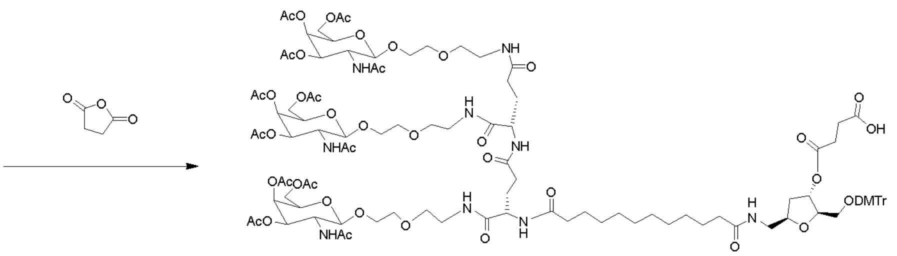

NAG0049

**Compound 3**

Compound **2** (293 mg, 1.20 mmol) was dissolved in DCM (3 mL), and DIEA (0.298 mL, 1.80 mmol) and HATU (457 mg, 1.20 mmol) were added. Then compound 1 (300 mg, 0.601 mmol, from Preparation Example 7) was added. The mixture was left to react at 20 °C for 1 h. The reaction solution was extracted with dichloromethane (60 mL) and water (60 mL). The organic phase was washed three times with water (60 mL × 3), dried over anhydrous sodium sulfate, filtered, and concentrated. The residue was purified by column chromatography (PE:EtOAc = 0:1) to give compound 3 (300 mg, 90% yield).

LCMS: chromatographic conditions 30-90CD_3min, retention time 2.225 min; MS (ESI) m/z = 698.4 $[M+Na]^+$.

**Compound 4**

Compound **3** (300 mg, 0.444 mmol) was dissolved in THF (3 mL) and $H_2O$ (1 mL), and $LiOH.E_2O$ (75 mg, 1.78 mmol) was added. The mixture was left to react at 20 °C for 12 h. The reaction solution was concentrated under reduced pressure, dissolved in water (5 mL) and methanol (5 mL), and purified by reversed-phase column chromatography (Boston C18 column, 0-100% $MeCN/H_2O$) to give compound 4 (212 mg, 100% yield).

LCMS: chromatographic conditions 10-80CD_3min, retention time 1.333 min; MS (ESI) m/z = 684.3 $[M+Na]^+$.
HPLC: chromatographic conditions 10-80CD_6min, retention time 1.853min.
$^1H$ NMR: (400 MHz, $CD_3OD$) $\delta$ ppm 7.47-7.38 (m, 2H), 7.35-7.25 (m, 6H), 7.24-7.17 (m, 1H), 6.86 (d, *J* = 8.8 Hz, 4H), 4.30-4.17 (m, 2H), 3.99-3.88 (m, 1H), 3.78 (s, 6H), 3.42-3.33 (m, 2H), 3.16-3.04 (m, 2H), 2.18-2.05 (m, 4H), 1.92-1.70 (m, 2H), 1.65-1.46 (m, 4H), 1.36-1.17 (m, 12H).

**Compound 5**

The compound **NAG0026** (300 mg, 0.197 mmol) was dissolved in anhydrous DMF (3 mL), and 3A molecular sieve (100 mg) was added. Then compound **4** (143 mg, 0.216 mmol), HOBt (32 mg, 0.236 mmol), DCC (53 mg, 0.256 mmol) and DIEA (0.1 mL, 0.590 mmol) were added sequentially. The reaction solution was left to react at 40 °C for 16 h. After the reaction was complete as shown by LC-MS, the reaction solution was quenched with water and filtered. The filtrate was concentrated and then purified by reversed-phase column chromatography (Boston C18 column, $H_2O$/MeCN, elution from 5% to 80%) to give compound **5** (110 mg, 28% yield).

**Compound NAG0049**

Compound **5** (110 mg, 0.051 mmol) was dissolved in anhydrous pyridine (3 mL), and 3A molecular sieve (100 mg), DMAP (31 mg, 0.255 mmol) and succinic anhydride (51 mg, 0.510 mmol) were added sequentially. The reaction solution was stirred at 50 °C for 48 h. LC-MS showed that about 50% of the starting material had been consumed. The reaction solution was filtered, concentrated, and purified by reversed-phase column chromatography (Boston C18 column, $H_2O$/MeCN, elution from 5% to 70%) to give **NAG0049** (45 mg, 39% yield).

MS (ESI) m/z = 2267.0 $[M-1]^-$, calculated: 2268.0.
$^1$H NMR (400 MHz, $CH_3CN$-$d_3$) δ 7.62-6.40 (m, 22H), 5.31 (s, 3H), 5.21-5.02 (m, 4H), 4.64 (dd, *J* = 8.7, 6.2 Hz, 3H), 4.16 (s, 2H), 4.13-3.94 (m, 14H), 3.79 (s, 10H), 3.72-3.38 (m, 24H), 3.32-3.11 (m, 5H), 2.56 (t, *J* = 3.4 Hz, 4H), 2.34-2.20 (m, 16H), 1.99 (d, *J* = 11.3 Hz, 24H), 1.41 (d, *J* = 127.6 Hz, 21H).

**Preparation Example 6: Synthesis of Compound NAG0050**

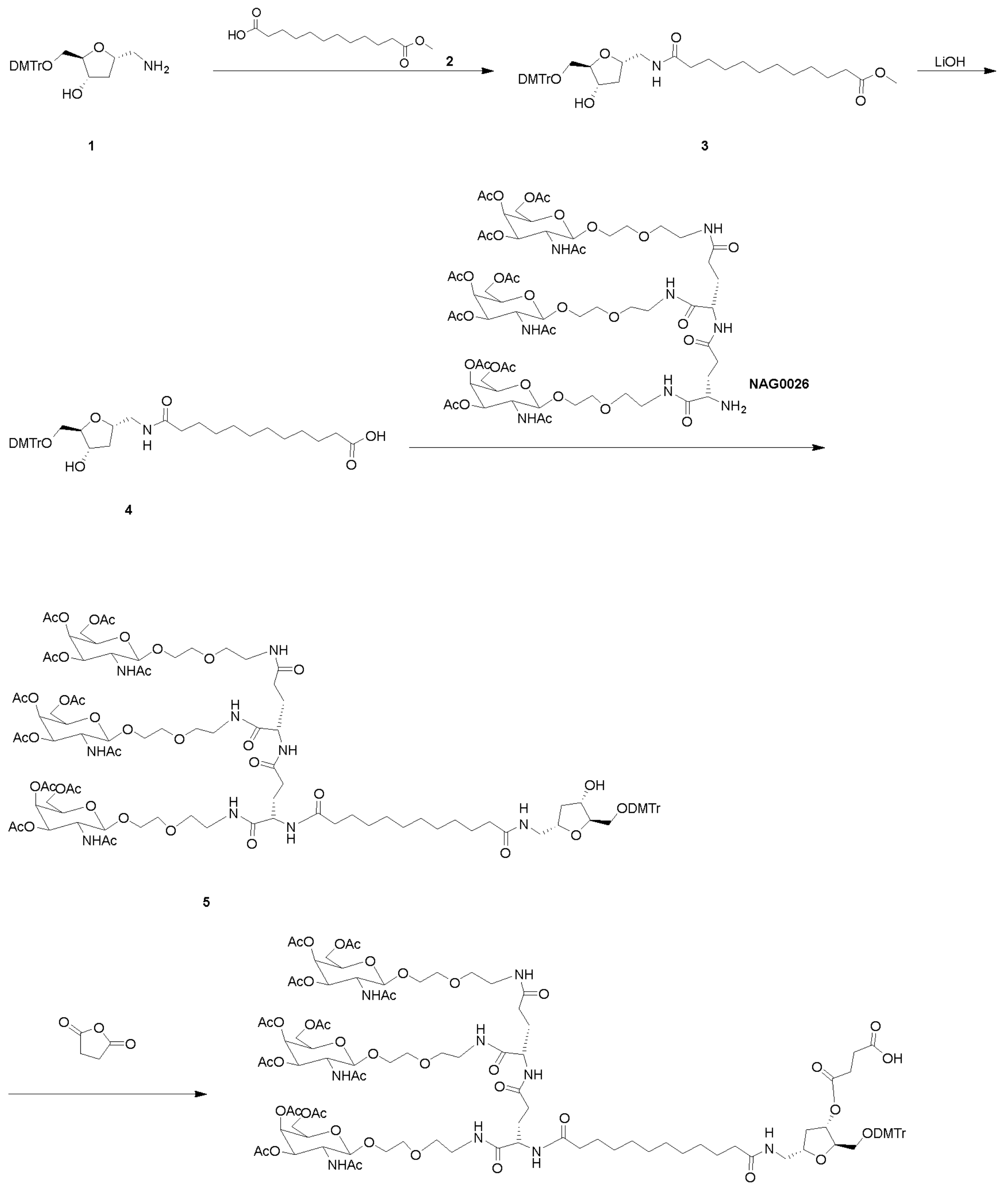


1

2

3

4

NAG0026

5

NAG0050

## Compound 3

Compound **2** (435 mg, 1.780 mmol) was dissolved in DCM (10 mL), and DIEA (0.441 mL, 2.67 mmol) and HATU (677 mg, 1.78 mmol) were added. Then compound 1 (400 mg, 0.890 mmol, from Preparation Example 8) was added. The mixture was left to react at 20 °C for 1 h. The reaction solution was extracted with dichloromethane (60 mL) and water (60 mL). The organic phase was washed three times with water (60 mL × 3), dried over anhydrous sodium sulfate, filtered, and concentrated. The residue was purified by column chromatography (PE:EtOAc = 0:1) to give compound **3** (600 mg, 90% yield).

LCMS: chromatographic conditions 30-90CD_3min, retention time 2.745 min; MS (ESI) m/z = 698.4 $[M+Na]^+$.
$^1H$ NMR: (400 MHz, $CD_3OD$) $\delta$ ppm 7.46-7.38 (m, 2H), 7.35-7.24 (m, 6H), 7.22-7.16 (m, 1H), 6.90-6.78 (m, 4H), 4.29-4.21 (m, 2H), 4.02-3.95 (m, 1H), 3.77 (s, 6H), 3.66-3.62 (m, 3H), 3.41 (s, 1H), 3.18-3.04 (m, 2H), 2.36-2.17 (m, 5H), 1.71-1.50 (m, 5H), 1.39-1.25 (m, 14H).

**Compound 4**

Compound **3** (600 mg, 0.799 mmol) was dissolved in THF (3 mL) and $H_2O$ (1 mL), and $LiOH.H_2O$ (134 mg, 3.20 mmol) was added. The mixture was left to react at 20 °C for 12 h. The reaction solution was concentrated under reduced pressure, dissolved in water (5 mL) and methanol (5 mL), and purified by reversed-phase column chromatography (Boston C18 column, 0-100% $MeCN/H_2O$) to give compound **4** (460 mg, 100% yield, lithium salt).

LCMS: chromatographic conditions 10-80CD_3min, retention time 1.346 min; MS (ESI) m/z = 684.3 $[M+Na]^+$.
HPLC: chromatographic conditions 10-80CD_6min, retention time 1.879 min.
$^1H$ NMR: (400 MHz, $CD_3OD$) $\delta$ ppm 7.47-7.39 (m, 2H), 7.35-7.24 (m, 6H), 7.22-7.15 (m, 1H), 6.91-6.79 (m, 4H), 4.31-4.18 (m, 2H), 4.02-3.95 (m, 1H), 3.78 (s, 6H), 3.44-3.33 (m, 2H), 3.18-3.04 (m, 2H), 2.35-2.27 (m, 1H), 2.24-2.10 (m, 4H), 1.70-1.51 (m, 5H), 1.31-1.23 (m, 12H).

**Compound 5**

The compound **NAG0026** (300 mg, 0.197 mmol) was dissolved in anhydrous DMF (3 mL), and 3A molecular sieve (100 mg) was added. Then compound **4** (143 mg, 0.216 mmol), HOBt (32 mg, 0.236 mmol), DCC (53 mg, 0.256 mmol) and DIEA (0.1 mL, 0.590 mmol) were added sequentially. The reaction solution was left to react at 40 °C for 16 h. After the reaction was complete as shown by LC-MS, the reaction solution was quenched with water and filtered. The filtrate was concentrated and then purified by reversed-phase column chromatography (Boston C18 column, $H_2O$/MeCN, elution from 5% to 80%) to give compound **5** (300 mg, 73% yield).

**Compound NAG0050**

Compound **5** (310 mg, 0.143 mmol) was dissolved in anhydrous pyridine (5 mL), and 3A molecular sieve (100 mg), DMAP (87 mg, 0.714 mmol) and succinic anhydride (143 mg, 1.429 mmol) were added sequentially. The reaction solution was stirred at 50 °C for 48 h. LC-MS showed that about 50% of the starting material had been consumed. The reaction solution was filtered, concentrated, and purified by reversed-phase column chromatography (Boston C18 column, H2O/MeCN, elution from 5% to 70%) to give **NAG0050** (140 mg, 43% yield).

MS (ESI) m/z = 2267.1 $[M-1]^-$, calculated: 2268.0.
$^1H$ NMR (400 MHz, $CH_3CN$-$d_3$) $\delta$ 7.54-7.14 (m, 14H), 6.97-6.64 (m, 8H), 5.32 (d, *J* = 3.4 Hz, 3H), 5.21-5.04 (m, 4H), 4.71-4.60 (m, 3H), 4.30 (d, *J* = 6.8 Hz, 3H), 4.17-3.95 (m, 14H), 3.93-3.84 (m, 3H), 3.79 (s, 7H), 3.74-3.65 (m, 3H), 3.63-3.08 (m, 26H), 2.57 (d, *J* = 2.0 Hz, 6H), 2.46-2.28 (m, 8H), 2.20 (dt, *J* = 15.1, 7.4 Hz, 5H), 2.11 (s, 2H), 2.01-1.97 (m, 16H), 1.90-1.88 (m, 9H), 1.65-1.55 (m, 4H), 1.39-1.20 (m, 14H).

**Preparation Example 7: Compound NAG0051**

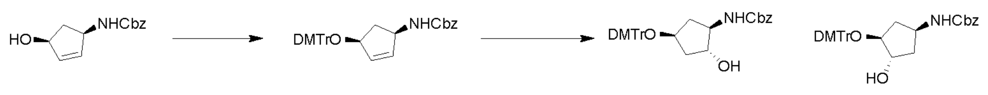

1 2 3 4

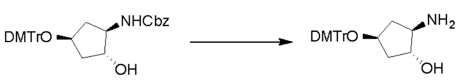

3 5

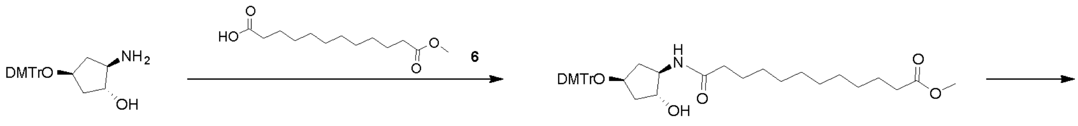

6

5 7

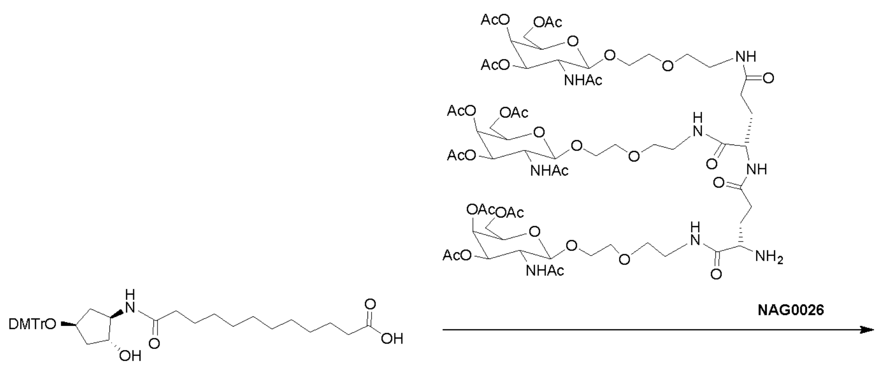

NAG0026

8

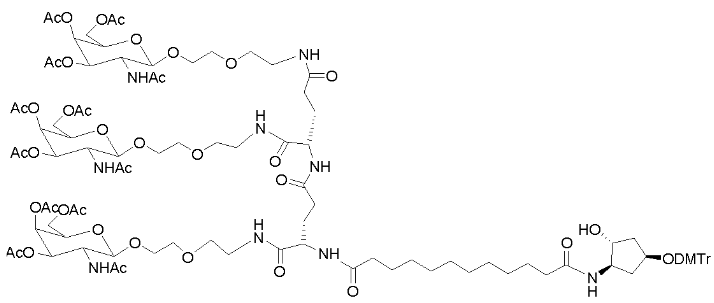

9

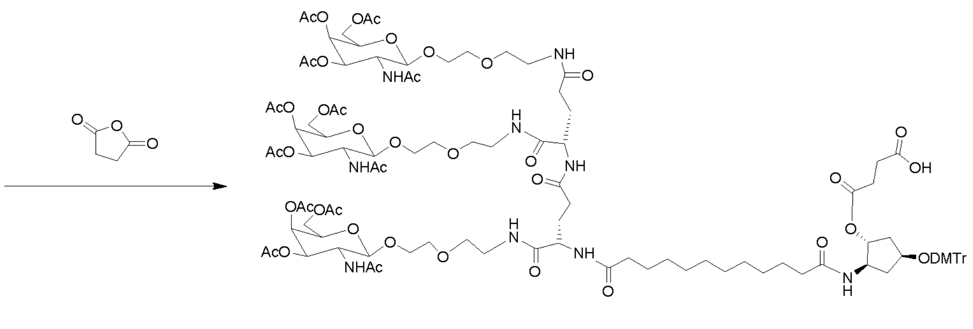

NAG0051

**Compound 2**

To the solvent dichloromethane (100 mL) were added compound **1** (5.00 g, 21.4 mmol) and TEA (8.04 mL, 57.9 mmol). DMTrCl (9.08 g, 26.8 mmol) was added slowly to the above system with stirring at 0 °C. After the addition, the mixture was heated to 25 °C and left to react for 12 h. Saturated sodium bicarbonate solution (100 mL) was added to the reaction solution, and the mixture was extracted with DCM (100 × 3). The organic phase was dried over anhydrous sodium sulfate, filtered, and concentrated. The residue was purified by column chromatography (PE:EtOAc 100:0-70:30) to give compound **2** (12 g, 94% yield).

LCMS: chromatographic conditions 5-95AB_1.5min, retention time 1.166 min; MS (ESI) m/z = 558.1 $[M+Na]^+$.
$^1$H NMR: (400 MHz, $CDCl_3$) $\delta$ ppm 7.52-7.47 (m, 2H), 7.42-7.35 (m, 8H), 7.34-7.27 (m, 3H), 7.26-7.19 (m, 1H), 6.89-6.81 (m, 4H), 5.68 (d, $J$ = 5.6 Hz, 1H), 5.18-5.08 (m, 3H), 4.83 (br d, $J$ = 9.2 Hz, 1H), 4.55-4.42 (m, 2H), 3.82 (s, 6H), 2.43-2.29 (m, 1H), 1.39-1.32 (m, 2H).

**Compound 3**

Compound **2** (6.00 g, 10.1 mmol) was added to the solvent THF (100 mL), and $BH_3THF$ (1 M, 20.2 mL, 20.2 mmol) was added to the above system at 0 °C. After the addition, the mixture was left to react at 20 °C for 12 h. The mixture was then cooled to 0 °C, and EtOH (6 mL) and NaOH (20 mL, 60.0 mmol) were slowly added in sequence at 0 °C. Finally, $H_2O_2$ (20 mL, 33.4 mmol) was slowly added dropwise to the reaction solution at 0 °C. The reaction solution was then slowly heated to 20 °C and left to react for 12 h. The reaction solution was filtered, and $H_2O$ (200 mL) and EtOAc (200 mL) were added. The mixture was extracted with EtOAc (3 × 200 mL), and the organic phase was washed with saturated $Na_2SO_3$ solution (100 mL), dried over anhydrous $Na_2SO_4$, filtered, and concentrated under reduced pressure. The resulting residue was purified by column chromatography (DCM/MeOH) to give a crude product (3.5 g), and the product was separated by basic preparative chromatography (column: Xbridge 150 × 50 mm, 5 μm; mobile phase: A: 0.1% $NH_3H_2O$ + 0.005% FA in water; B: MeCN; gradient: 20% B-95% B in 9 min) to give compound **3** (850 mg, 15% yield) and compound **4** (800 mg, 14% yield).

**Compound 3**

LCMS: chromatographic conditions 10-80AB_7min, retention time 4.214 min; MS (ESI) m/z = 576.3 $[M+Na]^+$.
$^1$H NMR: (400 MHz, $CDCl_3$) $\delta$ ppm 7.31-7.23 (m, 6H), 7.23-7.19 (m, 5H), 7.12-7.08 (m, 3H), 6.79-6.73 (m, 4H), 5.12 (s, 1H), 5.01 (s, 2H), 4.13 (s, 2H), 4.01-3.95 (m, 1H), 3.75-3.71 (m, 6H), 2.48-2.27 (m, 1H), 2.12-1.84 (m, 2H), 1.57 (br d, $J$ = 14.6 Hz, 1H).

**Compound 4**

LCMS: chromatographic conditions 10-80AB_7min, retention time 4.305 min; MS (ESI) m/z = 576.3 $[M+Na]^+$.
$^1$H NMR: (400 MHz, $CDCl_3$) $\delta$ ppm 7.37-7.33 (m, 1H), 7.31-7.19 (m, 11H), 7.15-7.08 (m, 2H), 6.78-6.69 (m, 4H), 5.08-4.98 (m, 3H), 4.39-4.05 (m, 2H), 3.71 (m, 6H), 3.54-3.36 (m, 1H), 2.37-1.97 (m, 1H), 1.87-1.65 (m, 2H), 1.14-0.99 (m, 1H).

**Compound 5**

Pd/C 5% (300 mg, 0.141 mmol) was added to a solution of compound **3** (600 mg, 1.030 mmol) in EtOAc (10 mL). The reaction solution was left to react at 20 °C for 2 h in a hydrogen atmosphere (15 psi). The reaction solution was filtered and concentrated to give a crude product, and the crude product was separated by basic preparative chromatography (column: Xbridge 150 × 50 mm, 5 μm; mobile phase: A: 0.1% $NH_3H_2O$ + 0.005% FA in water; B: MeCN; gradient: 20% B-95% B in 9 min) to give compound **5** (301 mg, 70% yield).

LCMS: chromatographic conditions 10-80CD_3min, retention time 2.444 min; MS (ESI) m/z = 839.5 $[2M+H]^+$.
HPLC: chromatographic conditions 10-80CD_7min, retention time 4.100 min.
$^1$H NMR: (400 MHz, $CD_3OD$) $\delta$ ppm 7.49-7.44 (m, 2H), 7.34 (d, $J$ = 8.8 Hz, 4H), 7.32-7.26 (m, 2H), 7.24-7.18 (m, 1H), 6.91-6.82 (m, 4H), 4.22-4.11 (m, 1H), 3.85-3.81 (m, 1H), 3.79 (s, 6H), 2.78-2.68 (m, 1H), 1.90-1.83 (m, 1H),

1.73-1.65 (m, 1H), 1.41-1.33 (m, 1H), 1.29-1.25 (m, 1H).

**Compound 7**

Compound **6** (233 mg, 0.953 mmol), HATU (272 mg, 0.715 mmol) and DIEA (0.236 mL, 1.430 mmol) were dissolved in DCM (10 mL), and compound **5** (200 mg, 0.477 mmol) was added. The mixture was left to react at 25 °C for 1 h. $H_2O$ (20 mL) was added, and the mixture was extracted with DCM (20 mL × 3). The organic phases were combined, dried over anhydrous sodium sulfate, and filtered. The filtrate was concentrated under reduced pressure, and the residue was separated by column chromatography (PE/EtOAc = 1/1-1/2) to give compound 7 (290 mg).

LCMS: chromatographic conditions 10-80CD_3min, retention time 2.834 min; MS (ESI) m/z = 668.4 $[M+Na]^+$.
$^1H$ NMR: (400 MHz, $CDCl_3$) $\delta$ ppm 7.49-7.44 (m, 2H), 7.41-7.30 (m, 6H), 7.25-7.18 (m, 1H), 6.89-6.79 (m, 4H), 4.30-4.07 (m, 2H), 3.87-3.79 (m, 7H), 3.73-3.64 (m, 4H), 2.8-2.80 (m, 7H), 2.32 (t, *J* = 7.6 Hz, 2H), 2.24-2.13 (m, 2H), 2.01-1.89 (m, 1H), 1.80-1.75 (m, 1H), 1.65-1.60 (m, 5H), 1.58 (s, 4H), 1.20-1.10 (m, 1H).

**Compound 8**

Compound 7 (290 mg, 0.404 mmol) was dissolved in THF (10 mL) and $H_2O$ (5 mL), and LiOH (51 mg, 1.212 mmol) was added. The mixture was left to react at 25 °C for 3 h. TLC (DCM/MeOH = 10/1) showed that about 20% of the starting material had not been consumed, and thus an additional 50 mg of LiOH was added. The mixture was left to react at 25 °C for 12 h, and TLC (DCM/MeOH = 10/1) showed that the starting material had been consumed completely. The reaction solution was concentrated, and the residue was separated by reversed-phase chromatography ($H_2O/CH_3CN$ = 5/1-3/1) to give compound **8** (217 mg).

LCMS: chromatographic conditions 5-95CDN_1.5min, retention time 0.879 min; MS (ESI) m/z = 630.3 $[M-H]^+$.
HPLC: chromatographic conditions 10-80CD_7min, retention time 2.677 min.
$^1H$ NMR: (400 MHz, $CD_3OD$) $\delta$ ppm 7.50-7.44 (m, 2H), 7.38-7.32 (m, 4H), 7.32-7.27 (m, 2H), 7.24-7.19 (m, 1H), 6.91-6.85 (m, 4H), 4.25-4.14 (m, 1H), 4.02-3.96 (m, 1H), 3.80 (s, 6H), 3.73-3.65 (m, 1H), 2.20-2.10 (m, 4H), 1.97-1.86 (m, 1H), 1.75-1.70 (m, 1H), 1.66-1.53 (m, 4H), 1.51-1.41 (m, 1H), 1.37-1.26 (m, 13H).

**Compound 9**

The compound **NAG0026** (300 mg, 0.197 mmol) was dissolved in anhydrous DMF (4 mL), and 3A molecular sieve (100 mg) was added. Then compound **8** (137 mg, 0.217 mmol), HOBt (32 mg, 0.236 mmol), DCC (53 mg, 0.256 mmol) and DIEA (0.10 mL, 0.591 mmol) were added sequentially. The reaction solution was left to react at 40 °C for 16 h. The reaction solution was quenched with water and filtered. The filtrate was concentrated and then purified by reversed-phase column chromatography (Boston C18 column, $H_2O$/MeCN, elution from 5% to 80%) to give compound **9** (330 mg).

**Compound NAG0051**

Compound **9** (330 mg, 0.154 mmol) was dissolved in anhydrous pyridine (5 mL), and 3A molecular sieve (100 mg), DMAP (94 mg, 0.771 mmol) and succinic anhydride (154 mg, 1.543 mmol) were added sequentially. The reaction solution was stirred at 50 °C for 48 h. LC-MS showed that about 60% of the starting material had been consumed. The reaction solution was filtered, concentrated, and purified by reversed-phase column chromatography (Boston C18 column, H2O/MeCN, elution from 5% to 70%) to give **NAG0051** (190 mg).

MS (ESI) m/z = 2237.3 $[M-1]^-$, calculated: 2238.0.
$^1H$ NMR (400 MHz, Acetonitrile-$d_3$) $\delta$ 7.54-7.16 (m, 14H), 7.01-6.53 (m, 8H), 5.32 (d, *J* = 5.1 Hz, 3H), 5.20-4.89 (m, 4H), 4.65 (q, *J* = 7.2 Hz, 3H), 4.38-3.20 (m, 52H), 2.51-2.44 (m, 4H), 2.36-2.20 (m, 20H), 2.02-1.97 (m, 20H), 1.64-1.20 (m, 22H).

**Preparation Example 8: Synthesis of NAG0052**

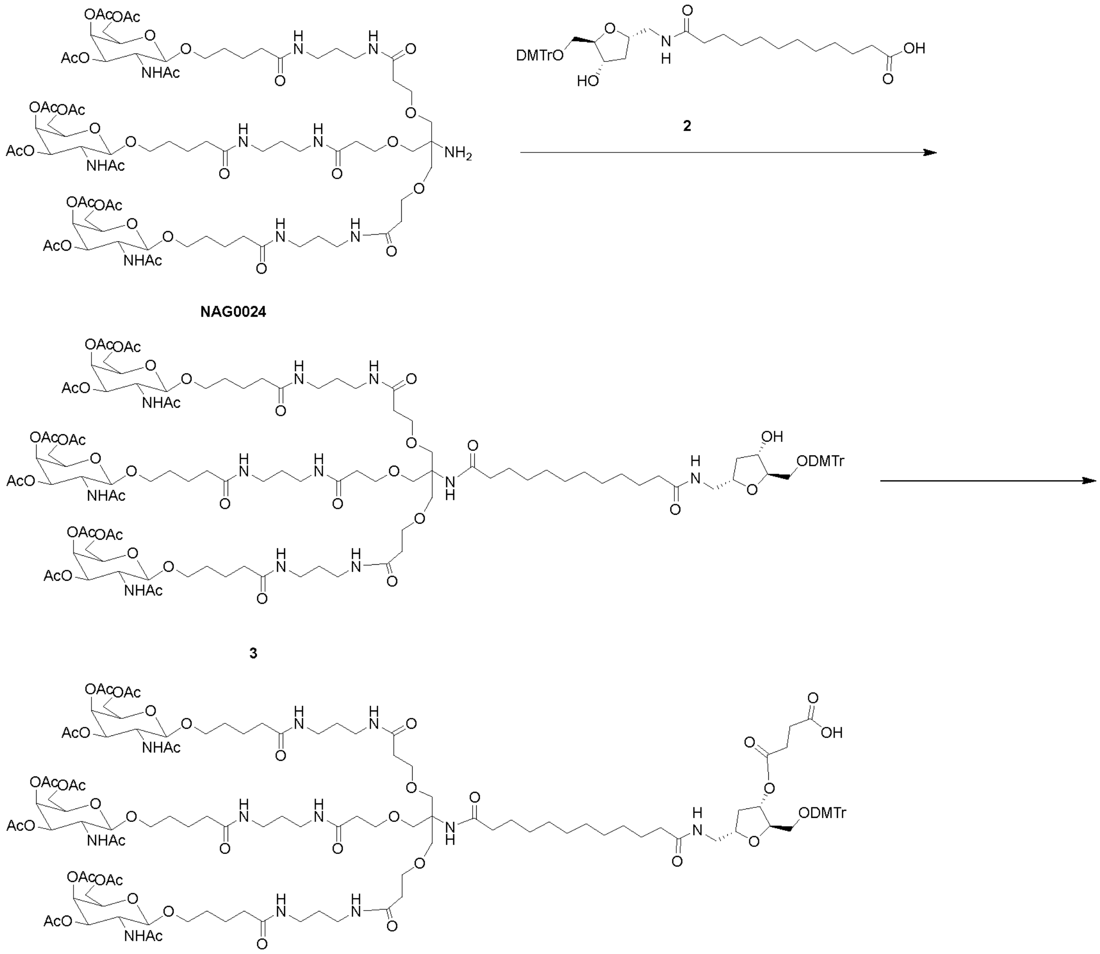
2
NAG0024
3
NAG0052

## Compound 3

The compound **NAG0024** (271 mg, 0.151 mmol) was dissolved in anhydrous THF (2 mL) and anhydrous DMF (4 mL) at room temperature in a nitrogen atmosphere, and 3A molecular sieve was added. Then compound **2** (100 mg, 0.151 mmol), HOBt (25 mg, 0.181 mmol), DCC (38 mg, 0.181 mmol) and DIEA (39 mg, 0.302 mmol) were added sequentially. The reaction solution was left to react at 45 °C for 16 h. After the reaction was complete as shown by LC-MS, the reaction solution was quenched with water and filtered. The filtrate was concentrated and then purified by reversed-phase column chromatography (Boston C18 column, 0-100% MeCN/$H_2O$) to give compound **3** (210 mg, 57% yield).

## Compound NAG0052

Compound **3** (230 mg, 0.094 mmol) was dissolved in pyridine (5 mL) at room temperature, and molecular sieve was added. DMAP (12 mg, 0.283 mmol) and succinic anhydride (28 mg, 0.283 mmol) were added. The mixture was stirred at 50 °C for 16 h in a nitrogen atmosphere. LCMS analysis showed that the reaction was complete. The reaction solution was filtered and concentrated. The residue was purified by reversed-phase column chromatography (Boston C18 column, 0-100% MeCN/$H_2O$) and then purified by preparative HPLC (column: Xbridge 150 $\times$ 50 mm, 5 $\mu$m; mobile phase: A: 0.1% $NH_3H_2O$ + 0.005% FA in water; B: MeCN; gradient: 20% B-95% B in 9 min) to give the compound **NAG0052** (123 mg, 0.048 mmol, 51% yield).

MS (ESI) m/z = 2535.3 $[M-1]^-$, calculated: 2536.2.
$^1H$ NMR (400 MHz, $CH_3CN$-$d_3$) $\delta$ 7.48-7.43 (m, 2H), 7.37-7.12 (m, 11H), 7.00-6.85 (m, 10H), 6.66 (s, 1H), 5.31 (dd, *J* = 3.4, 1.1 Hz, 3H), 5.20-5.13 (m, 1H), 5.05 (dd, *J* = 11.3, 3.4 Hz, 3H), 4.56 (d, *J* = 8.5 Hz, 3H), 4.30 (dd, *J* = 7.7, 5.3 Hz, 1H), 4.18-3.93 (m, 14H), 3.79 (s, 10H), 3.65 (q, *J* = 4.7, 3.6 Hz, 13H), 3.56-3.07 (m, 24H), 2.56 (s, 6H), 2.37

(t, $J$ = 5.8 Hz, 10H), 2.17 (t, $J$ = 7.5 Hz, 9H), 2.02-1.96 (m, 20H), 1.88 (s, 8H), 1.82-1.73 (m, 2H), 1.60 (dt, $J$ = 15.0, 7.3 Hz, 16H), 1.27 (s, 13H).

**Preparation Example 9: Synthesis of L96 and NAG1**

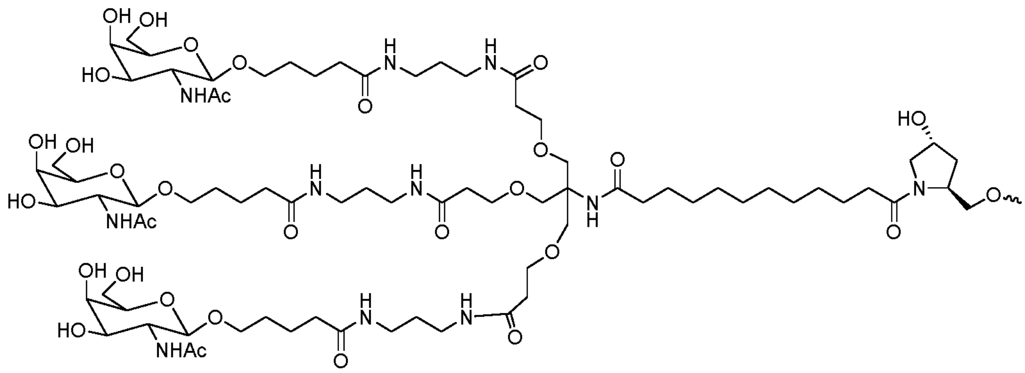

L96

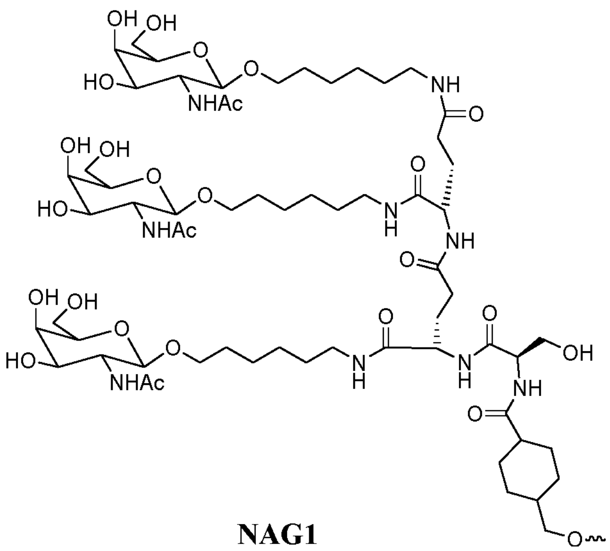

NAG1

L96 was prepared according to the method described in the patent application WO2014025805A1, and NAG1 was prepared according to the method described in the patent application WO2021254360A1. These patent applications are incorporated herein by reference in their entirety.

**Preparation Example 10: Synthesis of Nucleic Acid Ligand Conjugates**

1. Preparation of resin with support

The carboxylic acid group-containing compound **NAG0050** (140 mg, 0.062 mmol) was dissolved in anhydrous DMF (3 mL). After the substrate was completely dissolved, anhydrous acetonitrile (4 mL), DIEA (0.03 mL, 0.154 mmol, 2.5 eq) and HBTU (35 mg, 0.093 mmol, 1.5 eq) were sequentially added. After the reaction solution was mixed well, macroporous aminomethyl resin (476 mg, blank loading 0.41 mmol/g, target loading 0.1 mmol/g) was added. The reaction solution was shaken on a shaker (temperature: 25 °C; rotational speed: 200 rpm) overnight. The reaction solution was filtered. The filter cake was washed with DCM and then with anhydrous acetonitrile. The solid was collected and dried under vacuum overnight.

The solid was then dispersed in anhydrous acetonitrile (5 mL), and pyridine (0.18 mL), DMAP (3 mg), NMI (0.12 mL) and CapB1 (2.68 mL) were added sequentially. The reaction solution was shaken on a shaker (temperature:

25 °C; rotational speed: 200 rpm) for 2 h. The reaction solution was filtered. The filter cake was washed with anhydrous acetonitrile. The solid was collected and dried under vacuum overnight to give resin with a support. The loading was measured at 0.1 mmol/g.

The compounds **NAG0051** and **NAG0052** were subjected to the same reaction conditions to give resin with a support. 2. For **NAG0050-NAG0052** that had been attached to the resin, the resin was used as a start, and nucleoside monomers were attached one by one in the 3'-5' direction in the order in which nucleotides are arranged. Each time a nucleoside monomer was attached, four reactions-deprotection, coupling, capping, and oxidation or sulfurization-were involved.

Instrument models: Biolytic Dr. Oligo 48 solid-phase synthesizer, Biocomma Embed™ CPG Frits universal synthesis column DS0200, and Biocomma 96-well plate desalting column DC189650 (80 mg).

Table 1. Reagents used in the synthesis of siRNA conjugates

| Reagent | Composition | Specification | Use | Manufacturer |
|---|---|---|---|---|
| ACT | 0.6 M ETT in ACN | 4 L | Catalyst | Kroma |
| Cap A | N-Methylimidazole:acetonitrile 2:8 | 4 L | Capping reagent | Kroma |
| Cap B1 | Acetic anhydride:acetonitrile 40:60 | 4 L | | Kroma |
| Cap B2 | Pyridine:acetonitrile 60:40 | 4 L | | Kroma |
| OXD | 50 nM iodine in pyridine:water 90:10 | 4 L | Oxidizing agent | Kroma |
| DCA | Dichloroacetic acid in dichloromethane (3% v/v) | 1 kg | DMT-removing protective reagent | Kroma |
| Acetonitrile | Water content < 20 ppm (around 10 ppm in reality) | 4 1 | Solvent | Kroma |
| PADS (diphenylacetyl disulfide) | After mixing an equal volume of trimethylpyridine and acetonitrile, prepare a 0.05 M solution of PADS | | Sulfurizing reagent | Kroma |
| Trimethylpyridine | | | | Kroma |

Synthesis conditions:

Nucleoside monomers were provided in the form of 0.05 M solutions in acetonitrile.

The conditions for each deprotection reaction were the same: the temperature was 25 °C; the reaction time was 3 minutes; the deprotection reagent was DCA; and the injection volume was 180 μL.

The conditions for each coupling reaction were the same: the temperature was 25 °C; the reaction time was 3 minutes; the injection volume of nucleoside monomers was 90 μL; and the injection volume of the catalyst ACT was 110 μL.

The conditions for each capping reaction were the same: the temperature was 25 °C; the reaction time was 2 minutes; the capping reagent was a 1:1 (molar ratio) mixed solution of CapA and CapB (CapB1:CapB2 = 1:1); and the injection volume of the capping reagent was 180 μL.

The conditions for each oxidation reaction were the same: the temperature was 25 °C; the reaction time was 3 minutes; and the injection volume of the oxidizing reagent OXD was 180 μL.

The conditions for each sulfurization reaction were the same: the temperature was 25 °C; the reaction time was 4 minutes; the sulfurizing reagent was a 0.05 M solution of PADS in pyridylacetonitrile; and the injection volume of the sulfurizing reagent was 180 μL.

3. After the last nucleoside monomer was attached, the nucleic acid sequence attached to the solid-phase support was cleaved, deprotected, purified, desalted, and then lyophilized to give a sense strand and an antisense strand, wherein:

3-1. Cleavage and deprotection conditions: The synthesized nucleotide sequence attached to the support was added to a mixed solution of ammonia water and ethanol
(3:1) until the volume was 0.8 mL. A reaction was carried out at 50 °C for 15 h. The remaining support was removed by filtration, and the supernatant was concentrated under vacuum to dryness.

3-2. Purification and desalting conditions: Desalting was performed using Biocomma 96-well plate desalting column DC189650 (80 mg). The specific conditions include:

3-2-1. Sample preparation
0.1 M TEAA (triethylamine acetate) was added to the oligonucleotide sample until the volume was 0.8 mL.
3-2-2. 96-well plate activation

Activation: 0.8 mL of acetonitrile was added to each well of the 96-well plate for activation.
Equilibration: The 96-well plate was equilibrated with 0.8 mL of TEAA (pH 7.0) solution.

3-2-3. Purification process:
0.8 mL of solution containing oligonucleotides was passed through the desalting column; the 96-well plate was washed twice with 0.8 mL of 6.5% ammonia water to remove failed sequences; the 96-well plate was rinsed twice with 0.8 mL of deionized water to remove salt; the 96-well plate was rinsed 3 times with 0.8 mL of 3% trifluoroacetic acid to remove DMT, and the adsorption layer was observed to turn orange-red; the 96-well plate was rinsed with 0.8 mL of 0.1 M TEAA; the 96-well plate was washed twice with 0.8 mL of deionized water to remove trifluoroacetic acid and residual salt; elution was performed with 0.6 mL of 20% acetonitrile, and the eluate was collected and lyophilized.

Detection method: Using Waters Acquity UPLC-SQD2 LCMS (column: ACQUITY UPLC BEH C18), the purity of the sense and antisense strands described above was determined and their molecular weight was analyzed. The found values agreed with the calculated values, which indicates that what had been synthesized were sense and antisense strands whose 3' ends are conjugated to conjugation molecules.

4. Annealing procedure:

The sense and antisense strands synthesized in step 3 were dissolved in water for injection to form 1000 ng/μL solutions, and the solutions were mixed in equimolar ratio on a QPCR instrument (Applied Biosystems QuantStudio 6&7 Pro), heated at 90 °C for 10 min, held for 3 min following every 5 °C decrease, and finally held at 25 °C for 10 min, so that they formed a double-stranded structure through hydrogen bonds. The found values agreed with the calculated values, which indicates that the synthesized siRNA conjugates were the designed double-stranded nucleic acid sequences with conjugation molecules. The siRNAs have the sense and antisense strands shown in Table 2 and Table 3.

Table 2

| Nucleic acid ligand conjugate No. | siRNA conjugate sense strand No. | siRNA conjugate antisense strand No. |
|---|---|---|
| TRD002218 | TJR4373-SS | TJR0414-AS |
| TRD007203 | TJR013483S | TJR0414-AS |
| TRD007204 | TJR013484S | TJR0414-AS |
| TRD007205 | TJR013485S | TJR0414-AS |

Table 3. The nucleic acid sequences of the sense and antisense strands

| | | Sequence direction 5'-3' |
|---|---|---|
| **Sense strand** | TJR4373-SS | CmsAmsGm UmGfUm UfCfUf UmGmCm UmCmUm AmUmAm Am-**L96** |
| | TJR013483S | CmsAmsGm UmGfUm UfCfUf UmGmCm UmCmUm AmUmAms Ams-**NAG0050'** |
| | TJR013484S | CmsAmsGm UmGfUm UfCfUf UmGmCm UmCmUm AmUmAms Ams-**NAG0051'** |
| | TJR013485S | CmsAmsGm UmGfUm UfCfUf UmGmCm UmCmUm AmUmAms Ams-**NAG0052'** |
| **Antisense strand** | TJR0414-AS | UmsUfsAm UmAmGf AmGmCm AmAmGm AmAfCm AfCmUm GmsUmsUm |

The structures of the above conjugates are as follows:

| siRNA conjugate sense strand No. | Structure |
|---|---|
| TJR4373-SS | 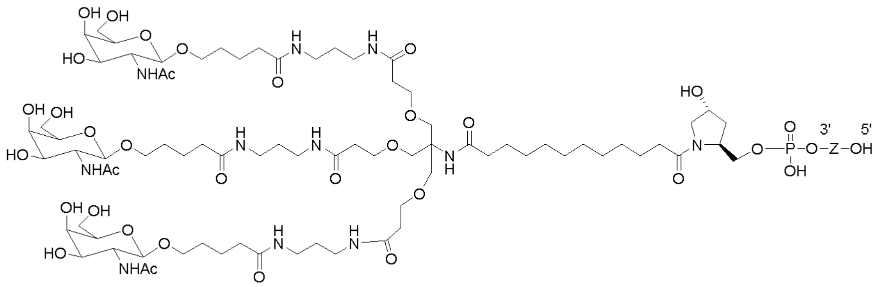 |
| TJR013483S | 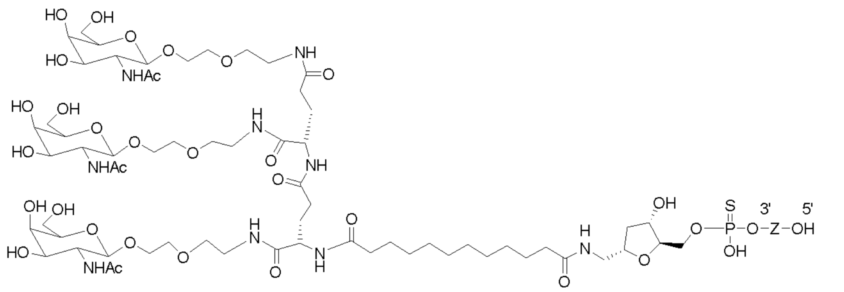 |
| TJR013484S | 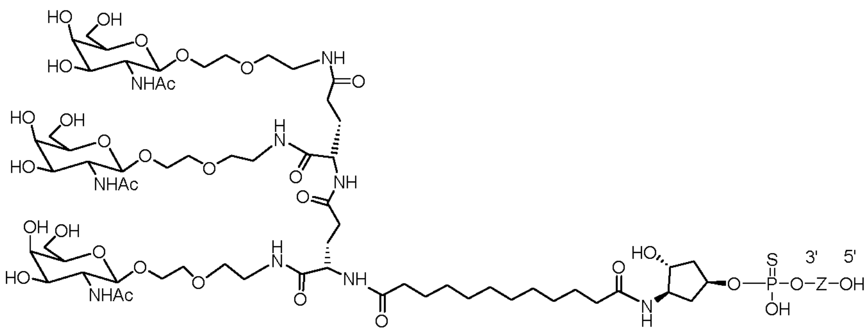 |

(continued)

| siRNA conjugate sense strand No. | Structure |
|---|---|
| TJR013485S | 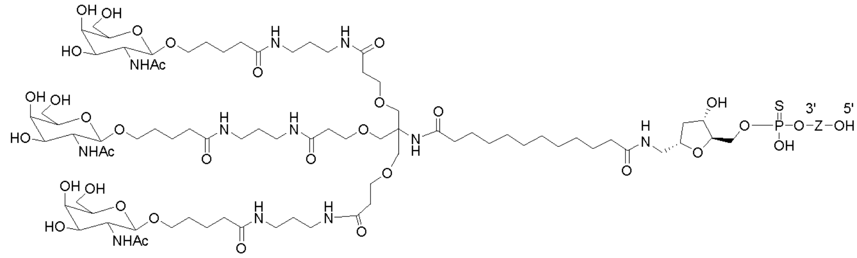 |

The conjugate TRD002218 was used as a reference positive compound.

**Test Example 1**

In this experiment, the inhibition efficiency of the siRNA conjugates of the present disclosure, which were conjugated with different structures, was investigated *in vivo* against the target gene mRNA expression level.

Six-to-eight-week-old male C57BL/6 mice were randomly divided into groups of 6, with 3 mice per time point. These groups of mice were administered the conjugates of the present disclosure (3 conjugates: TRD007203, TRD007204 and TRD007205), the reference positive nucleic acid-ligand conjugate TRD002218 and PBS.

All animals were administered a single subcutaneous injection according to their body weight. The doses of the siRNA conjugates (on an siRNA basis) were 1 mg/kg, and the volume was 5 mL/kg. The mice were sacrificed 7 days and 28 days after administration, and their livers were collected and stored with RNA later (Sigma Aldrich). Then, the liver tissue was homogenized using a tissue homogenizer, and the total RNA was extracted from the liver tissue using a tissue RNA extraction kit (FireGen Biomedicals, FG0412) by following the procedure described in the instructions. The total RNA was reverse-transcribed into cDNA, and the TTR mRNA expression level in liver tissue was measured by real-time fluorescence quantitative PCR. In the fluorescence quantitative PCR method, the glyceraldehyde 3-phosphate dehydrogenase (GAPDH) gene was used as an internal reference gene, and the TTR and GAPDH mRNA expression levels were measured using Taqman probe primers for TTR and GAPDH, respectively.

Table 4. Grouping for the compounds used in the *in vivo* test in mice

| Compound No. | Dose | mRNA quantification | Number of animals | Note |
|---|---|---|---|---|
| PBS | - | D7, 28 | 6 | 3 mice per time point |
| TRD002218 | 1 mpk s.c. | D7, 28 | 6 | 3 mice per time point |
| TRD007203 | 1 mpk s.c. | D7, 28 | 6 | 3 mice per time point |
| TRD007204 | 1 mpk s.c. | D7, 28 | 6 | 3 mice per time point |
| TRD007205 | 1 mpk s.c. | D7, 28 | 6 | 3 mice per time point |

Table 5. The sequences of detection primers

| Primer name | Forward primer |
|---|---|
| mTTR-F | GGGAAGACCGCGGAGTCT |
| mTTR-R | CAGTTCTACTCTGTACACTCCTTCTACAAA |
| mTTR-P | 5'6-FAM-CTGCACGGGCTCACCACAGATGA-3'BHQ1 |
| mGAPDH-F | CGGCAAATTCAACGGCACAG |
| mGAPDH-R | CCACGACATACTCAGCACCG |
| mGAPDH-P | 5 TET-ACCATCTTCCAGGAGCGAGACCCCACT-3 BHQ2 |

The TTR mRNA expression level was calculated according to the equation below:

TTR mRNA expression = [(TTR mRNA expression in test group/GAPDH mRNA expression in test group)/(TTR mRNA expression in control group/GAPDH mRNA expression in control group)] × 100%

The inhibition efficiencies of the siRNA conjugates of the present disclosure, which are conjugated with different structures, against the target gene mRNA expression level *in vivo* 7 days and 28 days after administration are shown in FIG. 1 and FIG. 2, respectively. As can be seen from the results in FIG. 1, both the conjugate TRD007203 of NAG0050 and the conjugate TRD007205 of NAG0052 well inhibited TTR mRNA expression 7 days after administration, and the conjugate TRD007203 of NAG0050 produced a significantly better effect than the conjugate TRD002218 of L96, indicating that it can mediate more efficient siRNA delivery. As can be seen from FIG. 2, 28 days after administration, the conjugate TRD007203 of NAG0050, the conjugate TRD007204 of NAG0051 and the conjugate TRD007205 of NAG0052 all produced better inhibitory effects on the target gene mRNA expression level than the conjugate TRD002218 of L96, and the superiority of the conjugate TRD007203 of NAG0050 is most significant.

**Preparation Example 11: Synthesis of Nucleic Acid Ligand Conjugates**

The following siRNA conjugates were synthesized by referring to the procedures of Preparation Example 10. The siRNAs have the sense and antisense strands shown in Tables 6 and 7.

Table 6. Nucleic acid compounds

| Gene | siRNA conjugate No. | siRNA conjugate sense strand No. | siRNA conjugate antisense strand No. |
|---|---|---|---|
| FXI | TRD008002 | TJR014937S | TJR013318A |
| | TRD008003 | TJR014938S | TJR013314A |
| ANGPTL3 | TRD008004 | TJR014939S | TJR013287A |
| | TRD008005 | TJR014940S | TJR013288A |
| | TRD008006 | TJR014941S | TJR013289A |
| MARC 1 | TRD008024 | TJR014963S | TJR014959A |
| | TRD008025 | TJR014964S | TJR014960A |
| | TRD008026 | TJR014965S | TJR014961A |
| | TRD008027 | TJR014966S | TJR014962A |
| | TRD6233 | TJR12296-SS | TJR12338-AS |
| | TRD6238 | TJR12301-SS | TJR12343-AS |
| | TRD6253 | TJR12316-SS | TJR12358-AS |

The conjugates TRD6233, TRD6238 and TRD6253 were used as reference positive compounds.

Table 7. The nucleic acid sequences of the sense and antisense strands

| | **No.** | **Sequence direction 5'-3'** |
|---|---|---|
| **Sense** | TJR014937S | CmsUmsUm GmCfAm AfCfAf AmAmGm AmCmAm |

(continued)

| | No. | Sequence direction 5'-3' |
|---|---|---|
| strand | | UmUmUm Am-NAG0052' |
| | TJR014938S | UmsCmsAm GmGfAm UfGfAf UmUmUm UmCmUm UmAmUm Um-NAG0052' |
| | TJR014939S | GmsAmsAm GmAfGm CfAfAf CmUmAm AmCmUm AmAmCm Um-NAG0052' |
| | TJR014940S | AmsGmsGm UmAfAm AfGfAf AmUmAm UmGmUm CmAmCm Um-NAG0052' |
| | TJR014941S | AmsGmsUm GmAfAm GfCfAf AmUmCm UmAmAm UmUmAm Um-NAG0052' |
| | TJR014963S | GmsUmsAm UmGfUm CfCfUf GmGmAm AmUmAm UmUmAm Am-NAG0052' |
| | TJR014964S | AmsCmsAm AmGfAm CfAfGf GmAmUm UmCmUm GmAmAm Am-NAG0052' |
| | TJR014965S | CmsUmsCm UmAfAm GfAfUf CmUmGm AmUmGm AmAmGm Um-NAG0052' |
| | TJR014966S | AmsGmsUm UmGfAm CfUfAf AmAmCm UmUmGm AmAmAm Am-NAG0052' |
| | TJR12296-SS | GmsUmsAm UmGfUm CfCfUf GmGmAm AmUmAm UmUmAm Am-NAG1 |
| | TJR12301-SS | AmsCmsAm AmGfAm CfAfGf GmAmUm UmCmUm GmAmAm Am-NAG1 |
| | TJR12316-SS | CmsUmsCm UmAfAm GfAfUf CmUmGm AmUmGm AmAmGm Um-NAG1 |

(continued)

| | No. | Sequence direction 5'-3' |
|---|---|---|
| Antisense strand | TJR013318A | UmsAfsAm AfUmGf U(036)CmUm UfUmGf UmUfGm CfAmAf GmsCmsGm |
| | TJR013314A | UmsAfsUm AfAmGf A(036)AmAm AfUmCf AmUfCm CfUmGf AmsAmsAm |
| | TJR013287A | AmsGfsUm UfAmGf U(036)UmAm GfUmUf GmCfUm CfUmUf CmsUmsAm |
| | TJR013288A | AmsGfsUm GfAmCf A(036)UmAm UfUmCf UmUfUm AfCmCf UmsCmsUm |
| | TJR013289A | AmsUfsAm AfUmUf A(036)GmAm UfUmGf CmUfUm CfAmCf UmsAmsUm |
| | TJR014959A | UmsUfsAm AfUmAf U(036)UmCm CfAmGf GmAfCm AfUmAf CmsGmsGm |
| | TJR014960A | UmsUfsUm CfAmGf A(036)AmUm CfCmUf GmUfCm UfUmGf UmsCmsAm |
| | TJR014961A | AmsCfsUm UfCmAf U(036)CmAm GfAmUf CmUfUm AfGmAf GmsUmsUm |
| | TJR014962A | UmsUfsUm UfCmAf A(036)GmUm UfUmAf GmUfCm AfAmCf UmsUmsCm |
| | TJR12338-AS | UmsUfsAm AfUmAf U(036)UmCf CmAmGf GmAfCm AfUmAf CmsGmsGm |
| | TJR12343-AS | UmsUfsUm CfAmGf A(036)AmUf CmCmUf GmUfCm UfUmGf UmsCmsAm |
| | TJR12358-AS | AmsCfsUm UfCmAf U(036)CmAf GmAmUf CmUfUm AfGmAf GmsUmsUm |

036 represents the (-)hmpNA(A) modification (bases change with the sequence) disclosed in WO2022028462A1.

**Test Example 2. siRNA Sequence psiCHECK 11 Concentration Point On-Target Activity**

The siRNA sequences in Table 6 were subjected to an *in vitro* molecular-level simulation of on-target activity screening in HEK293A cells (Nanjing Cobioer) using 11 concentrations.

Corresponding on-target siRNA sequences were constructed from the human FXI, ANGPTL3 and MARC1 genes and inserted into psiCHECK-2 plasmids (Sangon Biotech Co., Ltd.). The plasmids contained the renilla luciferase gene and the firefly luciferase gene. The plasmids were dual reporter gene systems. The target sequence of siRNA was inserted into the 3' UTR region of the renilla luciferase gene. The activity of siRNA for the target sequence was reflected by measuring the renilla luciferase expression after calibration with firefly luciferase. The measurement used Dual-Luciferase Reporter Assay System (Promega, E2940).

HEK293A cells were cultured at 37 °C with 5% $CO_2$ in a DMEM high glucose medium containing 10% fetal bovine serum. 24 h prior to transfection, the HEK293A cells were inoculated onto a 96-well plate, each well of which contained 100 μL of medium, at a density of 8 × $10^3$ cells per well.

The cells were co-transfected with the siRNAs and the corresponding plasmids using Lipofectamine2000 (ThermoFisher, 11668019); 0.2 μL of Lipofectamine2000 and 20 ng of plasmids were used per well. For the on-target sequence plasmids, a total of 11 concentration points of siRNA were set up. The highest concentration point final concentration was 20 nM, and 3-fold serial dilution was carried out. 24 h after transfection, the on-target levels were determined using Dual-Luciferase Reporter Assay System (Promega, E2940). Data were analyzed using GraphPad Prism5, and the results are shown in Table 8.

Table 8. siRNA sequence psi-CHECK on-target activity screening results

| siRNA conjugate No. | Remaining percentages of target gene mRNA (GSCM) expression (mean) at different siRNA concentrations | | | | | |
|---|---|---|---|---|---|---|
| | 20 nM | 6.6667 nM | 2.2222 nM | 0.7407 nM | 0.2469 nM | 0.0823 nM |
| TRD008002 | 6.4% | 5.9% | 6.5% | 7.8% | 9.7% | 15.0% |
| TRD008003 | 3.9% | 3.4% | 3.8% | 4.1% | 5.1% | 9.2% |
| TRD008004 | 5.4% | 4.9% | 4.0% | 5.6% | 6.8% | 17.5% |
| TRD008005 | 5.5% | 3.7% | 3.7% | 5.0% | 5.5% | 12.7% |
| TRD008006 | 4.2% | 3.1% | 3.3% | 3.8% | 5.7% | 10.6% |
| TRD008024 | 26.91% | 20.16% | 18.02% | 16.21% | 17.50% | 25.18% |
| TRD008025 | 24.41% | 21.09% | 22.07% | 18.86% | 20.28% | 29.57% |
| TRD008026 | 10.66% | 10.26% | 9.51% | 11.47% | 15.20% | 24.28% |
| TRD008027 | 20.33% | 15.44% | 15.61% | 14.39% | 15.31% | 27.01% |
| siRNA conjugate No. | Remaining percentages of target gene mRNA (GSCM) expression (mean) at different siRNA concentrations | | | | | GSCM $IC_{50}$ value (nM) |
| | 0.0274 nM | 0.0091 nM | 0.0030 nM | 0.0010 nM | 0.0003 nM | |
| TRD008002 | 34.7% | 74.2% | 93.6% | 98.7% | 93.7% | 0.0179 |
| TRD008003 | 20.2% | 54.0% | 77.3% | 96.3% | 88.0% | 0.0099 |
| TRD008004 | 38.0% | 73.3% | 97.3% | 102.2% | 84.5% | 0.0196 |
| TRD008005 | 33.6% | 69.4% | 112.9% | 124.6% | 103.1% | 0.0157 |
| TRD008006 | 23.5% | 51.2% | 75.9% | 89.2% | 88.7% | 0.0093 |
| TRD008024 | 44.20% | 71.85% | 99.14% | 95.71% | 100.03% | 0.0204 |
| TRD008025 | 48.80% | 88.00% | 100.83% | 98.83% | 100.45% | 0.0267 |
| TRD008026 | 49.72% | 99.29% | 103.55% | 104.57% | 100.83% | 0.0288 |
| TRD008027 | 47.05% | 81.86% | 114.93% | 122.14% | 100.28% | 0.0238 |

The results show that the ligand NAG0052 of the present disclosure, when conjugated with siRNAs of different structural sequences, can effectively inhibit target gene mRNA expression on cells.

**Test Example 3. Inhibition of Human FXI, ANGPTL3, MACR1 in Primary Human Hepatocytes (PHH) by siRNAs**

The siRNAs in Table 6 were subjected to primary human hepatocyte (PHH) activity screening in primary human hepatocyte (PHH, Novabiosis) using 5 or 7 concentrations.

The primary human hepatocytes (PHH) were cryopreserved in liquid nitrogen, and 24 h prior to transfection, the primary human hepatocytes (PHH) were thawed and then inoculated onto a 96-well plate, each well of which contained

80 μL of medium, at a density of 3 × $10^4$ cells per well. Lipofectamine RNAi MAX (ThermoFisher, 13778150) was used for siRNA transfection. The siRNA transfection gradient final concentrations for the 7-concentration-point experiment were 10 nM, 2 nM, 0.4 nM, 0.08 nM, 0.016 nM, 0.0032 nM and 0.00064 nM. The siRNA transfection gradient final concentrations for the 5-concentration-point experiment were 5 nM, 0.625 nM, 0.0781 nM, 0.00977 nM and 0.00122 nM. After 24 h of treatment, cell total RNA extraction was performed using a high throughput cellular RNA extraction kit (FireGen, FG0417), reverse transcription was performed using an RNA reverse transcription kit (Takara, 6210A), and the mRNA levels of human FXI, ANGPTL3 and MACR1 were determined using a Taqman probe Q-PCR kit (ThermoFisher, 4444964). The mRNA levels of human FXI, ANGPTL3 and MACR1 were corrected according to the GAPDH internal reference gene level. The Taqman probe primers are shown in Table 9. Data processing was performed using the $2^{-\Delta\Delta Ct}$ method, and the results are expressed as the remaining percentages of human FXI, ANGPTL3 and MACR1 mRNA expression relative to cells that had undergone control siRNA treatment. The inhibition rate $IC_{50}$ results are shown in Table 10 and Table 11.

ΔΔCt = [(Ct experimental group target gene - Ct experimental group internal reference) - (Ct control group target gene - Ct control group internal reference)].

$$\text{Inhibition rate (\%)} = (1 - \text{remaining amount of target gene expression}) \times 100\%.$$

Table 9. Taqman primers

| Primer name | Primer sequence |
|---|---|
| hFXI-PF | TTTGCTGGGAGAGGGTGTTG |
| hFXI-PR | TACAAACACCAAGCCCCTTCA |
| hFXI-P | CCAGCATGCTTCCTCCACAGTAACACG |
| hANG3-PF1-MGB | TTACTGGCAATGTCCCCAATG |
| hANG3-PR1-MGB | TGAAGTGTCCTTTTGCTTTGTGA |
| hANG3-P1-MGB | ACAAAGATTTGGTGTTTTC |
| hMARC1-PF | GCTCAGGAGGATGGTTGTGTAGT |
| hMARC1-PR | GAAGGAGCACTCCGTCATTAGC |
| hMARC1-P | CCCTGGATCCTTGCCATTCCCCTC |
| hGAPDH-PF1-MGB | GACCCCTTCATTGACCTCAACTAC |
| hGAPDH-PR1-MGB | TTGACGGTGCCATGGAATTT |
| hGAPDH-P1-MGB | TTACATGTTCCAATATGATTCC |

Table 10. The multi-dose inhibitory activity of siRNAs in PHH cells

| siRNA conjugate No. | Remaining percentages of target gene mRNA (PHH) expression (mean) at different siRNA concentrations | | | | | | | $IC_{50}$ values (nM) |
|---|---|---|---|---|---|---|---|---|
| | 10 nM | 2 nM | 0.4 nM | 0.08 nM | 0.016 nM | 0.0032 nM | 0.00064 nM | |
| TRD008002 | 16.61% | 23.16% | 34.70% | 38.35% | 52.77% | 91.37% | 105.17% | 0.0295 |
| TRD008003 | 19.15% | 25.65% | 39.67% | 42.81% | 55.62% | 80.31% | 91.47% | 0.0465 |
| TRD008004 | 4.27% | 6.24% | 7.34% | 15.42% | 41.23% | 68.30% | 81.65% | 0.0098 |
| TRD008005 | 5.85% | 5.66% | 8.71% | 24.52% | 47.34% | 88.18% | 84.60% | 0.0176 |
| TRD008006 | 6.52% | 5.99% | 8.44% | 10.76% | 33.06% | 58.39% | 63.74% | 0.0066 |

(continued)

| siRNA conjugate No. | Remaining percentages of target gene mRNA (PHH) expression (mean) at different siRNA concentrations | | | | | | | $IC_{50}$ values (nM) |
|---|---|---|---|---|---|---|---|---|
| | 10 nM | 2 nM | 0.4 nM | 0.08 nM | 0.016 nM | 0.0032 nM | 0.00064 nM | |
| TRD008024 | 12.72% | 8.30% | 8.86% | 9.68% | 19.98% | 36.58% | 65.77% | 0.0015 |
| TRD008025 | 9.63% | 9.62% | 9.93% | 20.08% | 28.44% | 38.41% | 55.92% | 0.0011 |
| TRD008026 | 7.44% | 9.15% | 10.37% | 15.59% | 33.72% | 53.33% | 68.26% | 0.0044 |

Table 11. The multi-dose inhibitory activity of siRNAs in PHH cells

| siRNA conjugate No. | Remaining percentages of MARC1 mRNA (PHH) expression (mean) at different siRNA concentrations | | | | | $IC_{50}$ values (nM) |
|---|---|---|---|---|---|---|
| | 5 nM | 0.63 nM | 0.078 nM | 0.0098 nM | 0.0012 nM | |
| TRD6233 | 5.70% | 6.45% | ND | 44.32% | 76.77% | 0.0074 |
| TRD6238 | 6.46% | 12.07% | 29.28% | 63.63% | 118.05% | 0.0186 |
| TRD6253 | 7.90% | 12.13% | 33.85% | 56.02% | 65.52% | 0.0206 |

The results show that the ligand NAG0052 of the present disclosure, when conjugated with siRNAs of different structural sequences, can effectively inhibit target gene mRNA expression on primary human hepatocytes. The conjugates TRD008024, TRD008025 and TRD008026 of NAG0052 showed better inhibitory activity on PHH cells than the conjugates TRD6233, TRD6238 and TRD6253 of NAG1.

**Test Example 4. Inhibition of FXI in Primary Cynomolgus Monkey Hepatocytes (PCH) by siRNAs-7-Concentration-Point Inhibitory Activity**

The 2 siRNAs of FXI in Table 6 were subjected to reverse transfection activity screening in primary monkey hepatocytes (PCH, Milestone) using 7 concentrations. The initial final concentration for transfection of each siRNA sample was 10 nM, and 5-fold serial dilution was carried out to obtain 7 concentration points.

The primary monkey hepatocytes (PCH) were cryopreserved in liquid nitrogen, and prior to transfection, the primary monkey hepatocytes (PCH) were thawed and then inoculated onto a 96-well plate, each well of which contained 90 μL of medium, at a density of $3 \times 10^4$ cells per well. Lipofectamine RNAi MAX (ThermoFisher, 13778150) was used for siRNA transfection, and the gradient final concentrations for siRNA transfection were 10 nM, 2 nM, 0.4 nM, 0.08 nM, 0.016 nM, 0.0032 nM and 0.00064 nM. After 24 h of treatment, cell total RNA extraction was performed using a high throughput cellular RNA extraction kit (FireGen, FG0417), reverse transcription was performed using an RNA reverse transcription kit (Takara, 6210A), and the mRNA level of monkey FXI was determined using a Taqman probe Q-PCR kit (ThermoFisher, 4444964). The mRNA level of monkey FXI was corrected according to the GAPDH internal reference gene level. The Taqman probe primers are shown in Table 12. Data processing was performed using the $2^{-\Delta\Delta Ct}$ method, and the results are expressed as the remaining percentages of monkey FXI mRNA expression relative to cells that had undergone control siRNA treatment. The inhibition rate $IC_{50}$ results are shown in Table 13.

$\Delta\Delta$Ct = [(Ct experimental group target gene - Ct experimental group internal reference) - (Ct control group target gene - Ct control group internal reference)].

$$\text{Inhibition rate (\%)} = (1 - \text{remaining amount of target gene expression}) \times 100\%.$$

Table 12. Monkey Taqman probe primers

| Primer name | Primer sequence |
|---|---|
| mkFXI-V1-PF1 | CTGGATATTGTTGCTGTGAAAGGT |
| mkFXI-V1-PR1 | CCTTCGTTGCAAGATGCTTGA |
| mkFXI-V1-P1 | CTGTGCACCAATGCCGTCCGC |
| mkGAPDH-PF1-MGB | AGTCAGCCGCATTTTCTCTTG |
| mkGAPDH-PR1-MGB | AAATCCGTTGACTCCGACCTT |
| mkGAPDH-P1-MGB | ATCGCCAGCGCATC |

Table 13. The multi-dose inhibitory activity of siRNAs in PCH

| siRNA conjugate No. | Remaining percentages of target gene mRNA (HCH) expression (mean) at different siRNA concentrations | | | | | | | $IC_{50}$ values (nM) |
|---|---|---|---|---|---|---|---|---|
| | 10 nM | 2 nM | 0.4 nM | 0.08 nM | 0.016 nM | 0.0032 nM | 0.00064 nM | |
| TRD008002 | 11.69% | 29.29% | 48.51% | 49.83% | 42.10% | 47.34% | 76.09% | 0.0398 |
| TRD008003 | 6.83% | 9.29% | 51.01% | 33.91% | 55.43% | 65.33% | 71.83% | 0.0369 |

The results show that the conjugates TRD008002 and TRD008003 of NAG0052 have very good inhibitory activity against FXI in PCH cells.

**Test Example 5. Inhibition of Human ANGPTL3 in Huh7 Cells by siRNAs-7-Concentration-Point Inhibitory Activity**

The siRNAs in Table 6 were subjected to Huh7 cell activity screening in Huh7 cells (Nanjing Cobioer) using 7 concentrations. The initial final concentration for transfection of each siRNA sample was 10 nM, and 5-fold serial dilution was carried out to obtain 7 concentration points.

Huh7 cells were cultured at 37 °C with 5% $CO_2$ in a DMEM high glucose medium containing 10% fetal bovine serum. 24 h prior to transfection, the Huh7 cells were inoculated onto a 96-well plate, each well of which contained 100 μL of medium, at a density of ten thousand cells per well. Lipofectamine RNAi MAX (ThermoFisher, 13778150) was used for siRNA transfection, and the final concentrations for siRNA transfection were 10 nM, 2 nM, 0.4 nM, 0.08 nM, 0.016 nM, 0.0032 nM and 0.00064 nM. After 24 h of treatment, cell total RNA extraction was performed using a high throughput cellular RNA extraction kit (FireGen, FG0417), reverse transcription was performed using an RNA reverse transcription kit (Takara, 6210A), and the mRNA level of human ANGPTL3 was determined using a Taqman probe Q-PCR kit (ThermoFisher, 4444964). The mRNA level of human ANGPTL3 was corrected according to the GAPDH internal reference gene level. Data processing was performed using the $2^{-\Delta\Delta Ct}$ method, and the results are expressed as the remaining percentages of human ANGPTL3 mRNA expression relative to cells that had undergone control siRNA treatment. The inhibition rate $IC_{50}$ results are shown in Table 14.

$\Delta\Delta Ct$ = [(Ct experimental group target gene - Ct experimental group internal reference) - (Ct control group target gene - Ct control group internal reference)].

$$\text{Inhibition rate (\%)} = (1 - \text{remaining amount of target gene expression}) \times 100\%.$$

Table 14. The multi-dose inhibitory activity of siRNAs against human ANGPTL3 in Huh7

| siRNA conjugate No. | Remaining percentages of target gene mRNA (Huh7) expression (mean) at different siRNA concentrations | | | | | | | $IC_{50}$ values (nM) |
|---|---|---|---|---|---|---|---|---|
| | 10 nM | 2 nM | 0.4 nM | 0.08 nM | 0.016 nM | 0.0032 nM | 0.00064 nM | |
| TRD008004 | 20.41% | 29.83% | 38.24% | 66.52% | 91.40% | 100.41% | 102.12% | 0.1995 |
| TRD008005 | 18.80% | 25.78% | 28.90% | 57.12% | 77.19% | 93.56% | 95.33% | 0.1047 |
| TRD008006 | 19.14% | 30.21% | 37.88% | 61.27% | 83.49% | 98.09% | 99.08% | 0.1738 |

The results show that the conjugates TRD008004, TR008005 and TRD008006 of NAG0052 exhibited effective inhibitory activity against ANGPTL3 in Huh7 cells.

**Preparation Example 12: Synthesis of Nucleic Acid Ligand Conjugates**

The following siRNA conjugates were synthesized for Test Examples 6 and 7 by referring to the procedures of Preparation Example 10. The siRNAs have the sense and antisense strands shown in Table 15.

Table 15

| siRNA conjugate No. | SS strand (5'-3') | AS strand (5'-3') |
|---|---|---|
| S-1 | CmsAmsGm UmGfUm UfCfUf UmGmCm UmCmUm AmUmAm Am -NAG1 | UmsUfsAm UmAmGf AmGmCm AmAmGm AmAfCm AfCmUm Gm sUm sUm |
| S-L96 | CmsAmsGm UmGfUm UfCfUf UmGmCm UmCmUm AmUmAm Am -L96 | UmsUfsAm UmAmGf AmGmCm AmAmGm AmAfCm AfCmUm Gm sUm sUm |

**Test Example 6. Inhibition of mRNA Expression in Primary Hepatocytes by Galactosamine Molecule Cluster-Conjugated siRNAs**

Fresh primary hepatocytes were isolated from mice using the method reported by Severgini et al. (Cytotechnology. 2012;64(2):187-195).

After isolation, the primary hepatocytes were inoculated onto a 24-well plate at 100 thousand cells per well. The test siRNA compounds were added at the final concentrations of 50 nM, 10 nM, 2 nM, 0.4 nM, 0.08 nM, 0.016 nM, 0.0032 nM and 0.00064 nM. Subsequently, the primary hepatocytes were cultured at 37 °C with 5% $CO_2$ for 24 h. After 24 h, the mTTR's mRNA expression level was determined using the qPCR method.

FIG. 3 shows the inhibition rates of different concentrations of the siRNA conjugates S-1 (NAG1-conjugated) and S-L96 (L96-conjugated) against mRNA. As shown in FIG. 3, S-1 exhibited excellent inhibition efficiency against mTTR gene expression. The $IC_{50}$ value of the control group S-L96 was 0.280 nM, while the $IC_{50}$ value of S-1 was 0.131 nM. This indicates that the NAG1-conjugated siRNA was freely taken in by primary hepatocytes *in vitro* with a better efficiency than the control group: NAG1 conjugation can more efficiently mediate entry of siRNA into primary hepatocytes. In Test Example 3, the NAG0052-conjugated siRNAs showed stronger inhibitory activity against target gene expression than the NAG1-conjugated siRNAs. As a whole, the data indicate that the NAG0052 conjugates have better inhibitory activity than the L96 conjugate.

**Test Example 7. *In Vivo* Inhibition of mRNA Expression by Galactosamine Molecule Cluster-Conjugated siRNAs**

Eight-week-old C57BL/6 mice (Joinnbio, SPF, female) were injected subcutaneously with the galactosamine molecule cluster-conjugated siRNAs described above. On day 1, 100 μL of solution containing PBS (referred to as the Mock group, i.e., the blank control group) or a dose (1 mg/kg (mpk) or 0.2 mpk) of a corresponding galactosamine molecule cluster-conjugated siRNA (S-L96 or S-1) formulated in PBS was injected subcutaneously into the loose skin on the neck and shoulder of the mice. In each group, 6 mice were given injections.

Three days after administration, the mice were sacrificed by cervical dislocation, and the mTTR's mRNA expression levels in the liver tissue of the mice were determined by qPCR.

FIG. 4 shows the expression levels of mRNA in mouse liver tissue after administration of different doses of S-1 and S-L96, respectively. As shown in FIG. 4, S-1 exhibited excellent inhibition efficiency against mTTR gene expression. S-1 showed better activity than the control group S-L96 when administered at 1 mpk and 0.2 mpk. In Test Example 3, the NAG0052-conjugated siRNAs showed stronger inhibitory activity against target gene expression than the NAG1-conjugated siRNAs. As a whole, the data indicate that the NAG0052 conjugates have better inhibitory activity than the L96 conjugate.

## Claims

**1.** A ligand having a structure represented by formula (I),

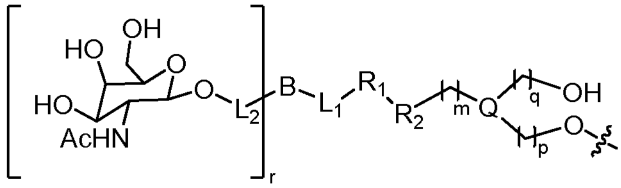

(I)

wherein $L_1$ is a $C_1$-$C_{30}$ alkyl chain, or a $C_1$-$C_{30}$ alkyl chain interrupted by one or more oxygen, sulfur or nitrogen atoms or C=O;
$R_1$ and $R_2$ are independently chemical bonds, -$NR_6$-, -C(=O)- or -OC(=O)-;
Q is

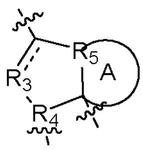 or 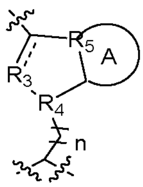 ;

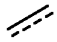 is a single or double bond; when 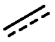 is a single bond, $R_3$ is independently $CR_7R_8$, $NR_6$, O or S; when 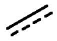 is a double bond, $R_3$ is independently $CR_9$ or N;
$R_4$ is independently $CR_9$ or N;
ring A is absent, or is cycloalkyl, heterocyclyl, aryl or heteroaryl; when ring A is present, $R_5$ is independently $CR_9$ or N; when ring A is absent, $R_5$ is independently $CR_7R_8$, $NR_6$ or O;
$R_6$ and $R_9$ are independently hydrogen, deuterium, alkyl, alkoxy, cycloalkyl, heterocyclyl, aryl, heteroaryl, SR', S(=O)R', $S(=O)_2R'$, $S(=O)_2NR'(R")$, NR'(R"), C(=O)R', C(=O)OR' or C(=O)NR'(R"), wherein the alkyl, alkoxy, cycloalkyl, heterocyclyl, aryl or heteroaryl is optionally substituted with one or more groups selected from the group consisting of halogen, hydroxy, oxo, nitro, cyano, $C_{1-6}$ alkyl, $C_{1-6}$ alkoxy, $C_{3-7}$ cycloalkyl, 3-12 membered heterocyclyl, 5-12 membered aryl, 5-12 membered heteroaryl, SR', S(=O)R', $S(=O)_2R'$, $S(=O)_2NR'(R")$, NR'(R"), C(=O)R', C(=O)OR' and C(=O)NR'(R");
$R_7$ and $R_8$ are independently hydrogen, deuterium, alkyl, alkoxy, cycloalkyl, heterocyclyl, aryl, heteroaryl, SR', S(=O)R', $S(=O)_2R'$, $S(=O)_2NR'(R")$, NR'(R"), C(=O)R', C(=O)OR' or C(=O)NR'(R"), wherein the alkyl, alkoxy, cycloalkyl, heterocyclyl, aryl or heteroaryl is optionally substituted with one or more groups selected from the group consisting of halogen, hydroxy, oxo, nitro, cyano, $C_{1-6}$ alkyl, $C_{1-6}$ alkoxy, $C_{3-7}$ cycloalkyl, 3-12 membered heterocyclyl, 5-12 membered aryl, 5-12 membered heteroaryl, SR', S(=O)R', $S(=O)_2R'$, $S(=O)_2NR'(R")$, NR'(R"), C(=O)R', C(=O)OR' and C(=O)NR'(R");
R' and R" are independently hydrogen, deuterium, hydroxy, alkyl, alkoxy, cycloalkyl, heterocyclyl, aryl or heteroaryl, wherein the alkyl, alkoxy, cycloalkyl, heterocyclyl, aryl or heteroaryl is optionally substituted with one or more substituents selected from the group consisting of halogen, hydroxy, oxo, nitro and cyano;
m, n, p and q are independently 0, 1, 2, 3 or 4;

B is

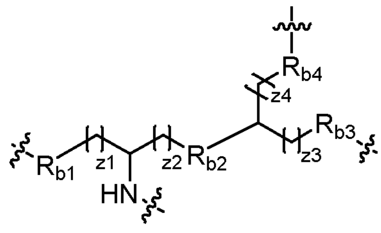

or

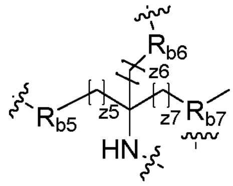

;

$R_{b1}$, $R_{b2}$, $R_{b3}$, $R_{b4}$, $R_{b5}$, $R_{b6}$ and $R_{b7}$ are independently -C(=O)-, -NHC(=O)-, -C(=O)O-, $-C(=O)-(CH_2)_{z8}-O-$ or $-NHC(=O)-(CH_2)_{z9}-O-$;
z1, z2, z3, z4, z5, z6, z7, z8 and z9 are independently integers of 0-10;
$L_2$ is a $C_1$-$C_{30}$ alkyl chain, or a $C_1$-$C_{30}$ alkyl chain interrupted by one or more oxygen, sulfur or nitrogen atoms or C=O;
r is an integer of 1-10.

**2.** The ligand according to claim 1, wherein $L_1$ is $L_3$ or $L_3$-$R_{10}$-$R_{11}$-$L_3$, wherein $L_3$ is independently a $C_1$-$C_{12}$ alkyl chain, $-(CH_2)_{j1}-C(=O)-(CH_2)_{j2}-$ or $-(CH_2)_{j3}-(CH_2CH_20)_{1-4}-(CH_2)_{j4}-$; $R_{10}$ and $R_{11}$ are independently chemical bonds, $-NR_{12}-$, -C(=O)- or -OC(=O)-; $R_{12}$ is hydrogen or $C_1$-$C_{12}$ alkyl; j 1, j2, j3 and j4 are independently integers of 0-10, preferably 0-2 or 4-10, and more preferably 0, 1, 2, 6, 7, 8, 9 or 10.

**3.** The ligand according to claim 2, wherein $L_1$ is $-(CH_2)_{j1}-C(=O)-(CH_2)_{j2}-$, preferably, $L_1$ is

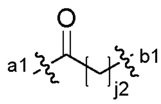

;

more preferably, $L_1$ is

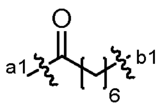

,

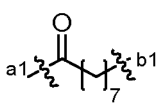

,

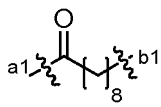

,

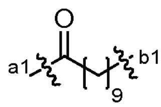

or

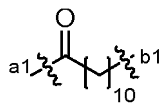

;

wherein the end a1 is attached to B, and the end b1 is attached to $R_1$.

**4.** The ligand according to any of claims 1-3, wherein $R_1$ and $R_2$ are of any of the following combinations:

combination 1: $R_1$ is a chemical bond and $R_2$ is C=O;
combination 2: $R_1$ is a chemical bond and $R_2$ is $NR_6$;
combination 3: $R_1$ is a chemical bond and $R_2$ is -OC(=O)-;
combination 4: $R_1$ is $NR_6$ and $R_2$ is C=O;
combination 5: $R_1$ is $NR_6$ and $R_2$ is -OC(=O)-;
combination 6: $R_2$ is $NR_6$ and $R_1$ is C=O; and
combination 7: $R_2$ is $NR_6$ and $R_1$ is -OC(=O)-.

**5.** The ligand according to any of claims 1-4, wherein $R_6$ is hydrogen or $C_{1-6}$ alkyl, preferably hydrogen, deuterium, methyl, ethyl, propyl or isopropyl, and more preferably hydrogen;

and/or, $R_7$ and $R_8$ are hydrogen;
and/or, $R_9$ is hydrogen.

**6.** The ligand according to any of claims 1-5, wherein m is 0 or 1.

**7.** The ligand according to any of claims 1-6, wherein p and q may be of any of the following combinations:

combination 1: p = 1 and q = 1;
combination 2: p = 1 and q = 0;
combination 3: p = 0 and q = 1; and
combination 4: p = 0 and q = 0.

**8.** The ligand according to any of claims 1-7, wherein

B is

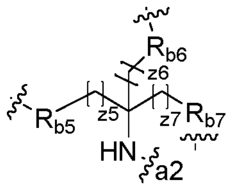
or
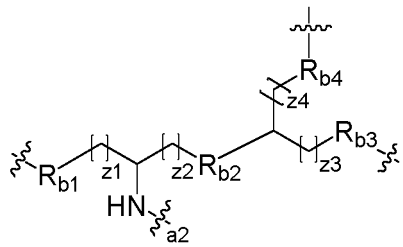
;

$R_{b1}$, $R_{b2}$, $R_{b3}$, $R_{b4}$, $R_{b5}$, $R_{b6}$ and $R_{b7}$ are independently -C(=O)- or -NHC(=O)-; the N atom of the end a2 is attached to $L_1$;
preferably, B is

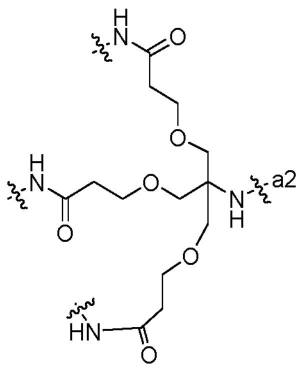
,
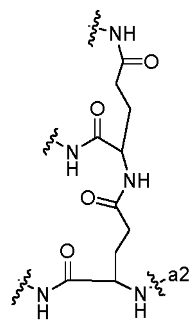
or
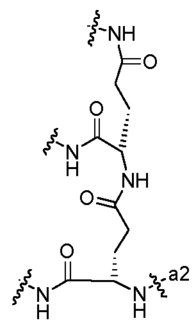
.

**9.** The ligand according to any of claims 1-8, wherein
$L_2$ is $L_4$ or $L_4$-$R_{13}$-$R_{14}$-$L_4$, wherein $L_4$ is independently a $C_1$-$C_{12}$ alkyl chain or $-(CH_2)_{j5}-(OCH_2CH_2)_{1-4}-(CH_2)_{j6}-$; $R_{13}$ and $R_{14}$ are independently chemical bonds, $-NR_{15}-$, -C(=O)- or -OC(=O)-; $R_{15}$ is independently hydrogen or $C_1$-$C_{12}$ alkyl; j5 and j6 are independently integers of 0-10, preferably 0-6, and more preferably 0, 1, 2, 3 or 4.

**10.** The ligand according to claim 9, wherein $L_2$ is $-(CH_2)_{j5}-(OCH_2CH_2)_{1-4}-(CH_2)_{j6}-$, preferably

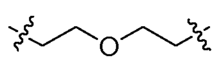
.

**11.** The ligand according to claim 9, wherein $L_2$ is

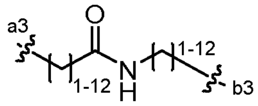
,

preferably

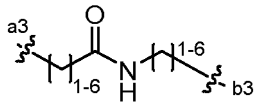
,

more preferably

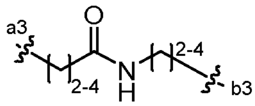
:

, and further preferably

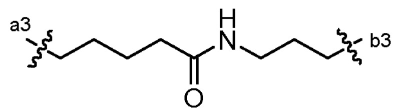
,

wherein the end a3 is attached to O, and the end b3 is attached to B.

**12.** The ligand according to any of claims 1-11, wherein r is 3.

**13.** The ligand according to any of claims 1-12, wherein

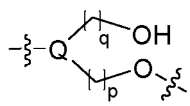

is

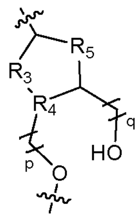
,

preferably

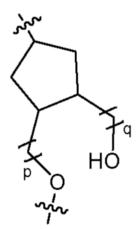
,
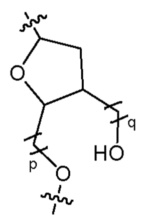
or
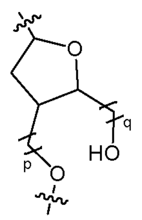
,

more preferably

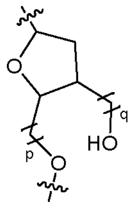 or 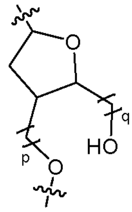 ,

and further preferably

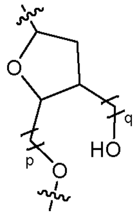 .

**14.** The ligand according to any of claims 1-13, wherein

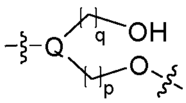

is

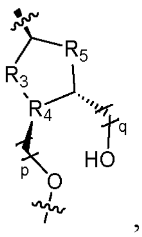 , 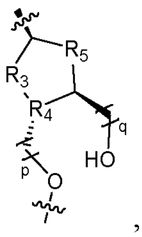 , 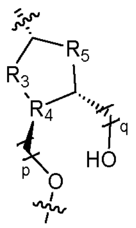 or 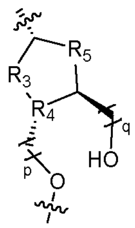 ,

preferably

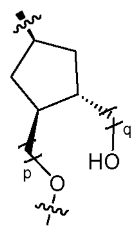 , 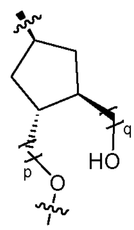 ,

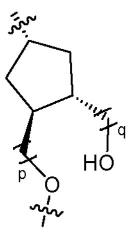 , 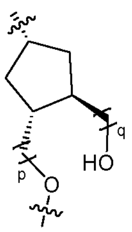 , 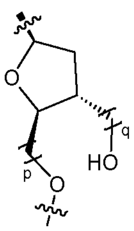 , 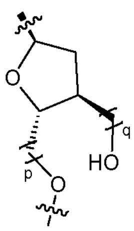 , 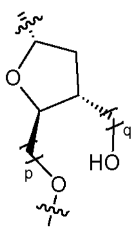 , 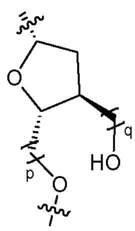 , 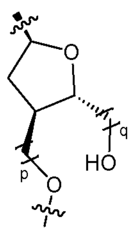 ,

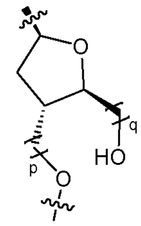, 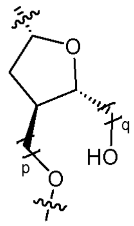 or 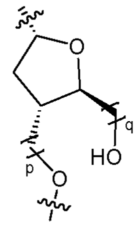,

more preferably

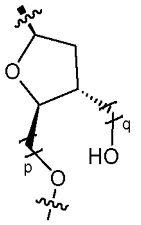, 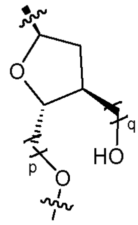,

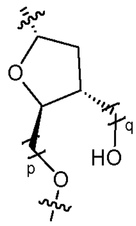, 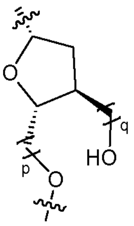, 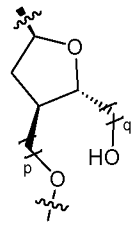, 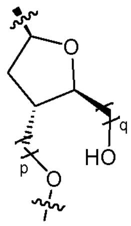, 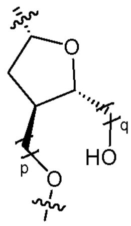 or 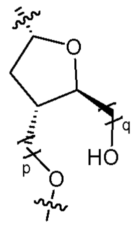,

and further preferably

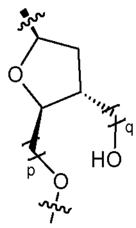, 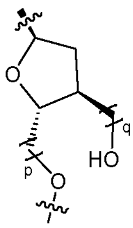, 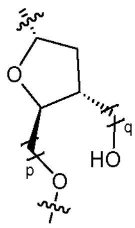 or 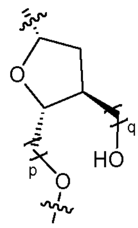.

**15.** The ligand according to any of claims 1-14, wherein the ligand is any of the following structures:

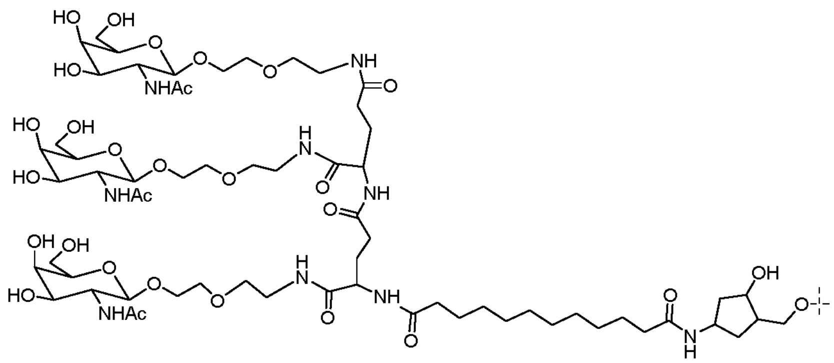,

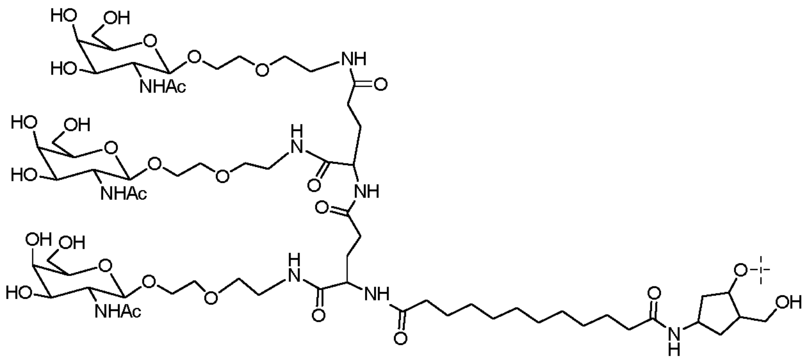

,

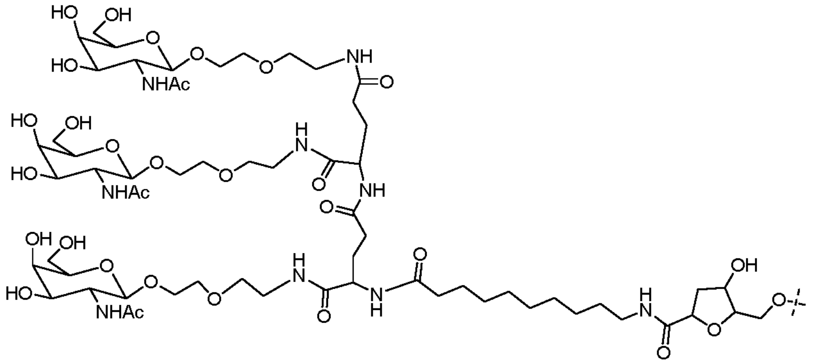

,

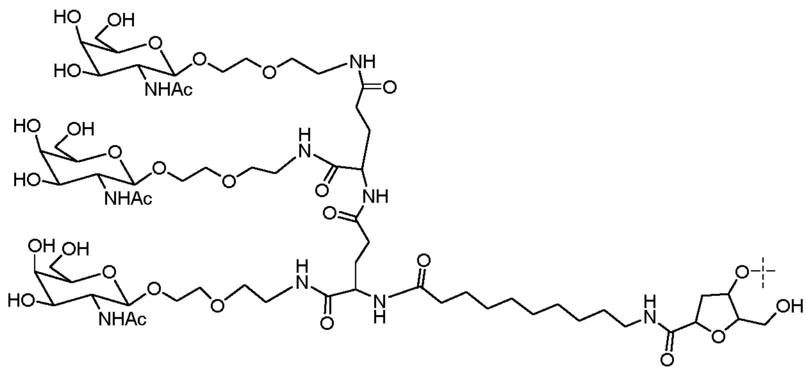

,

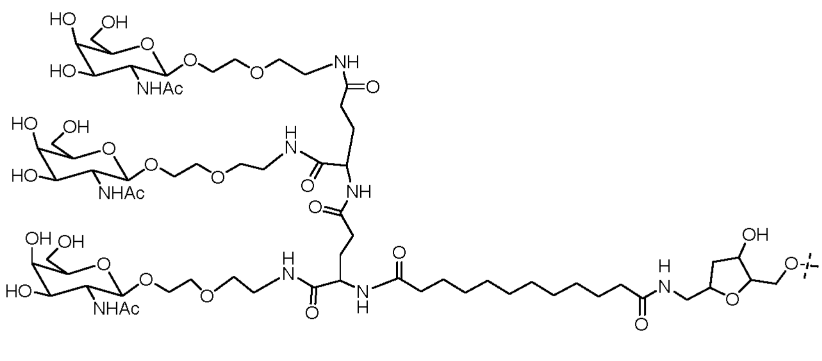

-

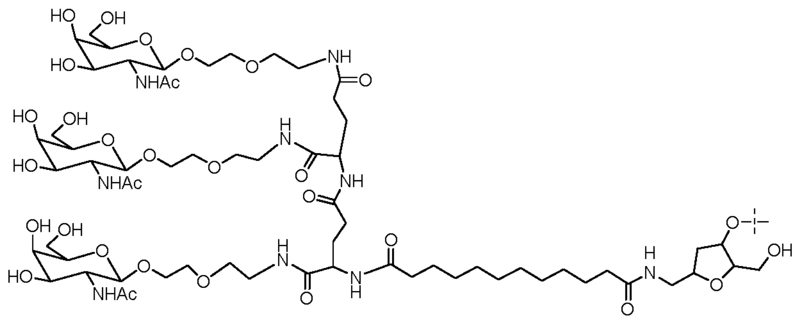

,

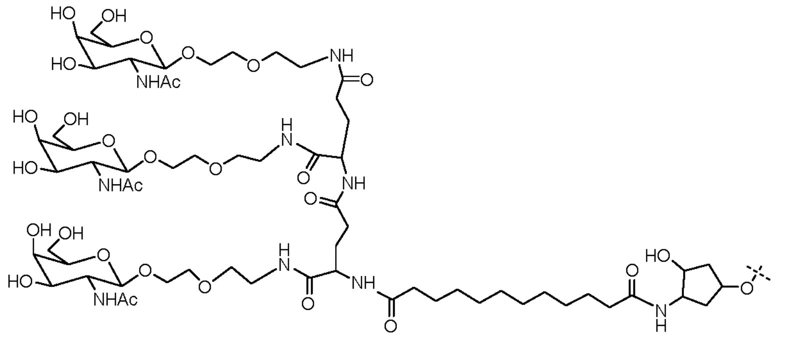

,

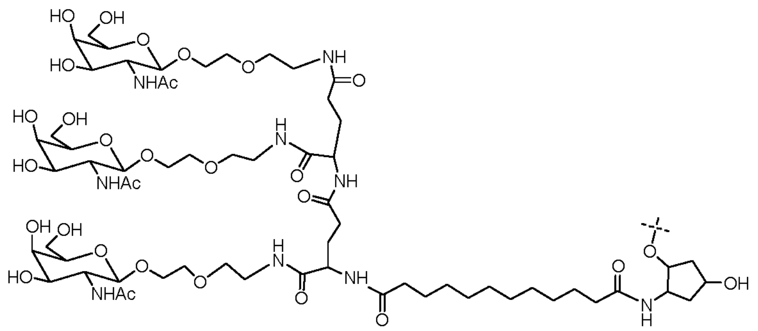

,

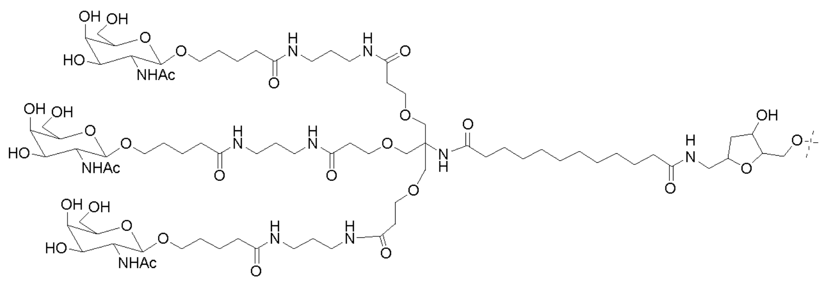

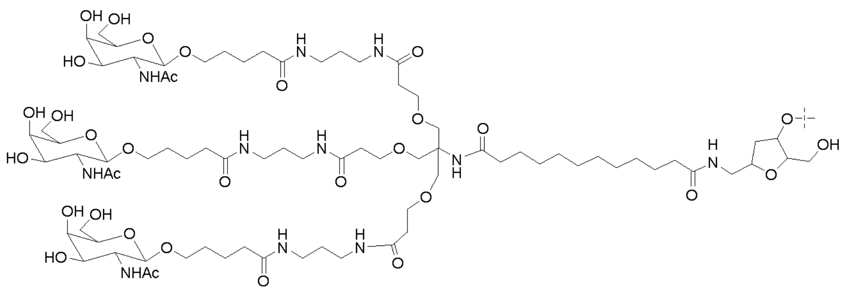

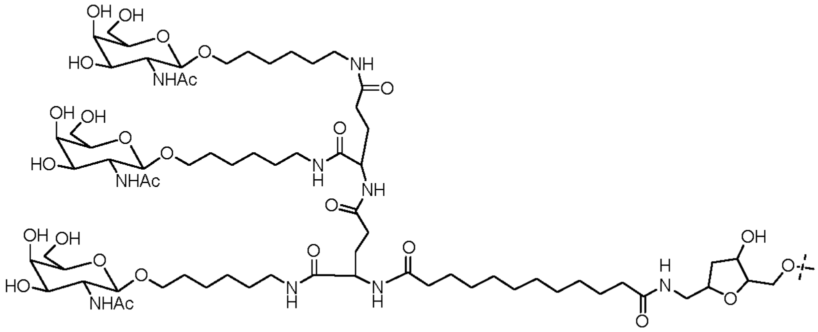

,

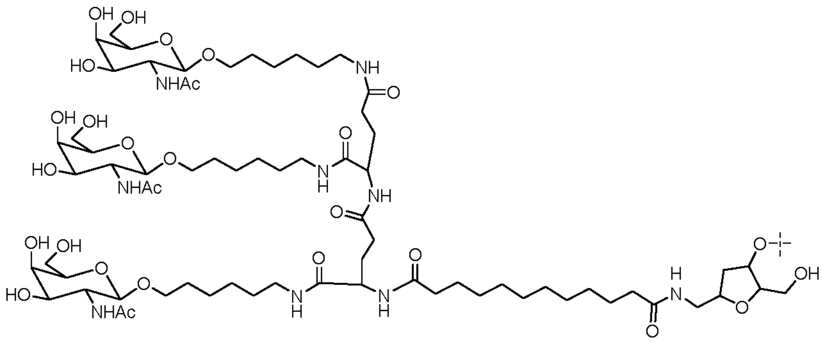

,

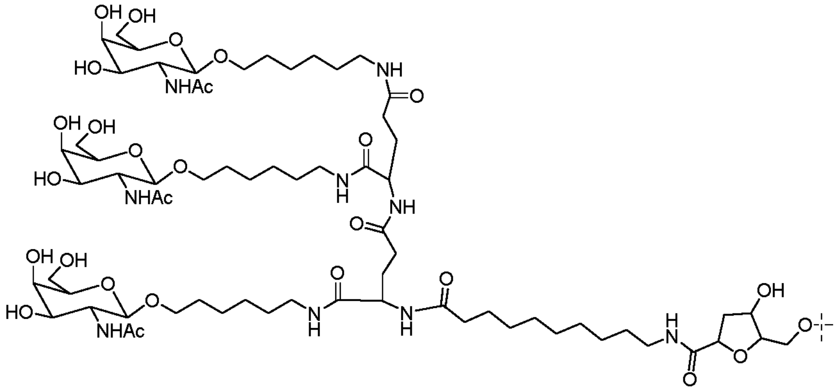

,

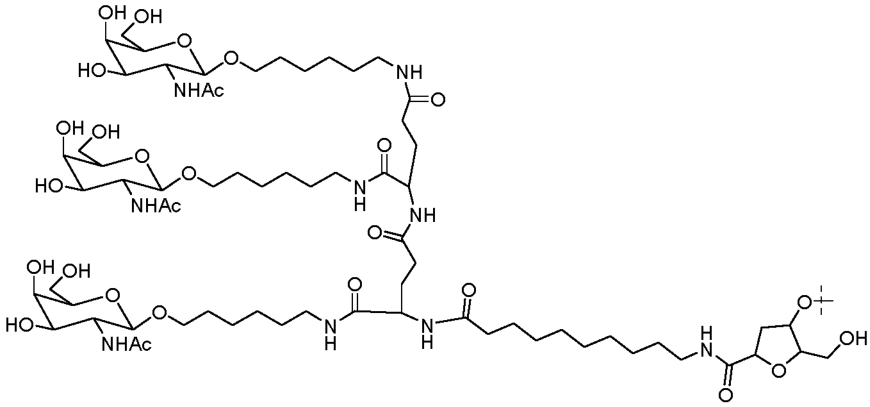

,

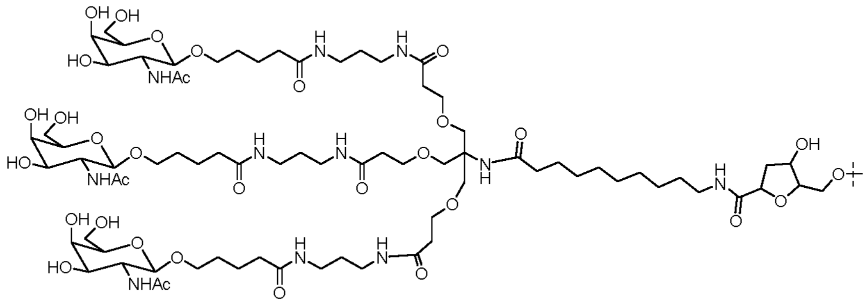

or

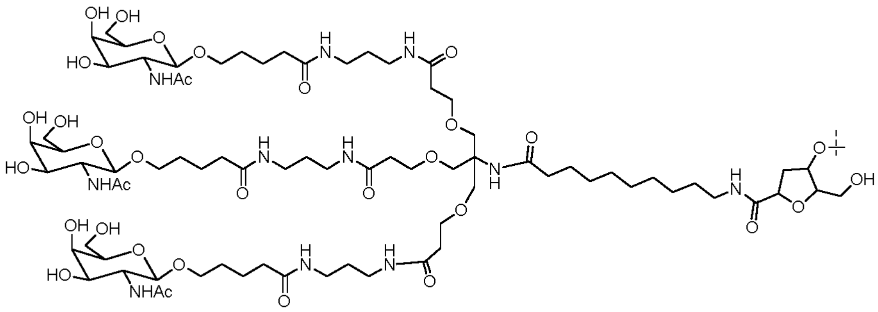

.

**16.** The ligand according to any of claims 1-15, wherein the ligand is any of the following structures:

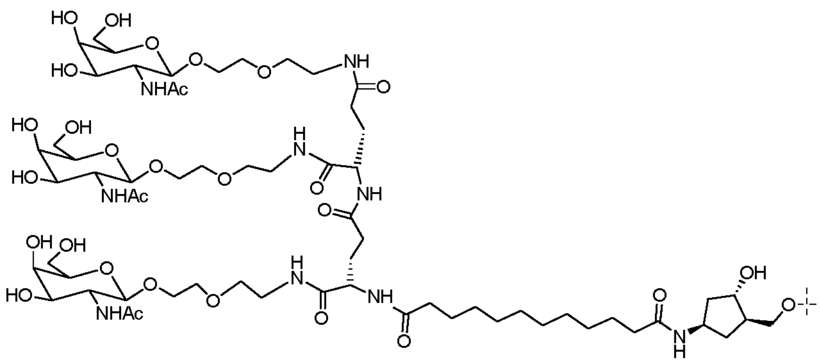

,

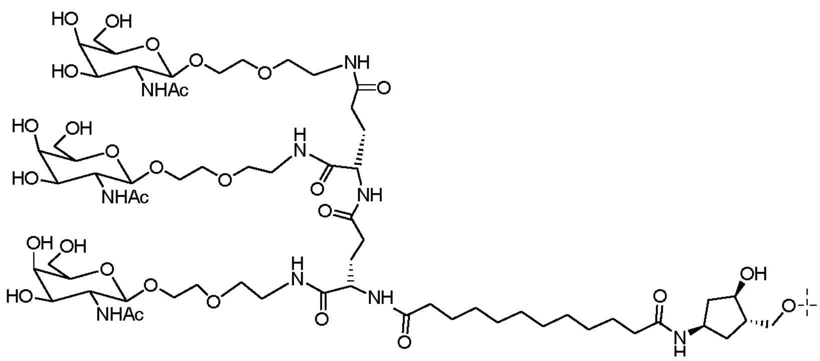

,

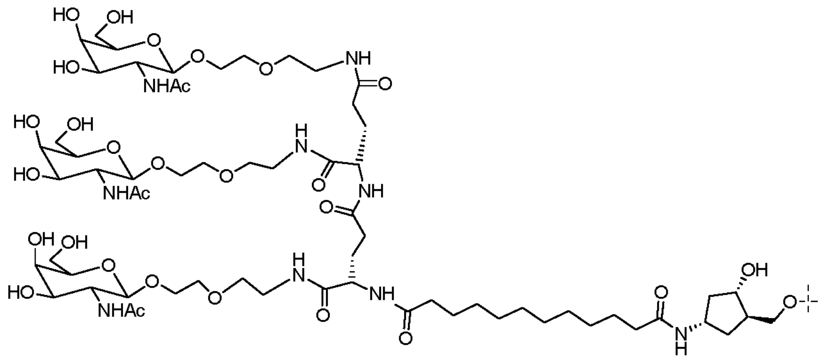

,

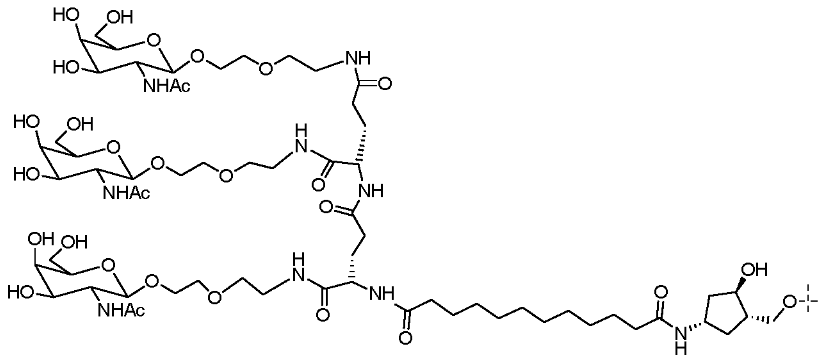

,

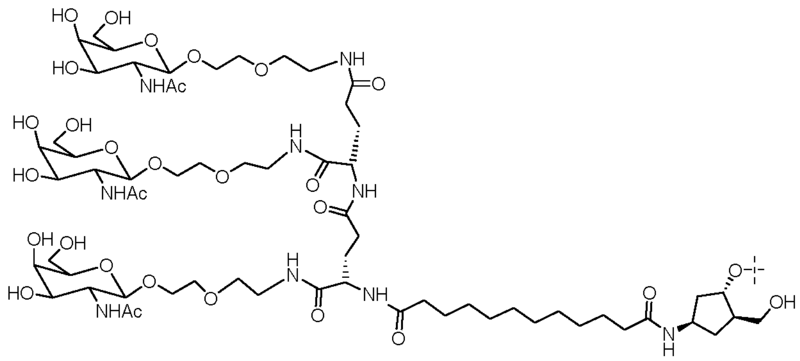

,

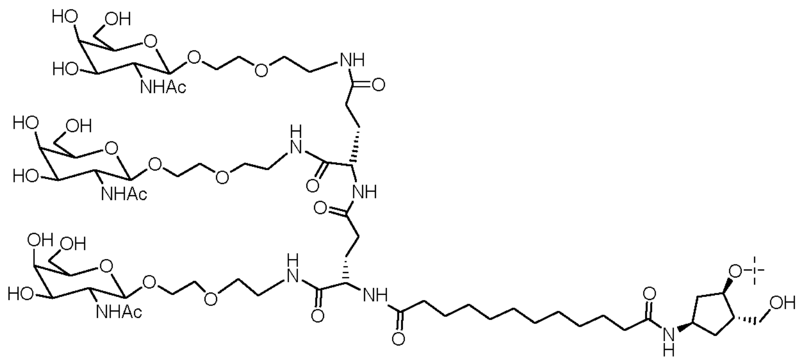

,

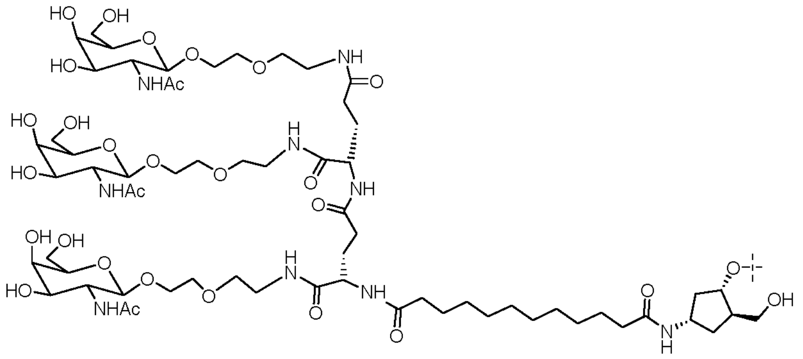

,

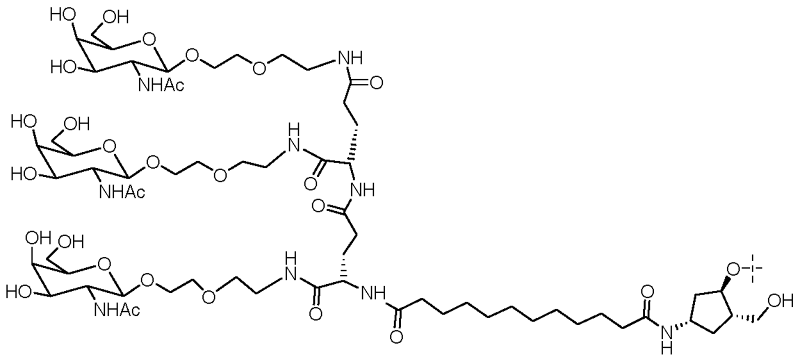

,

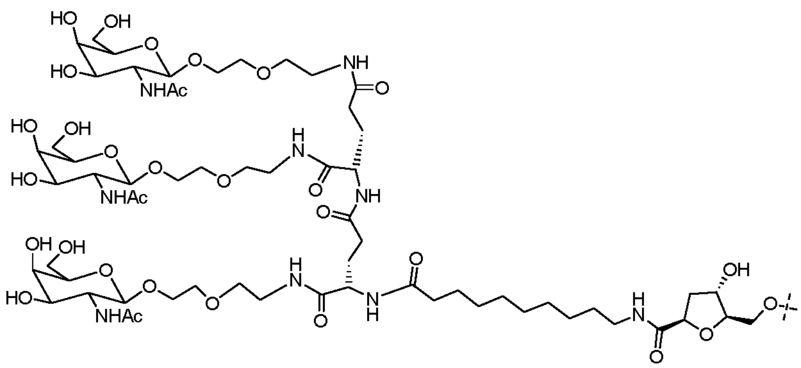

,

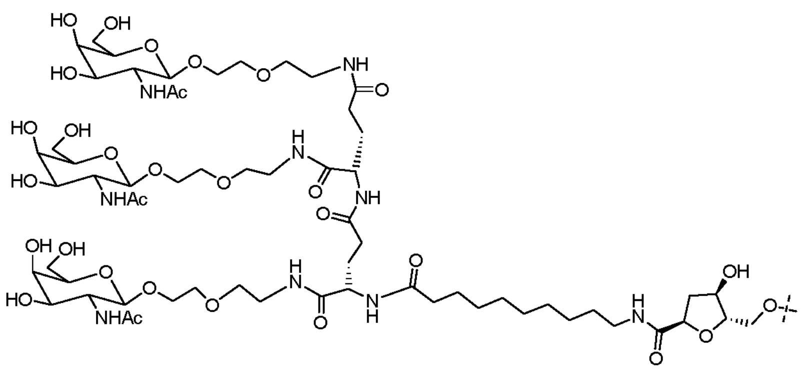

,

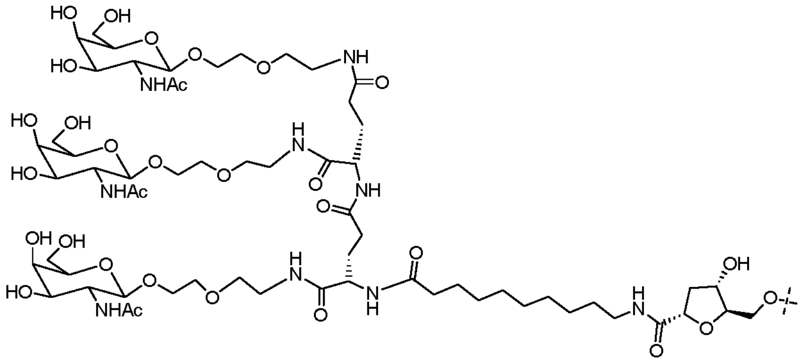

,

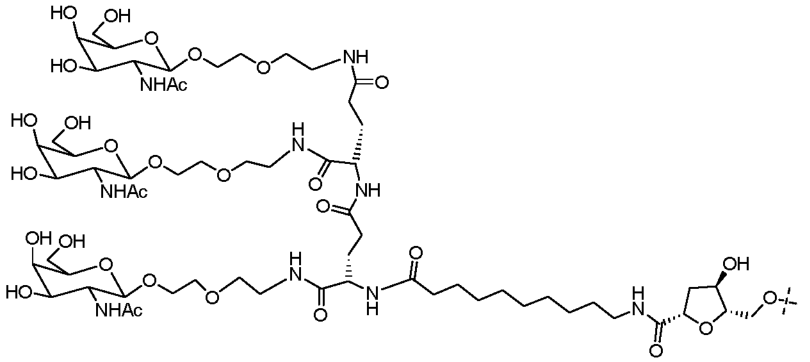

,

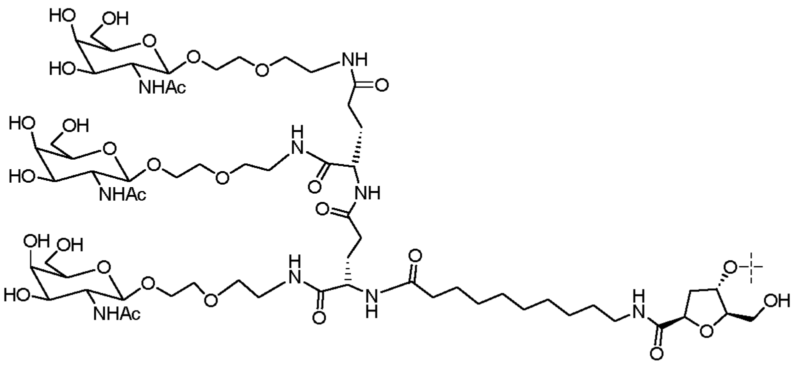

,

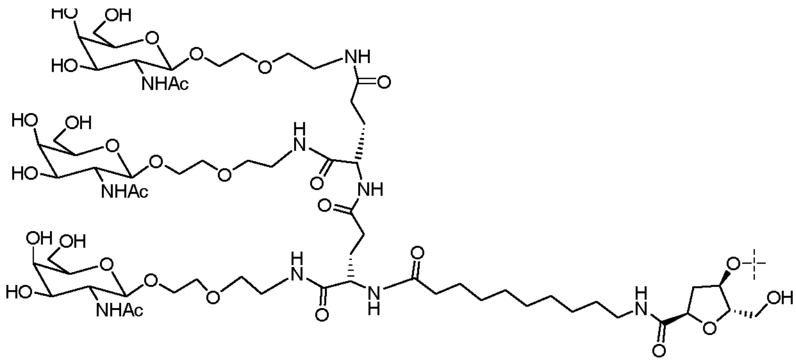

,

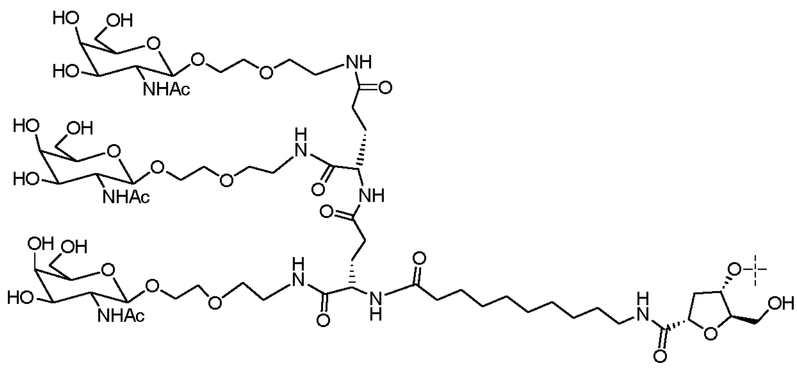

,

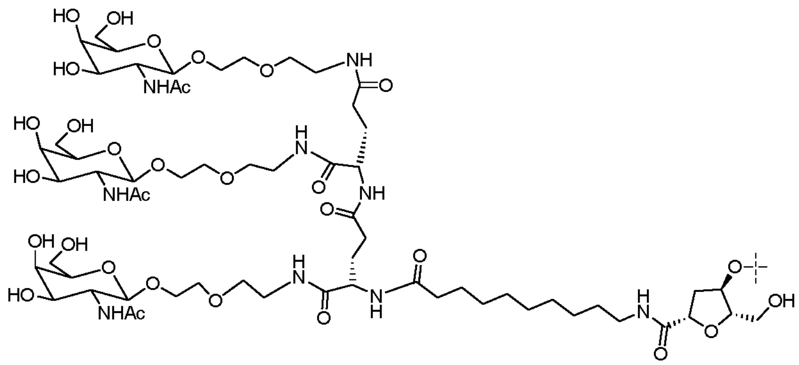

,

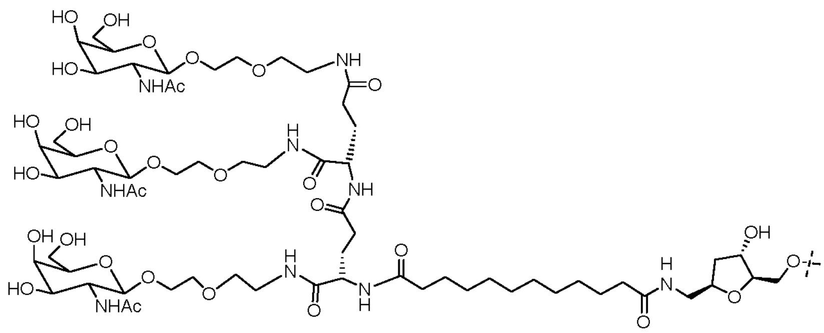

,

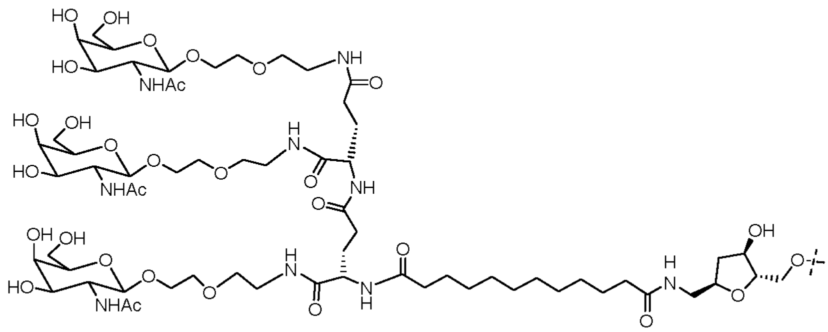

,

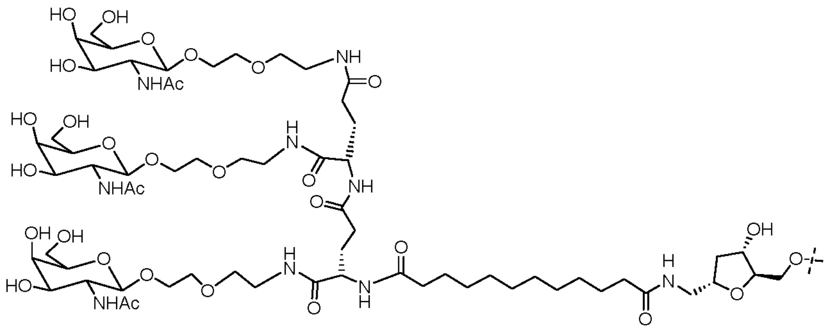

,

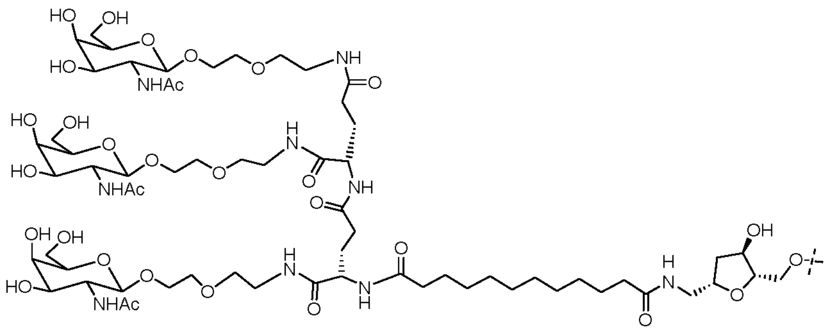

,

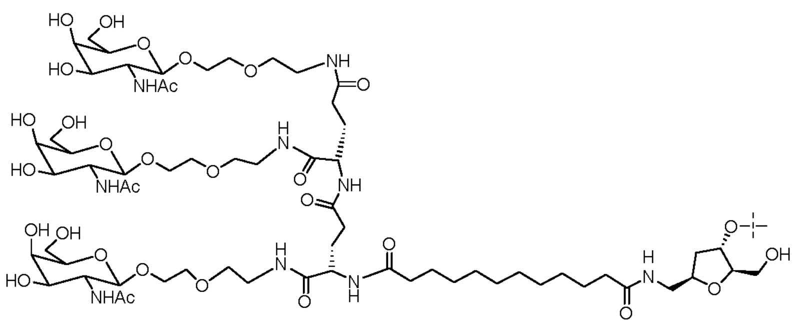

,

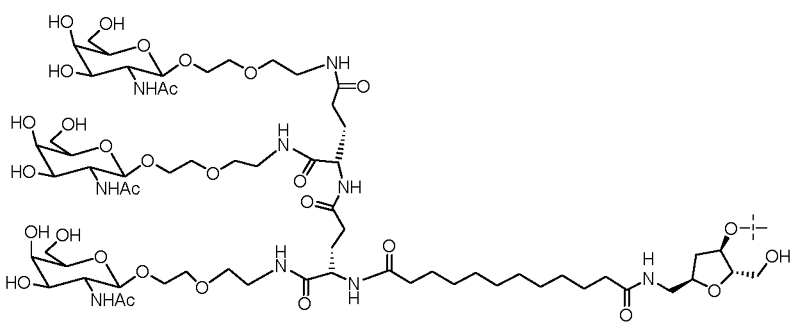

,

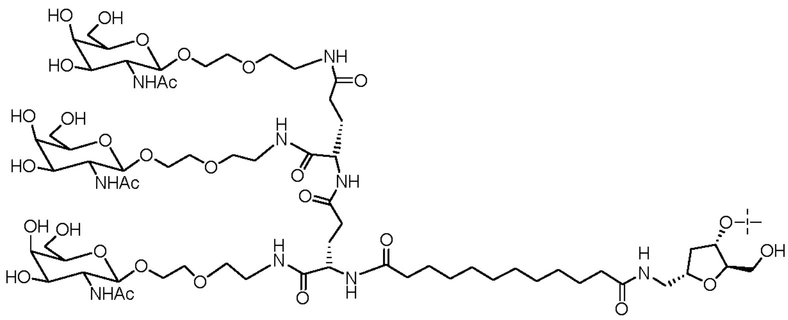

,

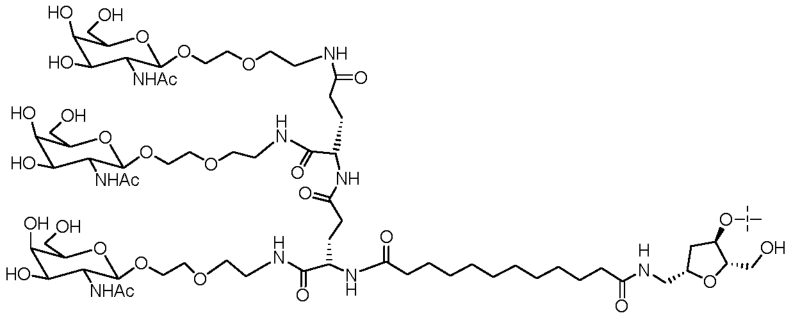

,

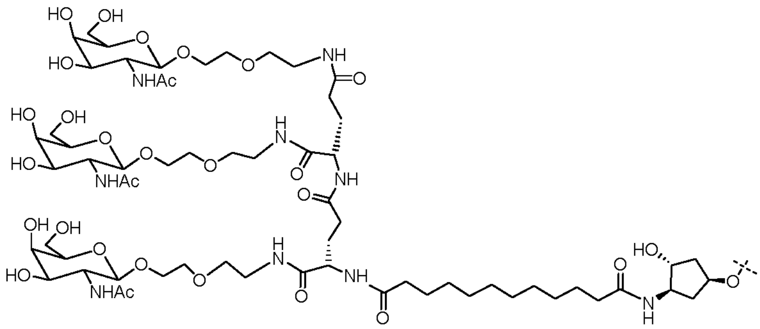

,

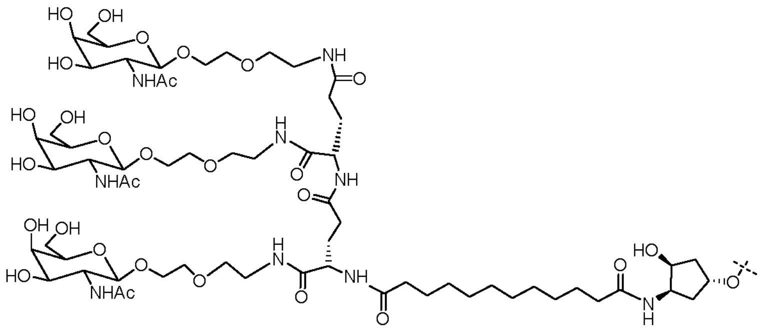

,

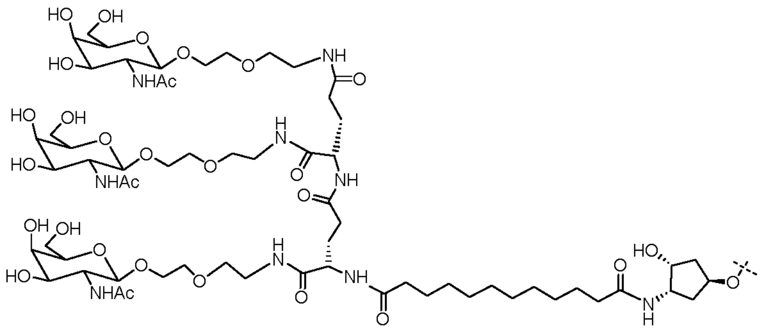

,

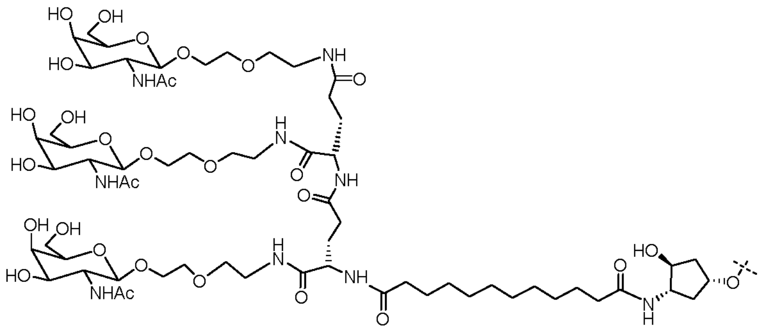

,

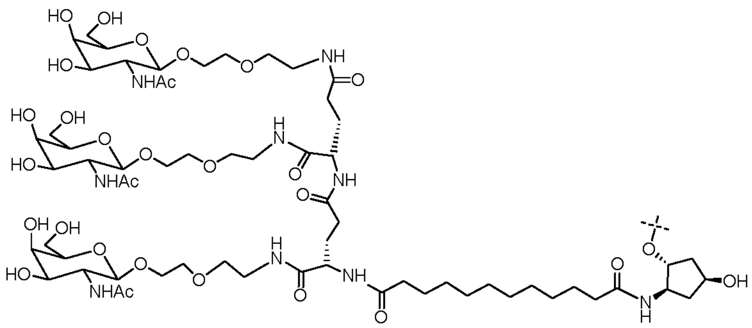

,

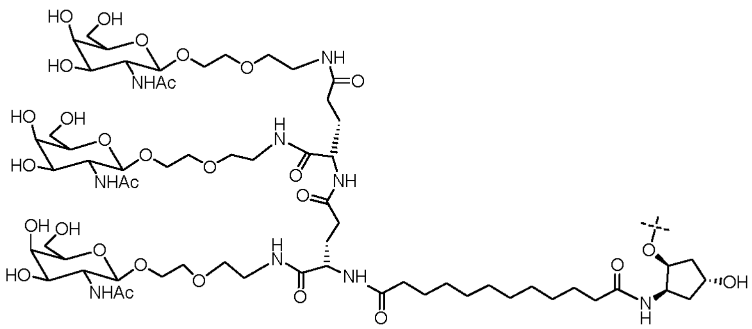

,

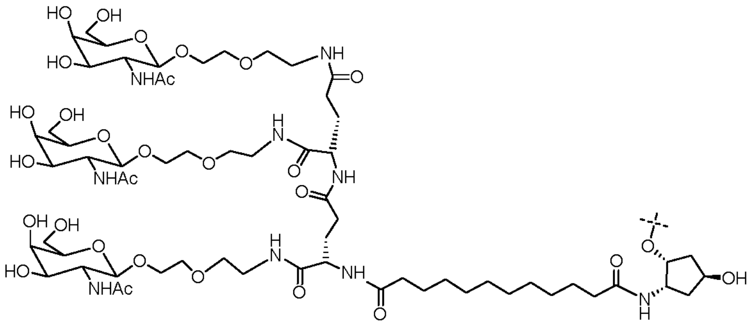

,

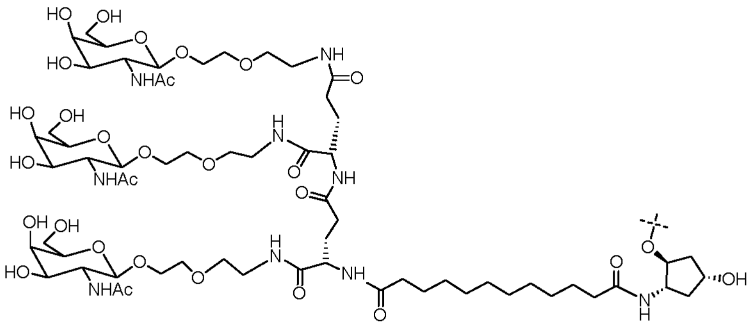

,

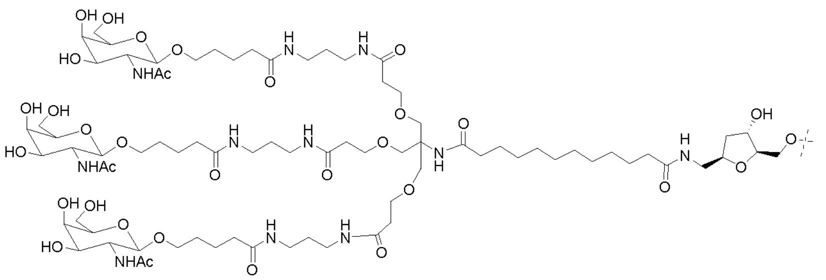

,

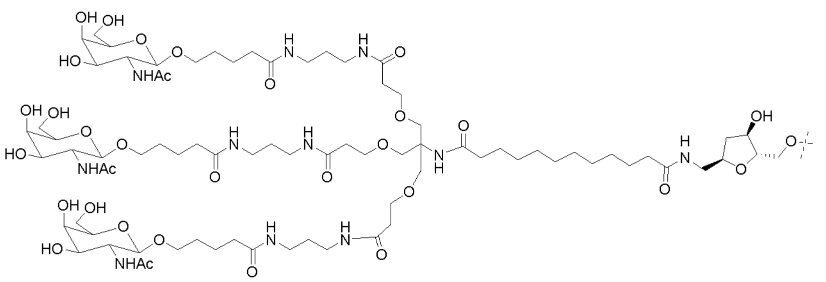

,

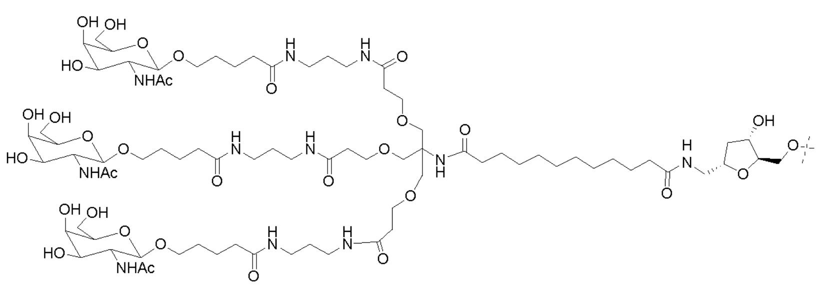

,

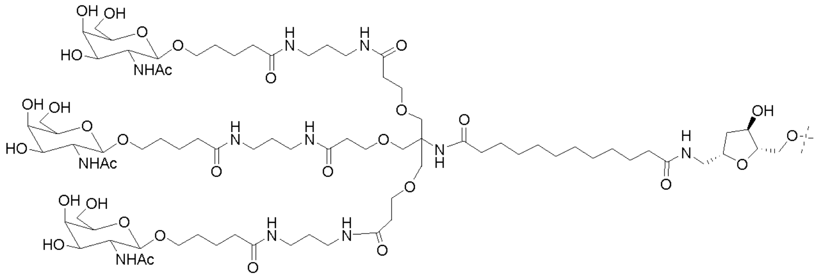

,

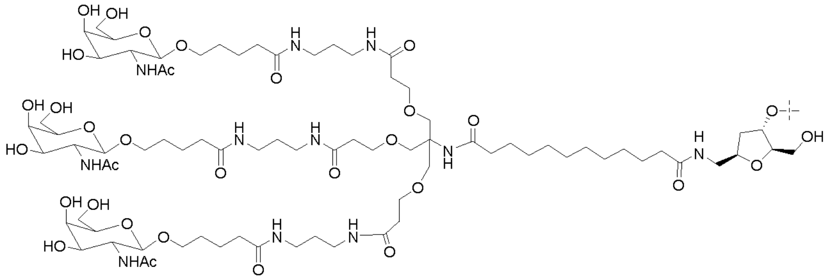

,

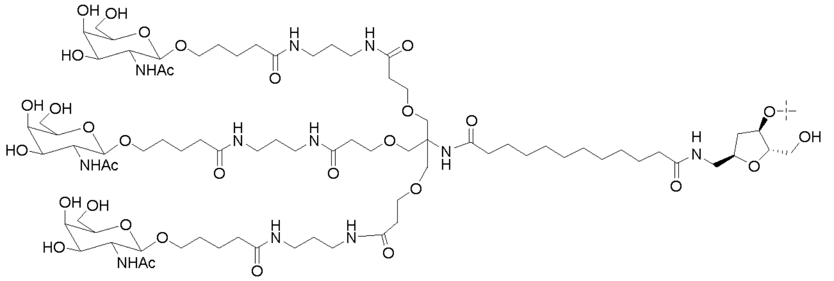

,

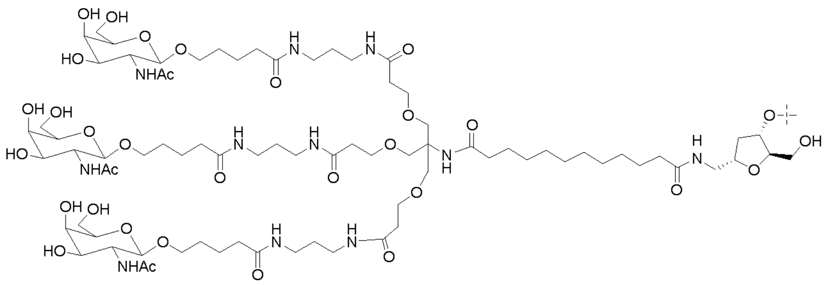

,

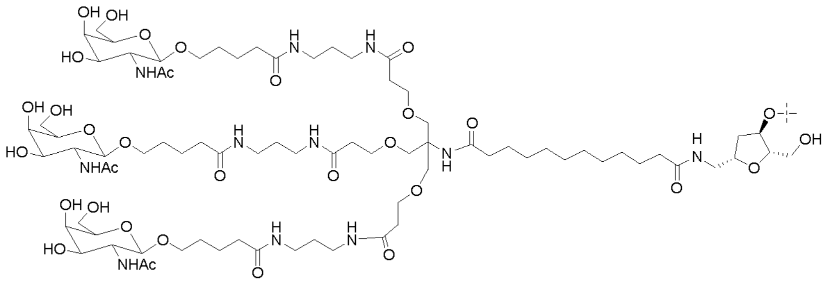

,

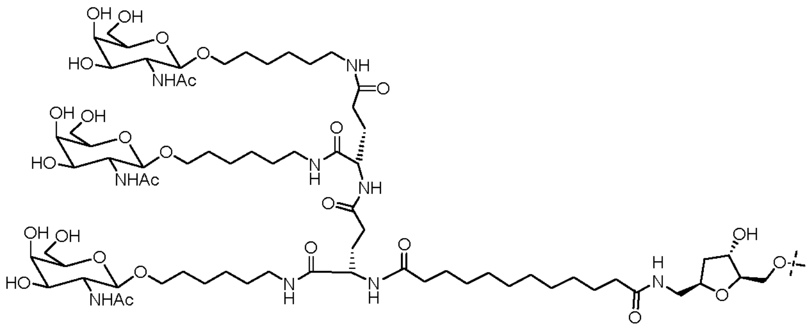

,

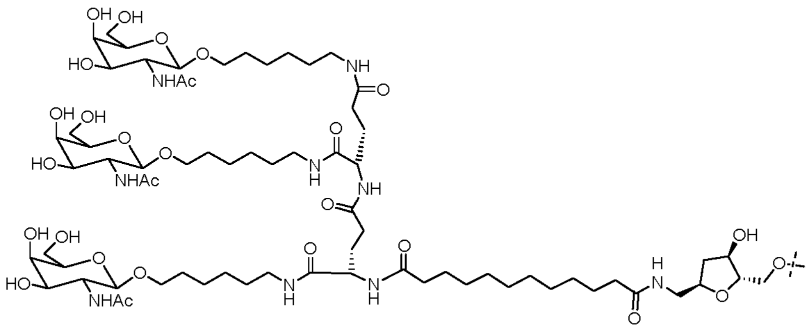

,

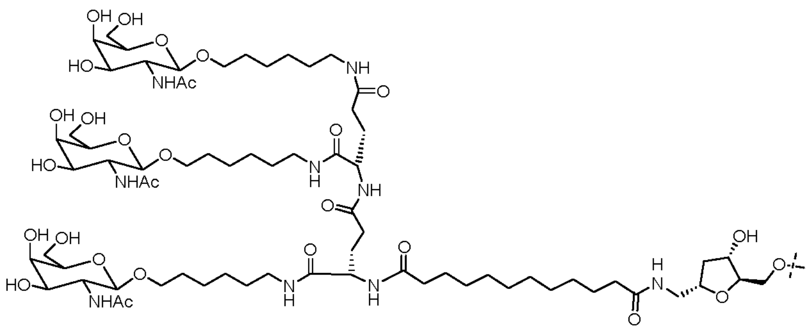

,

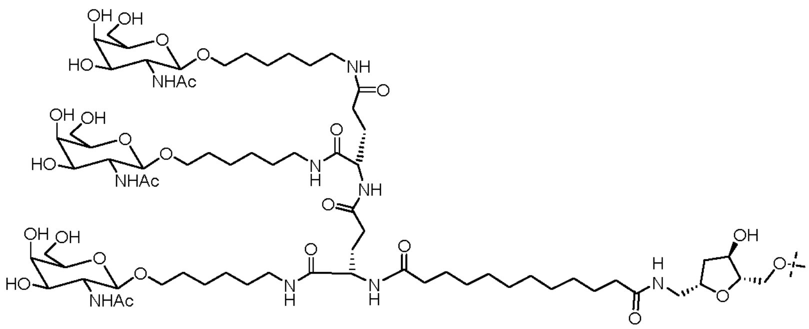

,

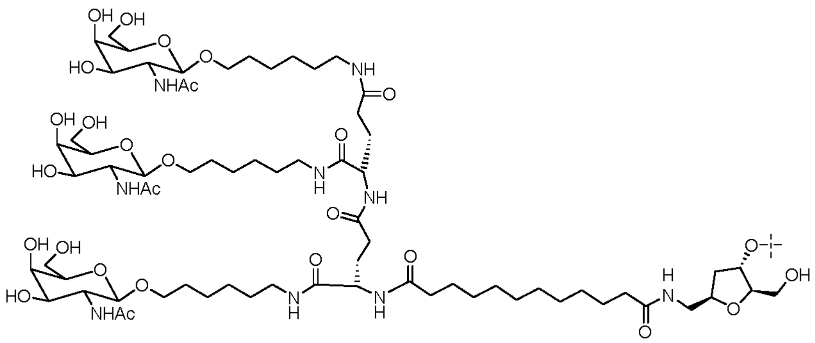

,

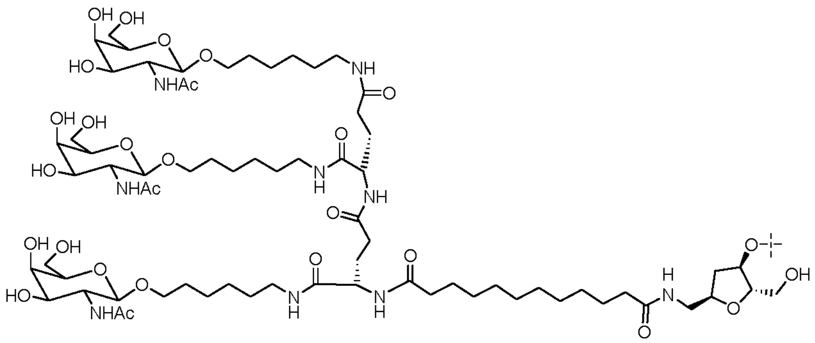

,

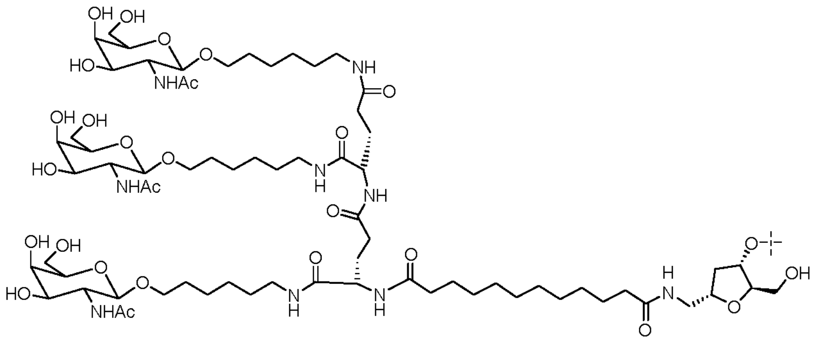

,

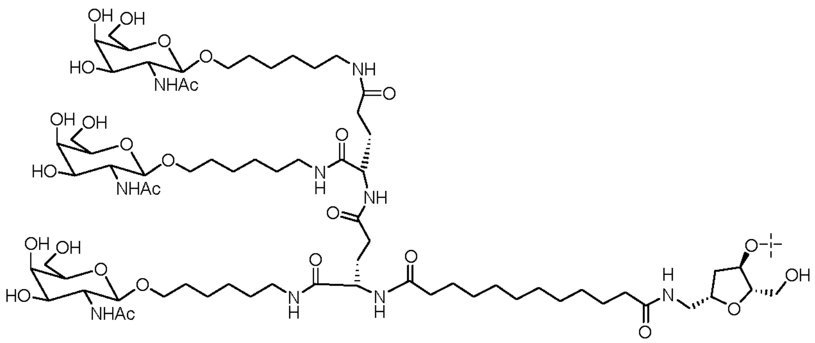

,

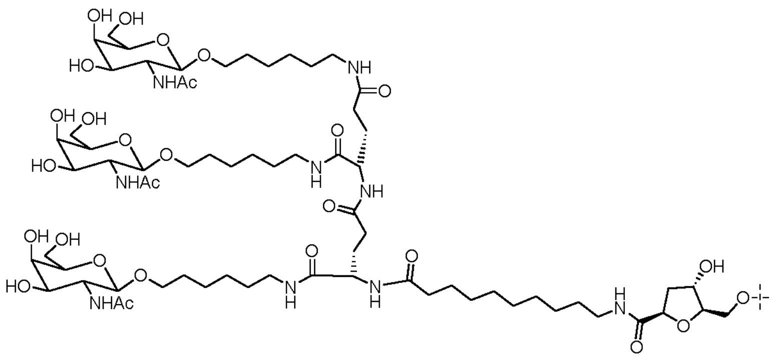

,

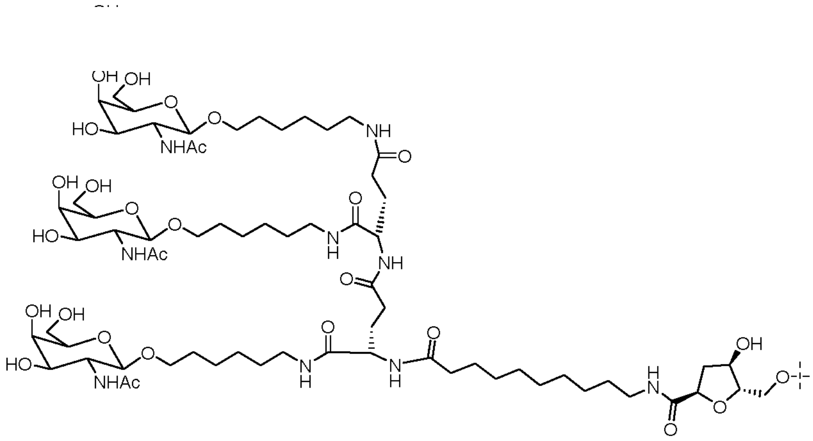

,

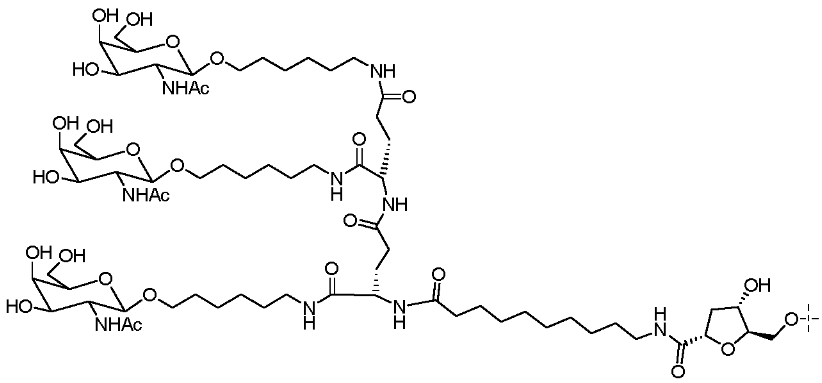

,

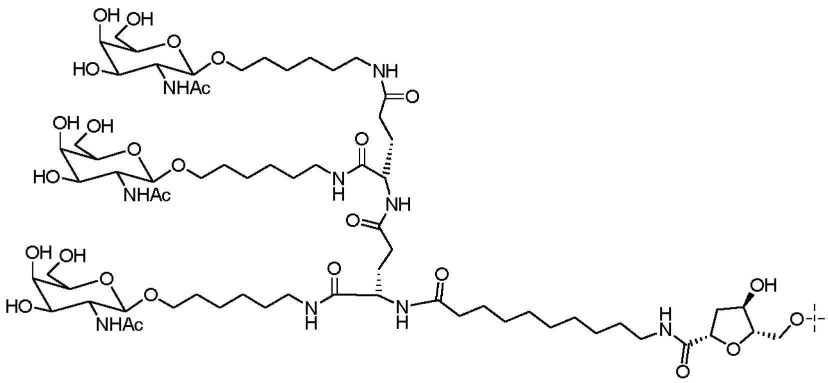

,

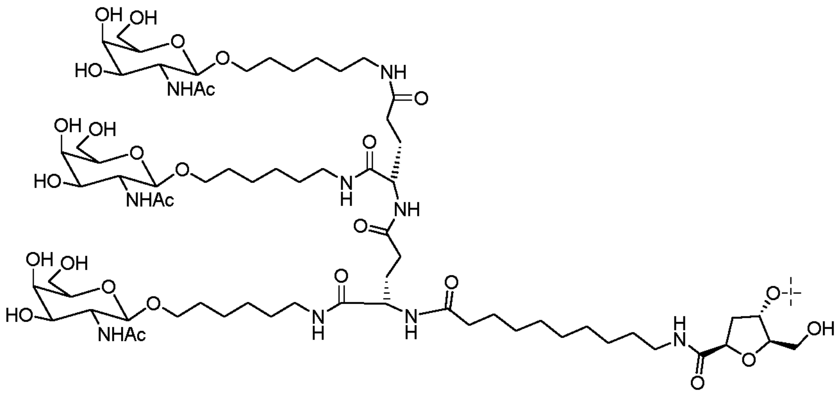

,

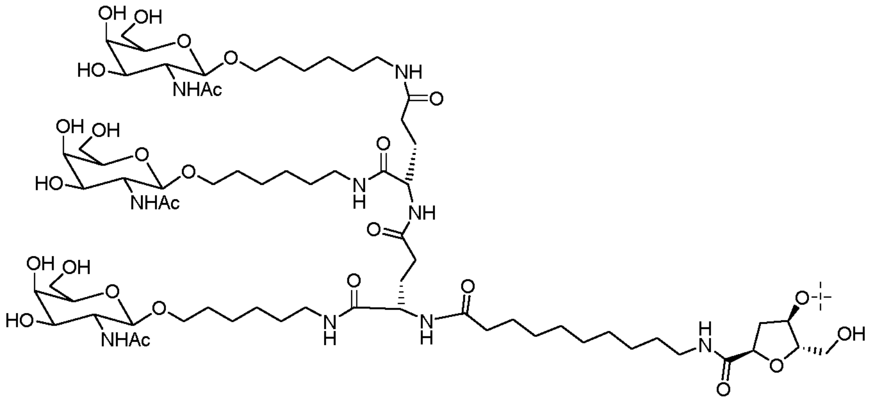

,

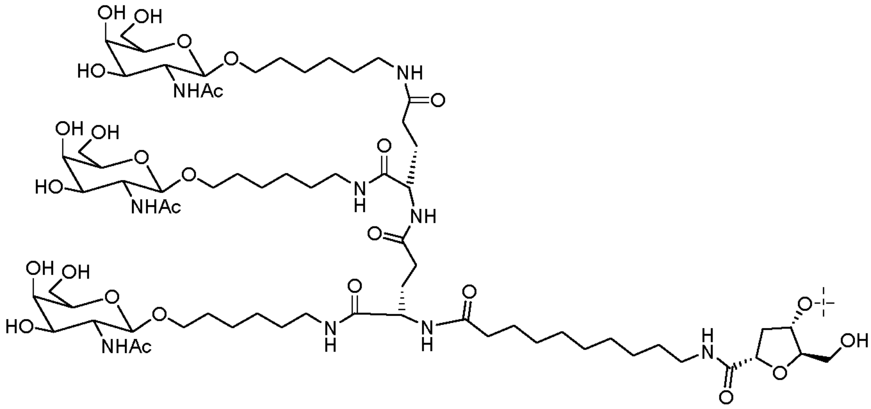

,

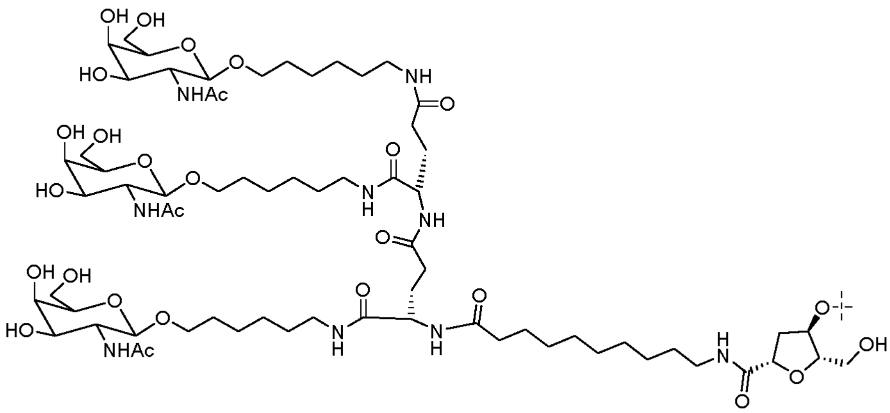

,

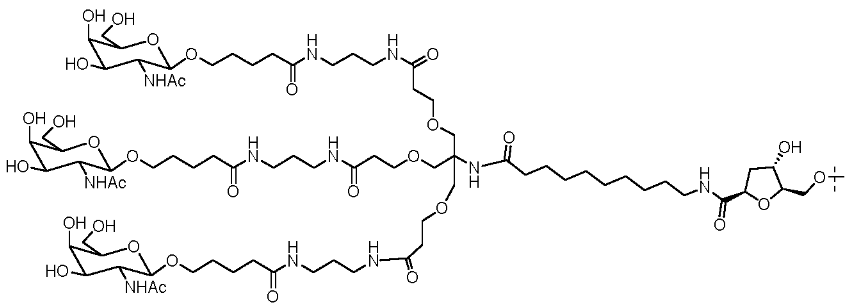

,

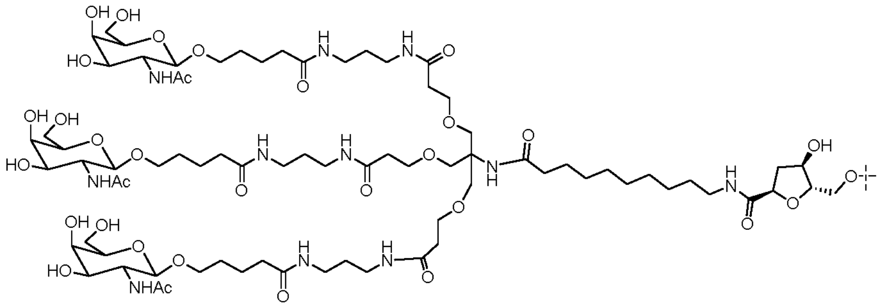

,

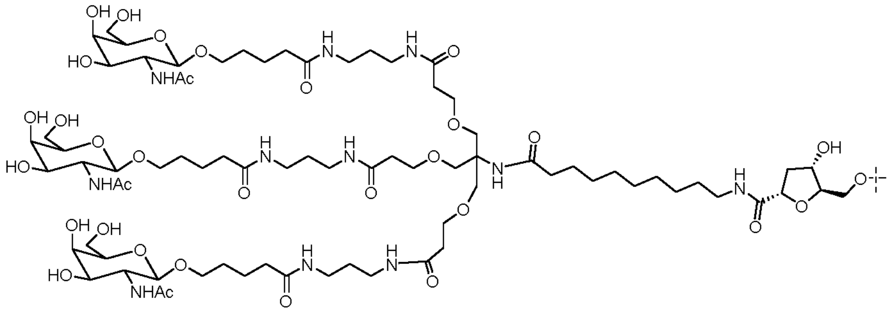

,

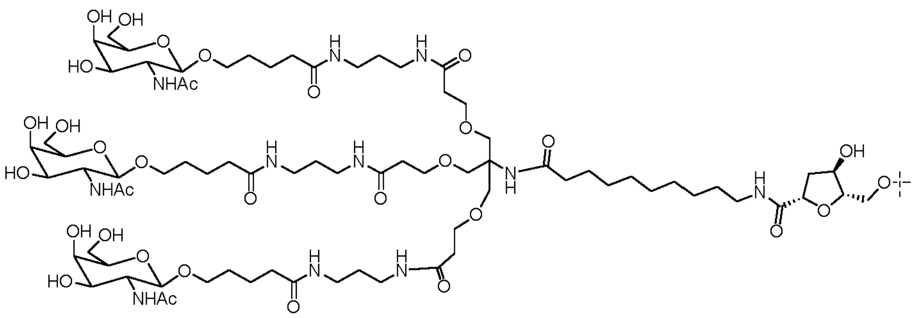

,

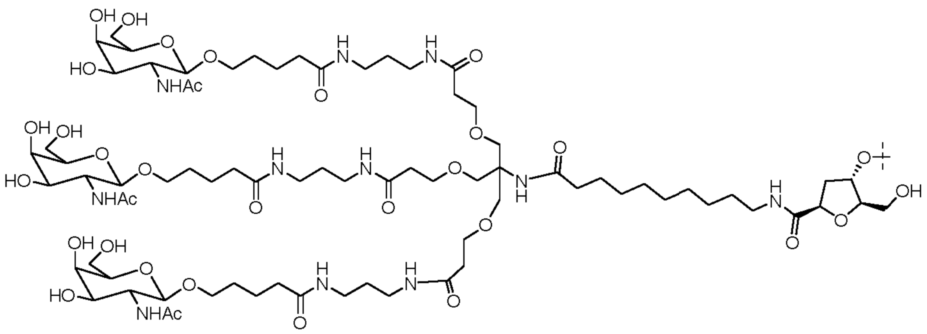

,

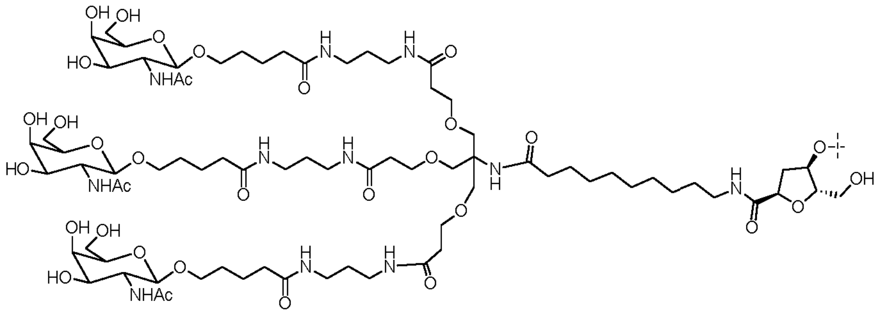

,

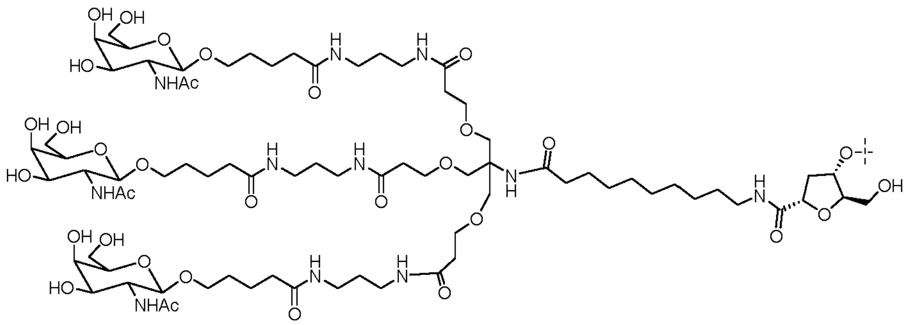

or

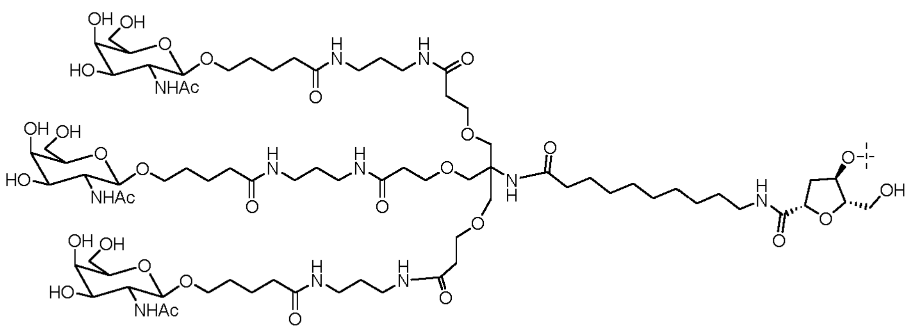

.

**17.** A compound represented by formula (II),

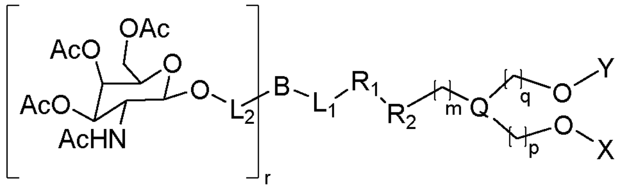

(II)

wherein X is a hydroxy protecting group, and the hydroxy protecting group is preferably an ester group protecting group, an alkoxymethyl protecting group, an alkyl protecting group, a silyl protecting group or an aryl protecting group, more preferably MMTr and DMTr, and further preferably DMTr;
Y is hydrogen, deuterium,

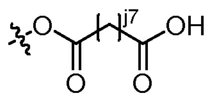 or 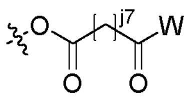 ;

j7 is 1, 2, 3 or 4, preferably 2;
W is a macromolecular compound, preferably resin, more preferably macroporous resin, and further preferably macroporous aminomethyl resin;
$L_1$, $R_1$, $R_2$, Q, B, $L_2$, m, p, q and r are as defined in any of claims 1-16.

**18.** The compound according to claim 17, being a compound represented by formula (II-1), (II-2) or (II-3),

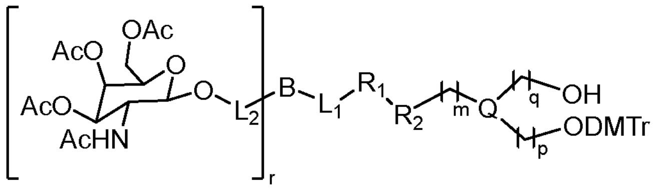

(II-1) ,

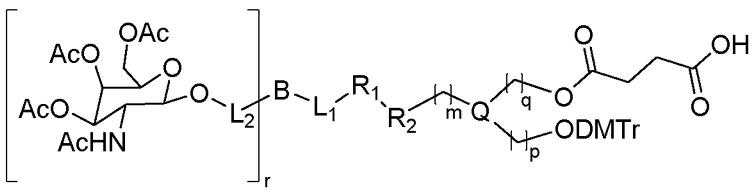

(II-2)

or

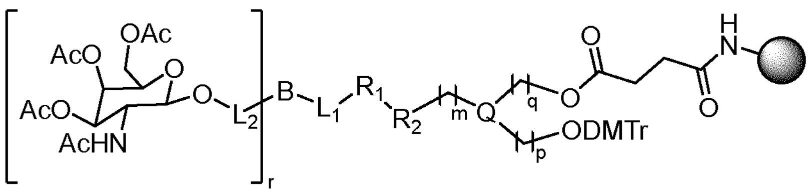

(II-3) ;

wherein

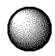

is resin, preferably macroporous resin, and more preferably macroporous aminomethyl resin; $L_1$, $R_1$, $R_2$, B, $L_2$, m, p, q and r are as defined in any of claims 1-16.

**19.** The compound according to claim 17 or 18, being any of the following structures:

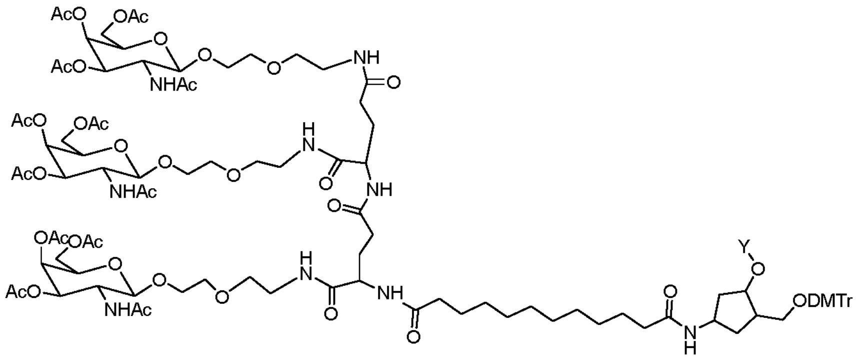

,

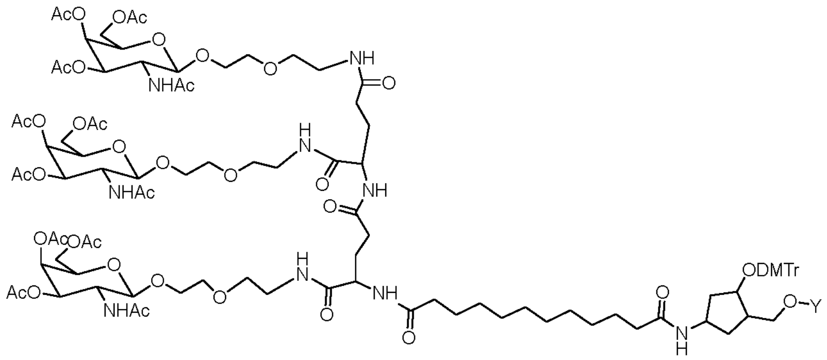

,

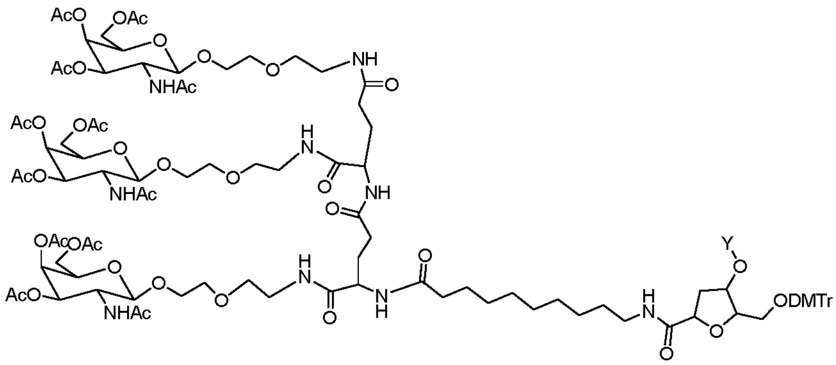

,

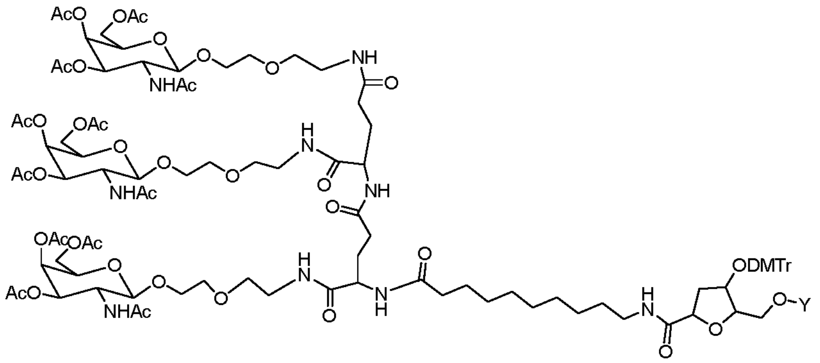

,

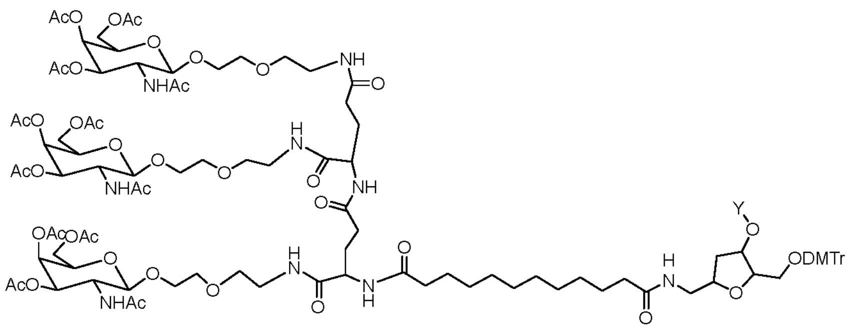

,

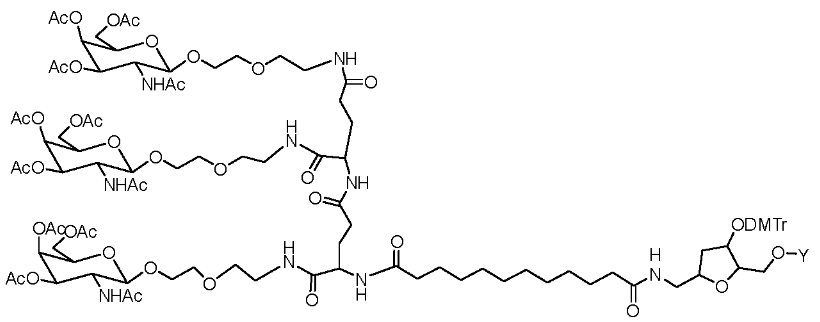

,

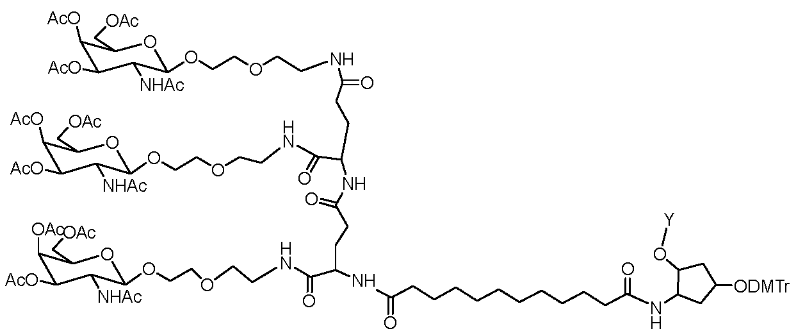

,

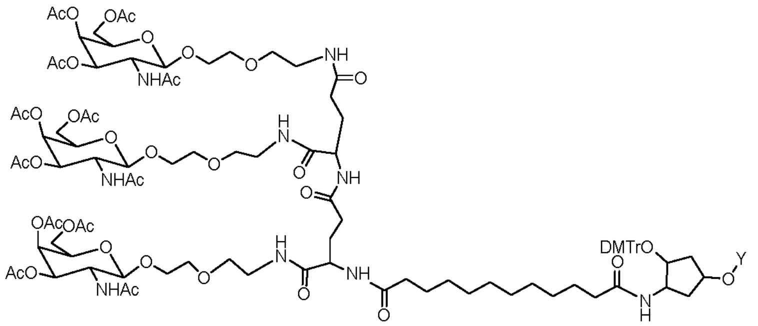

,

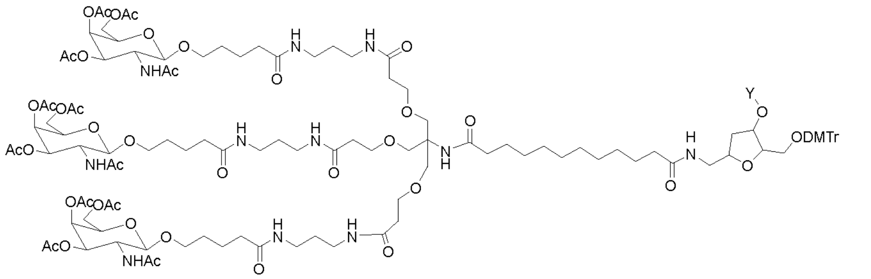

,

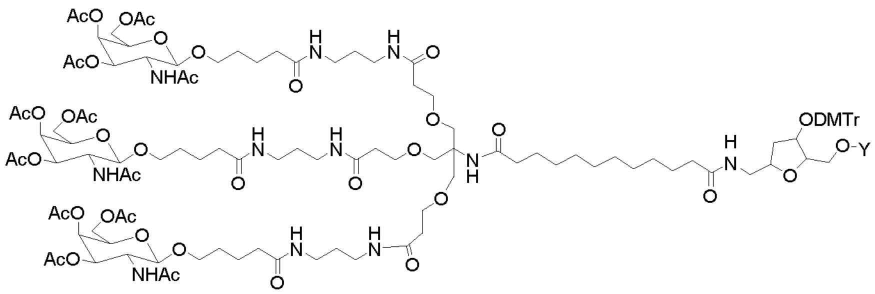

,

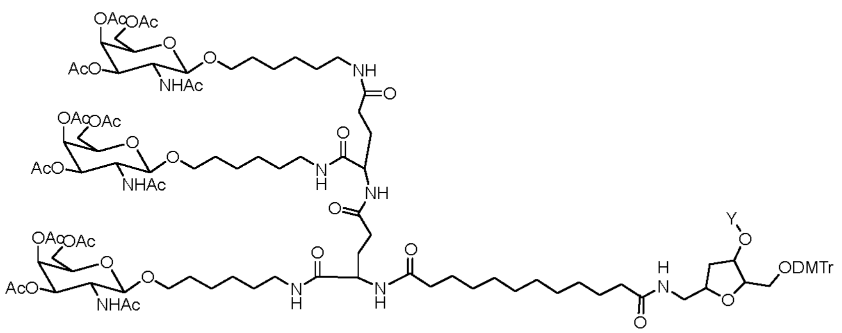

,

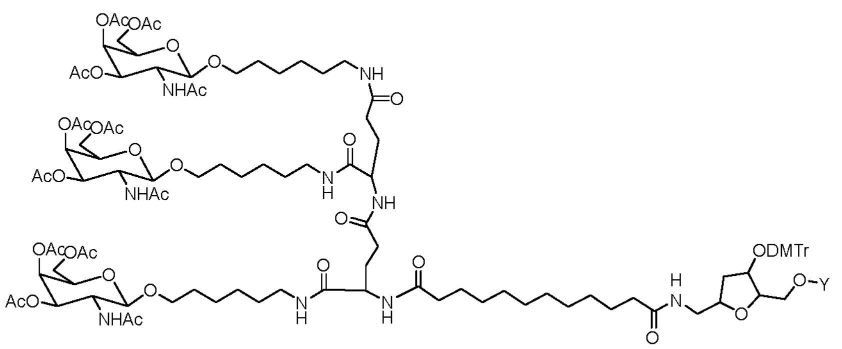

,

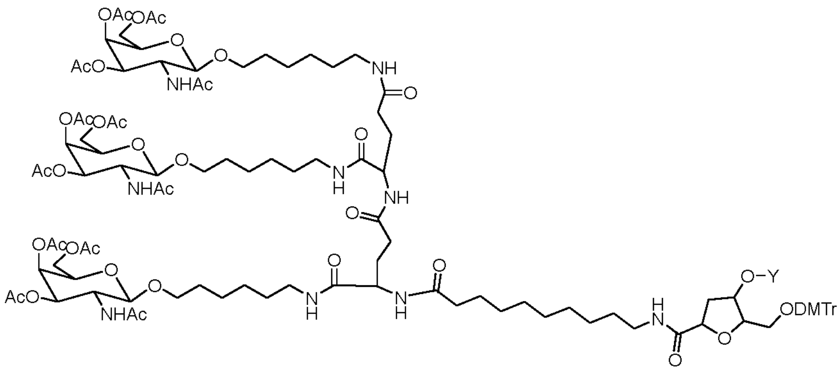

,

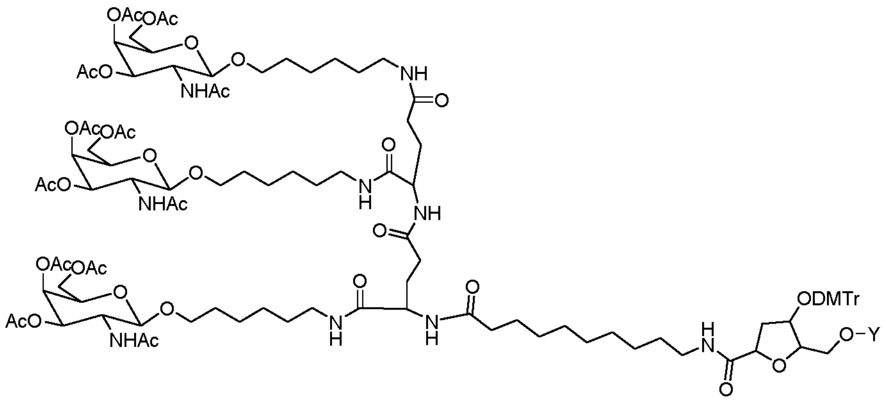

,

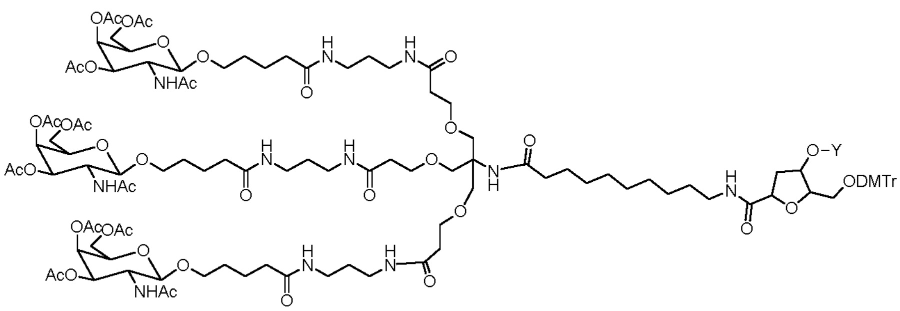

or

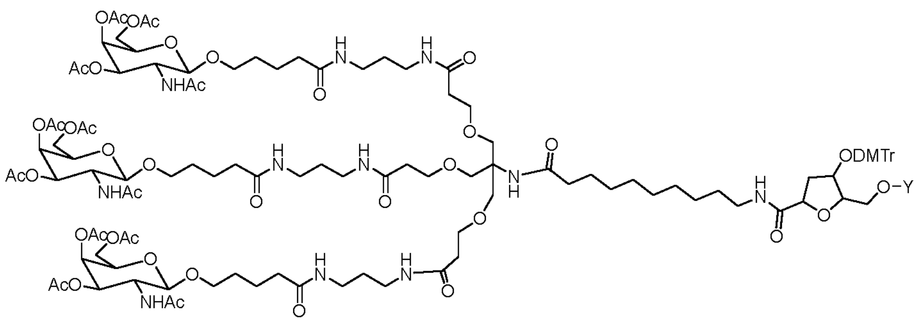

;

wherein Y is hydrogen or

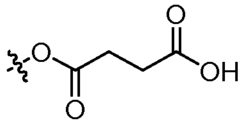

;

or, Y is

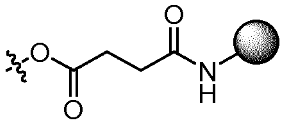

;

wherein

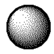

is resin, preferably macroporous resin, and more preferably macroporous aminomethyl resin.

**20.** The compound according to any of claims 17-19, being any of the following structures:

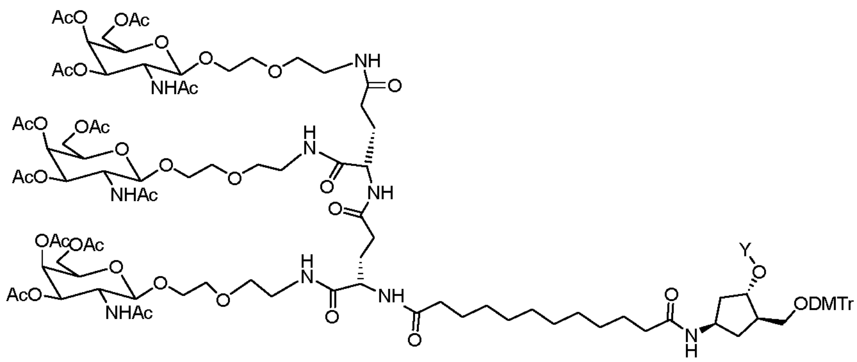

,

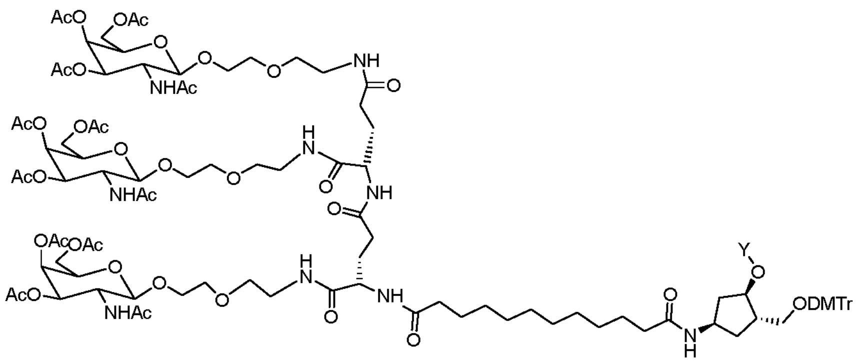

,

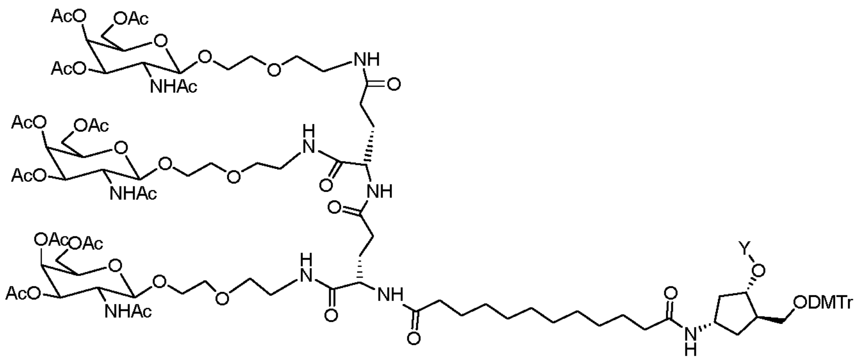

,

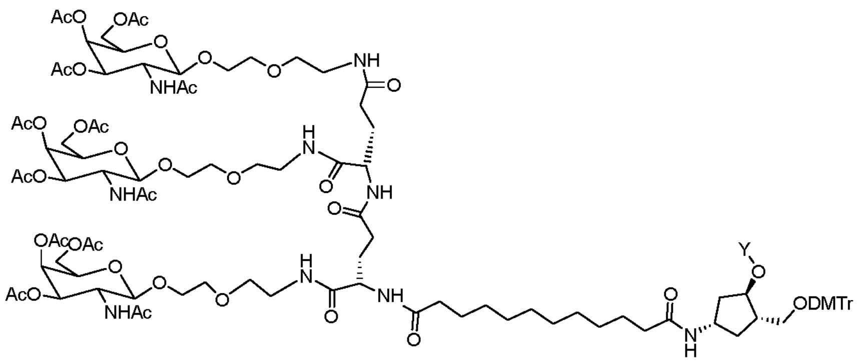

,

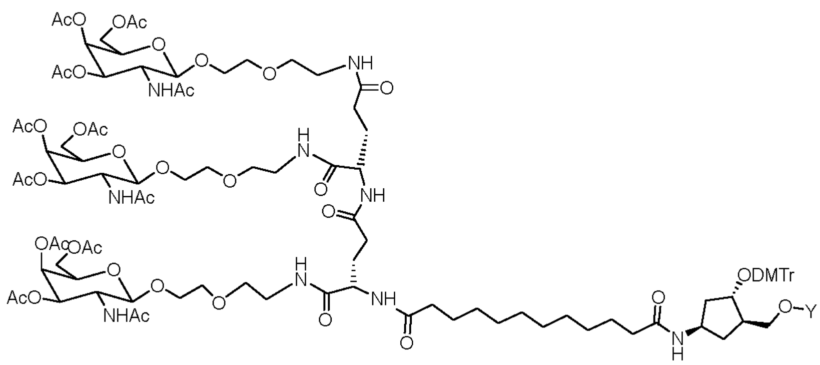

,

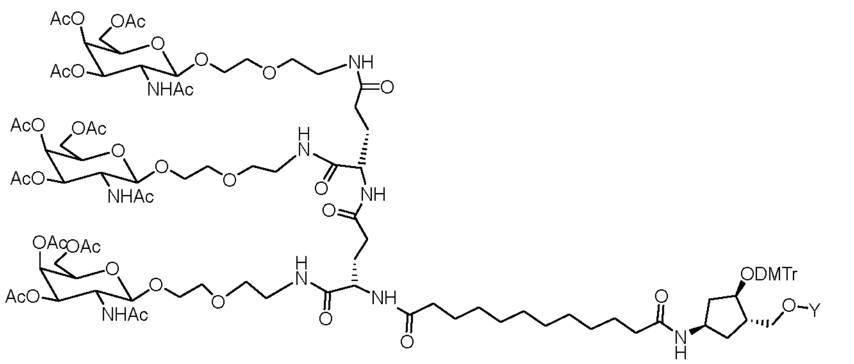

.

,

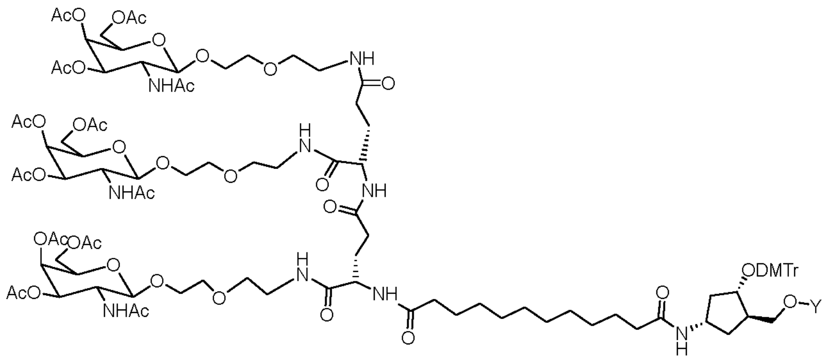

,

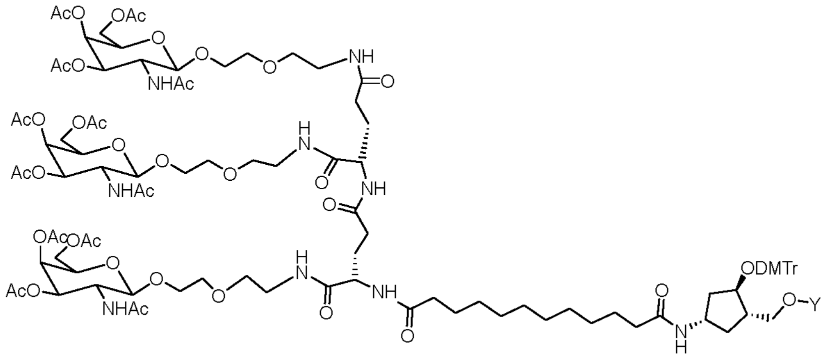

,

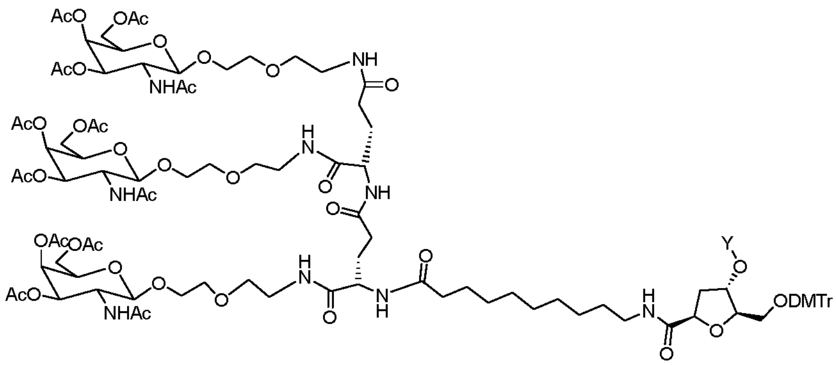

,

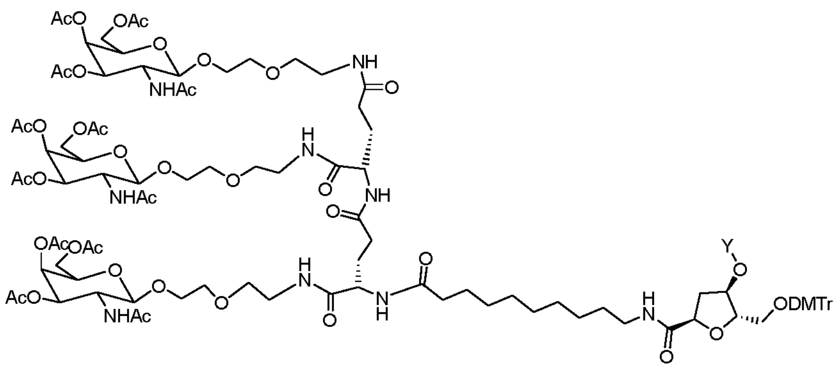

,

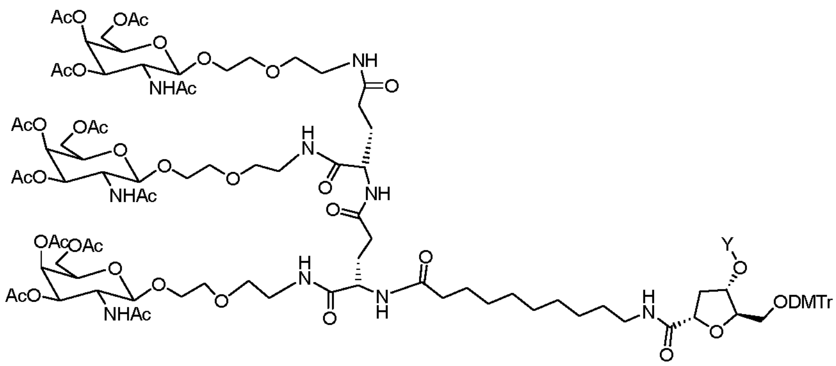

,

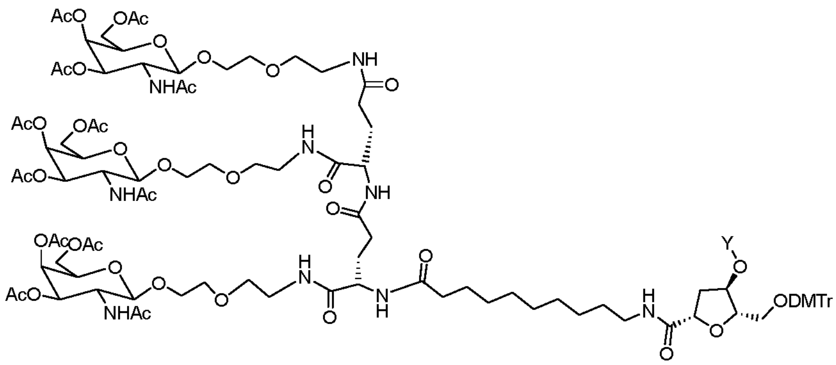

,

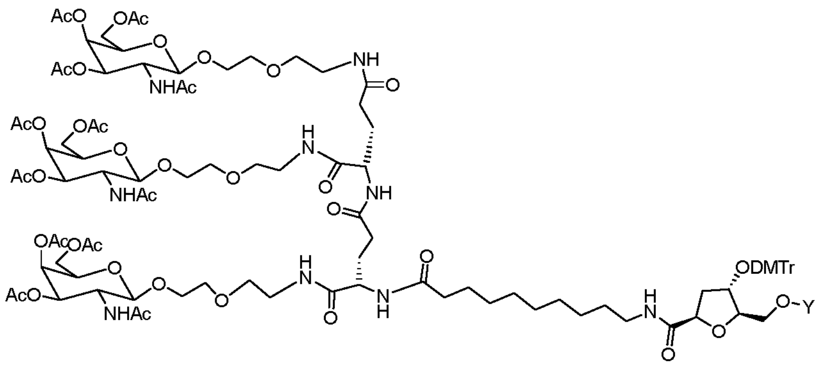

,

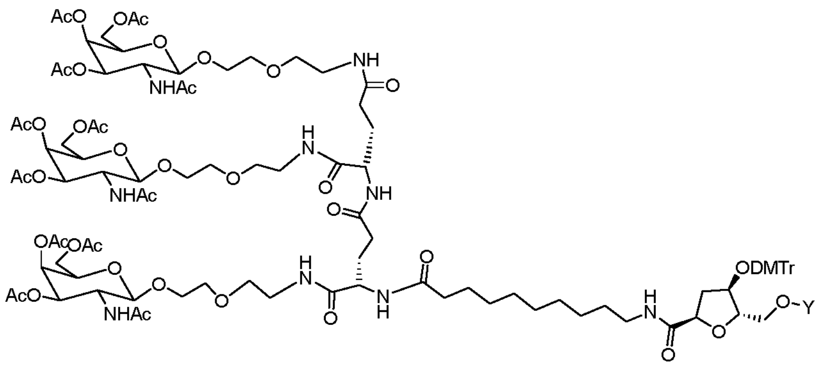

,

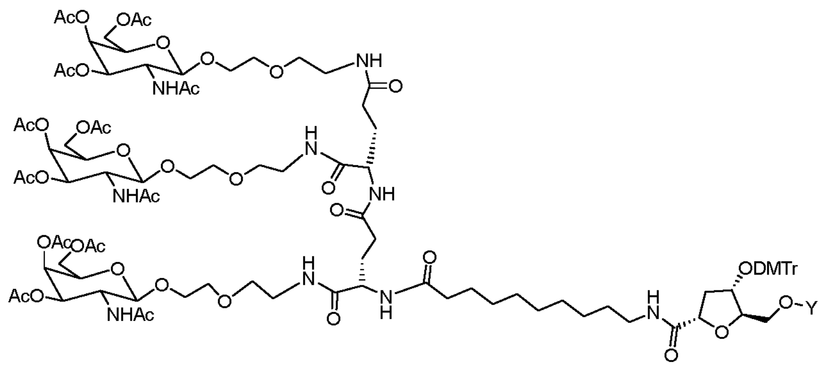

,

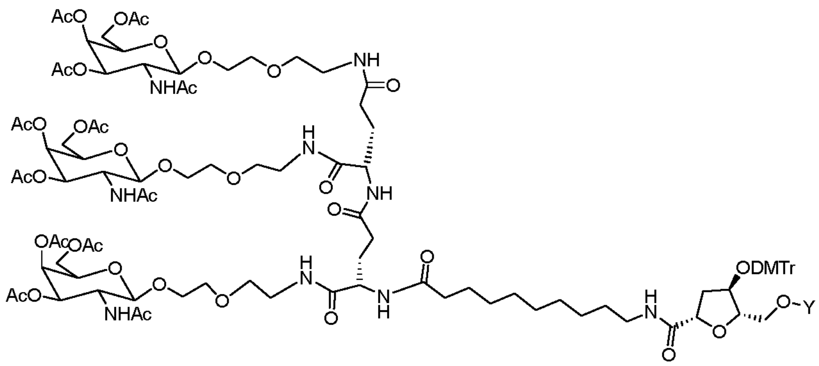

,

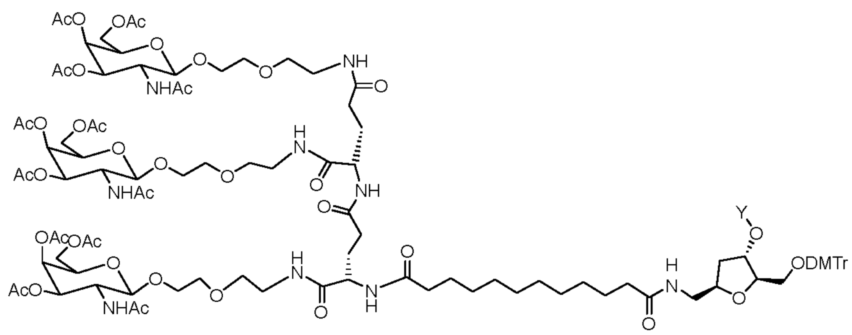

,

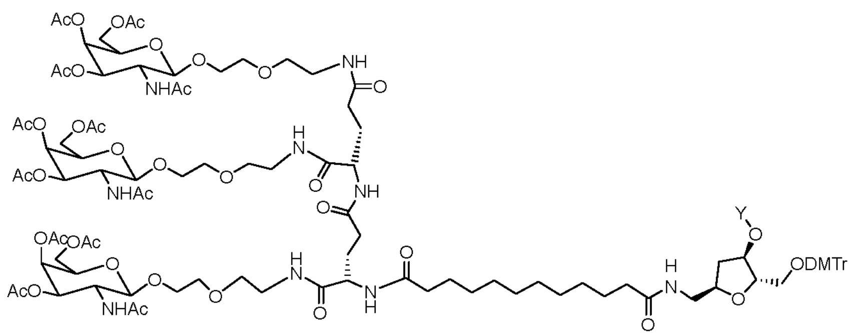

,

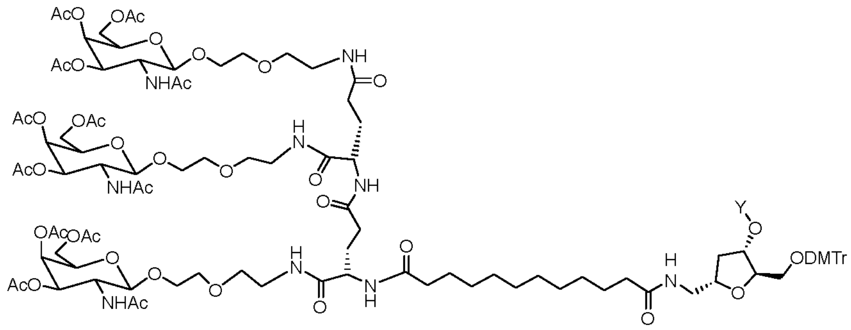

,

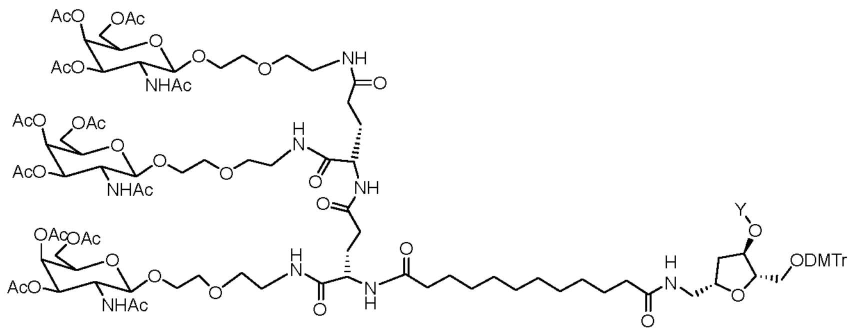

,

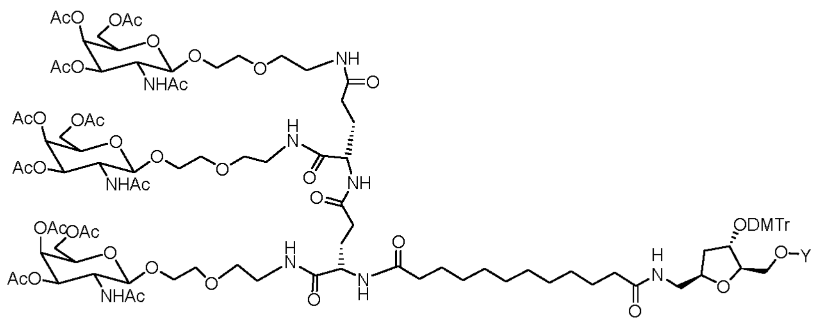

,

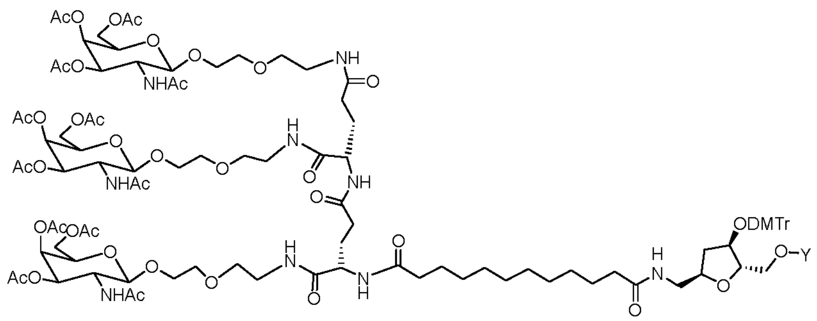

,

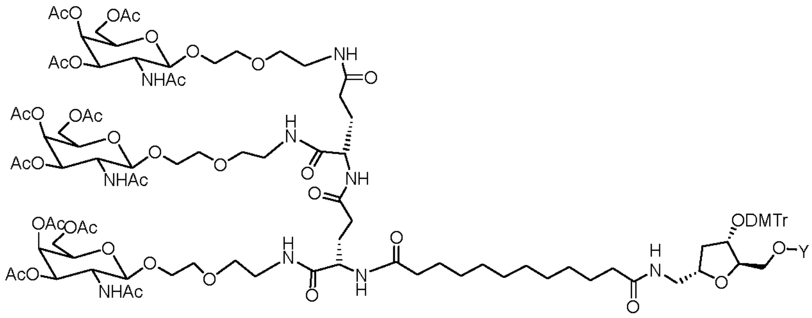

,

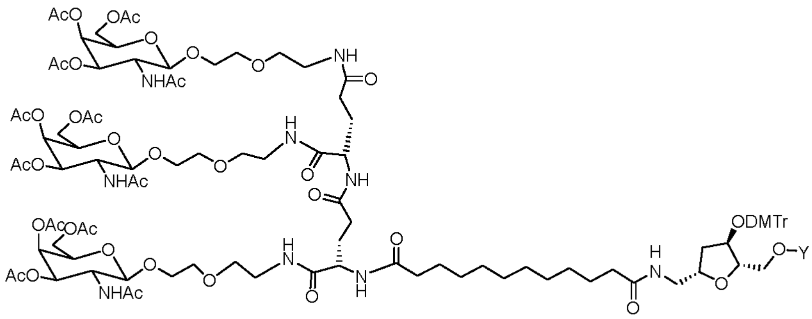

,

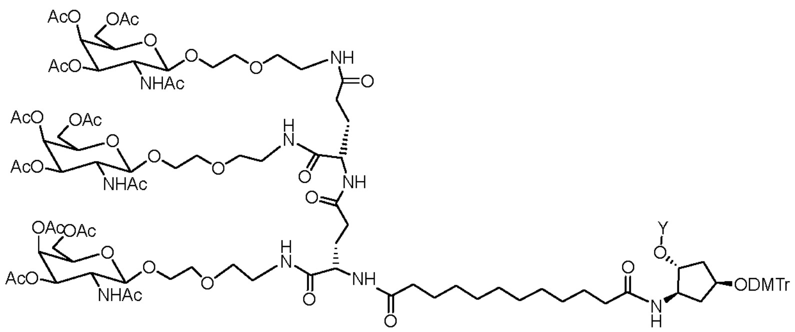

,

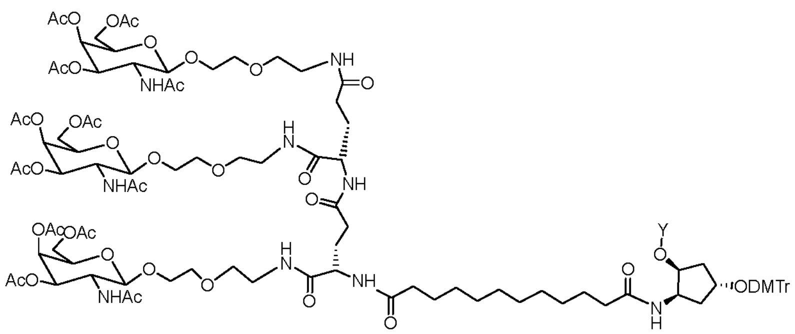

,

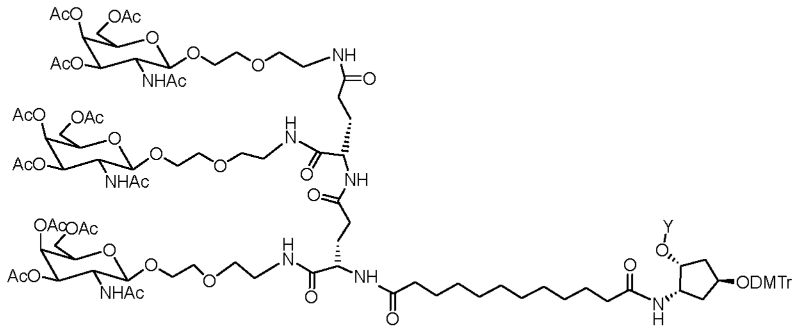

,

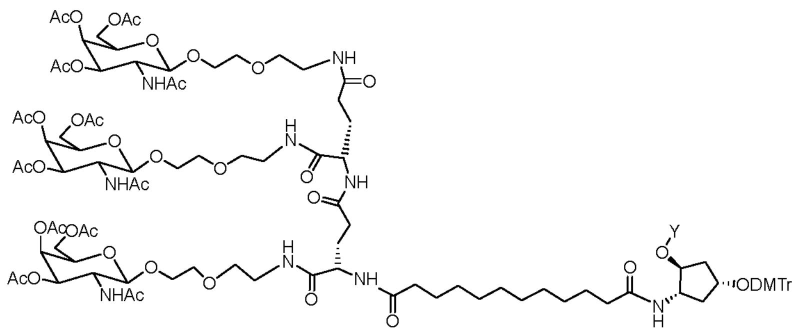

,

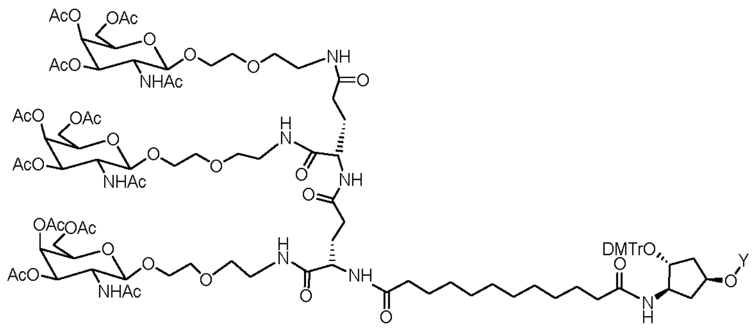

,

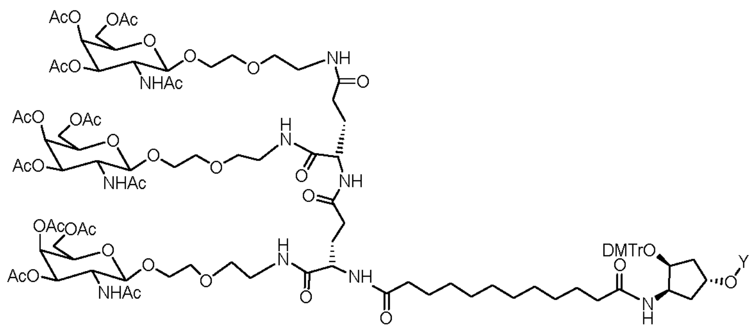

,

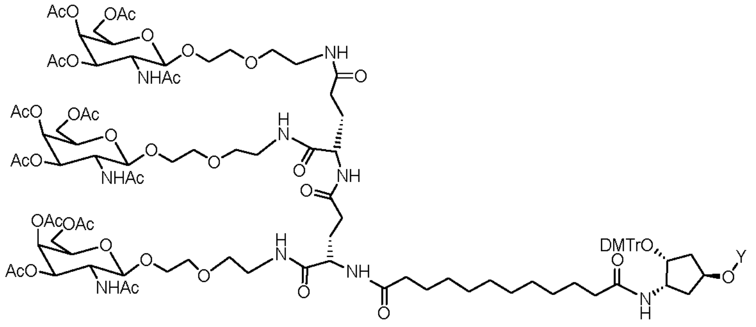

,

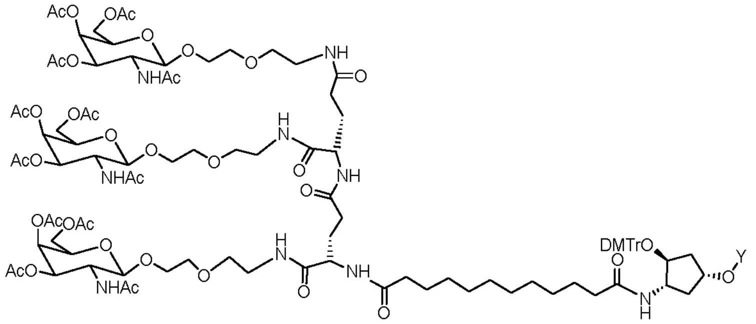

,

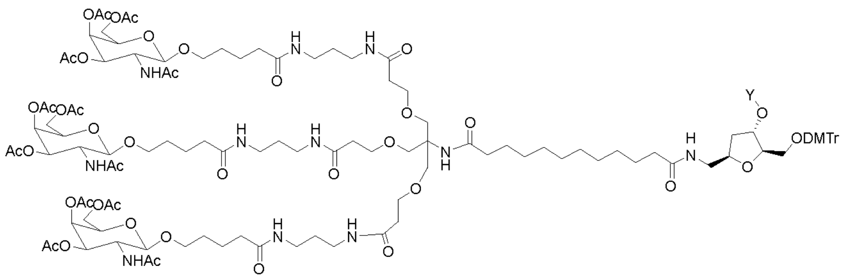

,

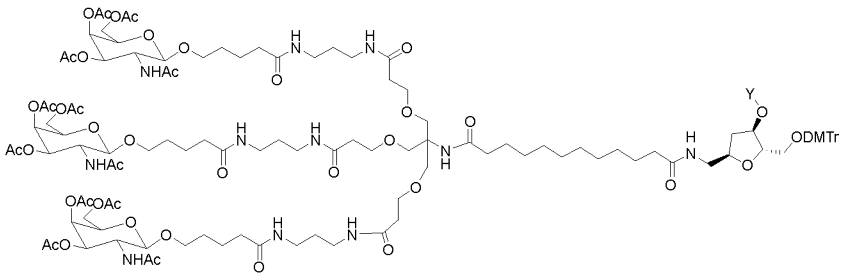

,

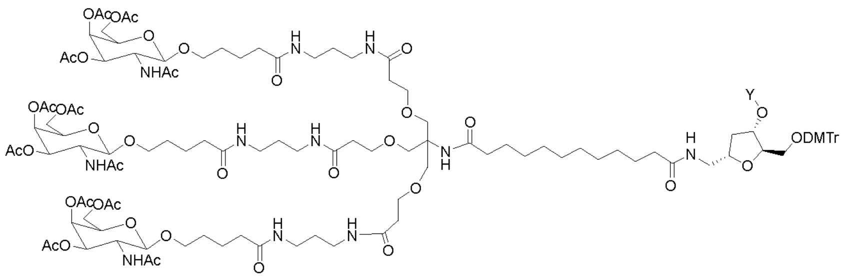

,

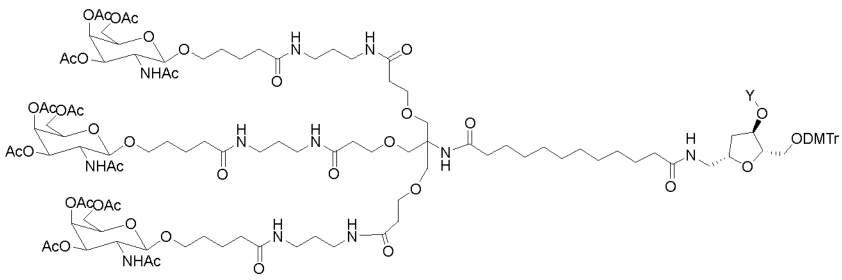

,

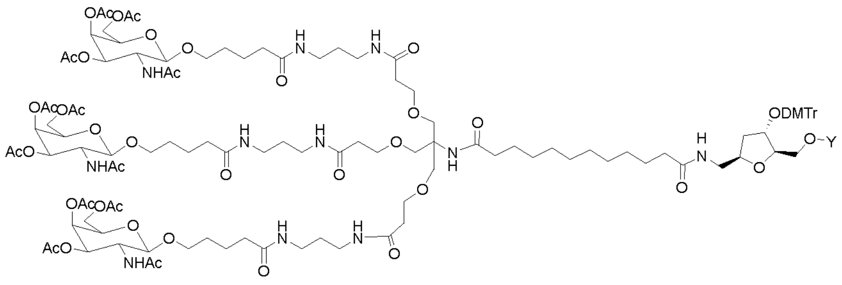

,

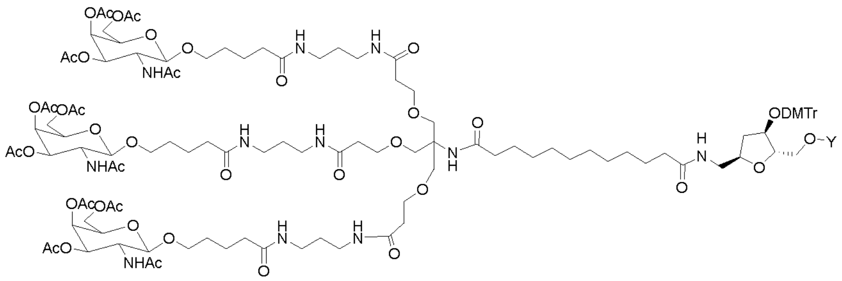

,

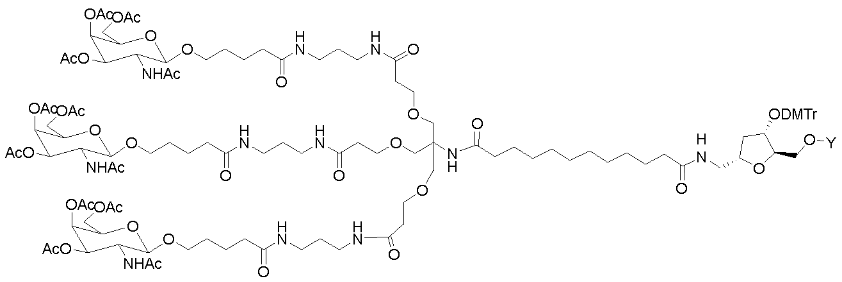

,

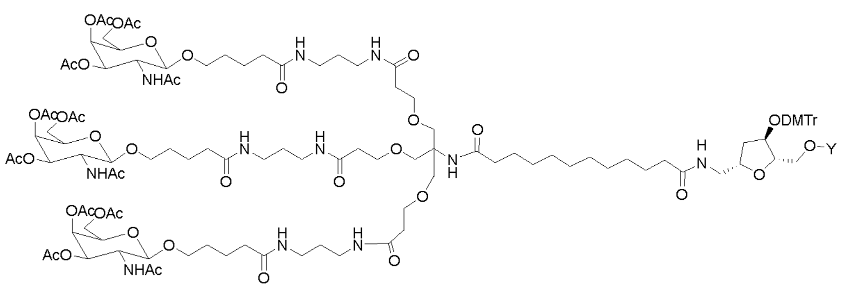

,

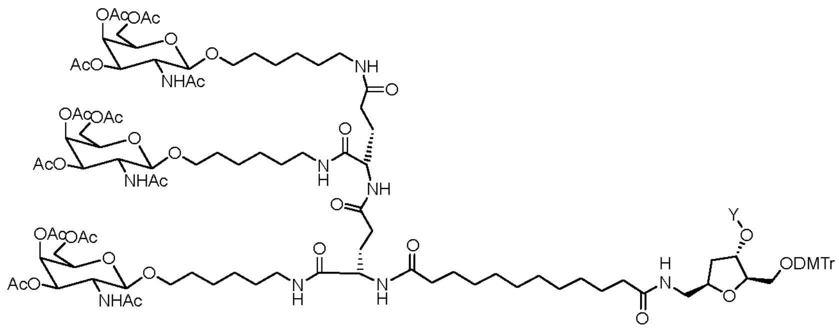

,

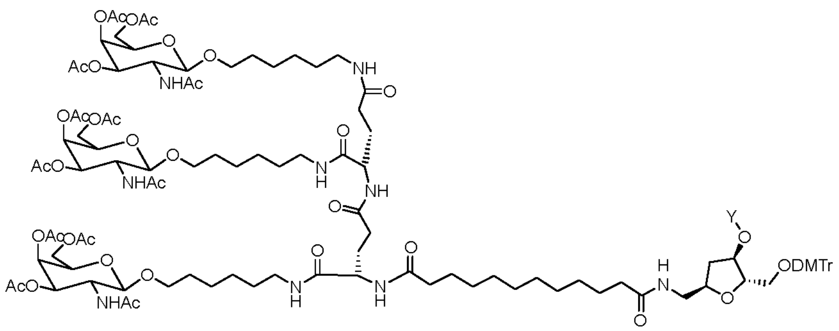

,

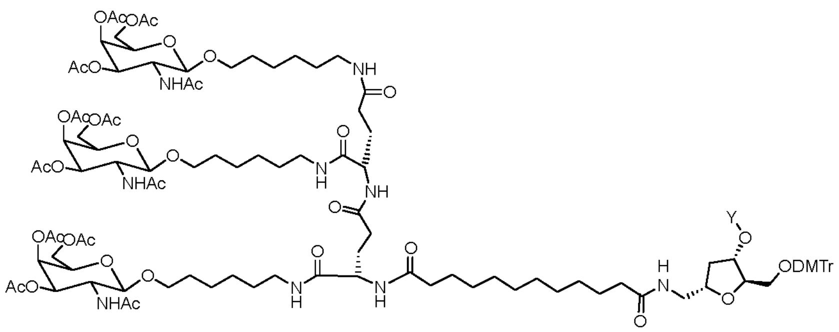

,

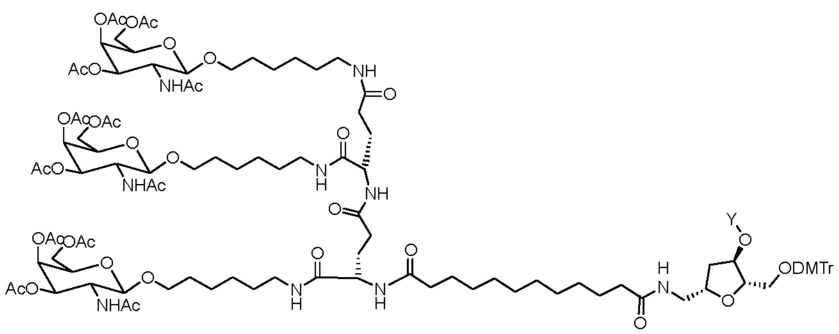

,

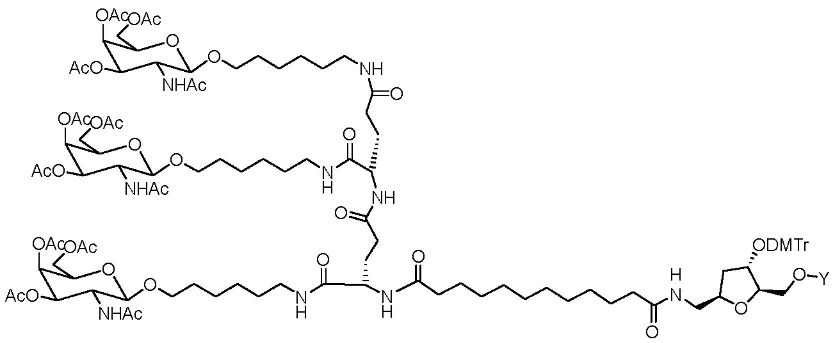

,

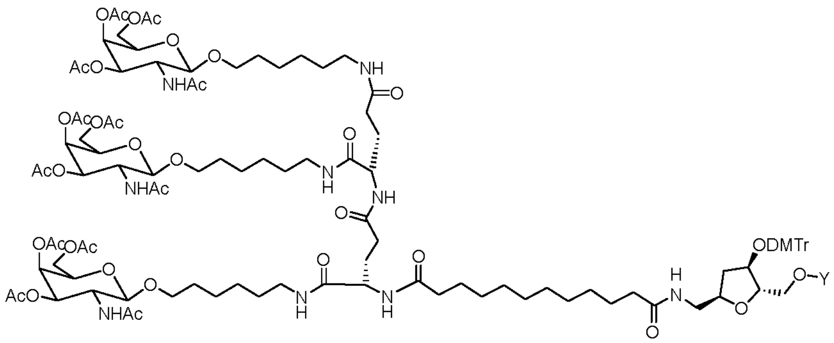

,

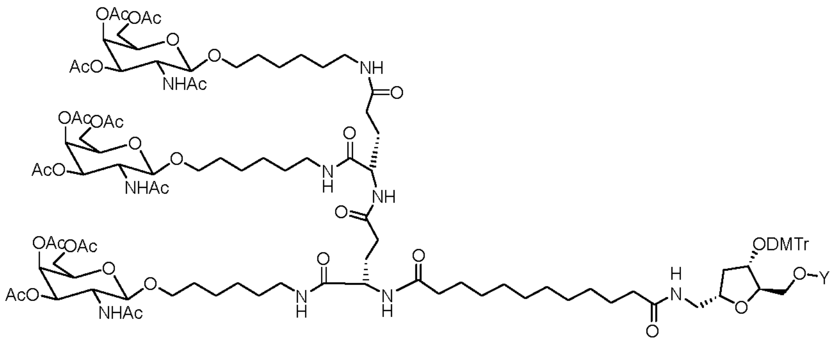

,

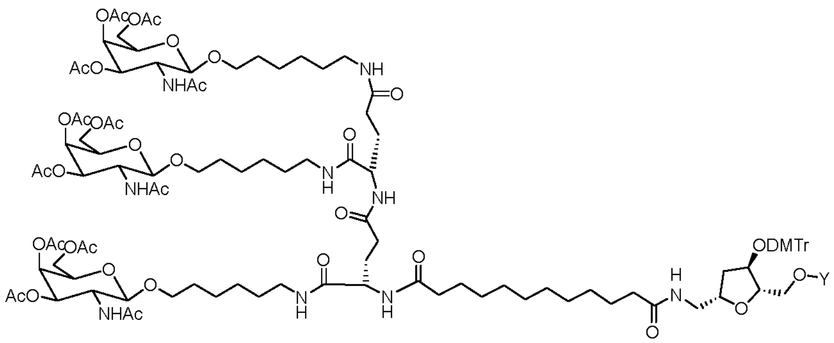

,

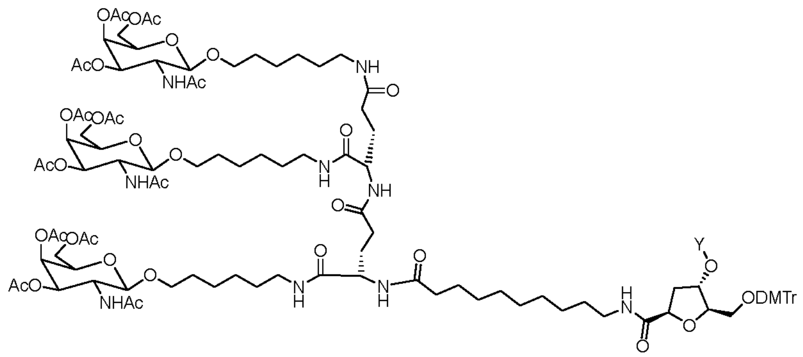

,

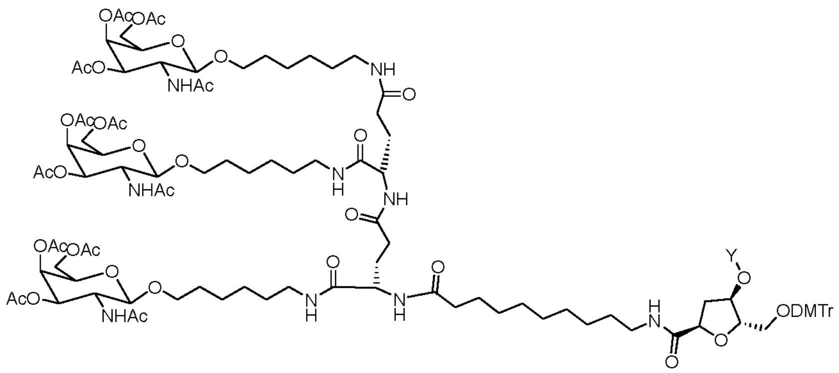

,

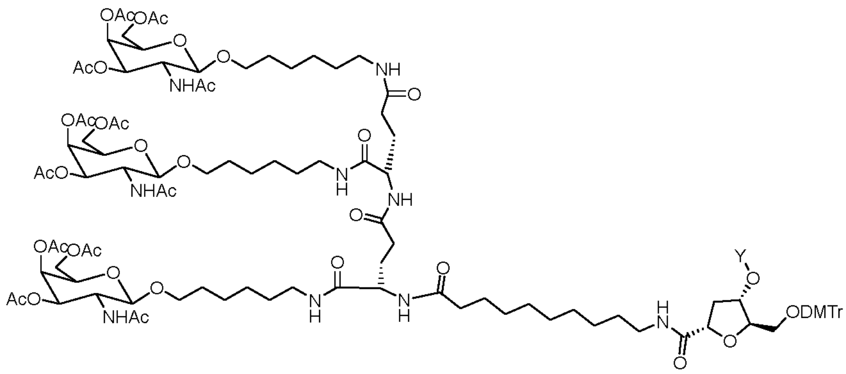

,

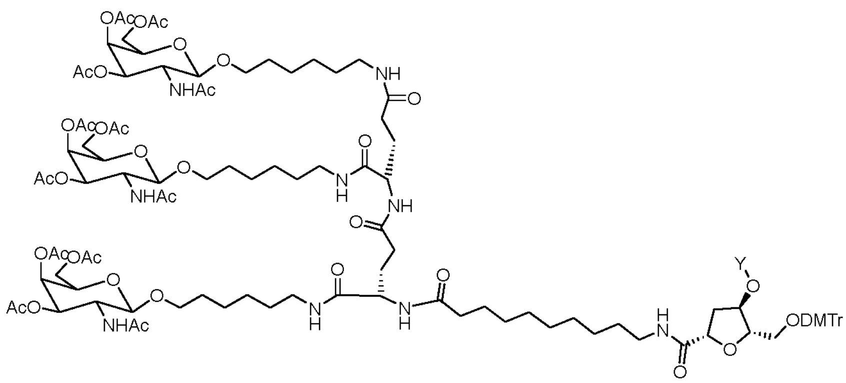

,

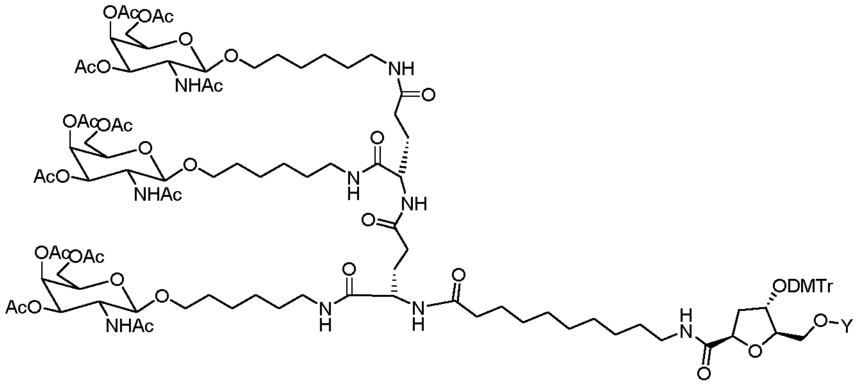

,

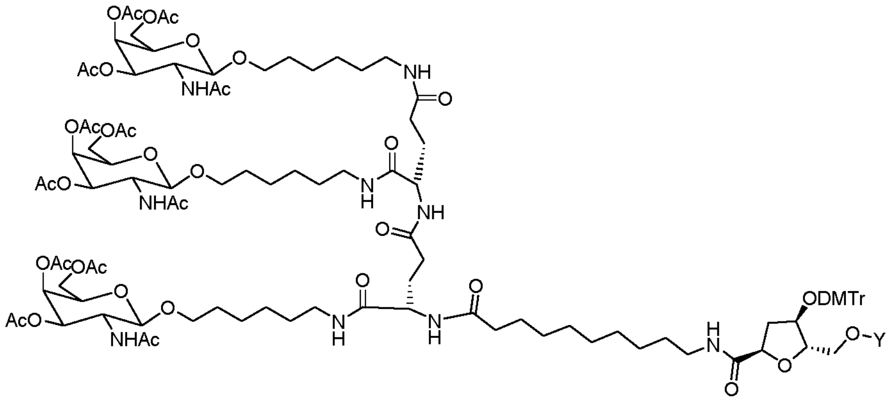

,

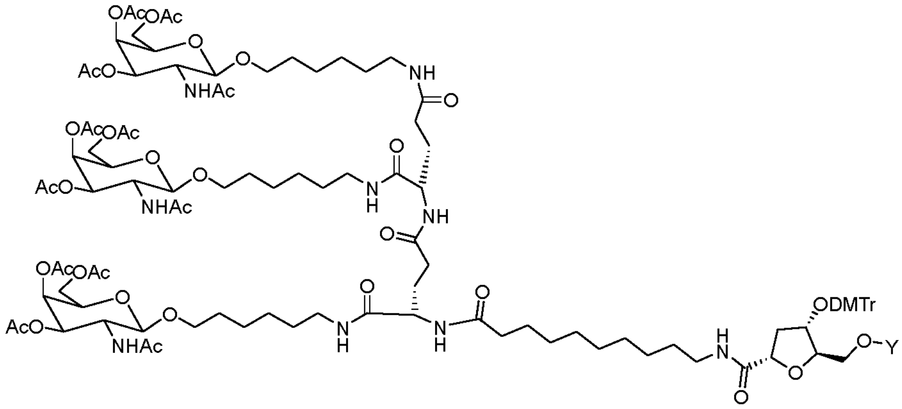

,

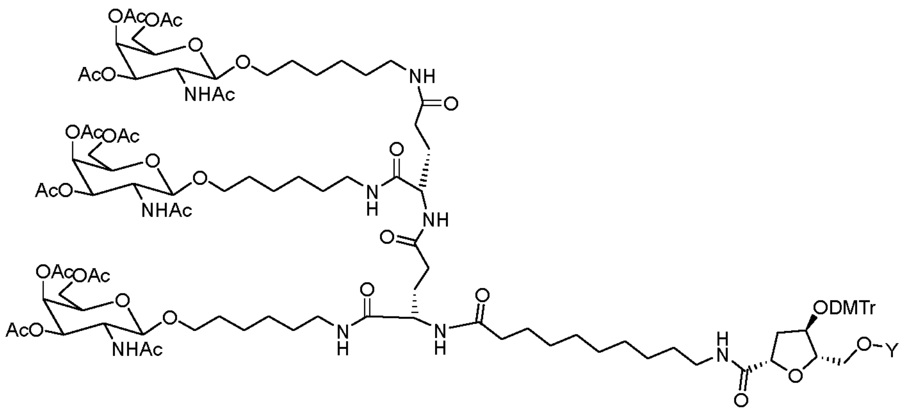

,

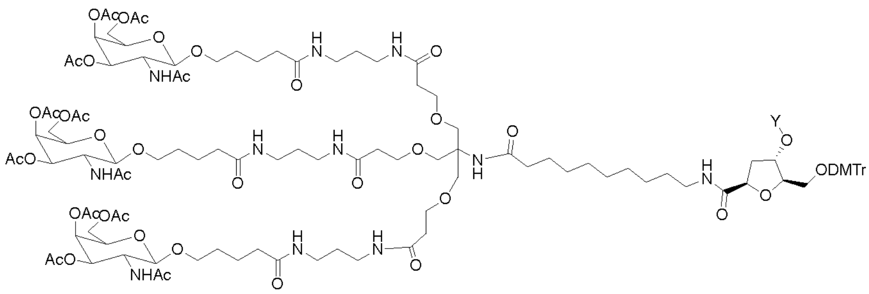

,

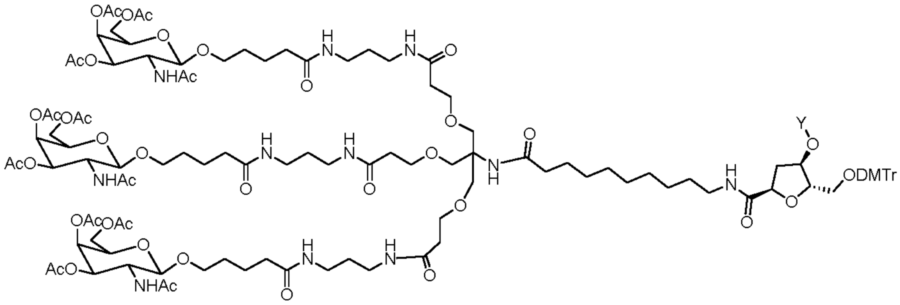

,

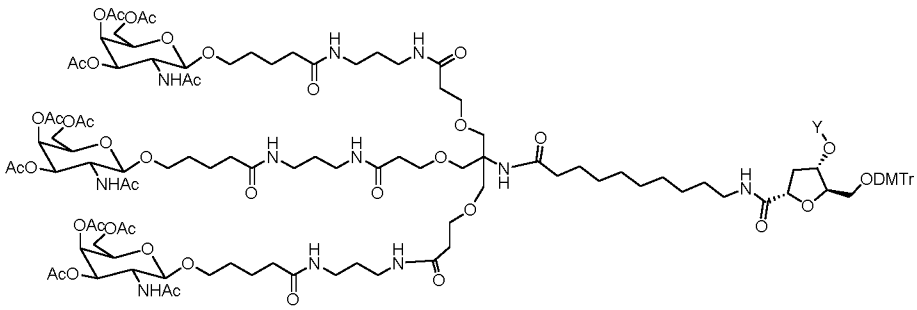

,

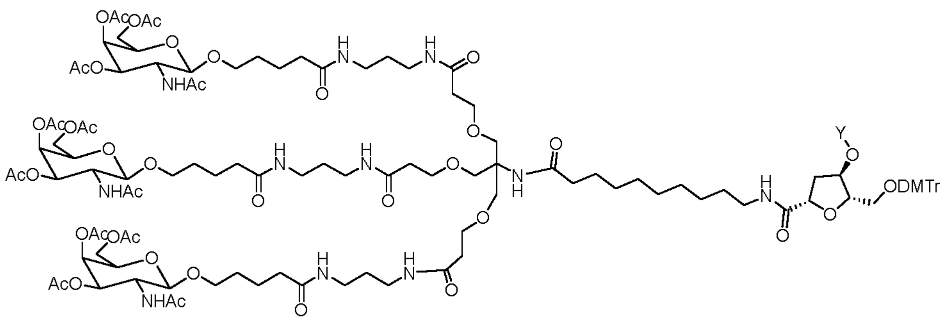

,

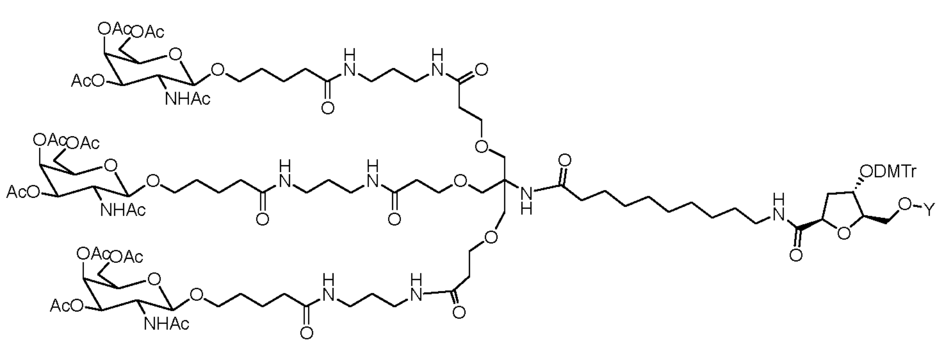

,

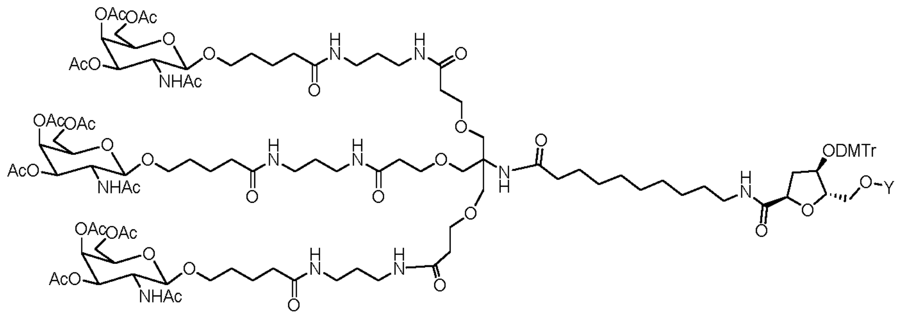

,

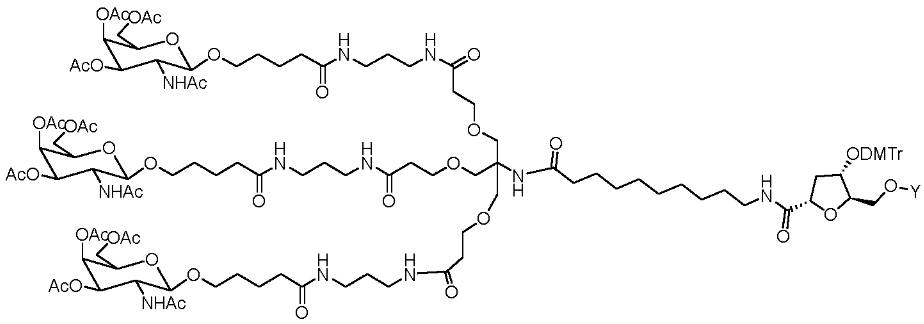

or

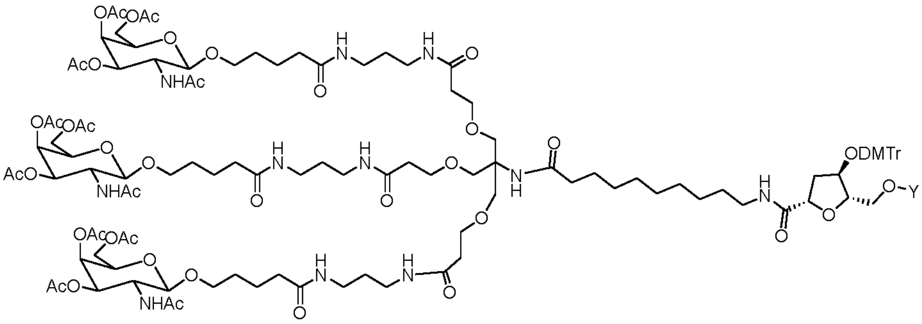

;

wherein Y is hydrogen or

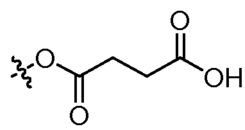

;

or, Y is

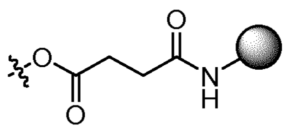

,

wherein

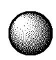

is resin, preferably macroporous resin, and more preferably macroporous aminomethyl resin.

**21.** A nucleic acid-ligand conjugate comprising a nucleic acid and one or more ligands according to any of claims 1-16, wherein the ligands are conjugated to an end of the nucleic acid, and the ligands are identical or different.

**22.** The nucleic acid-ligand conjugate according to claim 21, wherein the nucleic acid and the ligands are linked by phosphoester groups, phosphorothioate groups or phosphonic acid groups;

and/or, the 3' end of the nucleic acid is conjugated to the ligand;
and/or, the nucleic acid is a single-stranded nucleic acid, preferably the sense strand of an siRNA.

**23.** The nucleic acid-ligand conjugate according to claim 21 or 22, wherein the nucleic acid-ligand conjugate is any of the following structures or a pharmaceutically acceptable salt thereof:

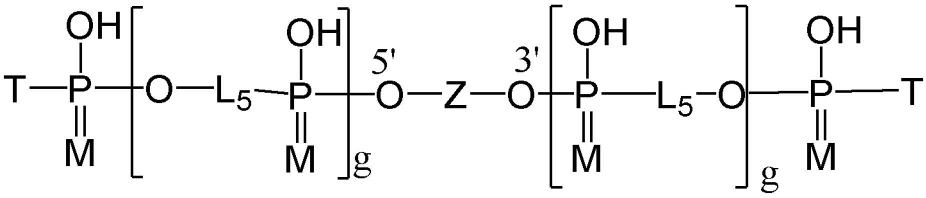
;

wherein T is the ligand according to any of claims 1-16, and each T is identical or different;
$L_5$ is independently a $C_1$-$C_{30}$ alkyl chain, or a $C_1$-$C_{30}$ alkyl chain interrupted by one or more oxygen, sulfur or nitrogen atoms or C=O, preferably

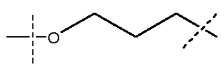
,

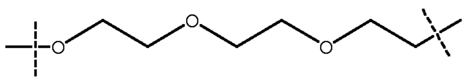

or

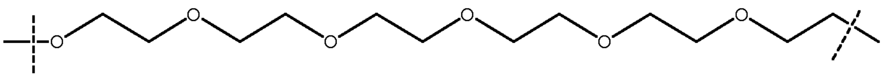
;

M is independently O or S;
g is independently an integer of 0-4, preferably 0 or 1, and more preferably 0;

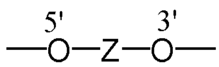
]

represents the nucleic acid.

**24.** The nucleic acid-ligand conjugate according to any of claims 21-23, wherein the nucleic acid-ligand conjugate is any of the following structures or a pharmaceutically acceptable salt thereof:

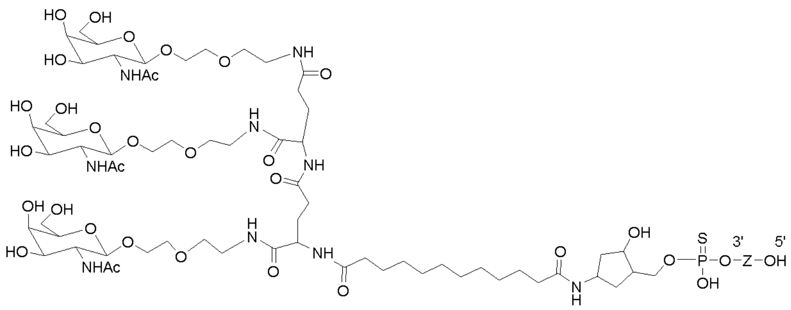

,

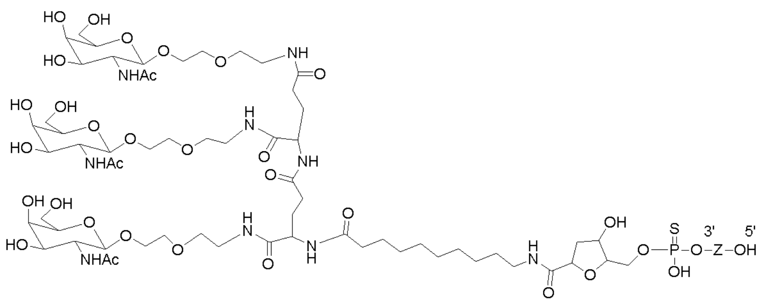

,

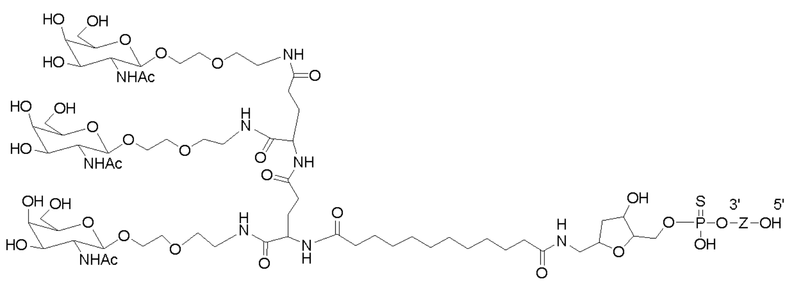

,

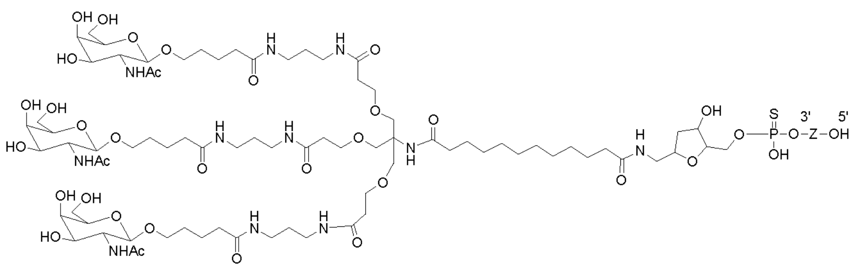

,

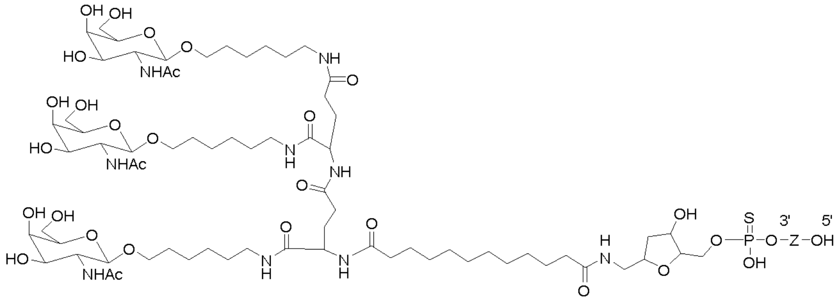

,

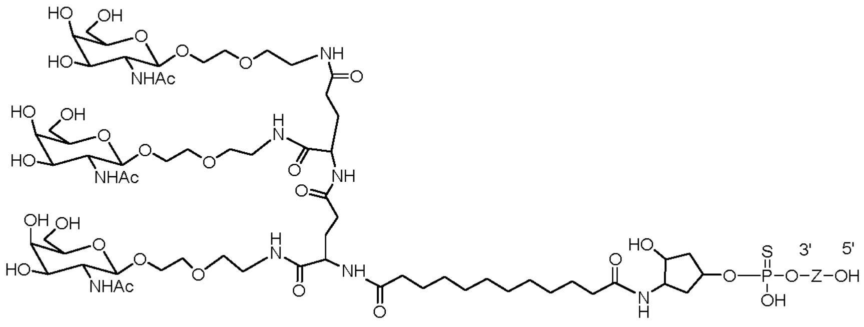

,

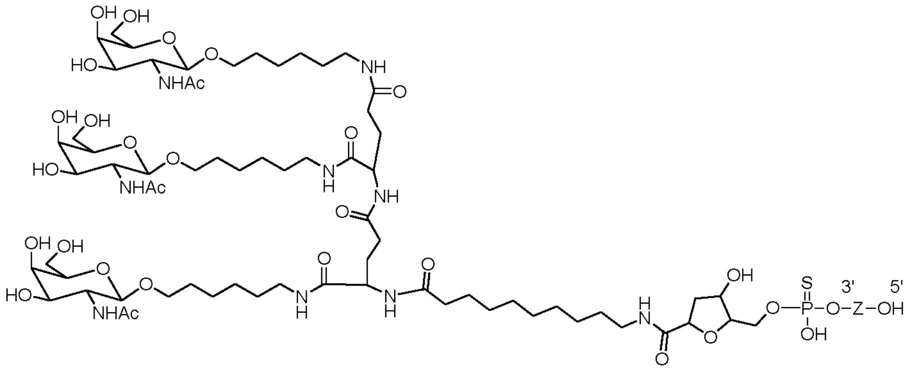

,

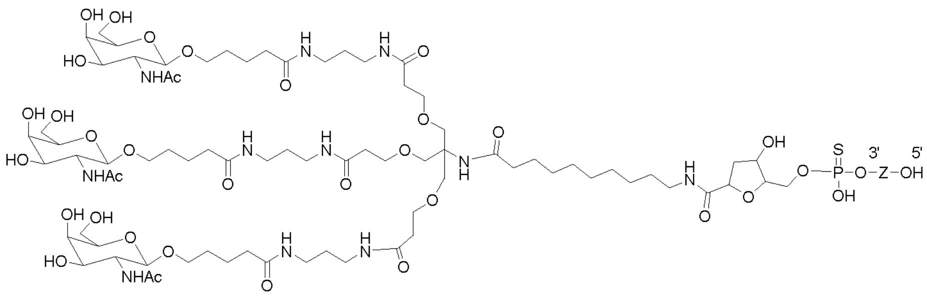

,

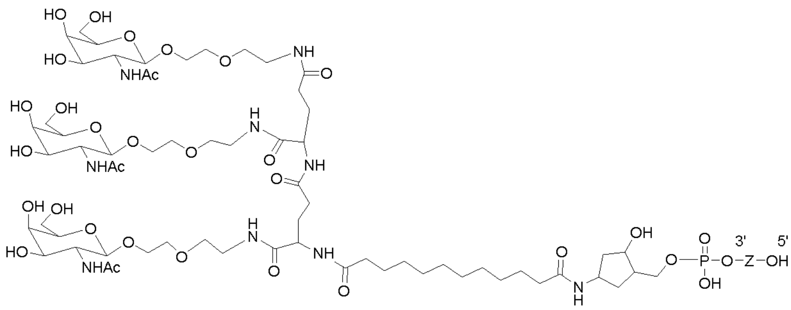

,

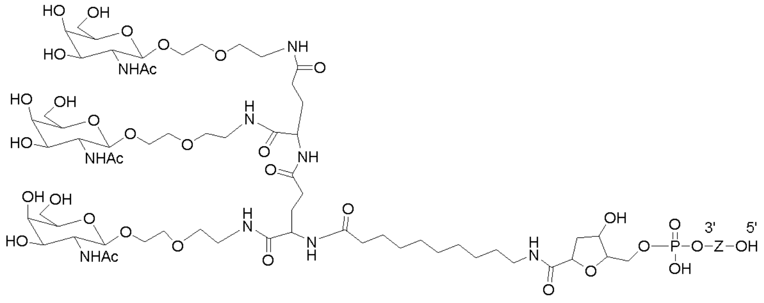

,

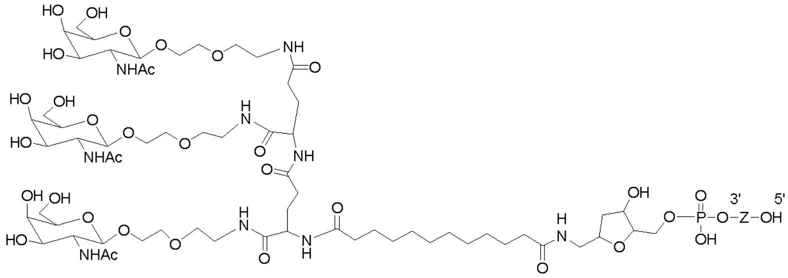

,

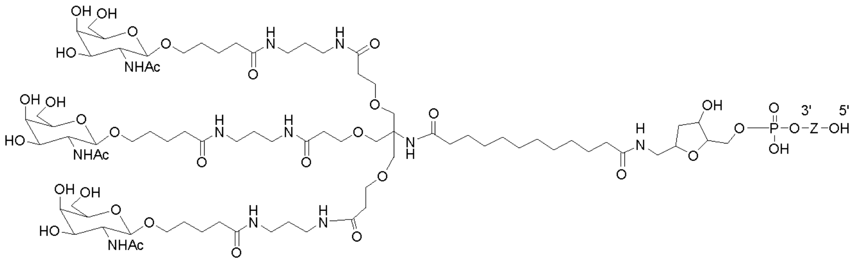

,

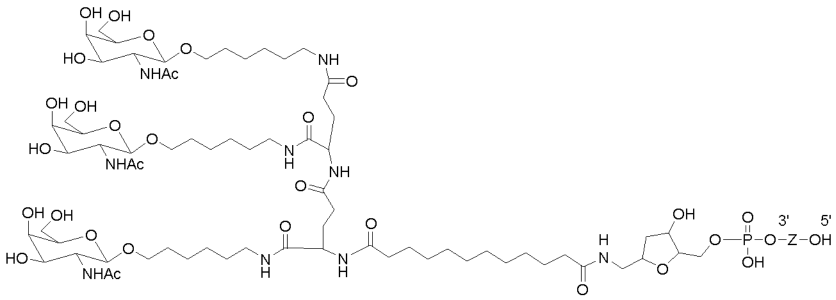

,

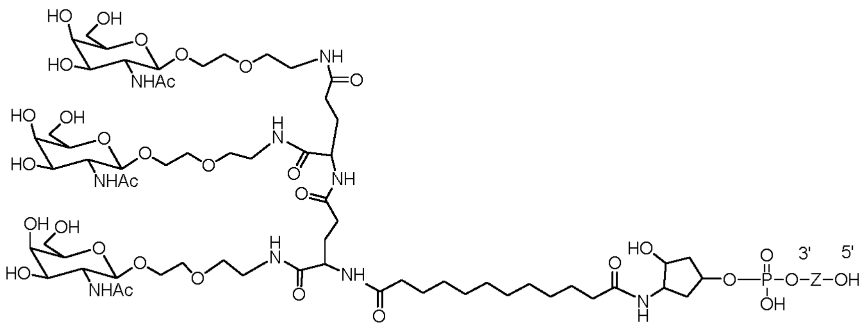

,

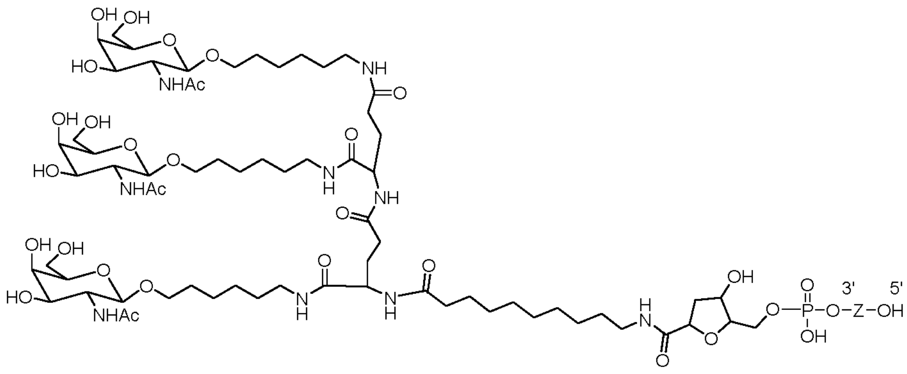

or

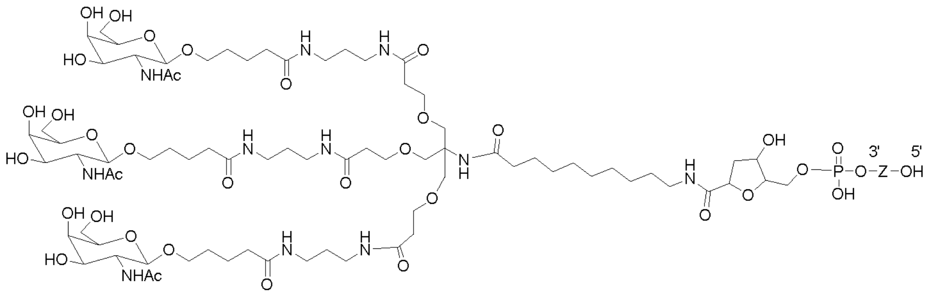

.

**25.** The nucleic acid-ligand conjugate according to any of claims 21-24, wherein the nucleic acid-ligand conjugate is any of the following structures or a pharmaceutically acceptable salt thereof:

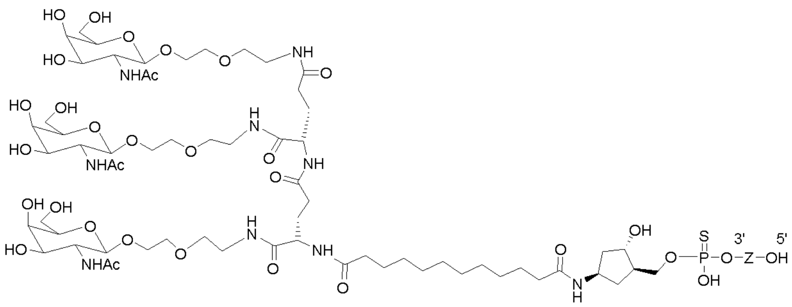

,

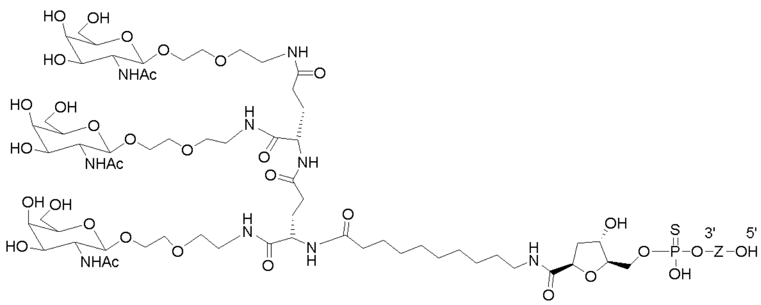

,

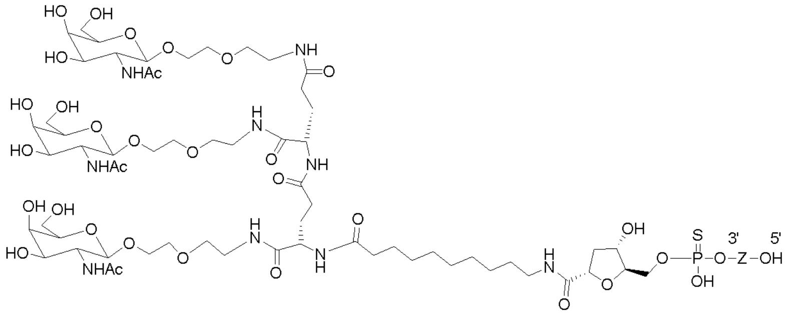

,

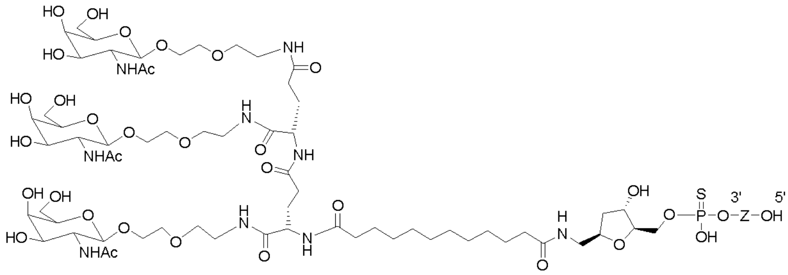

,

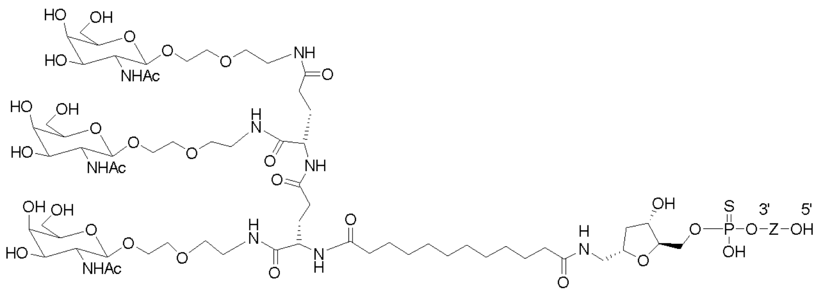

,

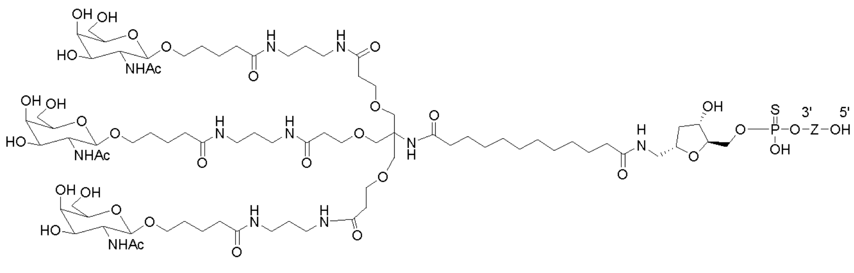

,

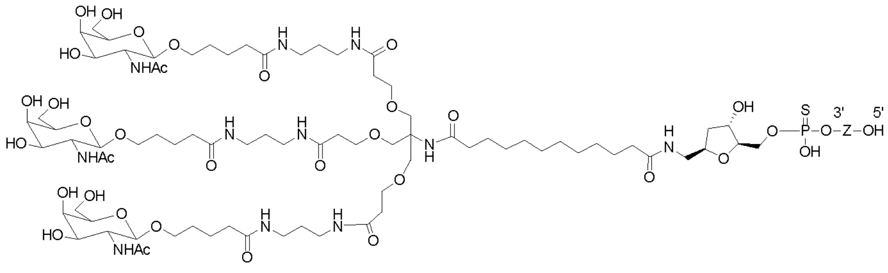

,

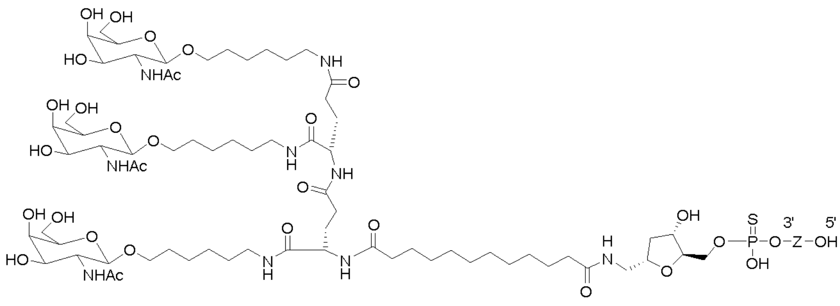

,

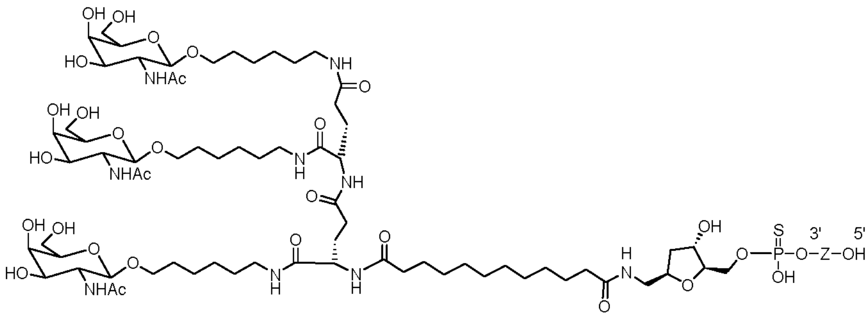

,

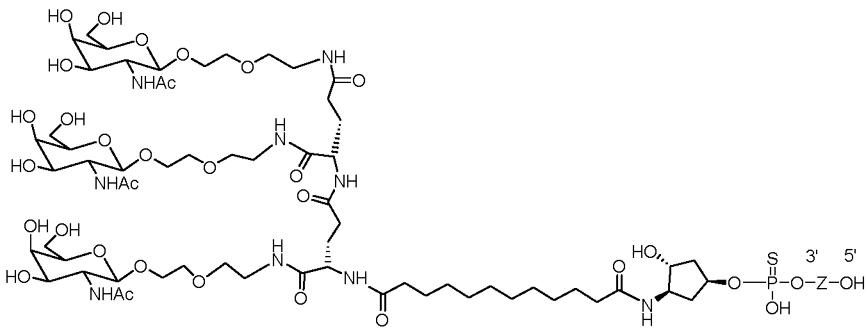

,

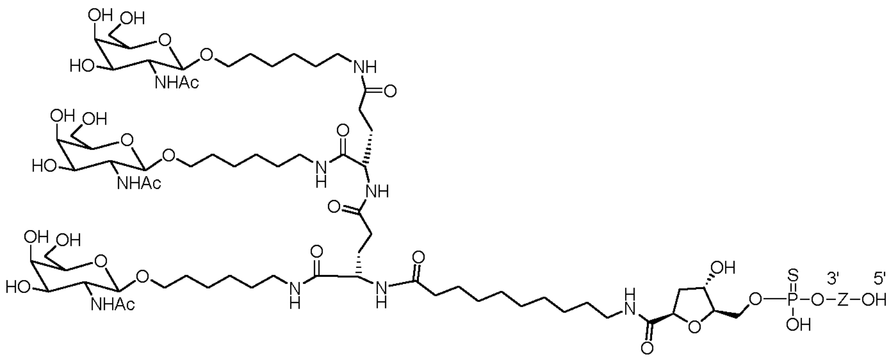

,

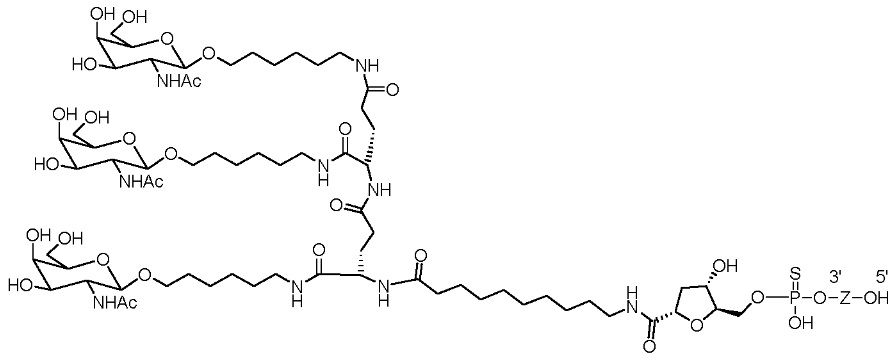

,

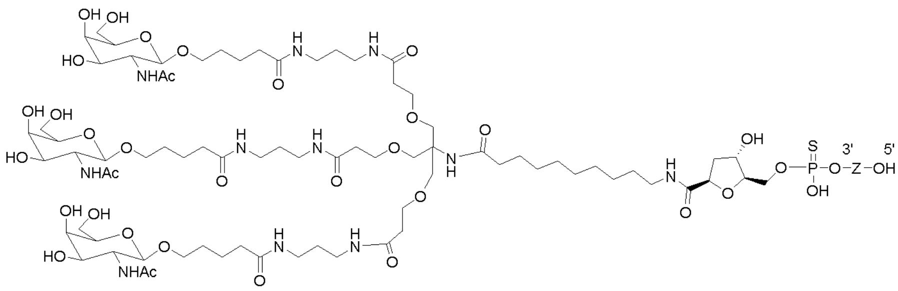

,

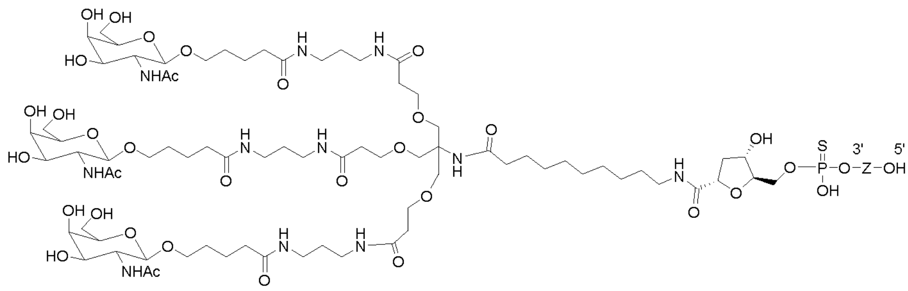

,

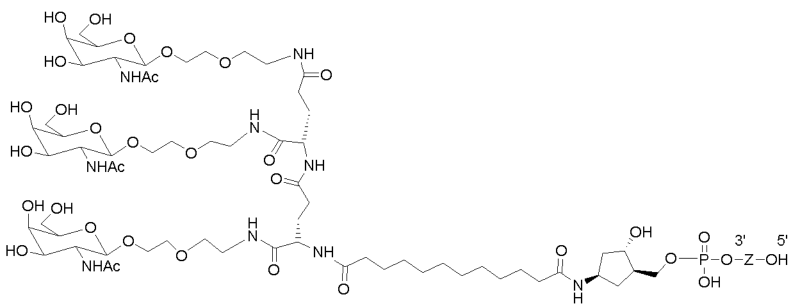

,

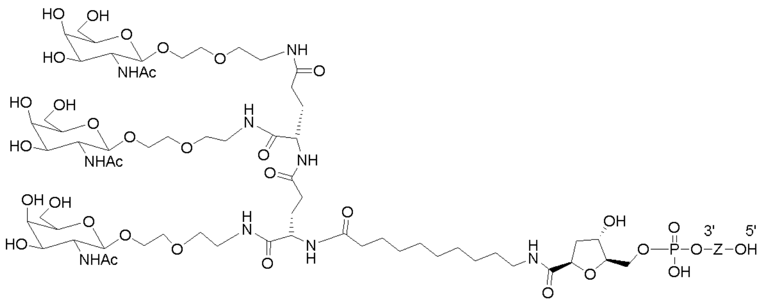

,

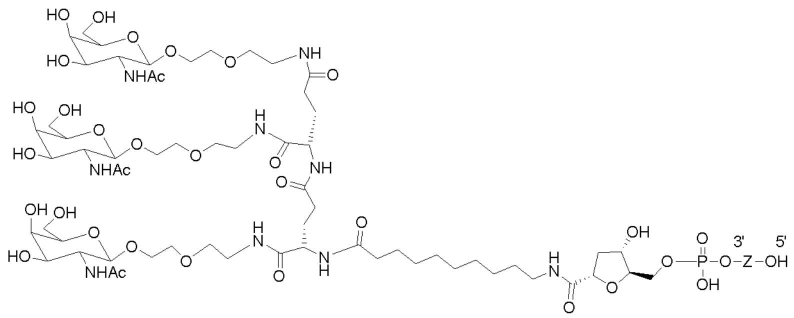

,

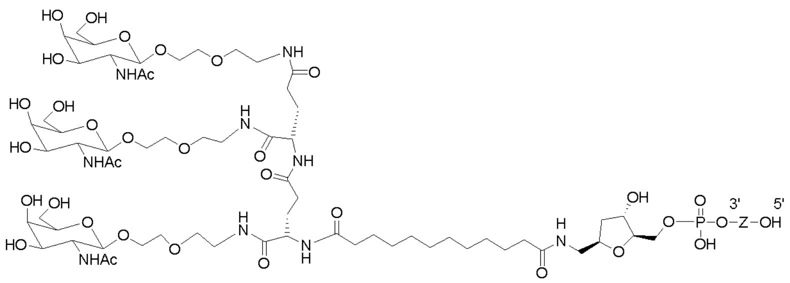

,

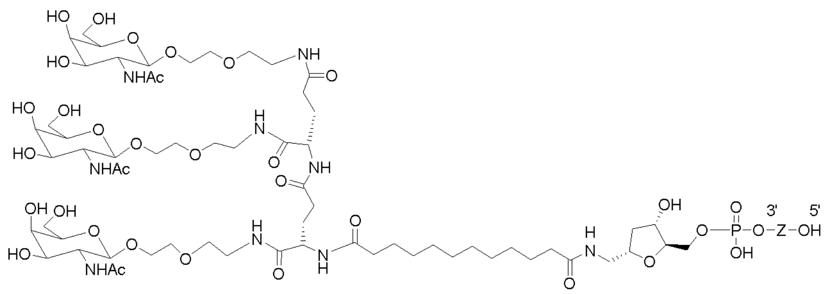

,

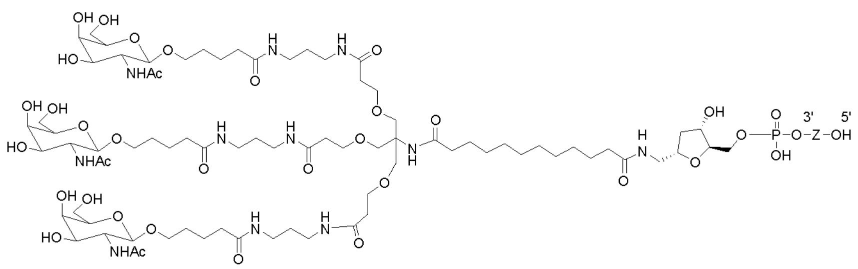

,

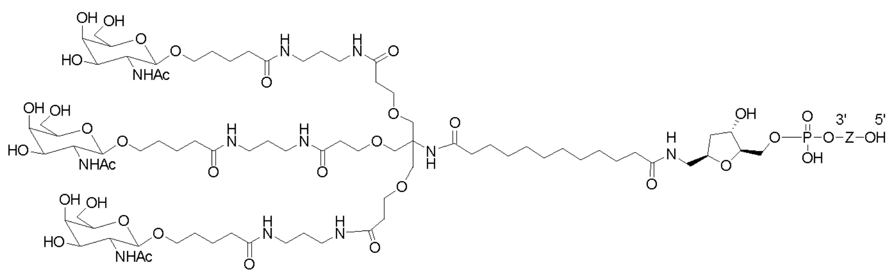

,

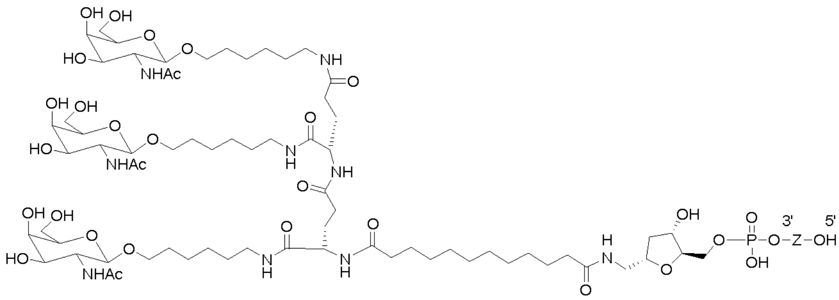

,

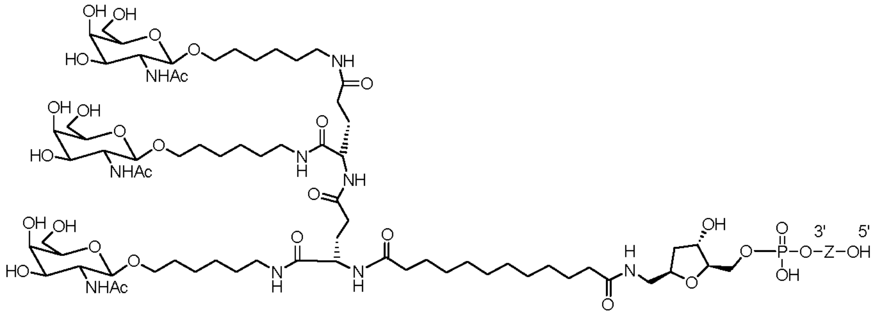

,

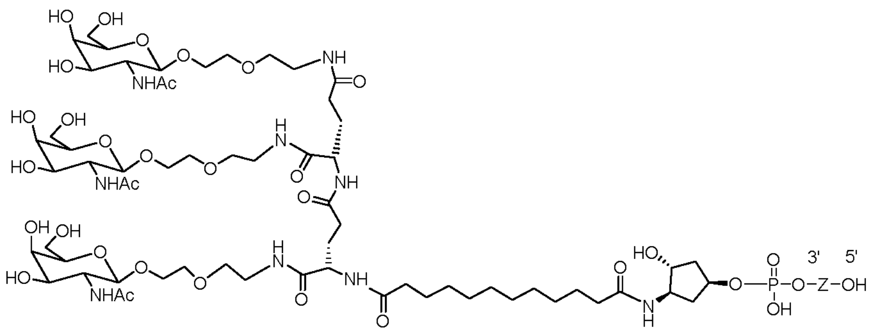

,

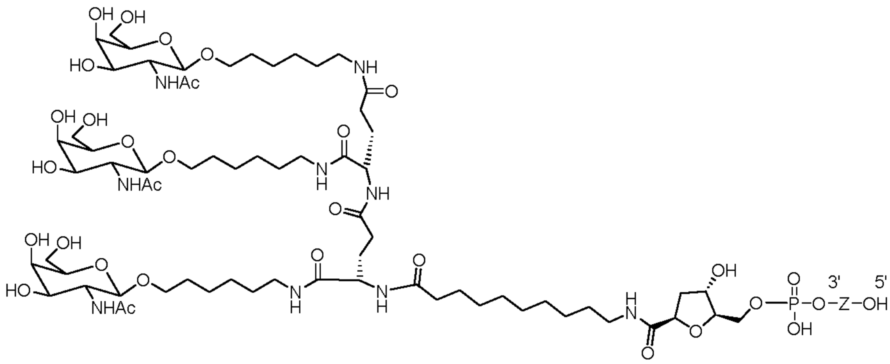

,

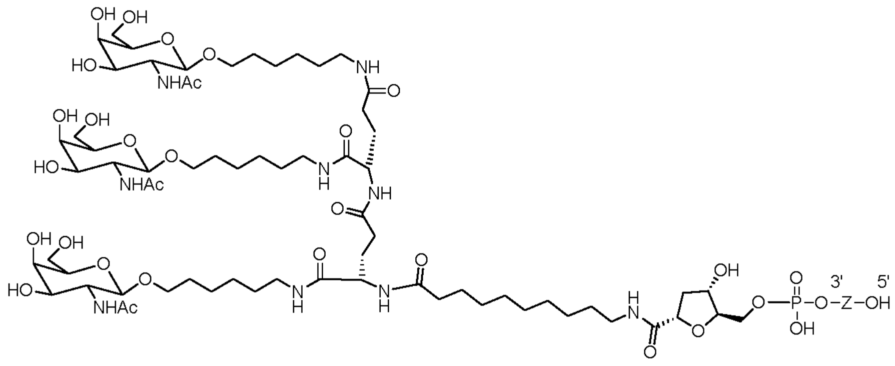

,

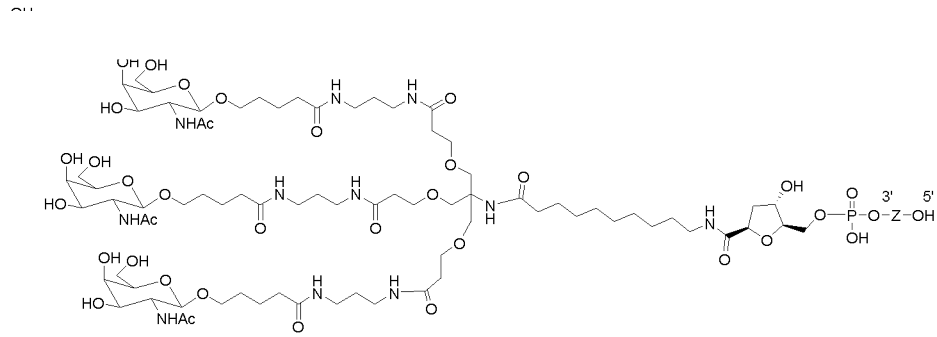

or

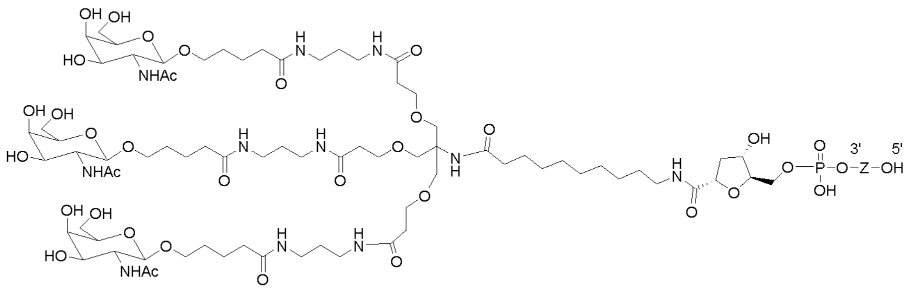

.

**26.** An RNAi agent comprising the nucleic acid-ligand conjugate according to any of claims 21-25, wherein the RNAi agent is preferably an siRNA.

**27.** A composition comprising the nucleic acid-ligand conjugate according to any of claims 21-25 or the RNAi agent according to claim 26, and one or more pharmaceutically acceptable excipients.

**28.** Use of the nucleic acid-ligand conjugate according to any of claims 21-25 or the RNAi agent according to claim 26 or the composition according to claim 27 in the preparation of a medicament for treating a disease in a patient, wherein the disease is preferably a hepatogenic disease.

**29.** A method for inhibiting mRNA expression in a patient or delivering an expression-inhibiting oligomeric compound to the liver *in vivo,* comprising administering to the patient the nucleic acid-ligand conjugate according to any of claims 21-25 or the RNAi agent according to claim 26 or the composition according to claim 27.

**30.** A method for preparing the nucleic acid-ligand conjugate according to any of claims 21-25, comprising the following steps: taking the compound according to any of claims 17-20 as a start and attaching nucleoside monomers one by one in the 3'-5' direction in the order in which nucleotides are arranged.

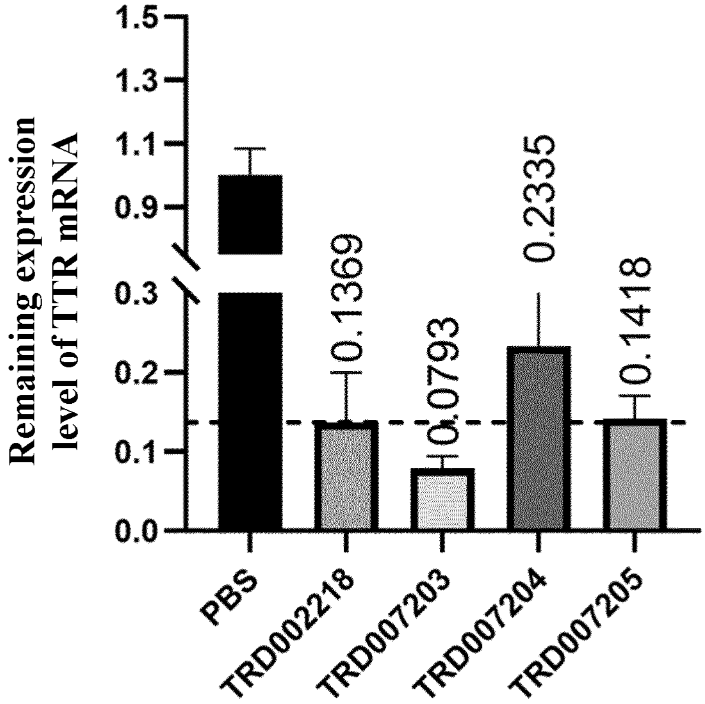
Remaining expression level of TTR mRNA
1.5
1.3
1.1
0.9
0.3
0.2
0.1
0.0
0.1369
0.0793
0.2335
0.1418
PBS
TRD002218
TRD007203
TRD007204
TRD007205

FIG. 1

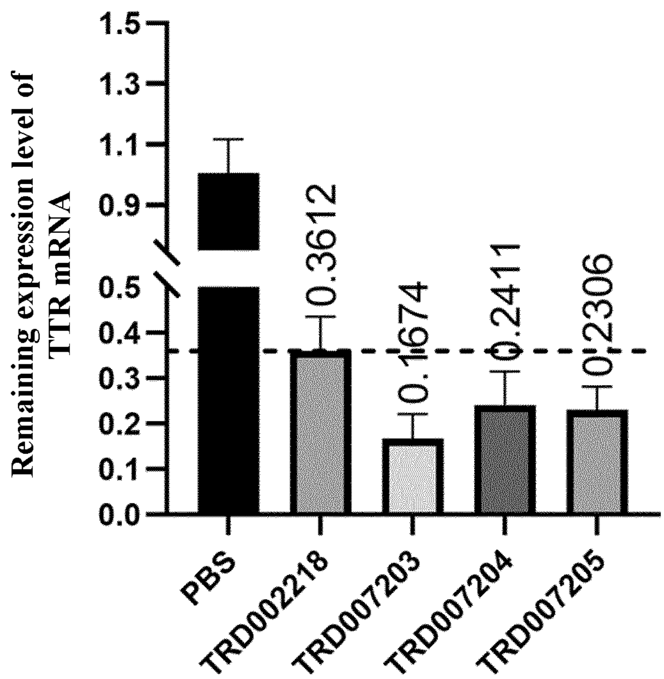
Remaining expression level of TTR mRNA
1.5
1.3
1.1
0.9
0.5
0.4
0.3
0.2
0.1
0.0
0.3612
0.1674
0.2411
0.2306
PBS
TRD002218
TRD007203
TRD007204
TRD007205

FIG. 2

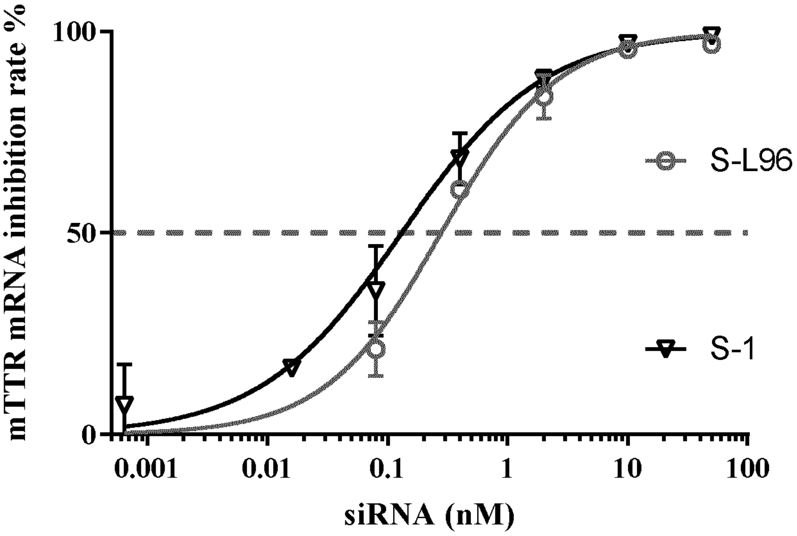
mTTR mRNA inhibition rate %
100
50
0
0.001
0.01
0.1
1
10
100
siRNA (nM)
S-L96
S-1

FIG. 3

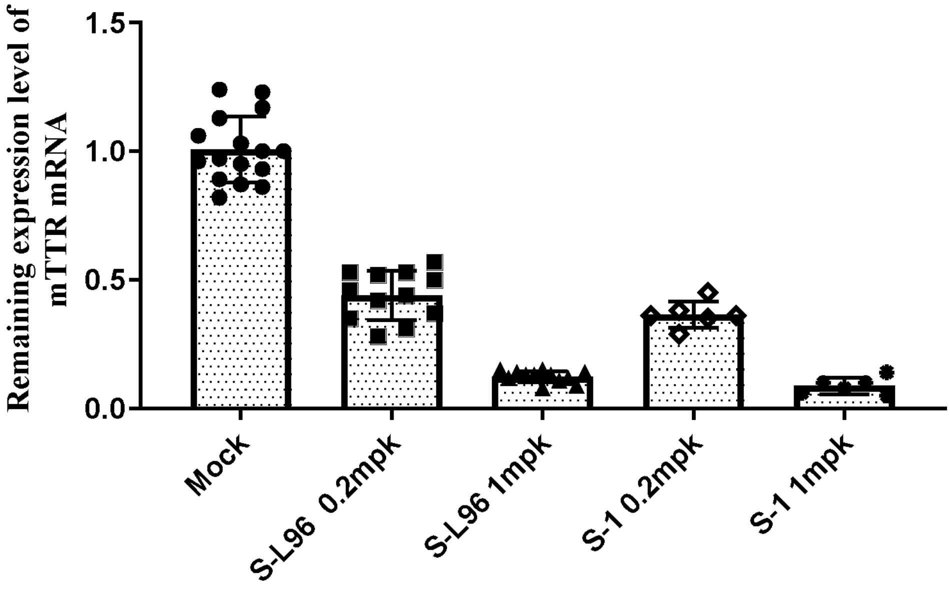
Remaining expression level of mTTR mRNA
1.5
1.0
0.5
0.0
Mock
S-L96 0.2mpk
S-L96 1mpk
S-1 0.2mpk
S-1 1mpk

FIG. 4

**INTERNATIONAL SEARCH REPORT**

International application No.
**PCT/CN2022/103275**

**A. CLASSIFICATION OF SUBJECT MATTER**

C12N 15/113(2010.01)i； A61K 47/54(2017.01)i； A61K 31/713(2006.01)i； A61P 1/16(2006.01)i

According to International Patent Classification (IPC) or to both national classification and IPC

**B. FIELDS SEARCHED**

Minimum documentation searched (classification system followed by classification symbols)

C12N; A61K; A61P

Documentation searched other than minimum documentation to the extent that such documents are included in the fields searched

Electronic data base consulted during the international search (name of data base and, where practicable, search terms used)

CNABS; CNTXT; DWPI; SIPOABS; STNext; CNKI; ISI Web of Science: 黄金宇; 上海拓界生物; 苏州瑞博生物; 核酸; 连接子; 肝靶向; siRNA; GalNac; linker; liver target

**C. DOCUMENTS CONSIDERED TO BE RELEVANT**

| Category* | Citation of document, with indication, where appropriate, of the relevant passages | Relevant to claim No. |
|---|---|---|
| X | CN 105392488 A (ISIS PHARMACEUTICALS INC.) 09 March 2016 (2016-03-09)<br>description, paragraphs 0043 and 0420-0438, and abstract | 1-30 |
| X | CN 107250362 A (ALNYLAM PHARMACEUTICALS INC.) 13 October 2017 (2017-10-13)<br>claims 32 and 63 and abstract | 1-30 |
| X | WO 2019105418 A1 (SUZHOU RIBO LIFE SCIENCE CO., LTD.) 06 June 2019 (2019-06-06)<br>claim 35 and abstract | 1-30 |
| Y | CN 105392488 A (ISIS PHARMACEUTICALS INC.) 09 March 2016 (2016-03-09)<br>description, paragraphs 0043 and 0420-0438, and abstract | 1-30 |
| Y | CN 107250362 A (ALNYLAM PHARMACEUTICALS INC.) 13 October 2017 (2017-10-13)<br>claims 32 and 63 and abstract | 1-30 |
| Y | WO 2019105418 A1 (SUZHOU RIBO LIFE SCIENCE CO., LTD.) 06 June 2019 (2019-06-06)<br>claim 35 and abstract | 1-30 |
| Y | CN 109069529 A (ARROWHEAD PHARMACEUTICALS INC.) 21 December 2018 (2018-12-21)<br>claims 1-3 and abstract | 1-30 |

☑ Further documents are listed in the continuation of Box C. ☑ See patent family annex.

* Special categories of cited documents:
"A" document defining the general state of the art which is not considered to be of particular relevance
"E" earlier application or patent but published on or after the international filing date
"L" document which may throw doubts on priority claim(s) or which is cited to establish the publication date of another citation or other special reason (as specified)
"O" document referring to an oral disclosure, use, exhibition or other means
"P" document published prior to the international filing date but later than the priority date claimed
"T" later document published after the international filing date or priority date and not in conflict with the application but cited to understand the principle or theory underlying the invention
"X" document of particular relevance; the claimed invention cannot be considered novel or cannot be considered to involve an inventive step when the document is taken alone
"Y" document of particular relevance; the claimed invention cannot be considered to involve an inventive step when the document is combined with one or more other such documents, such combination being obvious to a person skilled in the art
"&" document member of the same patent family

| Date of the actual completion of the international search | Date of mailing of the international search report |
|---|---|
| **20 September 2022** | **29 September 2022** |

| Name and mailing address of the ISA/CN | Authorized officer |
|---|---|
| **China National Intellectual Property Administration (ISA/CN)**<br>**No. 6, Xitucheng Road, Jimenqiao, Haidian District, Beijing 100088, China** | |
| Facsimile No. **(86-10)62019451** | Telephone No. |

Form PCT/ISA/210 (second sheet) (January 2015)

**INTERNATIONAL SEARCH REPORT**

International application No.
**PCT/CN2022/103275**

**C. DOCUMENTS CONSIDERED TO BE RELEVANT**

| Category* | Citation of document, with indication, where appropriate, of the relevant passages | Relevant to claim No. |
|---|---|---|
| A | WO 2015179693 A1 (ISIS PHARMACEUTICALS INC.) 26 November 2015 (2015-11-26) entire document | 1-30 |
| A | CN 112390835 A (SUZHOU RIBO LIFE SCIENCE CO., LTD.) 23 February 2021 (2021-02-23) entire document | 1-30 |
| A | CN 110959011 A (SUZHOU RIBO LIFE SCIENCE CO., LTD.) 03 April 2020 (2020-04-03) entire document | 1-30 |

Form PCT/ISA/210 (second sheet) (January 2015)

**INTERNATIONAL SEARCH REPORT**

International application No.
**PCT/CN2022/103275**

**Box No. II Observations where certain claims were found unsearchable (Continuation of item 2 of first sheet)**

This international search report has not been established in respect of certain claims under Article 17(2)(a) for the following reasons:

1. ☑ Claims Nos.: **29**
because they relate to subject matter not required to be searched by this Authority, namely:

[1] Claim 29 relates to a method for inhibiting mRNA expression in a patient or delivering an expression-inhibiting oligomeric compound to a liver in vivo, and thus does not comply with PCT Rule 39.1(iv). A search was conducted on the basis that the title of the subject matter is a use of the nucleic acid ligand conjugate according to any one of claims 21-25, or the RNAi reagent according to claim 26, or the composition according to claim 27 in the preparation of a drug for treating diseases of a patient.

2. ☐ Claims Nos.:
because they relate to parts of the international application that do not comply with the prescribed requirements to such an extent that no meaningful international search can be carried out, specifically:

3. ☐ Claims Nos.:
because they are dependent claims and are not drafted in accordance with the second and third sentences of Rule 6.4(a).

**INTERNATIONAL SEARCH REPORT**
**Information on patent family members**

International application No.
**PCT/CN2022/103275**

| Patent document cited in search report | Publication date (day/month/year) | Patent family member(s) | Publication date (day/month/year) |
|---|---|---|---|
| CN 105392488 A | 09 March 2016 | MX 2019010441 A | 17 October 2019 |
| | | IL 261901 A | 31 October 2018 |
| | | RU 2019124314 A | 21 August 2019 |
| | | US 2016076032 A1 | 17 March 2016 |
| | | RS 60796 B1 | 30 October 2020 |
| | | WO 2014179620 A1 | 06 November 2014 |
| | | EA 201891479 A1 | 30 November 2018 |
| | | JP 2016526874 A | 08 September 2016 |
| | | JP 2020058370 A | 16 April 2020 |
| | | ES 2778442 T3 | 10 August 2020 |
| | | KR 20190084138 A | 15 July 2019 |
| | | IL 263843 A | 31 January 2019 |
| | | JP 2016523515 A | 12 August 2016 |
| | | IL 242124 B | 28 February 2019 |
| | | HR P20201378 T1 | 27 November 2020 |
| | | AU 2017200365 A1 | 23 February 2017 |
| | | HU E050394 T2 | 30 November 2020 |
| | | JP 2021020901 A | 18 February 2021 |
| | | US 2018273953 A1 | 27 September 2018 |
| | | EP 2991656 A2 | 09 March 2016 |
| | | EP 3633039 A1 | 08 April 2020 |
| | | US 2016017323 A1 | 21 January 2016 |
| | | US 2015176007 A1 | 25 June 2015 |
| | | AU 2001259750 A1 | 14 February 2002 |
| | | AU 2014259759 A1 | 22 October 2015 |
| | | US 2021395734 A1 | 23 December 2021 |
| | | US 2021130823 A1 | 06 May 2021 |
| | | PT 2992098 T | 05 July 2019 |
| | | IL 242132 B | 31 October 2018 |
| | | IL 274064 A | 30 June 2020 |
| | | CN 105378085 A | 02 March 2016 |
| | | LT 2992098 T | 10 July 2019 |
| | | AU 2014259757 A1 | 22 October 2015 |
| | | CA 2921162 A1 | 06 November 2014 |
| | | HK 1221404 A1 | 02 June 2017 |
| | | UA 120287 C2 | 11 November 2019 |
| | | IL 264241 A | 28 February 2019 |
| | | US 2018273952 A1 | 27 September 2018 |
| | | JP 2020039355 A | 19 March 2020 |
| | | AU 2022202770 A1 | 19 May 2022 |
| | | AU 2020207820 A1 | 06 August 2020 |
| | | KR 20210151260 A | 13 December 2021 |
| | | MY 178929 A | 23 October 2020 |
| | | US 2020224198 A1 | 16 July 2020 |
| | | AU 2014259755 A1 | 22 October 2015 |
| | | US 2016076030 A1 | 17 March 2016 |
| | | PT 3524680 T | 04 January 2021 |
| | | AU 2019200820 A1 | 28 February 2019 |
| | | ES 2819213 T3 | 15 April 2021 |
| | | CR 20190269 A | 13 September 2019 |

Form PCT/ISA/210 (patent family annex) (January 2015)

**INTERNATIONAL SEARCH REPORT**
**Information on patent family members**

International application No.
**PCT/CN2022/103275**

| Patent document cited in search report | | | Publication date (day/month/year) | Patent family member(s) | | | Publication date (day/month/year) |
|---|---|---|---|---|---|---|---|
| CN | 107250362 | A | 13 October 2017 | US | 11408001 | B1 | 09 August 2022 |
| | | | | US | 2020199591 | A1 | 25 June 2020 |
| | | | | HK | 1244843 | A1 | 17 August 2018 |
| | | | | US | 2020080088 | A1 | 12 March 2020 |
| | | | | JP | 2020141685 | A | 10 September 2020 |
| | | | | WO | 2016081444 | A1 | 26 May 2016 |
| | | | | AU | 2021212094 | A1 | 26 August 2021 |
| | | | | JP | 2017535552 | A | 30 November 2017 |
| | | | | AU | 2015350120 | A1 | 18 May 2017 |
| | | | | EP | 3221451 | A1 | 27 September 2017 |
| | | | | US | 2021108203 | A1 | 15 April 2021 |
| | | | | CN | 113846101 | A | 28 December 2021 |
| | | | | US | 2017260527 | A1 | 14 September 2017 |
| | | | | CA | 2968114 | A1 | 26 May 2016 |
| WO | 2019105418 | A1 | 06 June 2019 | CN | 110997919 | A | 10 April 2020 |
| | | | | KR | 20200091414 | A | 30 July 2020 |
| | | | | EP | 3719128 | A1 | 07 October 2020 |
| | | | | CA | 3083968 | A1 | 06 June 2019 |
| | | | | TW | 201925469 | A | 01 July 2019 |
| | | | | US | 2022049249 | A1 | 17 February 2022 |
| | | | | JP | 2021504415 | A | 15 February 2021 |
| | | | | AU | 2018374219 | A1 | 21 May 2020 |
| CN | 109069529 | A | 21 December 2018 | EP | 3426261 | A1 | 16 January 2019 |
| | | | | US | 2021189394 | A1 | 24 June 2021 |
| | | | | BR | 112018068230 | A2 | 30 July 2019 |
| | | | | AU | 2017229469 | A1 | 09 August 2018 |
| | | | | US | 2019211333 | A1 | 11 July 2019 |
| | | | | KR | 20220005633 | A | 13 January 2022 |
| | | | | TW | 202203972 | A | 01 February 2022 |
| | | | | WO | 2017156012 | A1 | 14 September 2017 |
| | | | | JP | 2022023242 | A | 07 February 2022 |
| | | | | KR | 20180121925 | A | 09 November 2018 |
| | | | | IL | 261598 | A | 31 October 2018 |
| | | | | CA | 3011946 | A1 | 14 September 2017 |
| | | | | US | 2017253875 | A1 | 07 September 2017 |
| | | | | JP | 2019511491 | A | 25 April 2019 |
| | | | | TW | 201735953 | A | 16 October 2017 |
| | | | | IL | 288341 | A | 01 January 2022 |
| | | | | UY | 37146 | A | 29 September 2017 |
| | | | | MX | 2018009854 | A | 09 November 2018 |
| | | | | CN | 113797348 | A | 17 December 2021 |
| WO | 2015179693 | A1 | 26 November 2015 | US | 2017355727 | A1 | 14 December 2017 |
| | | | | US | 2021054017 | A1 | 25 February 2021 |
| CN | 112390835 | A | 23 February 2021 | None | | | |
| CN | 110959011 | A | 03 April 2020 | AU | 2018394875 | A1 | 09 April 2020 |
| | | | | TW | 201929905 | A | 01 August 2019 |
| | | | | JP | 2021509402 | A | 25 March 2021 |
| | | | | US | 2020338201 | A1 | 29 October 2020 |
| | | | | CA | 3087106 | A1 | 04 July 2019 |

Form PCT/ISA/210 (patent family annex) (January 2015)

**INTERNATIONAL SEARCH REPORT**
**Information on patent family members**

International application No.
**PCT/CN2022/103275**

| Patent document cited in search report | Publication date (day/month/year) | Patent family member(s) | | | Publication date (day/month/year) |
|---|---|---|---|---|---|
| | | ZA | 202003833 | B | 26 January 2022 |
| | | KR | 20200105812 | A | 09 September 2020 |
| | | EP | 3732185 | A1 | 04 November 2020 |
| | | RU | 2020121741 | A | 01 February 2022 |
| | | WO | 2019128611 | A1 | 04 July 2019 |

Form PCT/ISA/210 (patent family annex) (January 2015)

## REFERENCES CITED IN THE DESCRIPTION

*This list of references cited by the applicant is for the reader's convenience only. It does not form part of the European patent document. Even though great care has been taken in compiling the references, errors or omissions cannot be excluded and the EPO disclaims all liability in this regard.*

### Patent documents cited in the description


- WO 2014025805 A1
- WO 2021254360 A1
- WO 2022028462 A1


### Non-patent literature cited in the description


- **YUANYU H ; LIANG ZICAI L.** Asialoglycoprotein Receptor and Its Application in Liver-targeted Drug Delivery. *Prog. Biochem. Biophys.,* 2015, vol. 42 (6
- **SEVERGINI et al.** *Cytotechnology,* 2012, vol. 64 (2), 187-195